# EUROPEAN PATENT APPLICATION

(11) **EP 4 703 359 A1**
(43) Date of publication of application: **04.03.2026**
(21) Application number: 24796091.7
(22) Date of filing: 24.04.2024
(51) Int. Cl.: C07D 471/04, C07D 235/00, A61K 31/437, A61K 31/519, A61P 25/00

(54) **IMIDAZOPYRIDINE OR IMIDAZOPYRAZINE COMPOUND, PREPARATION METHOD THEREFOR, AND PHARMACEUTICAL COMPOSITION AND USE THEREOF**

(30) Priority: 24.04.2023 CN 202310452097; 06.07.2023 CN 202310824648
(71) Applicant: Shanghai Simr Biotechnology Co., Ltd., Shanghai 201318 (CN)
(72) Inventor: CHEN, Nanyang, Shanghai 201318 (CN); WANG, Fei, Shanghai 201318 (CN); GUO, Yanghui, Shanghai 201318 (CN); CHEN, Jun, Shanghai 201318 (CN); CHEN, Tingting, Shanghai 201318 (CN)
(74) Representative: Dai, Simin
(86) International application number: PCT/CN2024/089449
(87) International publication number: WO 2024/222712

(57) **Abstract**

Disclosed in the present invention are an imidazopyridine or imidazopyrazine compound, a preparation method therefor, and a pharmaceutical composition and use thereof. The present invention provides an imidazopyridine or imidazopyrazine compound and an isotope derivative, a nitrogen oxide, a pharmaceutically acceptable salt, a solvate, or a pharmaceutically acceptable salt solvate thereof. The imidazopyridine or imidazopyrazine compound provided by the present invention has excellent affinity activity and positive regulation activity on an α2/3-GABA_{A} receptor.

## Description

The present application claims the priority of Chinese Patent Application No. 2023104520979, filed on April 24, 2023, and Chinese Patent Application No. 202310824648X, filed on July 6, 2023. The contents of the above Chinese patent applications are incorporated herein by reference in their entireties.

### TECHNICAL FIELD

The present disclosure relates to an imidazopyridine or imidazopyrazine compound, a preparation method therefor, a pharmaceutical composition thereof, and a use thereof.

### BACKGROUND

γ-Aminobutyric acid (GABA) is an important inhibitory neurotransmitter in the mammalian central nervous system. Substances that modulate GABAergic neurotransmission are widely used for the treatment of various conditions, such as epilepsy, anxiety disorders, and depression. In nature, there are two types of GABA receptors: one is the GABA_{A} receptor (GABA_{A}R), which is a member of the ligand-gated ion channel superfamily, and the other is the GABA_{B} receptor (GABA_{B}R), which is a member of the G protein-coupled receptor superfamily. The known GABA_{A} receptor subunits in mammals include α1-6, β1-4, γ1-3, δ, ε, 0, and ρ1-2 subunits. Among these, the α, β, and γ subunits are essential for forming a fully functional GABA_{A} receptor, while the α subunit is critical for the binding of benzodiazepines to the GABA_{A} receptor.

Drugs that bind to allosteric sites can be categorized into positive allosteric modulators (or positive allosteric regulators), which enhance receptor activity; negative allosteric modulators (or inverse allosteric regulators), which reduce receptor activity; or neutral allosteric modulators (referring to compounds that bind to allosteric sites but do not modulate receptor activity). Recent evidence suggests that GABA_{A} receptors containing α2 or α3 subunits (herein referred to as α2/3-GABA_{A} receptors) may be implicated in certain pain states, and positive allosteric modulators of these receptors could serve as effective analgesics (Mirza, N. R.; Munro, G., Drug News and Perspectives, 2010, 23(6), 351-360).

The international patent applications PCT/SE2006/001433 (published as WO2007/073283) and PCT/SE2010/050892 (published as WO2011/021979) disclose certain cinnoline derivatives, which are considered to be useful as GABA_{A}R modulators.

The prevailing view holds that the modulatory activity on GABA_{A} receptors containing the α1 subunit is the primary source of side effects-such as sedation, addiction, drowsiness, and amnesia-associated with current GABA_{A} modulators, such as benzodiazepines (Rudolph, U.; Frederic Knoflach, F., *Nature Reviews: Drug Discovery*, 2011, 10(9), 685-697). The identification of novel compounds that interact with GABA_{A} receptors while exhibiting reduced side effects associated with α1-GABA_{A} receptors holds significant therapeutic potential.

### SUMMARY

The technical problem to be addressed by the present disclosure is that in the prior art, compounds having a modulatory function on GABA_{A} receptors exhibit poor affinity and poor positive modulatory activity toward α2/3-GABA_{A} receptors. The invention therefore provides an imidazopyridine or imidazopyrazine compound, a preparation method thereof, a pharmaceutical composition thereof, and a use thereof. The imidazopyridine or imidazopyrazine compound provided by the present disclosure exhibits excellent affinity and positive modulatory activity for the α2/3-GABA_{A} receptor.

The present disclosure employs the following technical solution to address the above-mentioned technical problem.

The present disclosure further provides a compound of formula (I), an isotopic derivative thereof, a nitrogen oxide thereof, a pharmaceutically acceptable salt thereof, a solvate thereof, or a solvate of the pharmaceutically acceptable salt thereof: 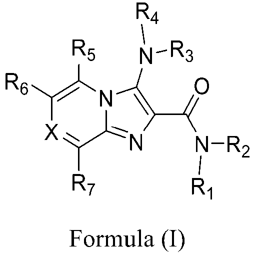 wherein
R₁ and R₂ are independently hydrogen, C₁₋₆ alkyl, C₃₋₆ cycloalkyl, C₂₋₆ alkenyl, C₂₋₆ alkynyl, 4- to 12-membered heterocycloalkyl, C₆₋₁₀ aryl, or 5- to 12-membered heteroaryl, wherein the C₁₋₆ alkyl, C₃₋₆ cycloalkyl, C₂₋₆ alkenyl, C₂₋₆ alkynyl, 4- to 12-membered heterocycloalkyl, C₆₋₁₀ aryl, and 5- to 12-membered heteroaryl are each substituted by 0, 1, 2, 3, 4 or 5 R₉;
alternatively, R₁ and R₂ together with the N atom to which they are attached form a 4-to 12-membered heterocycloalkyl or a 4- to 12-membered heterocycloalkyl substituted by 1, 2, or 3 R₁₋₁; R₁₋₁ is independently deuterium, -CN, -OH, halogen, C₁₋₆ alkyl, or -O-C₁₋₆ alkyl;
R₃ and R₄ are H, C₁₋₆ alkyl, C₃₋₆ cycloalkyl, or -O-C₁₋₆ alkyl, wherein the C₁₋₆ alkyl, C₃₋₆ cycloalkyl, and -O-C₁₋₆ alkyl are each substituted by 0, 1, 2, or 3 R₃₋₁; R₃₋₁ is independently deuterium, -CN, -OH, halogen, C₁₋₆ alkyl, or -O-C₁₋₆ alkyl;
R₅ and R₆ are independently hydrogen, deuterium, halogen, or C₁₋₆ alkyl;
X is N or CRs;
R₈ is hydrogen, deuterium, halogen, -CN, C₁₋₆ alkyl, C₃₋₆ cycloalkyl, or -O-C₁₋₆ alkyl, wherein the C₁₋₆ alkyl, C₃₋₆ cycloalkyl, and -O-C₁₋₆ alkyl are each substituted by 0, 1, 2, or 3 R₈₋₁; R₈₋₁ is independently deuterium, -CN, -OH, halogen, C₁₋₆ alkyl, or -O-C₁₋₆ alkyl;
R₇ is -CN, C₁₋₆ alkyl, C₃₋₆ cycloalkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, -Z-(CRₐR_{b})ₘ-C₆₋₁₀ aryl, or -Z-(CRₐR_{b})ₘ-5- to 12-membered heteroaryl, wherein the C₁₋₆ alkyl, C₃₋₆ cycloalkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, -Z-(CRₐR_{b})ₘ-C₆₋₁₀ aryl, and -Z-(CRₐR_{b})ₘ-5- to 12-membered heteroaryl are each substituted by 0, 1, 2, 3, 4, or 5 R₁₀;
Z is independently -O- or -NR_{c}; Rₐ and R_{b} are independently H or C₁₋₆ alkyl; R_{c} is H or C₁₋₆ alkyl;
m is independently 0, 1, or 2;
R₉ is halogen, -OH, -CN, C₁₋₆ alkyl, -O-C₁₋₆ alkyl, C₃₋₆ cycloalkyl, 5- to 12-membered heterocycloalkyl, C₆₋₁₀ aryl, or 5- to 12-membered heteroaryl, wherein the C₁₋₆ alkyl, -O-C₁₋₆ alkyl, C₃₋₆ cycloalkyl, 5- to 12-membered heterocycloalkyl, C₆₋₁₀ aryl, and 5- to 12-membered heteroaryl are each substituted by 0, 1, 2, or 3 R₉₋₁; R₉₋₁ is independently deuterium, -CN, -OH, halogen, C₁₋₆ alkyl, or -O-C₁₋₆ alkyl;
R₁₀ is oxo (-C=O), deuterium, halogen, -OH, -CN, 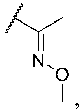 C₁₋₆ alkyl, C₂₋₆ alkenyl, C₂₋₆ alkynyl, -O-C₁₋₆ alkyl, -S-C₁₋₆ alkyl, C₆₋₁₀ aryl, 5- to 12-membered heteroaryl, C₃₋₆ cycloalkyl, or 5- to 12-membered heterocycloalkyl, wherein the C₁₋₆ alkyl, C₂₋₆ alkenyl, C₂₋₆ alkynyl, -O-C₁₋₆ alkyl, -S-C₁₋₆ alkyl, C₆₋₁₀ aryl, 5- to 12-membered heteroaryl, C₃₋₆ cycloalkyl, and 5- to 12-membered heterocycloalkyl are each substituted by 0, 1, 2, 3, 4, or 5 R₁₁;
R₁₁ is deuterium, halogen, -CN, -OH, C₁₋₆ alkyl, -O-C₁₋₆ alkyl, or oxo (-C=O);
each of said 5- to 12-membered heteroaryl is independently 5- to 12-membered heteroaryl containing 1, 2, 3, or 4 heteroatoms independently selected from the group consisting of N, O, and S; each of said 4- to 12-membered heterocycloalkyl is independently 4- to 12-membered heterocycloalkyl containing 1, 2, 3, or 4 heteroatoms independently selected from the group consisting of N, O, and S; each of said 5- to 12-membered heterocycloalkyl is independently 5- to 12-membered heterocycloalkyl containing 1, 2, 3, or 4 heteroatoms independently selected from the group consisting of N, O, and S; each of said 5-to 12-membered heterocycloalkenyl is independently 5- to 12-membered heterocycloalkenyl containing 1, 2, 3, or 4 heteroatoms independently selected from the group consisting of N, O, and S.

The present disclosure further provides a compound of formula (I), an isotopic derivative thereof, a nitrogen oxide thereof, a pharmaceutically acceptable salt thereof, a solvate thereof, or a solvate of the pharmaceutically acceptable salt thereof: 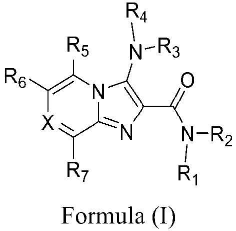 wherein
R₁ and R₂ are independently hydrogen, C₁₋₆ alkyl, C₃₋₆ cycloalkyl, C₂₋₆ alkenyl, C₂₋₆ alkynyl, 5- to 12-membered heterocycloalkyl, C₆₋₁₀ aryl, or 5- to 12-membered heteroaryl, wherein the C₁₋₆ alkyl, C₃₋₆ cycloalkyl, C₂₋₆ alkenyl, C₂₋₆ alkynyl, 5- to 12-membered heterocycloalkyl, C₆₋₁₀ aryl, and 5- to 12-membered heteroaryl are each substituted by 0, 1, 2, 3, 4, or 5 R₉;
alternatively, R₁ and R₂ together with the nitrogen atom to which they are attached form a 5- to 12-membered heterocycloalkyl;
R₃ and R₄ are H;
R₅ and R₆ are independently hydrogen or methyl;
X is N or CRs;
R₈ is hydrogen, deuterium, halogen, -CN, methoxy, methyl, or -CHF₂;
R₇ is -CN, C₁₋₆ alkyl, C₃₋₆ cycloalkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, or - Z-(CRₐR_{b})ₘ₋C₆₋₁₀ aryl, wherein the C₁₋₆ alkyl, C₃₋₆ cycloalkyl, C₃₋₆ cycloalkenyl, C₆₋₁₀ aryl, 5-to 12-membered heteroaryl, 5- to 12-membered heterocycloalkyl, 5- to 12-membered heterocycloalkenyl, and -Z-(CRₐR_{b})ₘ-C₆₋₁₀ aryl are each substituted by 0, 1, 2, 3, 4, or 5 R₁₀;
Z is -O- or -NR_{c}; Rₐ and R_{b} are independently H or C₁₋₆ alkyl; R_{c} is H or C₁₋₆ alkyl;
m is 0, 1, or 2;
R₉ is halogen, -OH, -CN, C₁₋₆ alkyl, -O-C₁₋₆ alkyl, C₃₋₆ cycloalkyl, 5- to 12-membered heterocycloalkyl, C₆₋₁₀ aryl, or 5- to 12-membered heteroaryl;
R₁₀ is oxo (-C=O), deuterium, halogen, hydroxyl, -CN, 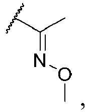 C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, or 5- to 12-membered heterocycloalkyl, wherein the C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, and 5- to 12-membered heterocycloalkyl are each substituted by 0, 1, 2, 3, 4, or 5 R₁₁;
R₁₁ is halogen, -CN, -OH, C₁₋₆ alkyl, or oxo (-C=O);
each of said 5- to 12-membered heteroaryl is independently 5- to 12-membered heteroaryl containing 1, 2, or 3 heteroatoms independently selected from the group consisting of N, O, and S; each of said 5- to 12-membered heterocycloalkyl is independently 5- to 12-membered heterocycloalkyl containing 1, 2, or 3 heteroatoms independently selected from the group consisting of N, O, and S; each of said 5- to 12-membered heterocycloalkenyl is independently 5- to 12-membered heterocycloalkenyl containing 1, 2, or 3 heteroatoms independently selected from the group consisting of N, O, and S.

The present disclosure provides a compound of formula (I), an isotopic derivative thereof, a nitrogen oxide thereof, a pharmaceutically acceptable salt thereof, a solvate thereof, or a solvate of the pharmaceutically acceptable salt thereof: 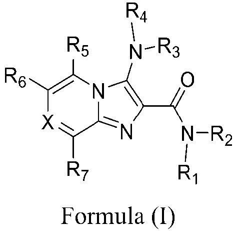 wherein
R₁, R₂, R₃, and R₄ are each independently hydrogen, C₁₋₆ alkyl, C₃₋₆ cycloalkyl, C₃₋₆ cycloalkyl C₁₋₃ alkyl, C₂₋₆ alkenyl, C₂₋₆ alkynyl, C₃₋₆ heterocycloalkyl, C₃₋₆ heterocycloalkyl C₁₋₃ alkyl, aryl, heteroaryl, aryl C₁₋₃ alkyl, or heteroaryl C₁₋₃ alkyl, wherein each of said groups is optionally substituted by 1, 2, 3, 4, or 5 R₉;
alternatively, R₁ and R₂ are connected to form a ring, and R₃ and R₄ are connected to form a ring;
X is selected from the group consisting of N and CRs;
R₅, R₆, and R₈ are each independently hydrogen, halogen, cyano, C₁₋₆ alkyl, C₁₋₆ alkoxy, C₁₋₆ haloalkyl, or C₁₋₆ haloalkoxy;
each R₇ is independently C₆₋₁₀ aryl, C₆₋₁₀ aryloxy, C₂₋₉ heteroaryl, C₂₋₉ heteroaryloxy, cycloalkyl, heterocycloalkyl, C₁₋₆ alkyl, C₁₋₆ alkoxy, C₆₋₁₀ arylamino, C₂₋₉ heteroarylamino, C₂₋₉ heteroaryl C₁₋₃ alkoxy, or C₆₋₁₀ aryl C₁₋₃ alkoxy, wherein each of said groups is optionally substituted by 1, 2, 3, 4, or 5 R₁₀;
R₉ is halogen, oxo, hydroxyl, cyano, nitro, C₁₋₆ alkyl, or C₁₋₆ alkoxy;
R₁₀ is halogen, oxo, hydroxy, cyano, nitro, C₁₋₆ alkyl, C₁₋₆ alkoxy, C₁₋₆ haloalkyl, C₁₋₆ haloalkoxy, C₂₋₆ alkenyl, C₂₋₆ alkynyl, -OR₁₂, -S(O)ₙR₁₁, -NR₁₁R₁₂, -C(O)R₁₁, -COOR₁₁, - OC(O)R₁₁, -NR₁₂C(O)R₁₁, -C(O)NR₁₁R₁₂, -NR₁₂C(O)OR₁₁, -OC(O)NR₁₁R₁₂, -NR₁₂S(O)ₙR₁₁, -S(O)ₙNR₁₁R₁₂, amino, C₁₋₄ alkylamino, C₂₋₈ dialkylamino, arylalkyl, cycloalkylalkyl, heteroarylalkyl, heterocycloalkylalkyl, aryl, cycloalkyl, heteroaryl, or heterocycloalkyl, wherein said C₁₋₆ alkyl, C₂₋₆ alkenyl, C₂₋₆ alkynyl, arylalkyl, cycloalkylalkyl, heteroarylalkyl, heterocycloalkylalkyl, aryl, cycloalkyl, heteroaryl, or heterocycloalkyl are each optionally substituted by 1, 2, 3, 4, or 5 substituents independently selected from the group consisting of halogen, oxo, hydroxy, cyano, nitro, C₁₋₆ alkyl, C₁₋₆ alkoxy, C₁₋₆ haloalkyl, C₁₋₆ haloalkoxy, C₂₋₆ alkenyl, C₂₋₆ alkynyl, -OR₁₁, -S(O)ₙR₁₁, -NR₁₁R₁₂, -C(O)R₁₁, -COOR₁₁, -OC(O)R₁₁, - NR₁₂C(O)R₁₁, -C(O)NR₁₁R₁₂, -NR₁₂C(O)OR₁₁, -OC(O)NR₁₁R₁₂, -NR₁₂S(O)ₙR₁₁, and - S(O)ₙNR₁₁R₁₂;
R₁₁ and R₁₂ are independently selected from the group consisting of hydrogen, C₁₋₆ alkyl, C₁₋₆ haloalkyl, aryl, heteroaryl, heteroaryl C₁₋₄ alkyl, aryl C₁₋₄ alkyl, cycloalkyl, heterocycloalkyl, and heterocycloalkylalkyl;
n is 1 or 2.

The present disclosure provides a compound of formula (I), an isotopic derivative thereof, a nitrogen oxide thereof, a pharmaceutically acceptable salt thereof, a solvate thereof, or a solvate of the pharmaceutically acceptable salt thereof: 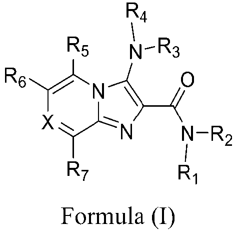 wherein
R₁ and R₂ are each independently hydrogen, C₁₋₆ alkyl, C₃₋₆ cycloalkyl, C₃₋₆ cycloalkyl C₁₋₃ alkyl, or C₂₋₆ alkenyl, wherein each of said groups is optionally substituted by 1, 2, 3, 4, or 5 R₉;
R₃, R₄, R₅, and R₆ are all hydrogen;
X is selected from the group consisting of N and CRs;
R₈ is a substituent selected from the group consisting of hydrogen, halogen, and cyano;
each R₇ is independently C₆₋₁₀ aryl, C₆₋₁₀ aryloxy, C₂₋₉ heteroaryl, C₂₋₉ heteroaryloxy, cycloalkyl, heterocycloalkyl, C₆₋₁₀ arylamino, C₂₋₉ heteroarylamino, C₂₋₉ heteroaryl C₁₋₃ alkoxy, or C₆₋₁₀ aryl C₁₋₃ alkoxy, wherein each of said groups is optionally substituted by 1, 2, 3, 4, or 5 R₁₀;
R₉ is halogen;
R₁₀ is deuterium, halogen, hydroxyl, cyano, C₁₋₆ alkyl, C₁₋₆ alkoxy, cycloalkyl, or heterocycloalkyl, wherein said C₁₋₆ alkyl, cycloalkyl, and heterocycloalkyl are each optionally substituted by 1, 2, 3, 4, or 5 substituents independently selected from the group consisting of halogen, oxo, hydroxy, and cyano.

In a preferred embodiment, for the compound of formula (I), isotopic derivative thereof, the nitrogen oxide thereof, the pharmaceutically acceptable salt thereof, the solvate thereof, or the solvate of the pharmaceutically acceptable salt thereof, the definitions of certain groups may be as described below, while the definitions of other groups may be as described in any embodiment of the present disclosure (hereinafter referred to as "in a preferred embodiment"): R₁ is H.

In a preferred embodiment, R₂ is C₁₋₆ alkyl, C₃₋₆ cycloalkyl, C₂₋₆ alkenyl, C₂₋₆ alkynyl, 4- to 12-membered heterocycloalkyl, C₆₋₁₀ aryl, or 5- to 12-membered heteroaryl, wherein the C₁₋₆ alkyl, C₃₋₆ cycloalkyl, C₂₋₆ alkenyl, 4- to 12-membered heterocycloalkyl, or 5- to 12-membered heteroaryl are each substituted by 0, 1, 2, 3, 4, or 5 R₉.

In a preferred embodiment, R₁₋₁ is C₁₋₆ alkyl.

In a preferred embodiment, R₃ and R₄ are H.

In a preferred embodiment, R₈ is hydrogen, deuterium, halogen, -CN, C₁₋₆ alkyl, -O-C₁₋₆ alkyl, or C₁₋₆ alkyl substituted by 1, 2, or 3 R₈₋₁.

In a preferred embodiment, R₇ is -CN, C₁₋₆ alkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, or -Z-(CRₐR_{b})ₘ-C₆₋₁₀ aryl, wherein the C₆₋₁₀ aryl, 5- to 12-membered heteroaryl, 5- to 12-membered heterocycloalkyl, 5- to 12-membered heterocycloalkenyl, and - Z-(CRₐR_{b})ₘ-C₆₋₁₀ aryl are each substituted by 1, 2, 3, 4, or 5 R₁₀.

In a preferred embodiment, Rₐ and R_{b} are H.

In a preferred embodiment, Z is -O-.

In a preferred embodiment, m is 0 or 1.

In a preferred embodiment, R₉ is halogen, -OH, -CN, C₁₋₆ alkyl, -O-C₁₋₆ alkyl, C₃₋₆ cycloalkyl, 5- to 12-membered heterocycloalkyl, C₆₋₁₀ aryl, 5- to 12-membered heteroaryl, or 5- to 12-membered heteroaryl substituted by 1, 2, or 3 R₉₋₁.

In a preferred embodiment, R₉₋₁ is independently C₁₋₆ alkyl.

In a preferred embodiment, R₁₀ is oxo (-C=O), deuterium, halogen, -OH, -CN, 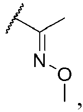 C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, or 5- to 12-membered heterocycloalkyl, wherein the C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, and 5- to 12-membered heterocycloalkyl are each substituted by 0, 1, 2, 3, 4, or 5 R₁₁.

In a preferred embodiment, R₁₁ is deuterium, halogen, -CN, -OH, C₁₋₆ alkyl, or oxo (-C=O).

In a preferred embodiment, in R₁, R₂, R₃, R₄, R₅, R₆, R₇, R₈, R₉, R₁₀, R₁₁, R₁₋₁, R₃₋₁, R₈₋₁, R₉₋₁, Rₐ, and R_{b}, the C₁₋₆ alkyl in said C₁₋₆ alkyl and substituted C₁₋₆ alkyl is independently methyl, ethyl, n-propyl, isopropyl, n-butyl, isobutyl, or tert-butyl; for example, methyl, ethyl, n-propyl, or isopropyl.

In a preferred embodiment, in R₁, R₂, R₃, R₄, R₇, R₈, R₉, and R₁₀, the C₃₋₆ cycloalkyl in said C₃₋₆ cycloalkyl and substituted C₃₋₆ cycloalkyl is independently cyclopropyl, cyclobutyl, cyclopentyl, 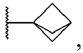 or cyclohexyl.

In a preferred embodiment, in R₁, R₂, and R₁₀, the C₂₋₆ alkenyl in said C₂₋₆ alkenyl and substituted C₂₋₆ alkenyl is independently vinyl, n-propenyl, isopropenyl, n-butenyl, or isobutenyl.

In a preferred embodiment, in R₁, R₂, and R₁₀, the C₂₋₆ alkynyl in said C₂₋₆ alkynyl and substituted C₂₋₆ alkynyl is independently ethynyl, n-propynyl, or n-butynyl.

In a preferred embodiment, in R₁, R₂, R₇, R₈, R₉, R₁₀, and R₁₁, the C₆₋₁₀ aryl in said C₆₋₁₀ aryl and substituted C₆₋₁₀ aryl is independently phenyl or naphthyl, for example, phenyl.

In a preferred embodiment, in R₃, R₄, R₈, R₉, R₁₀, R₁₁, R₁₁, R₃₋₁, R₈₋₁, and R₉₋₁, the C₁₋₆ alkyl in said -O-C₁₋₆ alkyl and substituted -O-C₁₋₆ alkyl is independently methyl, ethyl, n-propyl, isopropyl, n-butyl, isobutyl, or tert-butyl; for example, methyl, ethyl, n-propyl, or isopropyl.

In a preferred embodiment, in R₃, R₄, R₅, R₆, R₈, R₉, R₁₀, R₁₁, R₁₋₁, R₃₋₁, R₈₋₁, and R₉₋1, the halogen is independently F, Cl, or Br, for example F or Cl.

In a preferred embodiment, in R₁ and R₂, the 4- to 12-membered heterocycloalkyl in said 4- to 12-membered heterocycloalkyl and 4- to 12-membered heterocycloalkyl substituted by 1, 2, 3, 4, or 5 R₉ is independently 4- to 6-membered heterocycloalkyl.

In a preferred embodiment, in R₁ and R₂, the 4- to 12-membered heterocycloalkyl in said 4- to 12-membered heterocycloalkyl and 4- to 12-membered heterocycloalkyl substituted by 1, 2, 3, 4, or 5 R₉ is independently 4- to 12-membered heterocycloalkyl containing 1, 2, or 3 heteroatoms, wherein the heteroatom is N, for example

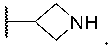

In a preferred embodiment, in R₁ and R₂, the 5- to 12-membered heteroaryl in said 5-to 12-membered heteroaryl and 5- to 12-membered heteroaryl substituted by 1, 2, 3, 4, or 5 R₉ is independently 5- to 6-membered heteroaryl.

In a preferred embodiment, in R₁ and R₂, the 5- to 12-membered heteroaryl in said 5-to 12-membered heteroaryl and 5- to 12-membered heteroaryl substituted by 1, 2, 3, 4, or 5 R₉ is independently 5- to 12-membered heteroaryl containing 1, 2, or 3 heteroatoms selected from the group consisting of N and O, for example,

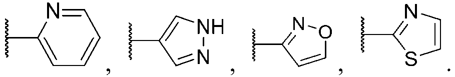

In a preferred embodiment, when R₁ and R₂ together with the N atom to which they are attached form a 4- to 12-membered heterocycloalkyl or a 4- to 12-membered heterocycloalkyl substituted by 1, 2, or 3 R₁₁, the 4- to 12-membered heterocycloalkyl in said 4- to 12-membered heterocycloalkyl and 4- to 12-membered heterocycloalkyl substituted by 1, 2, or 3 R₁₋₁ is independently 4- to 6-membered heterocycloalkyl.

In a preferred embodiment, when R₁ and R₂ together with the N atom to which they are attached form a 4- to 12-membered heterocycloalkyl or a 4- to 12-membered heterocycloalkyl substituted by 1, 2, or 3 R₁₁, the 4- to 12-membered heterocycloalkyl in said 4- to 12-membered heterocycloalkyl and 4- to 12-membered heterocycloalkyl substituted by 1, 2, or 3 R₁₋₁ is independently 4- to 12-membered heterocycloalkyl containing 1, 2, or 3 heteroatoms, wherein the heteroatom is N, for example,

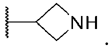

In a preferred embodiment, in R₇, the C₃₋₆ cycloalkenyl in said C₃₋₆ cycloalkenyl and C₃₋₆ cycloalkenyl substituted by 1, 2, 3, 4, or 5 R₁₀ is independently cyclopropenyl, cyclobutenyl, cyclopentenyl, or cyclohexenyl, for example cyclohexenyl, or for example

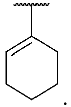

In a preferred embodiment, in R₇, the 5- to 12-membered heteroaryl group in said 5-to 12-membered heteroaryl and 5- to 12-membered heteroaryl substituted by 1, 2, 3, 4, or 5 R₁₀ is independently 5- to 12-membered heteroaryl containing 1, 2, or 3 heteroatoms selected from the group consisting of N and O, for example,

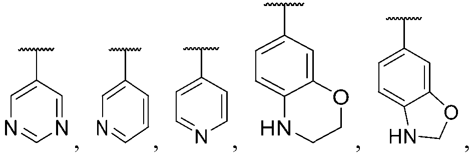

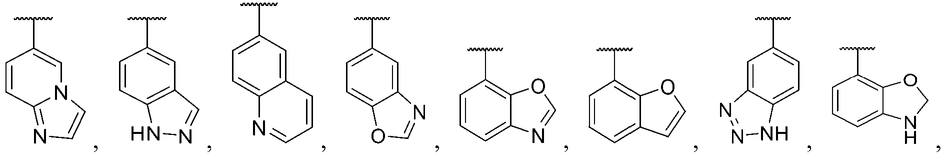

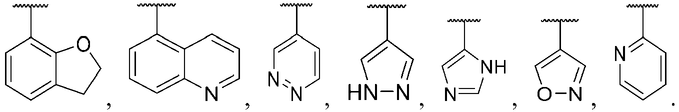

In a preferred embodiment, in R₇, the 5- to 12-membered heterocycloalkyl in said 5-to 12-membered heterocycloalkyl and 5- to 12-membered heterocycloalkyl substituted by 1, 2, 3, 4, or 5 R₁₀ is independently 5- to 6-membered heterocycloalkyl.

In a preferred embodiment, in R₇, the 5- to 12-membered heterocycloalkyl in said 5-to 12-membered heterocycloalkyl and 5- to 12-membered heterocycloalkyl substituted by 1, 2, 3, 4, or 5 R₁₀ is independently 5- to 12-membered heterocycloalkyl containing 1, 2, or 3 heteroatoms selected from the group consisting of N and O, for example,

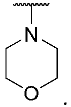

In a preferred embodiment, in R₇, the 5- to 12-membered heterocycloalkenyl in said 5- to 12-membered heterocycloalkenyl and 5- to 12-membered heterocycloalkenyl substituted by 1, 2, 3, 4, or 5 R₁₀ is independently 5- to 6-membered heterocycloalkyl.

In a preferred embodiment, in R₇, the 5- to 12-membered heterocycloalkenyl in said 5- to 12-membered heterocycloalkenyl and 5- to 12-membered heterocycloalkenyl substituted by 1, 2, 3, 4, or 5 R₁₀ is independently 5- to 12-membered heterocycloalkyl containing 1, 2, or 3 heteroatoms, wherein the heteroatom is N, for example,

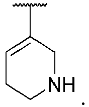

In a preferred embodiment, in R₇, the -Z-(CRₐR_{b})ₘ-C₆₋₁₀ aryl in said -Z-(CRₐR_{b})ₘ-C₆₋₁₀ aryl and -Z-(CRₐR_{b})ₘ-C₆₋₁₀ aryl substituted by 1, 2, 3, 4, or 5 R₁₀ is independently 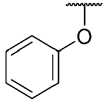 or

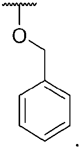

In a preferred embodiment, in R₉, the 5- to 12-membered heteroaryl in said 5- to 12-membered heteroaryl and 5- to 12-membered heteroaryl substituted by 1, 2, or 3 R₉₋₁ is independently 5- to 6-membered heteroaryl.

In a preferred embodiment, in R₉, the 5- to 12-membered heteroaryl in said 5- to 12-membered heteroaryl and 5- to 12-membered heteroaryl substituted by 1, 2, or 3 R₉₋₁ is independently 5- to 12-membered heteroaryl containing 1, 2, or 3 heteroatoms selected from the group consisting of N and O, for example,

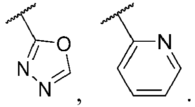

In a preferred embodiment, in R₁₀, the 5- to 12-membered heteroaryl in said 5- to 12-membered heteroaryl and 5- to 12-membered heteroaryl substituted by 1, 2, or 3 R₉₋₁ is independently 5- to 12-membered heteroaryl containing 1, 2, 3, or 4 heteroatoms selected from the group consisting of N and O, for example,

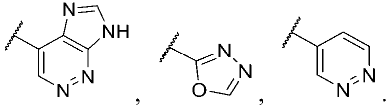

In a preferred embodiment, in R₁₀, the 5- to 12-membered heterocycloalkyl in said 5-to 12-membered heterocycloalkyl and 5- to 12-membered heterocycloalkyl substituted by 1, 2, or 3 R₉₋₁ is independently 5- to 6-membered heterocycloalkyl.

In a preferred embodiment, in R₁₀, the 5- to 12-membered heterocycloalkyl in said 5-to 12-membered heterocycloalkyl and 5- to 12-membered heterocycloalkyl substituted by 1, 2, or 3 R₉₋₁ is independently 5- to 12-membered heterocycloalkyl containing 1, 2, or 3 heteroatoms selected from the group consisting of N and O, for example, 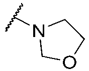 or

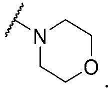

In a preferred embodiment, in R₁₀, the C₁₋₆ alkyl in said -S-C₁₋₆ alkyl and -S-C₁₋₆ alkyl substituted by 1, 2, 3, 4, or 5 R₁₁ is independently methyl, ethyl, n-propyl, isopropyl, n-butyl, isobutyl, or tert-butyl, for example, methyl.

In a preferred embodiment, R₂ is

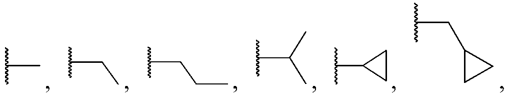

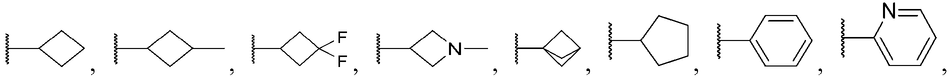

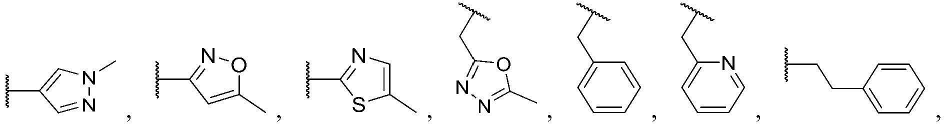

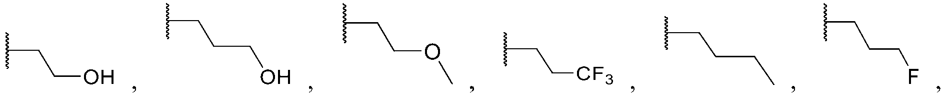

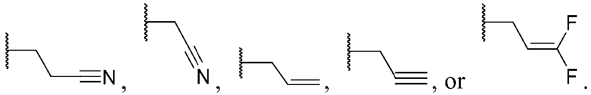

In a preferred embodiment, R₁ and R₂ together with the N atom to which they are attached form

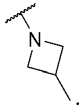

In a preferred embodiment, R₅ is H, deuterium, or methyl.

In a preferred embodiment, R₆ is H, deuterium, F, or methyl.

In a preferred embodiment, 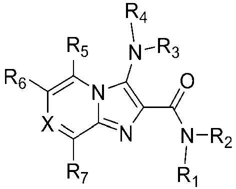 is

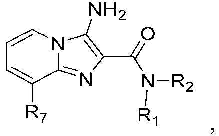

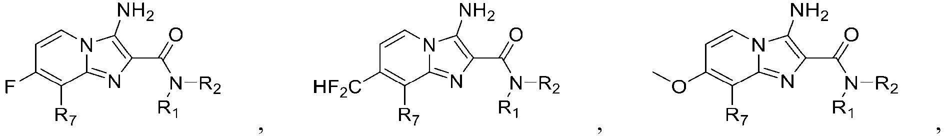

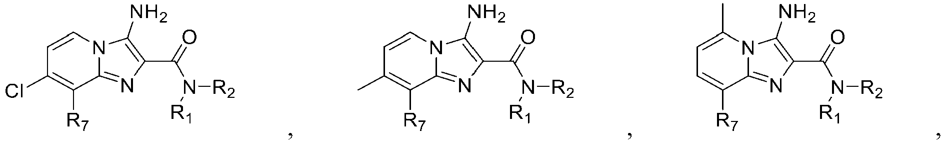

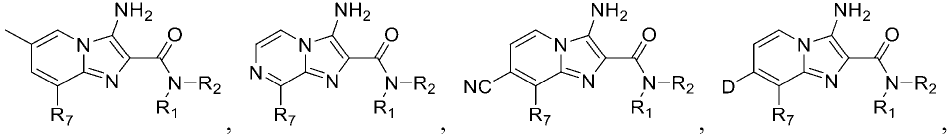

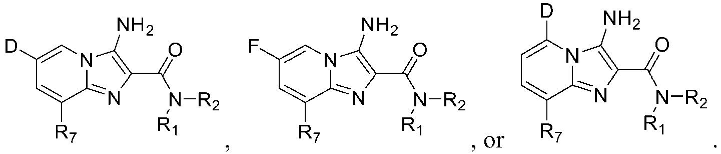

In a preferred embodiment, R₇ is 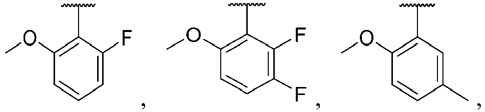 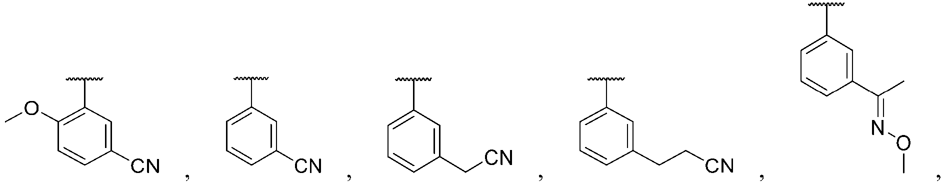 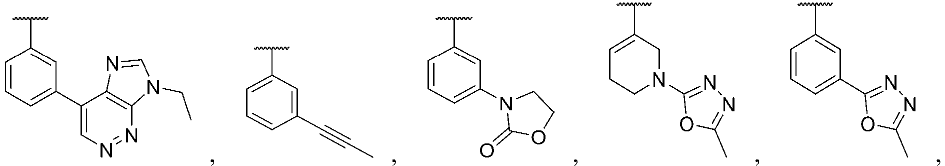 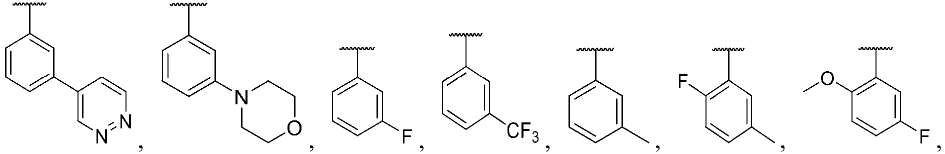 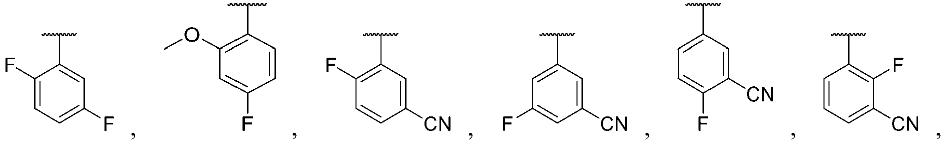 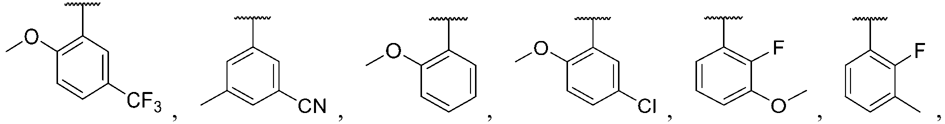 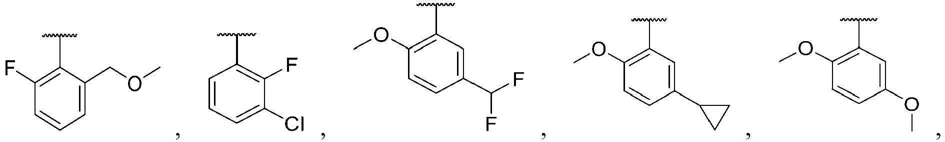 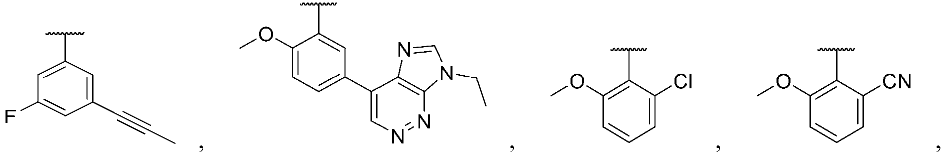 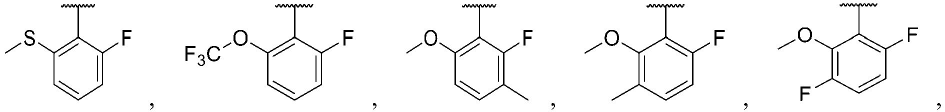 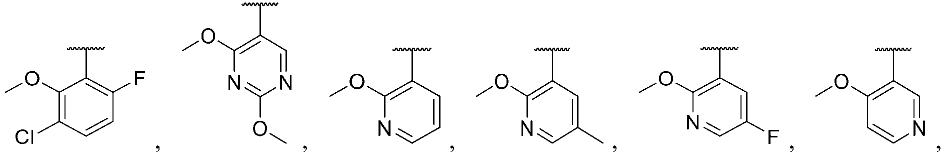 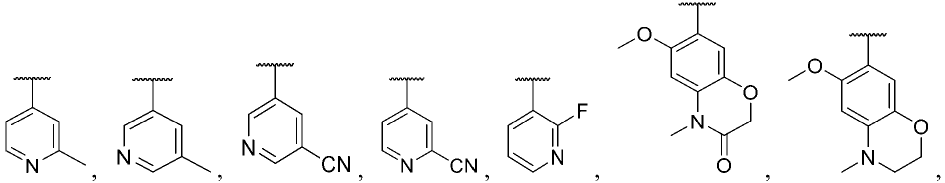 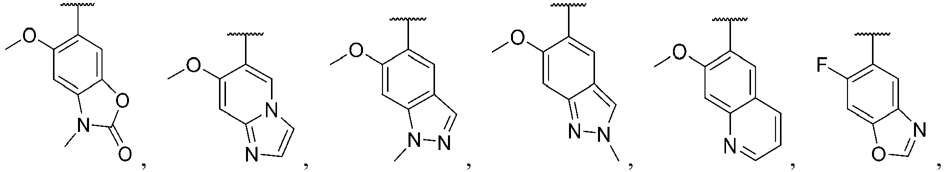 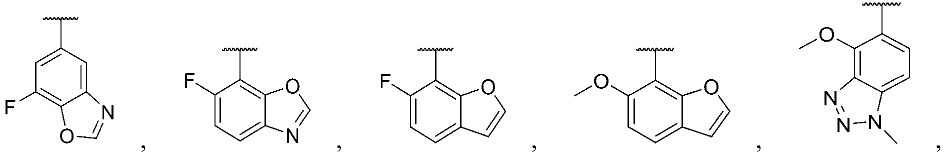 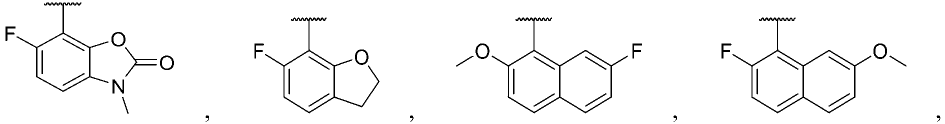 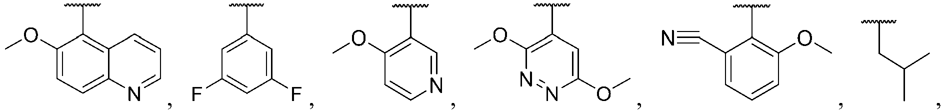 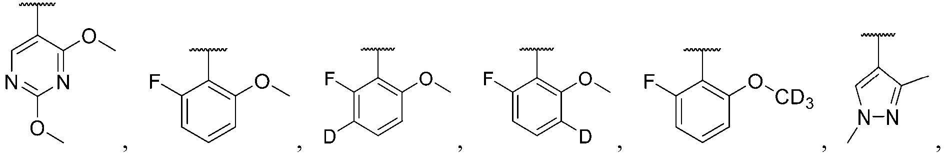 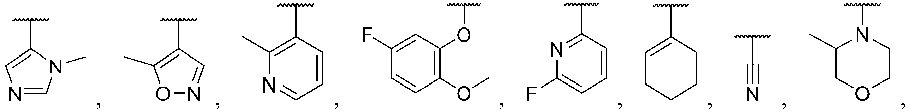 or

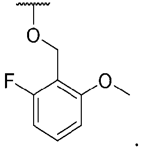

In a preferred embodiment, R₈ is H, D, F, Cl, -CN, -CH₃, -OCH₃, or -CHF₂.

In a preferred embodiment, R₉ is F, -OH, -CN, methyl, methoxy, cyclopropyl, phenyl,

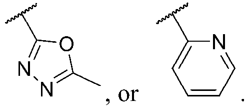

In a preferred embodiment, R₁₀ is oxo (-C=O), deuterium, F, Cl, -OH, -CN, 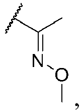 methyl, methoxy, -CF₃, 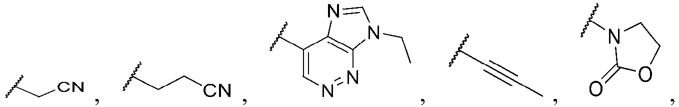 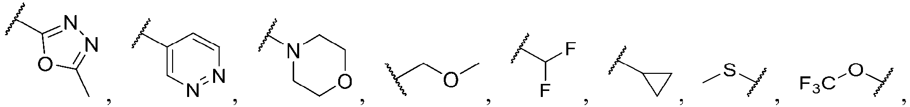 or

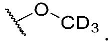

In a preferred embodiment, R₁₁ is deuterium, F, -CN, methyl, ethyl, or oxo (-C=O).

In a preferred embodiment, 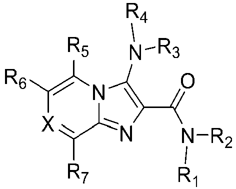 is 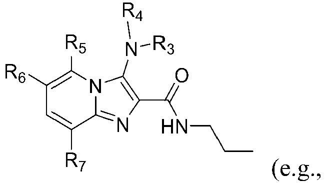 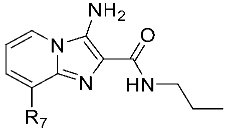 );
R₇ is 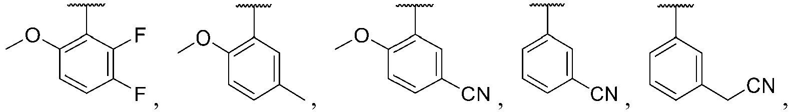 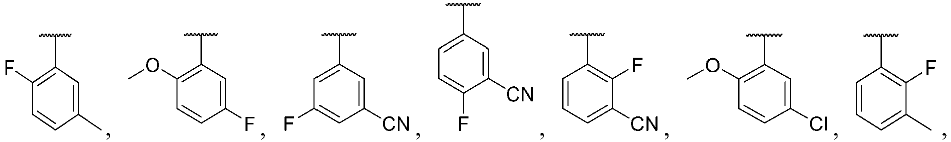 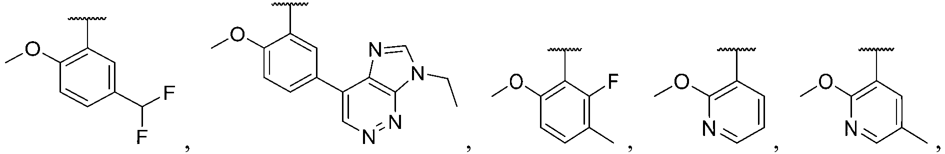 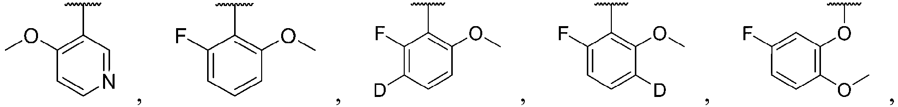 or

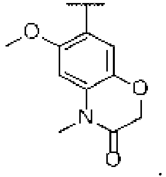

In one embodiment, the compound of formula (I) is any one selected from the group consisting of the following compounds:

| No. | Structure | No. | Structure |
|---|---|---|---|
| 1 | 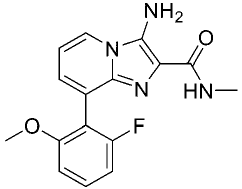 | 81 | 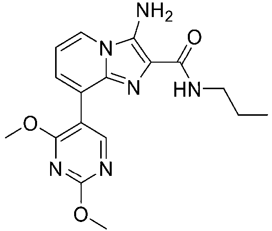 |
| 2 | 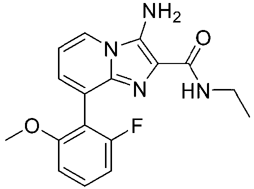 | 82 | 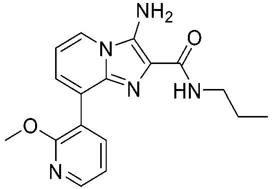 |
| 3 | 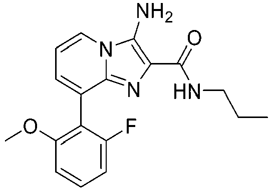 | 83 | 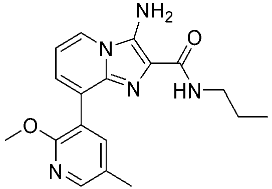 |
| 4 | 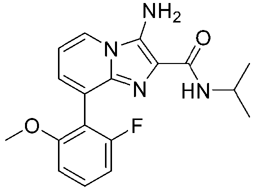 | 84 | 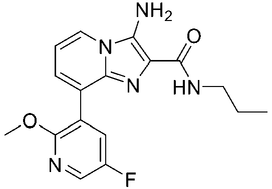 |
| 5 | 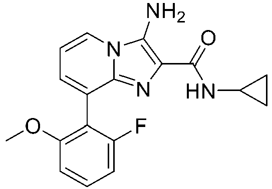 | 85 | 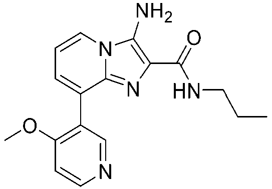 |
| 6 | 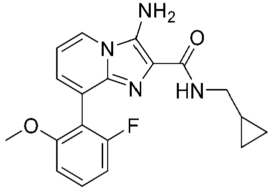 | 86 | 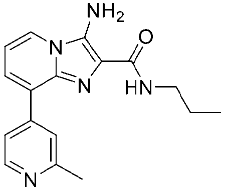 |
| 7 | 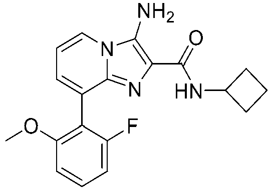 | 87 | 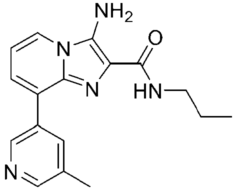 |
| 8 | 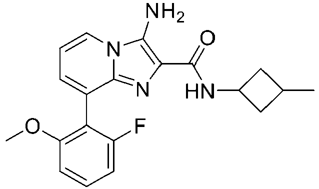 | 88 | 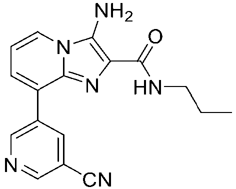 |
| 9 | 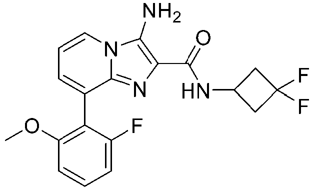 | 89 | 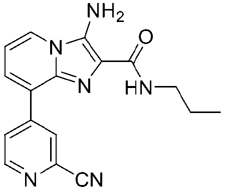 |
| 10 | 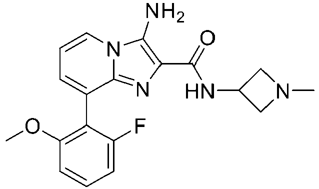 | 90 | 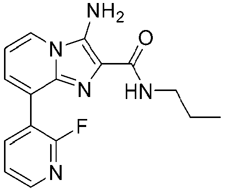 |
| 11 | 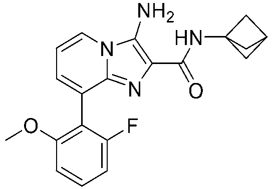 | 91 | 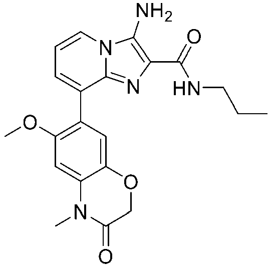 |
| 12 | 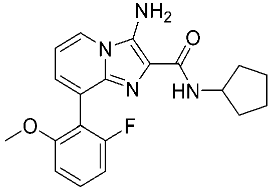 | 92 | 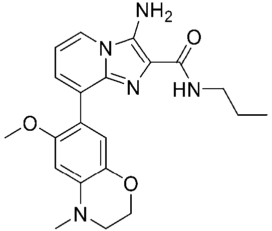 |
| 13 | 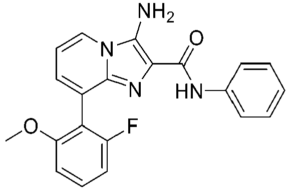 | 93 | 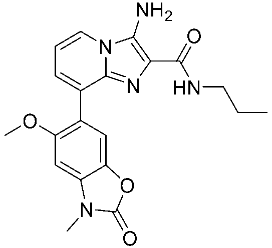 |
| 14 | 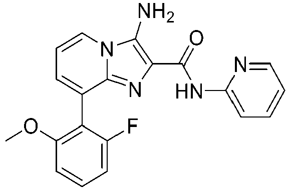 | 94 | 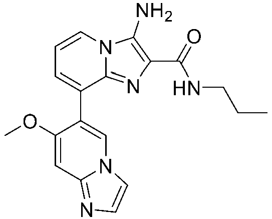 |
| 15 | 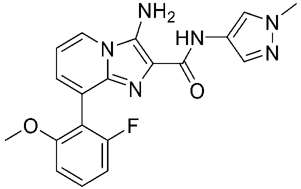 | 95 | 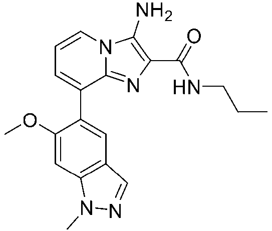 |
| 16 | 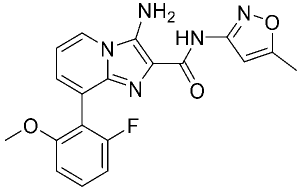 | 96 | 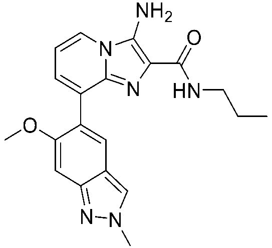 |
| 17 | 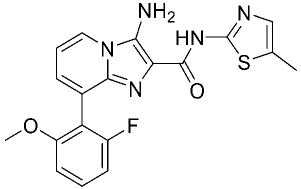 | 97 | 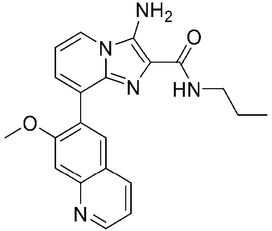 |
| 18 | 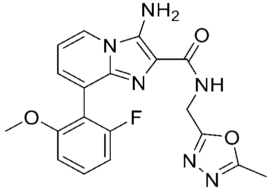 | 98 | 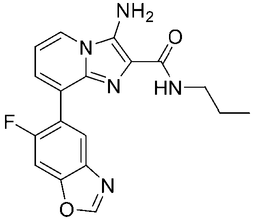 |
| 19 | 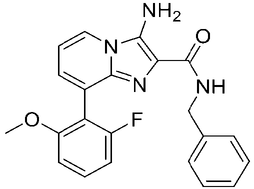 | 99 | 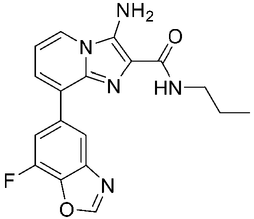 |
| 20 | 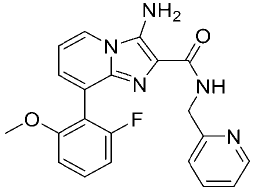 | 100 | 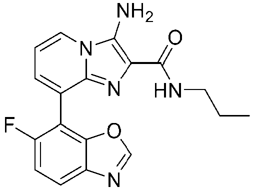 |
| 21 | 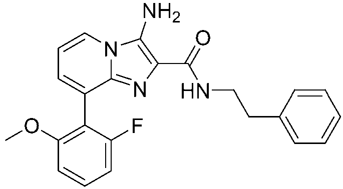 | 101 | 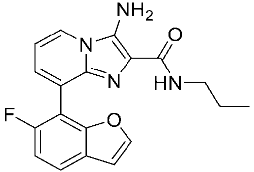 |
| 22 | 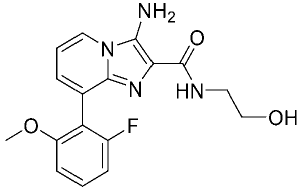 | 102 | 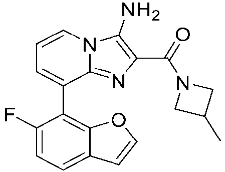 |
| 23 | 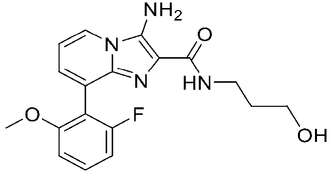 | 103 | 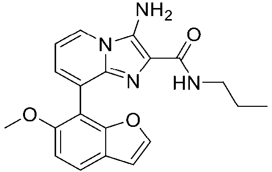 |
| 24 | 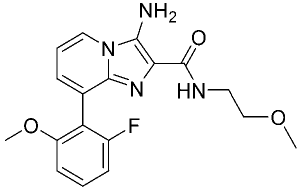 | 104 | 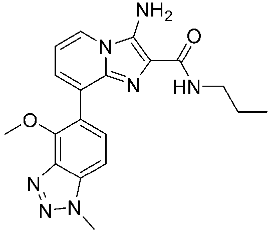 |
| 25 | 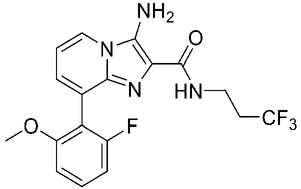 | 105 | 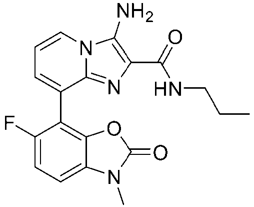 |
| 26 | 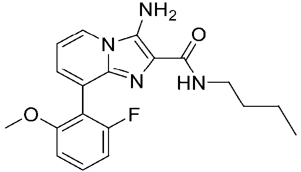 | 106 | 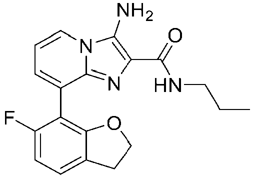 |
| 27 | 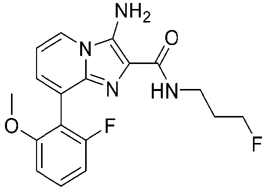 | 107 | 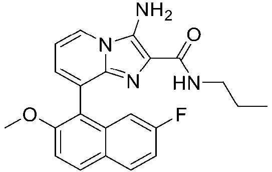 |
| 28 | 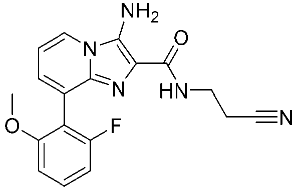 | 108 | 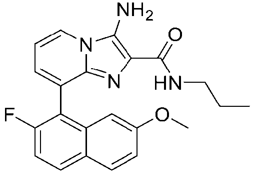 |
| 29 | 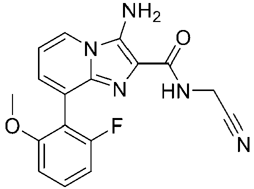 | 109 | 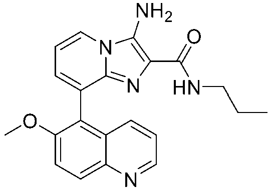 |
| 30 | 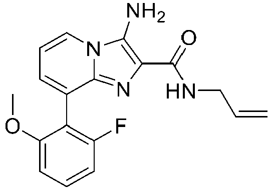 | 110 | 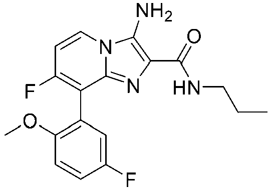 |
| 31 | 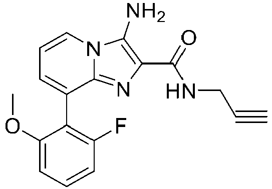 | 111 | 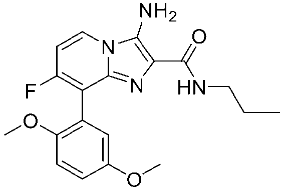 |
| 32 | 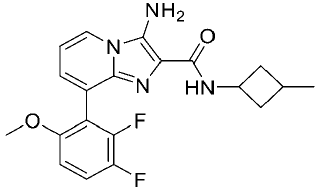 | 112 | 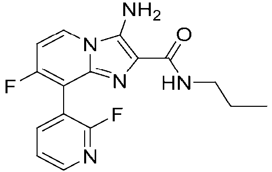 |
| 33 | 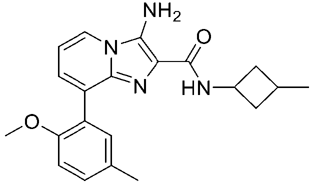 | 113 | 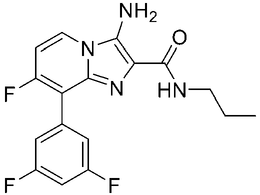 |
| 34 | 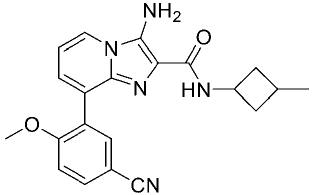 | 114 | 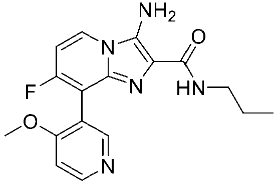 |
| 35 | 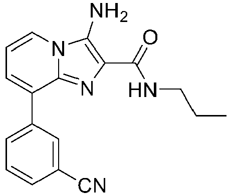 | 115 | 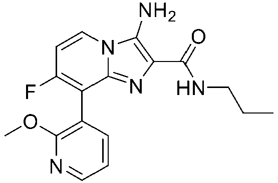 |
| 36 | 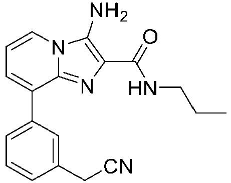 | 116 | 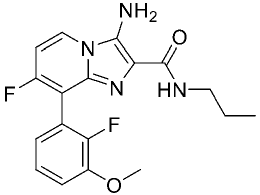 |
| 37 | 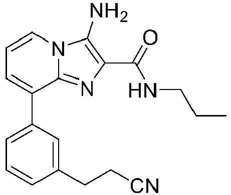 | 117 | 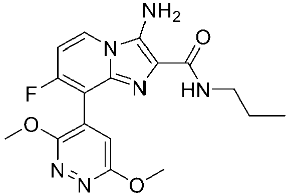 |
| 38 | 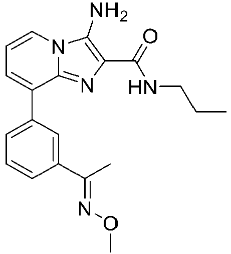 | 118 | 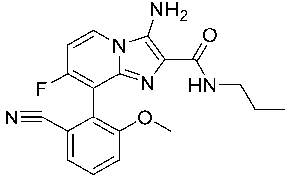 |
| 39 | 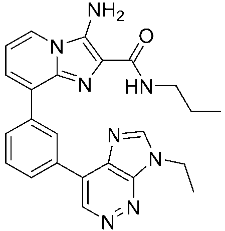 | 119 | 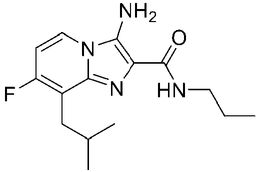 |
| 40 | 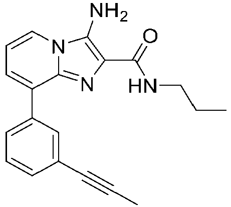 | 120 | 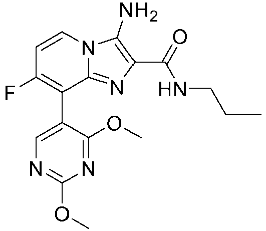 |
| 41 | 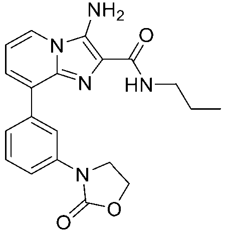 | 121 | 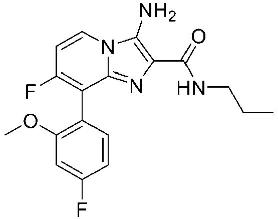 |
| 42 | 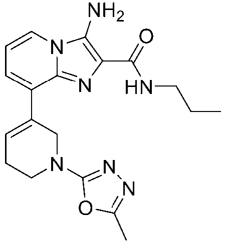 | 122 | 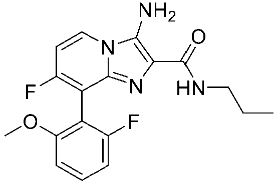 |
| 43 | 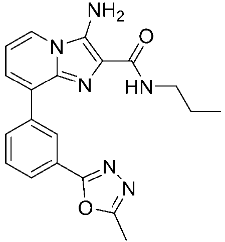 | 123 | 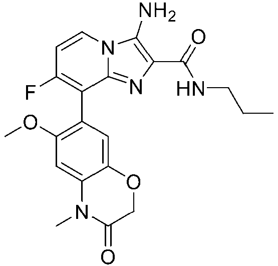 |
| 44 | 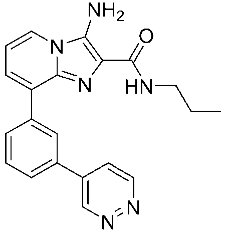 | 124 | 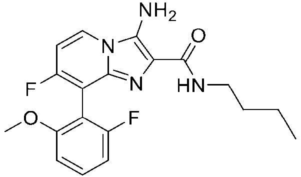 |
| 45 | 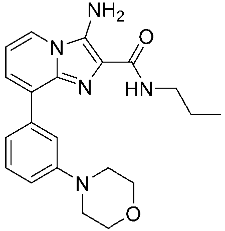 | 125 | 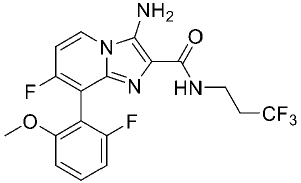 |
| 46 | 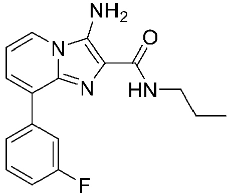 | 126 | 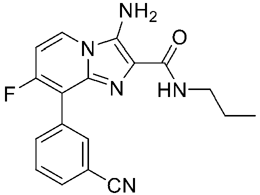 |
| 47 | 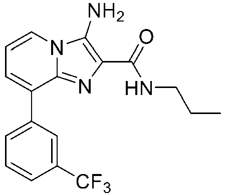 | 127 | 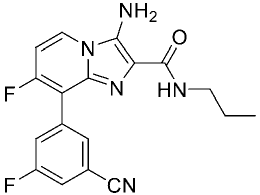 |
| 48 | 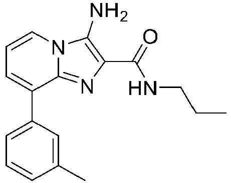 | 128 | 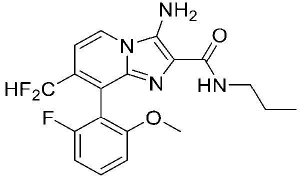 |
| 49 | 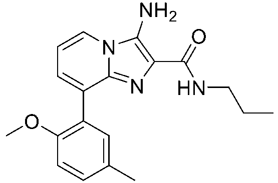 | 129 | 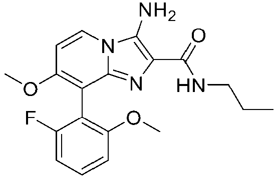 |
| 50 | 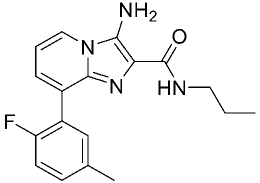 | 130 | 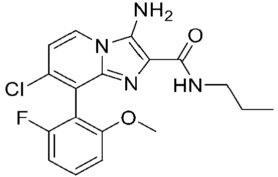 |
| 51 | 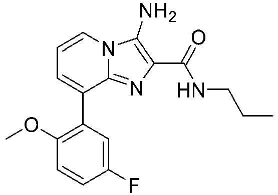 | 131 | 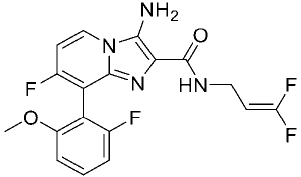 |
| 52 | 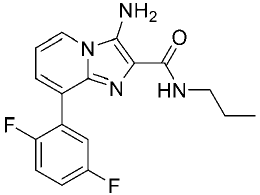 | 132 | 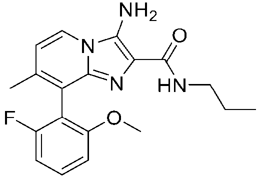 |
| 53 | 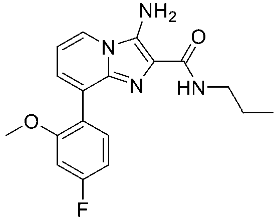 | 133 | 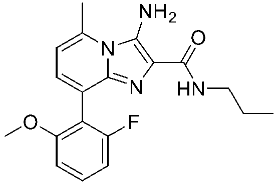 |
| 54 | 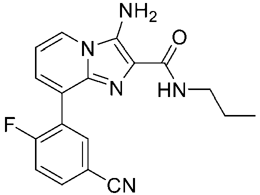 | 134 | 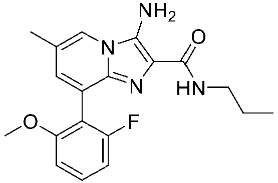 |
| 55 | 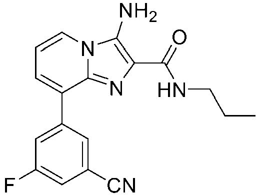 | 135 | 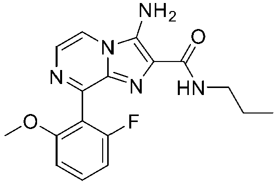 |
| 56 | 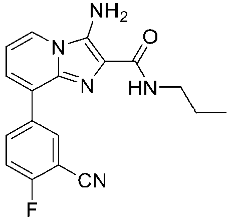 | 136 | 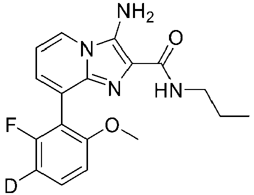 |
| 57 | 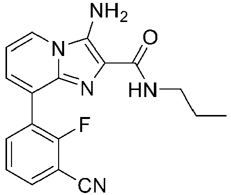 | 137 | 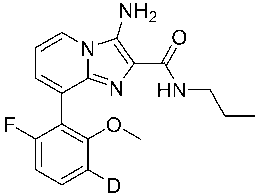 |
| 58 | 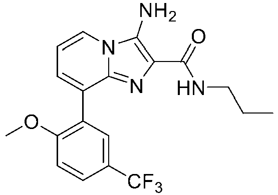 | 138 | 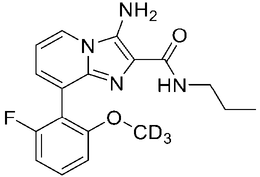 |
| 59 | 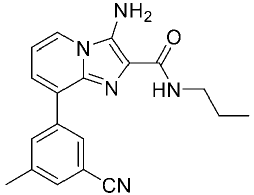 | 139 | 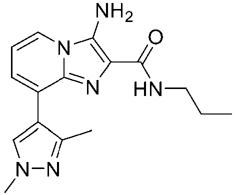 |
| 60 | 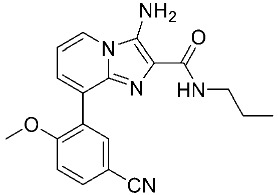 | 140 | 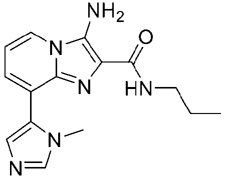 |
| 61 | 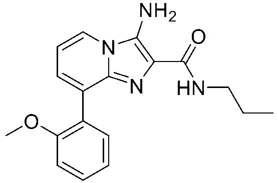 | 141 | 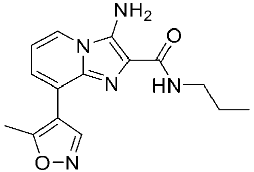 |
| 62 | 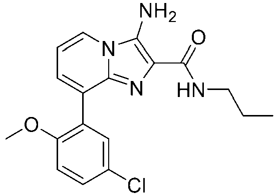 | 142 | 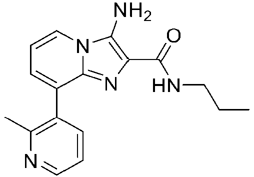 |
| 63 | 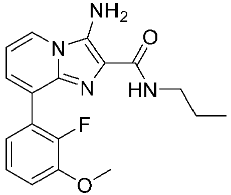 | 143 | 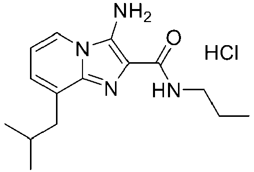 |
| 64 | 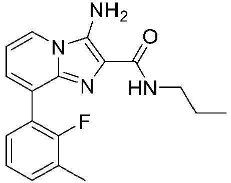 | 144 | 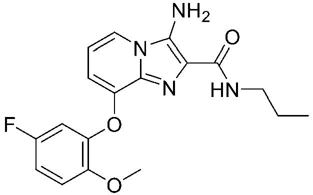 |
| 65 | 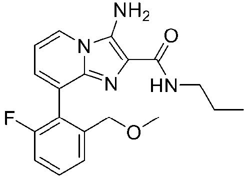 | 145 | 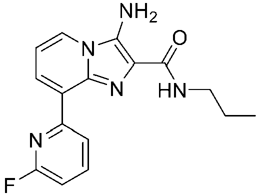 |
| 66 | 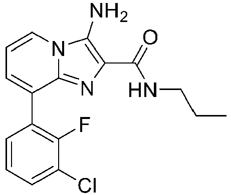 | 146 | 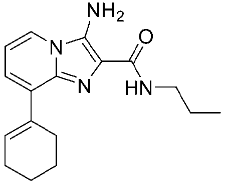 |
| 67 | 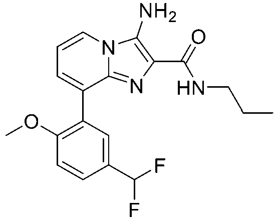 | 147 | 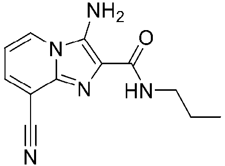 |
| 68 | 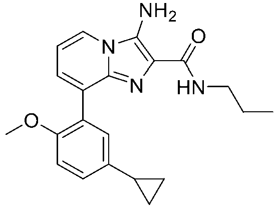 | 148 | 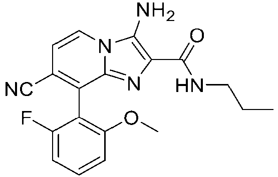 |
| 69 | 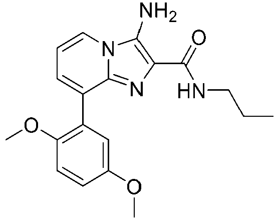 | 149 | 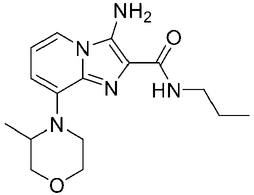 |
| 70 | 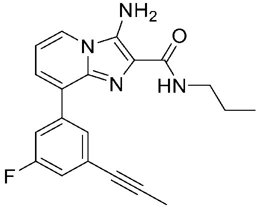 | 150 | 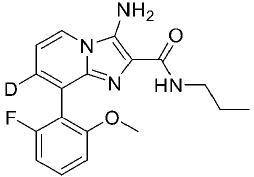 |
| 71 | 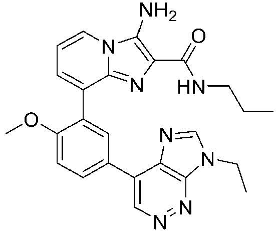 | 151 | 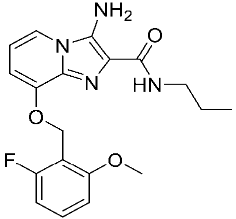 |
| 72 | 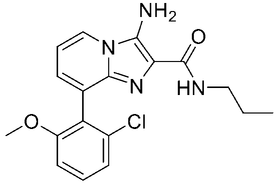 | 154 | 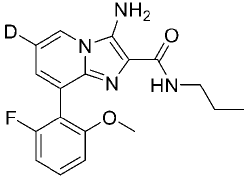 |
| 73 | 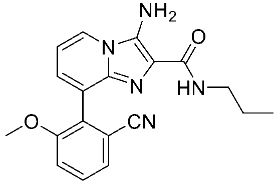 | 155 | 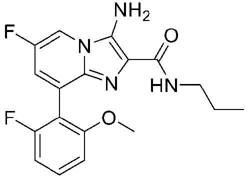 |
| 74 | 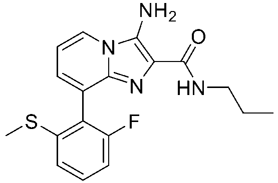 | 156 | 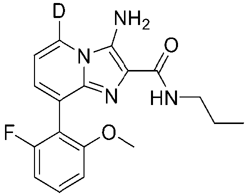 |
| 75 | 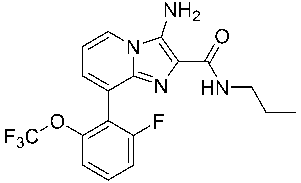 | | |
| 76 | 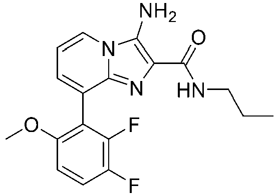 | | |
| 77 | 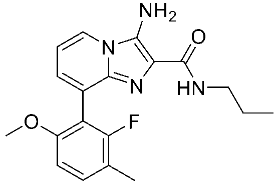 | | |
| 78 | 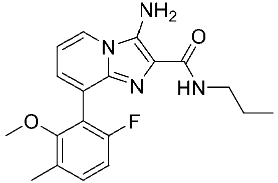 | | |
| 79 | 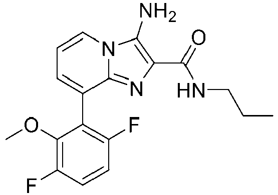 | | |
| 80 | 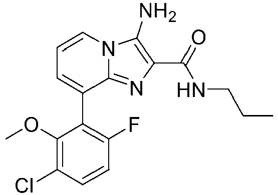 | | |

The present disclosure provides a use of a substance X or the aforementioned pharmaceutical composition in the manufacture of a medicament for treating or preventing a GABA_{A} receptor-associated disease; wherein the substance X is the compound as described in any preceding embodiment, an isotopic derivative thereof, a nitrogen oxide thereof, a pharmaceutically acceptable salt thereof, a solvate thereof, or a solvate of the pharmaceutically acceptable salt thereof.

Further, the GABA_{A} receptor-associated disease is selected from the group consisting of pain, Alzheimer's disease, multi-infarct dementia, stroke, epilepsy, anxiety, pruritus, and depression.

Further, the GABA_{A} receptor-associated disease is a disease associated with α2/3-GABA_{A} receptors, for example, pain, epilepsy, anxiety, pruritus, and depression.

The present disclosure provides a use of the aforementioned substance X or the aforementioned pharmaceutical composition in the manufacture of a medicament for treating or preventing pain, Alzheimer's disease, multi-infarct dementia, stroke, epilepsy, anxiety, pruritus, or depression.

The present disclosure provides a use of the aforementioned substance X or the aforementioned pharmaceutical composition for treating or preventing a GABA_{A} receptor-associated disease.

Further, the GABA_{A} receptor-associated disease is selected from the group consisting of pain, Alzheimer's disease, multi-infarct dementia, and stroke.

Further, the GABA_{A} receptor-associated disease is a disease associated with α2/3-GABA_{A} receptors, for example, pain, epilepsy, anxiety, pruritus, and depression.

The present disclosure provides a use of the aforementioned substance X or the aforementioned pharmaceutical composition in the treatment or prevention of pain, Alzheimer's disease, multi-infarct dementia, stroke, epilepsy, anxiety, pruritus, or depression.

The present disclosure provides a method for treating or preventing a GABA_{A} receptor-associated disease, comprising administering the aforementioned substance X or the aforementioned pharmaceutical composition to a subject in need thereof.

Further, the GABA_{A} receptor-associated disease is selected from the group consisting of pain, Alzheimer's disease, multi-infarct dementia, and stroke.

Further, the GABA_{A} receptor-associated disease is a disease associated with α2/3-GABA_{A} receptors, for example, pain, epilepsy, anxiety, pruritus, and depression.

The present disclosure provides a method for treating or preventing a disease, comprising administering the aforementioned substance X or the aforementioned pharmaceutical composition to a subject in need thereof;
the disease is pain, Alzheimer's disease, multi-infarct dementia, stroke, pain, epilepsy, anxiety, pruritus, or depression.

Unless otherwise specified, the terms used in the present disclosure have the following meanings:
Unless otherwise specified, the following definitions are provided to illustrate and define the meaning and scope of the various terms used in describing the present disclosure herein.

The following definitions of general terms apply whether they appear alone or in combination.

The nomenclature employed in the present disclosure follows the IUPAC system of nomenclature. Any open valence in the carbon, oxygen, sulfur, or nitrogen atoms within the structures provided in this text indicates the presence of a hydrogen atom.

As used herein, the term "patient" is defined as any warm-blooded animal, including but not limited to mice, guinea pigs, dogs, horses, or humans, with the patient preferably being a human.

The term "halogen" refers to fluorine, chlorine, bromine, and iodine, preferably fluorine.

The term "alkyl" refers to a saturated straight or branched chain hydrocarbon group consisting solely of carbon atoms and hydrogen atoms, having the specified number of carbon atoms (e.g., C₁₋₆, more preferably C₁₋₄). Alkyl includes, but is not limited to, methyl, ethyl, n-propyl, isopropyl, n-butyl, tert-butyl, isobutyl, sec-butyl, n-pentyl, or n-hexyl.

The term "alkenyl" refers to a straight or branched hydrocarbon group having at least one double bond, consisting solely of carbon and hydrogen atoms, with a specified number of carbon atoms (e.g., C₂₋₆, more preferably C₂₋₄), attached to the rest of the molecule via a single bond, and including cis-trans or Z-E isomers. For example, alkenyl includes but is not limited to vinyl (-CH=CH₂), propenyl (-CH=CH-CH₃), allyl (-CH₂-CH=CH₂), isopropenyl (-C(CH₃)=CH₂), -CH=CH-CH₂-CH₃, -CH₂-CH=CH-CH₃, -CH=CH-CH₂-CH₂-CH₃, -CH₂-CH=CH-CH₂-CH₃, -CH=CH-CH(CH₃)₂, -CH₂-CH=C(CH₃)₂, -CH=CH-(CH₂)₃-CH₃, etc.

The term "alkynyl" refers to a straight or branched hydrocarbon group having at least one triple bond, consisting solely of carbon and hydrogen atoms, containing a specified number of carbon atoms (e.g., C₂₋₆, more preferably C₂₋₄), and attached to the rest of the molecule via a single bond. For example, alkynyl includes but is not limited to ethynyl (-C≡CH), propynyl (-C≡C-CH₃), -C≡C-CH₂-CH₃, -CH₂C≡C-CH₃, -C≡C-CH₂-CH₂-CH₃, -CH₂C≡C-CH₂-CH₃, - C≡C-CH₂-CH₂-CH₂-CH₃, -C≡C-CH₂-CH(CH₃)₂, -C≡C-C(CH₃)₃, -C≡C-CH(CH₃)₂, -CH₂C≡C-CH₂-CH₂-CH₃, etc.

The term "alkoxy" refers to the group -O-R, wherein R is alkyl.

The term "cycloalkyl" refers to a saturated monocyclic group consisting solely of carbon atoms, having the specified number of carbon atoms (e.g., C₃ to C₆). Cycloalkyl includes but is not limited to cyclopropyl, cyclobutyl, cyclopentyl, and cyclohexyl.

The term "heterocycloalkyl" refers to a saturated cyclic group having a specified number of ring atoms (e.g., 5- to 12-membered), a specified number of heteroatoms (e.g., 1, 2, or 3), and specified heteroatoms (one, two, or three selected from the group consisting of N, O, and S).

The term "heterocycloalkenyl" refers to an unsaturated cyclic group having a specified number of ring atoms (e.g., 5- to 12-membered), a specified number of heteroatoms (e.g., 1, 2, or 3), and specified heteroatoms (one, two, or three selected from the group consisting of N, O, and S).

The term "heteroaryl" refers to a cyclic group having a specified number of ring atoms (e.g., 5- to 12-membered, 5- to 10-membered, preferably 6- to 10-membered, more preferably 5- to 8-membered), a specified number of heteroatoms (e.g., 1, 2, 3, or 4), and specified heteroatoms (one, two, or three selected from the group consisting of N, O, and S), which may be monocyclic or bicyclic, and wherein at least one ring is aromatic (conforming to Hückel's rule). Heteroaryl is connected to other moieties in the molecule via an aromatic ring or a non-aromatic ring.

The term "aryl" refers to a cyclic group consisting solely of carbon atoms with the specified number of carbon atoms (e.g., C₆-C₁₀), which may be monocyclic or polycyclic, and in which at least one ring is aromatic (conforming to Hückel's rule). Aryl is connected to other fragments in the molecule via an aromatic ring or a non-aromatic ring. Aryl includes but is not limited to phenyl, naphthyl, such as

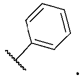

When any variable (e.g., the group R₁₁) appears more than once in the definition of a compound, their definitions are independent of and do not affect each other. For example, a phenyl substituted by 3 R₁₁ means that the phenyl is substituted by 3 R₁₁, and the definitions of the 3 R₁₁ are independent of each other and do not affect one another.

The term "pharmaceutically acceptable salt" refers to a salt formed by the reaction of a compound with a pharmaceutically acceptable acid or base, which is relatively non-toxic, safe, and suitable for patient use. When a compound contains a relatively acidic functional group, a base addition salt can be obtained by contacting the free form of the compound with a sufficient amount of a pharmaceutically acceptable base in a suitable inert solvent. When a compound contains relatively basic functional groups, an acid addition salt can be obtained by contacting the free form of the compound with a sufficient amount of a pharmaceutically acceptable acid in a suitable inert solvent. For details, refer to Handbook of Pharmaceutical Salts: Properties, Selection, and Use (P. Heinrich Stahl, 2002).

The term "solvate" refers to a substance formed when a compound crystallizes with a solvent. Solvates are classified into stoichiometric solvates and non-stoichiometric solvates.

The term "solvate of the pharmaceutically acceptable salt" refers to a substance formed by the combination of a compound with a pharmaceutically acceptable (relatively non-toxic, safe, and suitable for patient use) acid, base, or solvent, wherein the pharmaceutically acceptable salt has the same meaning as the term "pharmaceutically acceptable salt" mentioned above, and the solvent is stoichiometric or non-stoichiometric. A solvate of a pharmaceutically acceptable salt includes but is not limited to hydrochloride monohydrate.

The term "pharmaceutical excipient" refers to vehicles and additives used in the manufacturing of pharmaceuticals and the formulation of prescriptions. These include all substances contained in a drug product other than the active ingredients. For details, refer to the Pharmacopoeia of the People's Republic of China (2020 Edition) or the Handbook of Pharmaceutical Excipients (Raymond C Rowe, 2009).

The term "treating" or "treatment" refers to any of the following: (1) alleviating one or more biological manifestations of a disease; (2) interfering with one or more points in the biological cascade that triggers the disease; (3) slowing the progression of one or more biological manifestations of the disease.

The term "preventing" or "prevention" refers to reducing the risk of developing a disease.

The present disclosure also encompasses isotopically labeled compounds of the present disclosure. An "isotopically labeled" or "radiolabeled" compound is a compound of the present disclosure having the following feature: one or more atoms are replaced or substituted by atoms having an atomic mass or mass number different from the atomic mass or mass number typically found in nature (i.e., naturally occurring). Suitable radionuclides that may be incorporated in the compounds of the present disclosure include, but are not limited to, ²H (also written as D for deuterium), ³H (also written as T for tritium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I, and ¹³¹I. The radionuclide incorporated in these radiolabeled compounds depends on the specific application of the radiolabeled compounds. For example, in the context of in vitro receptor labeling and competition assays, compounds labeled with ³H, ¹⁴C, ⁸²Br, ¹²⁵I, ¹³¹I, or ³⁵S are typically the most useful. For radiological imaging applications, ¹¹C, ¹⁸F, ¹²⁵I, ¹²³I, ¹²⁴I, ¹³¹I, ⁷⁵Br, ⁷⁶Br, or ⁷⁷Br are typically the most useful.

It should be understood that a "radiolabeled compound" is a compound bound with at least one radionuclide. In some embodiments, the radionuclide is selected from the group consisting of ³H, ¹⁴C, ¹²⁵I, ³⁵S, and ⁸²Br.

Compounds containing amine functional groups and/or nitrogen-containing heteroaryl groups may form N-oxides. The compounds involved in the present disclosure, which contain amine functional groups and/or nitrogen-containing heteroaryl groups, may also form N-oxides.

When the compound contains several amine functional groups and/or nitrogen-containing heteroaryl groups, one or more nitrogen atoms may be oxidized to N-oxides. Specific examples of N-oxides include N-oxides of tertiary amines or N-oxides of nitrogen atoms in nitrogen-containing heterocycles.

N-Oxides may be prepared by treating the corresponding amine with an oxidizing agent, such as hydrogen peroxide or a peracid (e.g., a peroxycarboxylic acid). For reference, see, for example, *Advanced Organic Chemistry* by Jerry March, 4th Edition, Wiley Interscience. More specifically, the N-oxides can be prepared according to the method of L.W. Deady (Syn. Comm., 1977, 7, 509-514), wherein a nitrogen-containing compound is reacted with meta-chloroperoxybenzoic acid (m-CPBA) in an inert solvent such as dichloromethane.

Without departing from common knowledge in the art, the above preferred conditions can be combined in any manner to obtain various preferred embodiments of the present disclosure.

All reagents and raw materials used in the present disclosure are commercially available.

The positive and progressive effect of the present disclosure lies in that the compounds of the present disclosure possess significant pharmacological properties, acting as positive allosteric modulators of the α2/3-GABA_{A} receptor. The compounds of the present disclosure exhibit excellent affinity and positive modulatory activity for the α2/3-GABA_{A} receptor.

### DETAILED DESCRIPTION OF THE PREFERRED EMBODIMENT

The following examples are provided to further illustrate the present disclosure, but are not intended to limit the scope of the invention as described herein. In the following examples, the experimental methods without specified conditions were conducted according to conventional methods and conditions, or selected according to product instructions.

### Conventional route

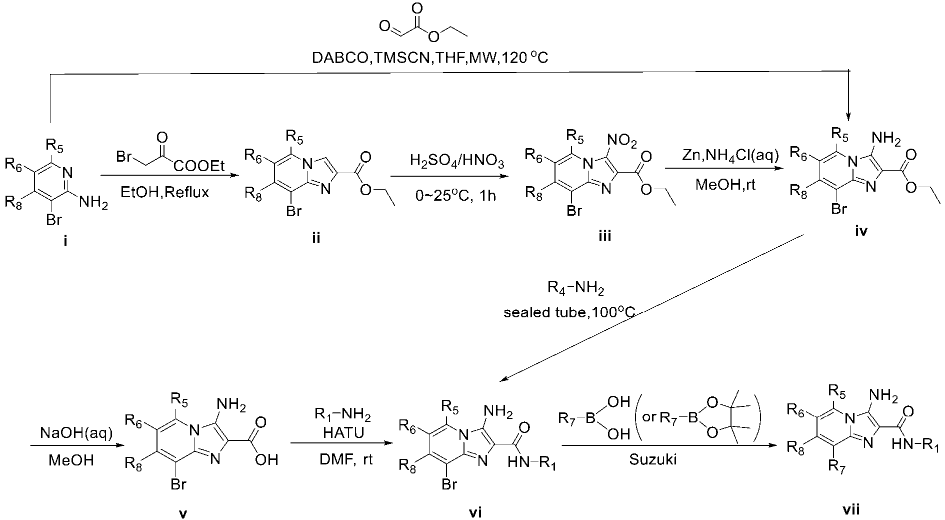

### Synthesis method of intermediate iv: (using ethyl 3-amino-8-bromoimidazo[1,2-a]pyridine-2-carboxylate as an example)

### Route 1:

### Step 1: ethyl 8-bromoimidazo[1,2-a]pyridine-2-carboxylate

3-bromopyridin-2-amine (50 g, 290.6 mmol) and ethyl bromopyruvate (169.2 g, 872 mmol) were sequentially added to anhydrous ethanol (300 mL). The mixture was refluxed for 3 hours. The mixture was concentrated, and a saturated aqueous sodium carbonate solution (300 mL) was added. The mixture was extracted with dichloromethane (300 mL × 3). The organic phase was dried over sodium sulfate and concentrated. The target compound was obtained as a yellow-brown solid (50 g, 64%) by column chromatography (petroleum ether:ethyl acetate = 3:1 to 1:3). LC-MS: m/z [M+H]⁺ = 269.

### Step 2: ethyl 8-bromo-3-nitroimidazo[1,2-a]pyridine-2-carboxylate

Under an ice bath, ethyl 8-bromoimidazo[1,2-a]pyridine-2-carboxylate (50 g, 186.6 mmol) was added portionwise to concentrated sulfuric acid (200 mL) and stirred to dissolve. The fuming nitric acid (20 mL) was added dropwise slowly while maintaining a low temperature. After the dropwise addition was completed, the reaction was allowed to proceed for 2 hours at room temperature. The reaction mixture was poured into ice water, and the pH was slowly adjusted to 6 with sodium hydroxide. The mixture was extracted with dichloromethane (300 mL × 3). The organic phase was dried over sodium sulfate and concentrated to obtain the target product as a yellow solid (51 g, 87%). LC-MS: m/z [M+H]⁺ =314

### Step 3: ethyl 3-amino-8-bromoimidazo[1,2-a]pyridine-2-carboxylate

Ethyl 8-bromo-3-nitroimidazo[1,2-a]pyridine-2-carboxylate (51 g, 163 mmol) was dissolved in methanol (300 mL). Zinc powder (53 g, 815 mmol) and a saturated aqueous solution of ammonium chloride (100 mL) were added sequentially, and the mixture was reacted at 50°C for 1 hour. The mixture was filtered to remove zinc powder, and evaporated to remove most of the methanol. Then, 300 mL of water was added, and the mixture was extracted with ethyl acetate (300 mL × 3). The organic phases were combined, dried over anhydrous sodium sulfate, concentrated, and purified by column chromatography to obtain the target product as a yellow solid (15 g, 32.5%). LC-MS: m/z [M+H]⁺ = 284.

### Route 2:

### Step 1: ethyl 3-amino-8-bromoimidazo[1,2-a]pyridine-2-carboxylate

3-Bromopyridin-2-amine (72 mg, 0.42 mmol) was dispersed in anhydrous tetrahydrofuran (3 mL), followed by sequential addition of ethyl 2-oxoacetate (64 mg, 0.63 mmol) and triethylenediamine (CAS No.: 280-57-9, 47 mg, 0.42 mmol). At -10°C, trimethylsilyl cyanide (CAS No.: 7677-24-9, 50 mg, 1.2 mmol) was added, and the mixture was reacted at 120°C under microwave irradiation for 2 hours. 20 mL of water was added. The mixture was extracted with ethyl acetate (20 mL × 3), concentrated, purified by column chromatography (petroleum ether : ethyl acetate = 2 : 1) to obtain ethyl 3-amino-8-bromoimidazo[1,2-a]pyridine-2-carboxylate as a yellow solid (60 mg, 50%). LC-MS: m/z [M+H]⁺ = 284

### Synthesis method of intermediate vi: (taking 3-amino-8-bromo-N-propylimidazo[1,2-a]pyridine-2-carboxamide as an example)

### Route 1:

### Step 1: 3-amino-8-bromoimidazo[1,2-a]pyridine-2-carboxylic acid

Ethyl 3-amino-8-bromoimidazo[1,2-a]pyridine-2-carboxylate (1.3 g, 4.59 mmol) and an aqueous sodium hydroxide solution (4 N, 5 mL) were sequentially added to MeOH (30 mL), and the mixture was stirred at 25°C for 3 hours. The mixture was neutralized to a pH of 6 with 1 M dilute hydrochloric acid, evaporated to remove methanol, and filtered. The filter cake was dried to obtain the title compound (842 mg, 72%) as a brown powder. LC-MS: m/z [M+H]⁺ = 256

### Step 2: 3-amino-8-bromo-N-propylimidazo[1,2-a]pyridine-2-carboxamide

3-Amino-8-bromoimidazo[1,2-a]pyridine-2-carboxylic acid (842 mg, 3.3 mmol) was dispersed in N,N-dimethylformamide (10 mL). HATU (CAS No.: 148893-10-1, 1.5 g, 3.96 mmol), n-propylamine (383 mg, 6.6 mmol), and triethylamine (660 mg, 6.6 mmol) were sequentially added, and the mixture was stirred for 16 hours. The reaction mixture was poured into water, extracted with dichloromethane (50 mL × 3), then dried and concentrated to obtain the title compound (625 mg, 64%, crude) as a yellow solid. LC-MS: m/z [M+H]⁺ = 297

### Route 2:

### Step 1: 3-amino-8-bromo-N-propylimidazo[1,2-a]pyridine-2-carboxamide

Ethyl 3-amino-8-bromoimidazo[1,2-a]pyridine-2-carboxylate (1 g, 3.53 mmol) was added to n-propylamine (30 mL), and the mixture was stirred in a sealed tube at 100°C for 3 hours. The mixture was cooled, concentrated, and purified by column chromatography (petroleum ether:ethyl acetate = 1:1 to 1:3) to obtain the target compound (627 mg, 60%) as a brown solid. LC-MS: m/z [M+H]⁺ = 297

The starting material substituents for Intermediate IV and Intermediate VI include, but are not limited to, the following cases: R₅=R₆=R₈=H or R₈=F; R₅=H, R₆=H or R₈=Me; R₅=H, R₆=H or R₈=H; R₅=Me, R₆=H or R₈=H; R₅=H, R₆=Me; or R₈=CHF₂, R₅=H, R₆=H, which can be prepared by the above method.

### General synthetic method of compound vii: (taking 3-amino-8-(2-fluoro-6-methoxyphenyl)-N-propylimidazo[1,2-a]pyridine-2-carboxamide as an example)

3-Amino-8-bromo-N-propylimidazo[1,2-a]pyridine-2-carboxamide (100 mg, 0.34 mmol), (2-fluoro-6-methoxyphenyl)boronic acid (115 mg, 0.68 mmol), cesium carbonate (221 mg, 0.68 mmol), and [1,1'-bis(di-tert-butylphosphino)ferrocene]dichloropalladium (CAS No.: 95408-45-0, 26 mg, 0.04 mmol) were sequentially added to a mixed solution of dioxane and water (2 mL/0.4 mL). The mixture was stirred at 100°C under argon atmosphere for 2 hours. 20 mL of water was added. The mixture was extracted with dichloromethane (20 mL × 3). The organic phase was concentrated, and purified by column chromatography (petroleum ether:ethyl acetate = 1:1 to 1:5) to obtain the target product (80 mg, 69%) as a yellow solid.
¹H NMR (400 MHz, DMSO-*d*₆) 8.15 (d, *J* =5.87 Hz, 1H) 7.55 - 7.63 (m, 1H) 7.39 - 7.48 (m, 1H) 6.82 - 7.03 (m, 4H) 6.15 (s, 2H) 3.70 (s, 3H) 3.13-3.17(m, 2H) 1.44-1.48 (m, 2H) 0.81 (t, *J* =7.34 Hz, 3H). LC-MS: m/z [M+1]⁺= 343

### Example 1

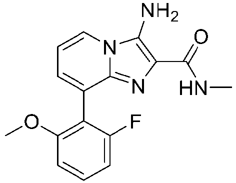

### 3-Amino-8-(2-fluoro-6-methoxyphenyl)-N-methylimidazo[1,2-a]pyridine-2-carboxamide

### Step 1: 3-amino-8-bromo-N-methylimidazo[1,2-a]pyridine-2-carboxamide

Ethyl 3-amino-8-bromoimidazo[1,2-a]pyridine-2-carboxylate (100 mg, 0.35 mmol) was added to a solution of methylamine in ethanol (20%, 3 mL). The tube was sealed and the mixture was stirred at 110°C for 2 hours, concentrated, and purified by column chromatography to obtain a light yellow powder (70 mg, 70%). LC-MS: m/z [M+H]⁺ = 270

**Step 2:** The operation method was the same as that in the synthesis example of compound vii, using 3-amino-8-bromo-N-methylimidazo[1,2-a]pyridine-2-carboxamide (70 mg, 0.26 mmol) and (2-fluoro-6-methoxyphenyl)boronic acid (44 mg, 0.26 mmol) as starting materials to obtain the target product (16 mg, 19%).
¹H NMR (400 MHz, DMSO-*d*₆) 8.15 (dd, *J* = 6.8, 1.2 Hz, 1H), 7.52 (d, *J* = 4.8 Hz, 1H), 7.43 (td, *J* = 8.4, 7.0 Hz, 1H), 6.98 (d, *J* = 8.5 Hz, 1H), 6.91 (ddd, *J* = 16.9, 10.1, 4.0 Hz, 3H), 6.12 (s, 2H), 3.70 (s, 3H), 2.71 (d, *J* = 4.8 Hz, 3H). LC-MS: m/z [M+H]⁺ = 315

### Example 2

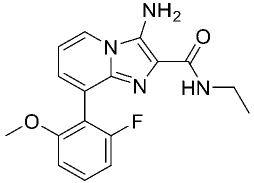

### 3-Amino-8-(2-fluoro-6-methoxyphenyl)-N-ethylimidazo[1,2-a]pyridine-2-carboxamide

### Step 1: 3-amino-8-bromo-N-ethylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and ethylamine hydrochloride (47 mg, 0.59 mmol) as starting materials to obtain the target product (50 mg, 45%) as a yellow-green solid.

**Step 2:** The operation method was the same as that in the synthesis example of compound vii, using 3-amino-8-bromo-N-ethylimidazo[1,2-a]pyridine-2-carboxamide (50 mg, 0.17 mmol) and (2-fluoro-6-methoxyphenyl)boronic acid (30 mg, 0.17 mmol) as starting materials to obtain the target product (25 mg, 44%).
¹H NMR (400 MHz, DMSO-*d*₆) 8.19 (d, *J* = 6.8 Hz, 1H), 8.07 (s, 1H), 7.63 (d, J= 7.0 Hz, 1H), 7.48 (dd, *J* = 15.2, 8.4 Hz, 1H), 7.21 (t, *J* = 6.9 Hz, 1H), 6.88 (dd, *J =* 15.0, 8.6 Hz, 2H), 6.18 (s, 2H), 3.83 (s, 3H), 3.44 - 3.27 (m, 2H), 1.19 (t, *J =* 7.3 Hz, 3H). LC-MS: m/z [M+H]⁺ = 329

### Example 3

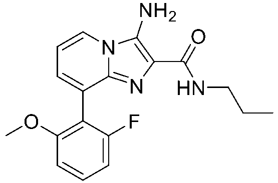

### 3-Amino-8-(2-fluoro-6-methoxyphenyl)-N-propylimidazo[1,2-a]pyridine-2-carboxamide

The operation method is the same as the synthesis example of compound vii.

### Example 4

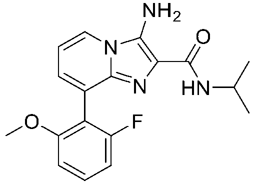

### 3-Amino-8-(2-fluoro-6-methoxyphenyl)-N-isopropylimidazo[1,2-a]pyridine-2-carboxamide

### Step 1: 3-amino-8-bromo-N-isopropylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and isopropylamine (35 mg, 0.59 mmol) as starting materials to obtain the target product (30 mg, 26%) as a yellow-green solid.

**Step 2:** The operation method was the same as that in the synthesis example of compound vii, using 3-amino-8-bromo-N-isopropylimidazo[1,2-a]pyridine-2-carboxamide (30 mg, 0.117 mmol) and (2-fluoro-6-methoxyphenyl)boronic acid (20 mg, 0.117 mmol) as starting materials to obtain the target product (11 mg, 27%).
¹H NMR (400 MHz, DMSO-*d*₆) 8.16 (d, *J* = 6.8 Hz, 1H), 7.44 (dd, *J* = 15.3, 8.3 Hz, 1H), 7.17 (d, *J* = 8.4 Hz, 1H), 7.09 - 6.78 (m, 4H), 6.15 (s, 2H), 4.08 (dd, *J* = 14.8, 6.7 Hz, 1H), 3.71 (s, 3H), 1.12 (dd, *J* = 6.5, 4.3 Hz, 6H). LC-MS: m/z [M+H]⁺ = 343

### Example 5

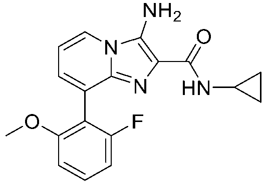

### 3-Amino-8-(2-fluoro-6-methoxyphenyl)-N-cyclopropylimidazo[1,2-a]pyridine-2-carboxamide

### Step 1: 3-amino-8-bromo-N-cyclopropylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and cyclopropylamine (35 mg, 0.59 mmol) as starting materials to obtain the target product (65 mg, 56%) as a yellow-green solid. LC-MS: m/z [M+H]⁺ = 295

**Step 2:** The operation method was the same as that in the synthesis example of compound vii, using 3-amino-8-bromo-N-cyclopropylimidazo[1,2-a]pyridine-2-carboxamide (65 mg, 0.22 mmol) and (2-fluoro-6-methoxyphenyl)boronic acid (37 mg, 0.22 mmol) as starting materials to obtain the target product (30 mg, 40%).
¹H NMR (400 MHz, DMSO-*d*₆) 8.15 (d, *J* = 6.6 Hz, 1H), 7.51 (d, *J* = 4.3 Hz, 1H), 7.42 (dd, *J* =15.3, 8.3 Hz, 1H), 6.97 (d, *J* = 8.4 Hz, 1H), 6.94 - 6.85 (m, 3H), 6.17 (s, 2H), 3.69 (s, 3H), 2.84 - 2.71 (m, 1H), 0.63 - 0.52 (m, 4H). LC-MS: m/z [M+H]⁺ = 341

### Example 6

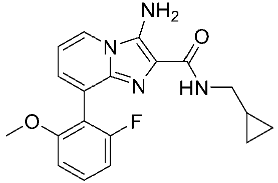

### 3-Amino-N-(cyclopropylmethyl)-8-(2-fluoro-6-methoxyphenyl)imidazo[1,2-a]pyridine-2-carboxamide

### Step 1: 3-amino-8-bromo-N-(cyclopropylmethyl)imidazo[1,2-a]pyridine-2-carboxamide

The operation method was the same as that in the synthesis of intermediate vi (route 1, step 2), using 3-amino-8-bromoimidazol[1,2-a]pyridine-2-carboxylic acid (100 mg, 0.39 mmol) and cyclopropylmethylamine (42 mg, 0.59 mmol) as starting materials to obtain the target product (100 mg, crude) as a yellow-green solid.

**Step 2:** The operation method was the same as that in the synthesis example of compound vii, using 3-amino-8-bromo-N-(cyclopropylmethyl)imidazo[1,2-a]pyridine-2-carboxamide (100 mg, 0.32 mmol) and (2-fluoro-6-methoxyphenyl)boronic acid (81 mg, 0.48 mmol) as starting materials to obtain the target product (34 mg, 30%) as a yellow solid.
¹H NMR (400 MHz, DMSO-*d*₆) 8.16 (d, *J* = 6.85 Hz, 2 H) 7.63 (t, *J* = 5.87 Hz, 2 H) 7.44 (d, *J* = 7.34 Hz, 2 H) 6.84 - 7.05 (m, 6 H) 6.17 (s, 2 H) 3.70 (s,4 H) 3.06 (t, *J* = 6.60 Hz, 3 H) 1.00 (br. s., 1 H) 0.33 - 0.38 (m, 2 H) 0.19 (d, *J* = 4.40 Hz, 2 H). LC-MS: m/z [M+1]⁺= 355

### Example 7

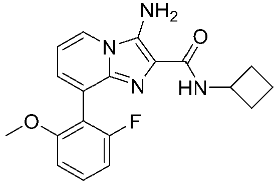

### 3-Amino-8-(2-fluoro-6-methoxyphenyl)-N-cyclobutylimidazo[1,2-a]pyridine-2-carboxamide

### Step 1: 3-amino-8-bromo-N-cyclobutylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and cyclobutylamine (42 mg, 0.59 mmol) as starting materials to obtain the target product (25 mg, 20%) as a yellow-green solid.

**Step 2:** The operation method was the same as that in the synthesis example of compound vii, using 3-amino-8-bromo-N-cyclobutylimidazo[1,2-a]pyridine-2-carboxamide (25 mg, 0.08 mmol) and (2-fluoro-6-methoxyphenyl)boronic acid (14 mg, 0.08 mmol) as starting materials to obtain the target product (6 mg, 21%).
¹H NMR (400 MHz, DMSO-*d*₆) 8.15 (d, *J* = 5.8 Hz, 1H), 7.66 (d, *J* = 8.5 Hz, 1H), 7.44 (dd, *J* = 15.3, 8.3 Hz, 1H), 6.99 (d, *J* = 8.4 Hz, 1H), 6.96 - 6.85 (m, 3H), 6.16 (s, 2H), 4.40 (dd, *J* = 16.7, 8.4 Hz, 1H), 3.71 (s, 3H), 2.15-2.09 (m, 2H), 1.62-1.57 (m, 2H), 1.15 (dd, *J* = 13.5, 4.4 Hz, 2H). LC-MS: m/z [M+H]⁺ = 355

### Example 8

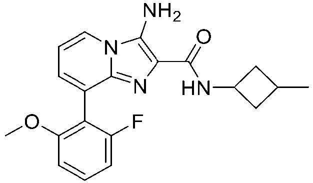

### 3-Amino-8-(2-fluoro-6-methoxyphenyl)-N-(3-methylcyclobutyl)imidazo[1,2-a]pyridine-2-carboxamide

### Step 1: 3-amino-8-bromo-N-(3-methylcyclobutyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and 3-methylcyclobutan-1-amine (50 mg, 0.59 mmol) as starting materials to obtain the target product (100 mg, crude) as a yellow-green solid.

**Step 2:** The operation method was the same as that in the synthesis example of compound vii, using 3-amino-8-bromo-N-(3-methylcyclobutyl)limidazo[1,2-a]pyridine-2-carboxamide (50 mg, 0.14 mmol) and (2-fluoro-6-methoxyphenyl)boronic acid (24 mg, 0.14 mmol) as starting materials to obtain the target product (10 mg, 19%).
¹H NMR (400 MHz, DMSO-*d*₆) 8.15 (d, *J* = 6.0 Hz, 1H), 7.61 (d, *J* = 8.4 Hz, 1H), 7.44 (dd, *J* = 15.4, 8.3 Hz, 1H), 6.99 (d, *J* = 8.4 Hz, 1H), 6.96 - 6.84 (m, 3H), 6.15 (s, 2H), 4.61 - 4.13 (m, 1H), 3.73 (d, *J* = 15.9 Hz, 3H), 2.34 - 2.20 (m, 2H), 1.94 (dd, *J* = 15.1, 8.3 Hz, 1H), 1.86 - 1.65 (m, 2H), 1.06 (d, *J* = 6.5 Hz, 3H). LC-MS: m/z [M+H]⁺ = 369

### Example 9

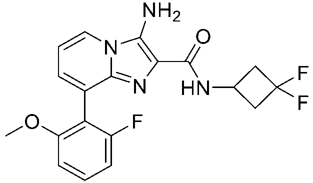

### 3-Amino-N-(3,3-difluorocyclobutyl)-8-(2-fluoro-6-methoxyphenyl)imidazo[1,2-a]pyridine-2-carboxamide

### Step 1: 3-amino-8-bromo-N-(3,3-difluorocyclobutyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and 3,3-difluorocyclobutan-1-amine (84 mg, 0.78 mmol) as starting materials to obtain the target product (100 mg, crude).

**Step 2:** The operation method was the same as that in the synthesis example of compound vii, using 3-amino-8-bromo-N-(3,3-difluorocyclobutyl)imidazo[1,2-a]pyridine-2-carboxamide (93 mg, 0.27 mmol) and (2-fluoro-6-methoxyphenyl)boronic acid (69 mg, 0.41 mmol) as starting materials to obtain the target product (31 mg, 29%) as a yellow solid.
¹H NMR (400 MHz, DMSO-*d*₆) 8.17 (d, *J* = 5.87 Hz, 2H) 7.35 - 7.53 (m, 1H) 6.80 - 7.02 (m, 4H) 6.25 (br. s., 2H) 4.28 (br. s., 1H) 3.70 (s, 3H)2.79 (br. s., 4H). LC-MS: m/z [M+1]⁺= 391

### Example 10

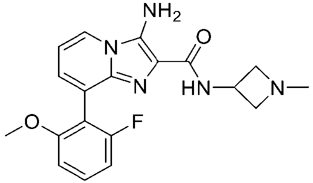

### 3-Amino-8-(2-fluoro-6-methoxyphenyl)-N-(1-methylazetidin-3-yl)imidazo[1,2-a]pyridine-2-carboxamide

### Step 1: tert-butyl 3-(3-amino-8-bromoimidazo[1,2-a]pyridine-2-carboxamido)azetidine-1-carboxylate

The operation method was the same as that in the synthesis of intermediate vi (route 1, step 2), using 3-amino-8-bromoimidazo[1,2-a]pyridine-2-carboxylic acid (100 mg, 0.39 mmol) and tert-butyl 3-aminoazetidine-1-carboxylate (100 mg, 0.58 mmol) as starting materials to obtain the crude product (70 mg, 43%). LC-MS: m/z [M+H]⁺ = 410

### Step 2: 3-amino-N-(azetidin-3-yl)-8-bromoimidazo[1,2-a]pyridine-2-carboxamide

tert-Butyl 3-(3-amino-8-bromoimidazo[1,2-a]pyridine-2-carboxamido)azetidine-1-carboxylate (70 mg, 0.17 mmol) was dissolved in dichloromethane (3 mL), and trifluoroacetic acid (1 mL) was added. The mixture was stirred for 16 hours and then concentrated to obtain the target product as a yellow oily crude product (70 mg, 100%). LC-MS: m/z [M+H]⁺ = 310

### Step 3: 3-amino-8-bromo-N-(1-methylazetidin-3-yl)imidazo[1,2-a]pyridine-2-carboxamide

At 25°C, 3-amino-N-(azetidin-3-yl)-8-bromoimidazo[1,2-a]pyridine-2-carboxamide (40 mg, 0.12 mmol) was dissolved in methanol (10 mL), and an aqueous formaldehyde solution (30%, 20 mg) and NaCNBH₃ (20 mg, 0.3 mmol) were sequentially added. The mixture was stirred at 25°C for 2 hours. The reaction mixture was added dropwise to water (20 mL), extracted with ethyl acetate (30 mL × 3), concentrated, and purified by column chromatography to obtain the target product (35 mg, 55%). LC-MS: m/z [M+H]⁺ = 324

**Step 4:** The operation method was the same as that in the synthesis example of compound vii, using 3-amino-8-bromo-N-(1-methylazetidin-3-yl)imidazo[1,2-a]pyridine-2-carboxamide (35 mg, 0.11 mmol) and (2-fluoro-6-methoxyphenyl)boronic acid (20 mg, 0.12 mmol) as starting materials to obtain the target product (7 mg, 17%).
¹H NMR (400 MHz, DMSO-*d*₆) 8.23 (d, *J* = 6.7 Hz, 1H), 7.95 (d, *J* = 7.7 Hz, 1H), 7.51 (dd, J= 15.4, 8.3 Hz, 1H), 7.06 (d, *J* = 8.5 Hz, 1H), 6.97 (dt, *J* = 13.4, 6.2 Hz,3H), 6.27 (s, 2H), 4.52 (d, *J* = 7.2 Hz, 1H), 3.77 (s, 3H), 3.61 (s, 2H), 3.18 (s, 2H),2.33 (s, 3H). LC-MS: m/z [M+H]⁺ = 370

### Example 11

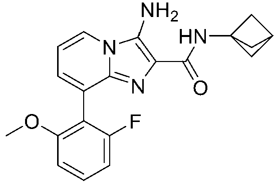

### 3-Amino-N-(bicyclo[1.1.1]pent-1-yl)-8-(2-fluoro-6-methoxyphenyl)imidazo[1,2-a]pyridine-2-carboxamide

**Step 1: 3-amino-N-(bicyclo[1.1.1]pentan-1-yl)-8-bromoimidazo[1,2-a]pyridine-2-carboxamide** The operation method was the same as that in the synthesis of intermediate vi (route 1, step 2), using 3-amino-8-bromoimidazo[1,2-a]pyridine-2-carboxylic acid (150 mg, 0.59 mmol) and 1-aminobicyclo[1.1.1]pentane hydrochloride (100 mg, 1.18 mmol) as starting materials to obtain the target product (200 mg, crude).

**Step 2:** The operation method was the same as that in the synthesis example of compound vii, using 3-amino-N-(bicyclo[1.1.1]pentan-1-yl)-8-bromoimidazo[1,2-a]pyridine-2-carboxamide (200 mg, 0.62 mmol) and (2-fluoro-6-methoxyphenyl)boronic acid (110 mg, 0.62 mmol) as starting materials to obtain the target product (173 mg, 76%) as a yellow solid.
¹H NMR (400 MHz, DMSO-*d*₆) 8.15 (d, *J* = 6.36 Hz, 1H) 7.88 (s, 1H) 7.38 - 7.48 (m, 1H) 6.98 (d, *J* = 8.31 Hz, 1H) 6.82 - 6.95 (m, 3H) 6.21 (s, 2H)3.70 (s, 3H) 2.04 (s, 6H) 1.88 (s, 1H).LC-MS: m/z [M+1]⁺= 367

### Example 12

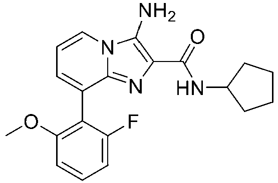

### 3-Amino-N-cyclopentyl-8-(2-fluoro-6-methoxyphenyl)imidazo[1,2-a]pyridine-2-carboxamide

### Step 1: 3-amino-8-bromo-N-cyclopentylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (150 mg, 0.59 mmol) and cyclopentylamine (100 mg, 1.18 mmol) as starting materials to obtain the target product (200 mg, crude).

**Step 2:** The operation method was the same as that in the synthesis example of compound vii, using 3-amino-8-bromo-N-cyclopentylimidazo[1,2-a]pyridine-2-carboxamide (200 mg, 0.62 mmol) and (2-fluoro-6-methoxyphenyl)boronic acid (210 mg, 1.24 mmol) as starting materials to obtain the target product (203 mg, 89%) as a yellow solid.
¹H NMR (400 MHz, DMSO-*d*₆) 8.15 (d, J = 6.85 Hz, 1H) 7.39 - 7.50 (m, 1H) 7.25 - 7.35 (m, 1H) 6.99 (d, *J* =8.31 Hz, 1H) 6.84 - 6.96 (m, 3H) 6.16(s, 2H) 4.08 - 4.26 (m, 1H) 3.70 (s, 3H) 1.74 - 1.90 (m, 2H) 1.62 (br. s., 2H) 1.48 (br. s., 4H). LC-MS: m/z [M+1]⁺= 369

### Example 13

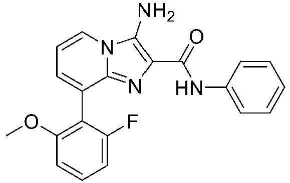

### 3-Amino-8-(2-fluoro-6-methoxyphenyl)-N-phenylimidazo[1,2-a]pyridine-2-carboxamide

### Step 1: 3-amino-8-bromo-N-phenylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid and aniline as starting materials to obtain the target product.

**Step 2:** The operation method was the same as that in the synthesis example of compound vii, using 3-amino-8-bromo-N-propylimidazo[1,2-a]pyridine-2-carboxamide (100 mg, 0.30 mmol) and (2-fluoro-6-methoxyphenyl)boronic acid (115 mg, 0.68 mmol) as starting materials to obtain the target product (92 mg, 81%) as a yellow solid.

¹HNMR (400MHz, DMSO-*d*₆) 9.40 (s, 1H), 8.23 (d, *J* = 6.8 Hz, 1H), 7.76 (d, *J* = 7.8 Hz, 2H), 7.57 - 7.39 (m, 1H), 7.28 (t, *J* = 7.8 Hz, 2H), 7.06 - 6.98 (m, 3H), 6.97 - 6.90 (m, 2H), 6.41 (s, 2H), 3.73 (s, 3H). LC-MS: m/z [M+H]⁺ = 377

### Example 14

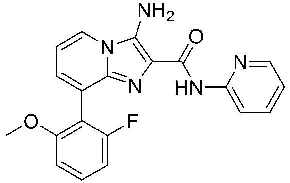

### 3-Amino-8-(2-fluoro-6-methoxyphenyl)-N-(pyridin-2-yl)imidazo[1,2-a]pyridine-2-carboxamide

### Step 1: 3-amino-8-bromo-N-(pyridin-2-yl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid and pyridin-2-amine as starting materials to obtain the target product.

**Step 2:** The operation method was the same as that in the synthesis example of compound vii, using 3-amino-8-bromo-N-(pyridin-2-yl)imidazo[1,2-a]pyridine-2-carboxamide (100 mg, 0.30 mmol) and (2-fluoro-6-methoxyphenyl)boronic acid (115 mg, 0.68 mmol) as starting materials to obtain the target product (50 mg, 44%) as a yellow solid.

¹HNMR (400MHz, DMSO-*d*₆) 9.13 (s, 1H), 8.33 - 8.17 (m, 3H), 7.82 (t, *J* = 7.6 Hz, 1H), 7.49 (q, *J* = 7.8 Hz, 1H), 7.16 - 7.02 (m, 3H), 7.00 - 6.92 (m, 2H), 6.50 (s, 2H), 3.73 (s, 3H). LC-MS: m/z [M+H]⁺ = 378

### Example 15

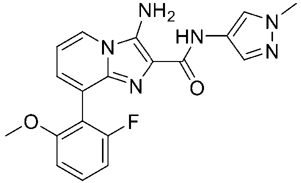

### 3-Amino-8-(2-fluoro-6-methoxyphenyl)-N-(1-methyl-1H-pyrazol-4-yl)imidazo[1,2-a]pyridine-2-carboxamide

### Step 1: 3-amino-8-bromo-N-(1-methyl-1H-pyrazol-4-yl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid and 1-methyl-1H-pyrazol-4-amine as starting materials to obtain the target product.

**Step 2:** The operation method was the same as that in the synthesis example of compound vii, using 3-amino-8-bromo-N-(1-methyl-1H-pyrazol-4-yl)imidazo[1,2-a]pyridine-2-carboxamide (100 mg, 0.30 mmol) and (2-fluoro-6-methoxyphenyl)boronic acid (115 mg, 0.68 mmol) as starting materials to obtain the target product (78 mg, 69%) as a yellow solid.
¹HNMR (400MHz, DMSO-*d*₆) 9.72 (s, 1H), 8.20 (d, *J* = 6.4 Hz, 1H), 7.96 (s, 1H), 7.64 (s, 1H), 7.45 (q, *J=* 7.8 Hz, 1H), 7.04 - 6.83 (m, 4H), 6.31 (br. s., 2H), 3.78 (s, 3H), 3.71 (s, 3H). LC-MS: m/z [M+H]⁺ = 381

### Example 16

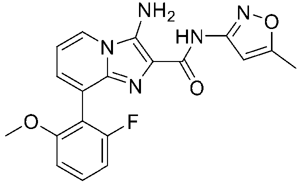

### 3-Amino-8-(2-fluoro-6-methoxyphenyl)-N-(5-methylisoxazol-3-yl)imidazo[1,2-a]pyridine-2-carboxamide

### Step 1: 3-amino-8-bromo-N-(5-methylisoxazol-3-yl)imidazo[1,2-a]pyridine-2-carboxamide

3-Amino-8-bromoimidazo[1,2-a]pyridine-2-carboxylic acid (110 mg, 0.40 mmol) was dissolved in pyridine (2 mL). 5-Methylisoxazol-3-amine (79 mg, 0.80 mmol) and N,N-dimethylpyridin-4-amine (49 mg, 0.40 mmol) were added, and the mixture was stirred for 10 minutes. Thionyl chloride (95 mg, 0.80 mmol) was then added dropwise, and the reaction mixture was stirred at room temperature for 16 hours. The reaction mixture was concentrated, added with water (20 mL), and extracted with ethyl acetate (50 mL × 3). The organic phases were combined, dried over anhydrous sodium sulfate, filtered, concentrated, and purified by column chromatography (dichloromethane:methanol = 10:1) to obtain the product (71 mg, 52.5%). LC-MS: m/z [M+H]⁺ = 336

**Step 2:** The operation method was the same as that in the synthesis example of compound vii, using 3-amino-8-bromo-N-(5-methylisoxazol-3-yl)imidazo[1,2-a]pyridine-2-carboxamide (100 mg, 0.30 mmol) and (2-fluoro-6-methoxyphenyl)boronic acid (115 mg, 0.68 mmol) as starting materials to obtain the target product (8 mg, 7%) as a gray solid.
¹HNMR (400MHz, DMSO-d6) 9.56 (br. s., 1 H), 8.26 (d, J = 6.8 Hz, 1 H), 7.47 (q, J = 7.7 Hz, 1 H), 7.07 - 6.98 (m, 2 H), 6.98 - 6.90 (m, 2 H), 6.70 (s, 1 H), 6.51 (br. s., 2 H), 3.72 (s, 3 H), 2.39 (s, 3 H). LC-MS: m/z [M+H]⁺ = 382

### Example 17

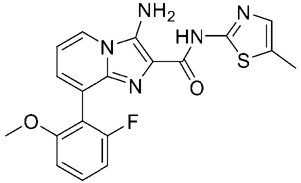

### 3-Amino-8-(2-fluoro-6-methoxyphenyl)-N-(5-methylthiazol-2-yl)imidazo[1,2-a]pyridine-2-carboxamide

### Step 1: 3-amino-8-bromo-N-(5-methylthiazol-2-yl)imidazo[1,2-a]pyridine-2-carboxamide

3-Amino-8-bromoimidazo[1,2-a]pyridine-2-carboxylic acid (150 mg, 0.59 mmol) was dissolved in DMF (1 mL), and 5-methylthiazol-2-amine (135 mg, 1.18 mmol), 1-(3-dimethylaminopropyl)-3-ethylcarbodiimide hydrochloride (CAS No.: 25952-53-8, 170 mg, 0.89 mmol), and N,N-dimethylpyridin-4-amine (144 mg, 1.18 mmol) were sequentially added. The reaction mixture was stirred at 50°C for 6 hours. The reaction mixture was quenched with water (20 mL). The aqueous phase was extracted with ethyl acetate (50 mL × 3). The organic layers were combined, dried over anhydrous sodium sulfate, filtered, concentrated, and purified by column chromatography (petroleum ether:ethyl acetate = 1:1) to obtain the product (91 mg, 44%). LC-MS: m/z [M+H]⁺ = 352

**Step 2:** The operation method was the same as that in the synthesis example of compound vii, using 3-amino-8-bromo-N-(5-methylthiazol-2-yl)imidazo[1,2-a]pyridine-2-carboxamide (100 mg, 0.32 mmol) and (2-fluoro-6-methoxyphenyl)boronic acid (115 mg, 0.68 mmol) as starting materials to obtain the target product (18 mg, 16%) as a yellow solid.
¹HNMR (400MHz, DMSO-d6) 10.35 (br. s., 1 H), 8.27 (d, J = 6.8 Hz, 1 H), 7.47 (d, J = 7.3 Hz, 1 H), 7.11 (s, 1 H), 7.02 (d, J = 8.3 Hz, 2 H), 7.05 (d, J = 6.8 Hz, 2 H), 6.97 - 6.90 (m, 2 H), 6.61 (br. s., 2 H), 3.72 (s, 3 H), 2.35 (s, 3 H). LC-MS: m/z [M+H]⁺ =398

### Example 18

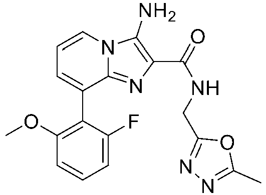

### 3-Amino-8-(2-fluoro-6-methoxyphenyl)-N-((5-methyl-1,3,4-diazol-2-yl)methyl)imidazo[1,2-a]pyridine-2-carboxamide

### Step 1: 3-amino-8-bromo-N-((5-methyl-1,3,4-diazol-2-yl)methyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid and (5-methyl-1,3,4-thiadiazol-2-yl)methanamine as starting materials to obtain the target product.

**Step 2:** The operation method was the same as that in the synthesis example of compound vii, using 3-amino-8-bromo-N-((5-methyl-1,3,4-diazol-2-yl)methyl)imidazo[1,2-a]pyridine-2-carboxamide (100 mg, 0.28 mmol) and (2-fluoro-6-methoxyphenyl)boronic acid (115 mg, 0.68 mmol) as starting materials to obtain the target product (81 mg, 72%) as a yellow solid.
¹H NMR (400MHz, DMSO-*d*₆) 8.30 - 8.09 (m, 2 H), 7.44 (q, J = 7.8 Hz, 1 H), 7.06 - 6.83 (m, 4 H), 6.24 (s, 2 H), 4.56 (d, J = 5.9 Hz, 2 H), 3.71 (s, 3 H), 2.44 (s, 3 H). LC-MS: m/z [M+H]⁺ = 397

### Example 19

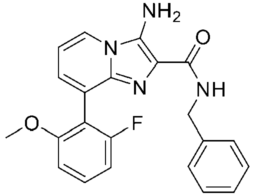

### 3-Amino-N-benzyl-8-(2-fluoro-6-methoxyphenyl)imidazo[1,2-a]pyridine-2-carboxamide

### Step 1: 3-amino-8-bromo-N-benzylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid and benzylamine as starting materials to obtain the target product.

**Step 2:** The operation method was the same as that in the synthesis example of compound vii, using 3-amino-8-bromo-N-benzylimidazo[1,2-a]pyridine-2-carboxamide (100 mg, 0.29 mmol) and (2-fluoro-6-methoxyphenyl)boronic acid (115 mg, 0.68 mmol) as starting materials to obtain the target product (48 mg, 43%) as a yellow solid.
¹HNMR (400MHz, DMSO-*d*₆) 8.23 - 8.07 (m, 2 H), 7.51 - 7.36 (m, 1 H), 7.28 (d, J = 4.4 Hz, 4 H), 7.23 - 7.17 (m, 1 H), 7.02 - 6.86 (m, 4 H), 6.19 (s, 2 H), 4.39 (d, J = 6.4 Hz, 2 H), 3.70 (s, 3 H). LC-MS: m/z [M+H]⁺ = 391

### Example 20

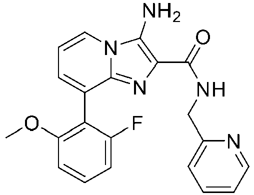

### 3-Amino-8-(2-fluoro-6-methoxyphenyl)-N-(pyridin-2-methyl)imidazo[1,2-a]pyridine-2-carboxamide

### Step 1: 3-amino-8-bromo-N-(pyridin-2-methyl)imidazo[1,2-a]pyridine-2-carboxamide

The operation was performed in the same manner as the synthesis of intermediate vi (Route 1, Step 2), using 3-amino-8-bromoimidazo[1,2-a]pyridine-2-carboxylic acid and pyridine-2-methanamine as starting materials to obtain the target product.

**Step 2:** The operation method was the same as that in the synthesis example of compound vii, using 3-amino-8-bromo-N-(pyridin-2-methyl)imidazo[1,2-a]pyridine-2-carboxamide (100 mg, 0.30 mmol) and (2-fluoro-6-methoxyphenyl)boronic acid (115 mg, 0.68 mmol) as starting materials to obtain the target product (63 mg, 56%) as a yellow solid.
¹HNMR (400MHz, DMSO-*d*₆) 8.47 (br. s., 1 H), 8.18 (d, J = 5.9 Hz, 2 H), 7.75 - 7.69 (m, 1 H), 7.44 (q, J = 7.8 Hz, 1 H), 7.34 - 7.16 (m, 2 H), 7.05 - 6.84 (m, 4 H), 6.19 (br. s., 2 H), 4.51 (d, J = 5.4 Hz, 2 H), 3.72 (s, 3 H). LC-MS: m/z [M+H]⁺ = 392

### Example 21

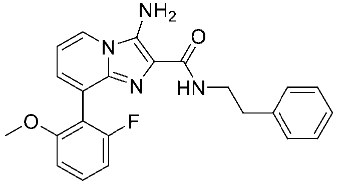

### 3-Amino-8-(2-fluoro-6-methoxyphenyl)-N-phenethylimidazo[1,2-a]pyridine-2-carboxamide

### Step 1: 3-amino-8-bromo-N-phenethylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and phenethylamine (71 mg, 0.59 mmol) as starting materials to obtain the target product (100 mg, crude) as a yellow-green solid.

**Step 2:** The operation method was the same as that in the synthesis example of compound vii, using 3-amino-8-bromo-N-phenethylimidazo[1,2-a]pyridine-2-carboxamide (0.05 g, 0.14 mmol) and (2-fluoro-6-methoxyphenyl)boronic acid (0.029 g, 0.17 mmol) as starting materials to obtain the product (20 mg, 35%) as a yellow solid.
¹H NMR (400MHz, DMSO-d6) 8.17 (d, J = 6.4 Hz, 1 H), 7.65 (s, 1 H), 7.48 - 7.40 (m, 1 H), 7.26 (d, J = 6.8 Hz, 2 H), 7.19 (d, J = 6.8 Hz, 3 H), 7.02 - 6.88 (m, 4 H), 6.18 (s, 2 H), 3.69 (s, 3 H), 3.42 (d, J = 6.8 Hz, 2 H), 2.78 (t, J = 6.8 Hz, 2 H). LC-MS: m/z [M+1]⁺ = 405

### Example 22

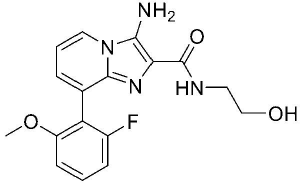

### 3-Amino-8-(2-fluoro-6-methoxyphenyl)-N-(2-hydroxyethyl)imidazo[1,2-a]pyridine-2-carboxamide

### Step 1: 3-amino-8-bromo-N-(2-hydroxyethyl)imidazo[1,2-a]pyridine-2-carboxamide

The operation method was the same as that in the synthesis of intermediate vi (route 2, step 1), using ethyl 3-amino-8-bromoimidazo[1,2-a]pyridine-2-carboxylate (100 mg, 0.35 mmol) and ethanolamine (1.1 g, 17.5 mmol) as starting materials to obtain the target product (100 mg) as a yellow solid.

**Step 2:** The operation method was the same as that in the synthesis example of compound vii, using 3-amino-8-bromo-N-(2-hydroxyethyl)imidazo[1,2-a]pyridine-2-carboxamide (100 mg, 0.33 mmol) and (2-fluoro-6-methoxyphenyl)boronic acid (56 mg, 0.33 mmol) as starting materials to obtain the target product (39 mg, 34%) as a yellow solid.
¹H NMR (400 MHz, DMSO-*d*₆) 8.08 - 8.24 (m, 1 H) 7.38 - 7.52 (m, 2 H) 6.82 - 7.03 (m, 4 H) 6.15 (s, 2 H) 5.75 (s, 1 H) 4.64 - 4.77 (m, 1 H) 3.70 (s,3 H) 3.44 (d, J=5.38 Hz, 2 H) 3.28 (d, J=5.87 Hz, 2 H).LC-MS: m/z [M+1]⁺= 345

### Example 23

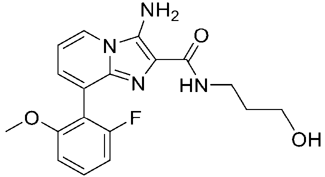

### 3-Amino-8-(2-fluoro-6-methoxyphenyl)-N-(3-hydroxypropyl)imidazo[1,2-a]pyridine-2-carboxamide

### Step 1: 3-amino-8-bromo-N-(3-hydroxypropyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and 3-amino-1-propanol (44 mg, 0.59 mmol) as starting materials to obtain the target product (50 mg, 41%) as a yellow-green solid.

**Step 2:** The operation method was the same as that in the synthesis example of compound vii, using 3-amino-8-bromo-N-(3-hydroxypropyl)imidazo[1,2-a]pyridine-2-carboxamide (50 mg, 0.16 mmol) and (2-fluoro-6-methoxyphenyl)boronic acid (27 mg, 0.16 mmol) as starting materials to obtain the target product (15 mg, 26%).
¹H NMR (400 MHz, DMSO-*d*₆) 8.16 (d, J= 6.0 Hz, 1H), 7.59 (t, *J* = 6.1 Hz, 1H), 7.43 (dd, *J=* 15.3, 8.3 Hz, 1H), 6.99 (d, *J* = 8.4 Hz, 1H), 6.96 - 6.84 (m, 3H), 6.14 (s, 2H), 4.44 (t, *J=* 5.4 Hz, 1H), 3.71 (s, 3H), 3.39 (dd, *J =* 11.9, 6.1 Hz, 2H), 3.27 (dd, *J =* 13.7, 6.8 Hz, 2H), 1.60 (p, *J=* 6.5 Hz, 2H). LC-MS: m/z [M+H]⁺ = 359

### Example 24

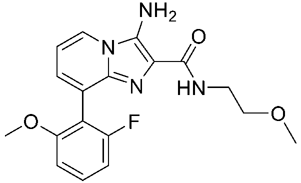

### 3-Amino-8-(2-fluoro-6-methoxyphenyl)-N-(2-methoxyethyl)imidazo[1,2-a]pyridine-2-carboxamide

### Step 1: 3-amino-8-bromo-N-(2-methoxyethyl)imidazo[1,2-a]pyridine-2-carboxamide

The operation method was the same as that in the synthesis of intermediate vi (route 2, step 1), using ethyl 3-amino-8-bromoimidazo[1,2-a]pyridine-2-carboxylate (100 mg, 0.35 mmol) and 2-methoxyethan-1-amine (1.3 g, 17.5 mmol) as starting materials to obtain the target product (100 mg) as a yellow solid.

**Step 2:** The operation method was the same as that in the synthesis example of compound vii, using 3-amino-8-bromo-N-(2-methoxyethyl)imidazo[1,2-a]pyridine-2-carboxamide (100 mg, 0.32 mmol) and (2-fluoro-6-methoxyphenyl)boronic acid (54 mg, 0.32 mmol) as starting materials to obtain the target product (52 mg, 45%) as a yellow solid.
¹H NMR (400 MHz, DMSO-*d*₆) 8.16 (d, J=5.87 Hz, 1 H) 7.37 - 7.52 (m, 2 H) 6.84 - 7.04 (m, 4 H) 6.17 (s, 2 H) 3.70 (s, 3 H) 3.38 (s, 4 H) 3.22 (s, 3H).LC-MS: m/z [M+1]⁺=359

### Example 25

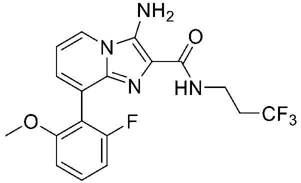

### 3-Amino-8-(2-fluoro-6-methoxyphenyl)-N-(3,3,3-trifluoropropyl)imidazo[1,2-a]pyridine-2-carboxamide

### Step 1: 3-amino-8-bromo-N-(3,3,3-trifluoropropyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and 3,3,3-trifluoropropan-1-amine (132 mg, 1.17 mmol) as starting materials to obtain the target product (50 mg, 36%) as a yellow-green solid. LC-MS: m/z [M+H]⁺ =351

**Step 2:** The operation method was the same as that in the synthesis example of compound vii, using 3-amino-8-bromo-N-(3,3,3-trifluoropropyl)imidazo[1,2-a]pyridine-2-carboxamide (50 mg, 0.14 mmol) and (2-fluoro-6-methoxyphenyl)boronic acid (24 mg, 0.14 mmol) as starting materials to obtain the target product (9 mg, 16%).
¹H NMR (400 MHz, DMSO-*d*₆) 8.17 (d, *J* = 6.0 Hz, 1H), 7.79 (t, *J* = 6.1 Hz, 1H), 7.43 (dd, *J* = 15.4, 8.3 Hz, 1H), 6.99 (d, *J* = 8.4 Hz, 1H), 6.91 (dt, *J* = 13.6, 6.3 Hz, 3H), 6.19 (s, 2H), 3.70 (s, 3H), 3.45 (dd, *J* = 13.5, 5.6 Hz, 2H), 2.49 - 2.41 (m, 2H). LC-MS: m/z [M+H]⁺ = 397

### Example 26

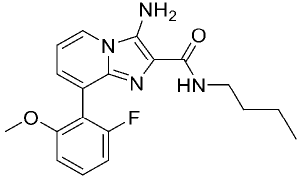

### 3-Amino-8-(2-fluoro-6-methoxyphenyl)-N-butylimidazo[1,2-a]pyridine-2-carboxamide

### Step 1: 3-amino-8-bromo-N-butylimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and butylamine (83 mg, 1.13 mmol) as starting materials to obtain the target product (40 mg, 33%) as a yellow-green solid. LC-MS: m/z [M+H]⁺ = 313

**Step 2:** The operation method was the same as that in the synthesis example of compound vii, using 3-amino-8-bromo-N-(3,3,3-trifluoropropyl)imidazo[1,2-a]pyridine-2-carboxamide (40 mg, 0.128 mmol) and (2-fluoro-6-methoxyphenyl)boronic acid (24 mg, 0.14 mmol) as starting materials to obtain the target product (21 mg, 46%).
¹H NMR (400 MHz, DMSO-*d*₆) 8.15 (d, *J* = 6.9 Hz, 1H), 7.54 (t, *J* = 5.9 Hz, 1H), 7.43 (dd, *J=* 15.5, 8.1 Hz, 1H), 6.99 (d, *J* = 8.4 Hz, 1H), 6.96 - 6.84 (m, 3H), 6.13 (s, 2H), 3.70 (s, 3H), 3.20 (dd, *J=* 13.6, 6.8 Hz, 2H), 1.43 (dd, *J=* 14.6, 7.4 Hz, 2H), 1.25 (dd, *J* = 14.7, 7.3 Hz, 2H), 0.86 (t, *J* = 7.3 Hz, 3H). LC-MS: m/z [M+H]⁺ = 357

### Example 27

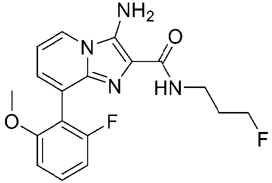

### 3-Amino-8-(2-fluoro-6-methoxyphenyl)-N-(3-fluoropropyl)imidazo[1,2-a]pyridine-2-carboxamide

### Step 1: 3-fluoro-N-(4-methoxybenzyl)propan-1-amine

(4-Methoxyphenyl)methanamine (150 mg, 1.1 mmol), potassium carbonate (303 mg, 2.2 mmol), and 1-fluoro-3-iodopropane (247 mg, 1.3 mmol) were sequentially added to N,N-dimethylformamide (5 mL). The mixture was stirred at 100°C for 16 hours. The mixture was filtered, concentrated, and purified by column chromatography (100 mg, 46%) to obtain the target product. LC-MS: m/z [M+H]⁺ = 198

### Step 2: 3-amino-8-bromo-N-(3-fluoropropyl)-N-(4-methoxybenzyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (130 mg, 0.5 mmol) and 3-fluoro-N-(4-methoxybenzyl)propan-1-amine (100 mg, 0.5 mmol) as starting materials to obtain the target product (70 mg, 32%) as a pale green solid. LC-MS: m/z [M+H]⁺ = 437

### Step 3: 3-Amino-8-(2-fluoro-6-methoxyphenyl)-N-(3-fluoropropyl)-N-(4-methoxybenzyl)imidazo[1,2-a]pyridine-2-carboxamide

The operation method was the same as that in the synthesis example of compound vii, using 3-amino-8-bromo-N-(3-fluoropropyl)-N-(4-methoxybenzyl)imidazo[1,2-a]pyridine-2-carboxamide (70 mg, 0.16 mmol) and (2-fluoro-6-methoxyphenyl)boronic acid (30 mg, 0.17 mmol) as starting materials to obtain the target product (60 mg, 78%). LC-MS: m/z [M+H]⁺ =481

**Step 4:** At 25°C, 3-amino-8-(2-fluoro-6-methoxyphenyl)-N-(3-fluoropropyl)-N-(4-methoxybenzyl)imidazo[1,2-a]pyridine-2-carboxamide (30 mg, 0.06 mmol) was dissolved in dichloromethane (3 mL), followed by dropwise addition of trifluoroacetic acid (1 mL). The mixture was concentrated and purified by column chromatography to obtain the target compound (5 mg, 23%).
¹H NMR (400 MHz, DMSO-*d*₆) 8.16 (d, *J* = 6.6 Hz, 1H), 7.70 (t, *J* = 6.1 Hz, 1H), 7.43 (dd, *J* = 15.3, 8.3 Hz, 1H), 6.99 (d, *J* = 8.5 Hz, 1H), 6.91 (dt, *J* = 13.6, 6.4 Hz, 3H), 6.16 (s, 2H), 4.50 (t, *J* = 5.9 Hz, 1H), 4.38 (t, *J* = 5.9 Hz, 1H), 3.70 (s, 3H), 3.29 (d, *J* = 6.7 Hz, 2H), 1.93 - 1.72 (m, 2H). LC-MS: m/z [M+H]⁺ = 361

### Example 28

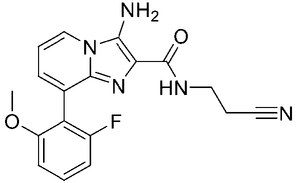

### 3-Amino-N-(2-cyanoethyl)-8-(2-fluoro-6-methoxyphenyl)imidazo[1,2-a]pyridine-2-carboxamide

### Step 1: 3-amino-8-bromo-N-(2-cyanoethyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (100 mg, 0.39 mmol) and 3-aminopropanenitrile (33 mg, 0.47 mmol) as starting materials to obtain the title product as a yellow oil (118 mg, yield 90%). LC-MS: m/z [M+H]⁺ = 308.

**Step 2:** The operation method was the same as that in the synthesis example of compound vii, using 3-amino-8-bromo-N-(2-cyanoethyl)imidazo[1,2-a]pyridine-2-carboxamide (118 mg, 0.38 mmol) and 2-fluoro-6-methoxyphenylboronic acid (73 mg, 0.43 mmol) as starting materials to obtain the title product as a pale yellow solid (32 mg, yield 24%).
¹HNMR (400 MHz, DMSO-d6) 8.18 (d, J = 6.7 Hz, 1H), 7.90 (t, J = 5.9 Hz, 1H), 7.43 (dd, J *=* 15.5, 8.1 Hz, 1H), 6.99 (d, J = 8.4 Hz, 1H), 6.97 - 6.86 (m, 3H), 6.21 (s, 2H), 3.71 (s, 3H), 3.45 (dd, J = 12.6, 6.2 Hz, 2H), 2.72 (t, J = 6.5 Hz, 2H). LC-MS: m/z [M+H]⁺ =354

### Example 29

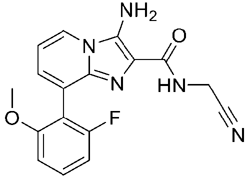

### 3-Amino-N-(cyanomethyl)-8-(2-fluoro-6-methoxyphenyl)imidazo[1,2-a]pyridine-2-carboxamide

### Step 1: 3-Amino-8-bromo-N-(cyanomethyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (200 mg, 0.78 mmol) and 2-aminoacetonitrile (87.8 mg, 1.56 mmol)as starting materials to obtain the target product (110 mg, 48%). LC-MS: m/z [M+1] =294

**Step 2:** The operation method was the same as that in the synthesis example of compound vii, using 3-amino-8-bromo-N-(cyanomethyl)imidazo[1,2-a]pyridine-2-carboxamide (100 mg, 0.34 mmol) and (2-fluoro-6-methoxyphenyl)boronic acid (116 mg, 0.68 mmol) as starting materials to obtain the target product (25 mg, 21.6%).
¹H NMR (400 MHz, DMSO-*d*₆) 9.15 (t, J = 6.0 Hz, 1H), 8.43 (d, J = 7.3 Hz, 1H), 7.83- 7.70 (m, 2H), 6.97 (d, J = 30.2 Hz, 2H), 4.68 (s, 2H), 3.70 (s, 3H), 3.44 (s, 3H), 3.32 (d, J = 6.8 Hz, 2H), 1.70 - 1.54 (m, 2H), 1.00 - 0.86 (m, 3H). LC-MS: m/z [M+1] = 340

### Example 30

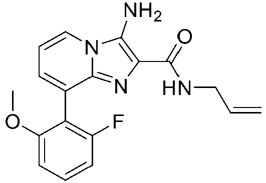

### N-Allyl-3-amino-8-(2-fluoro-6-methoxyphenyl)imidazo[1,2-a]pyridine-2-carboxamide

### Step 1: N-allyl-3-amino-8-bromoimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (300 mg, 1.17 mmol) and prop-2-en-1-amine (134 mg, 2.34 mmol) as starting materials to obtain the target product (164 mg, 47%) as a yellow solid. LCMS: m/z [M+H]⁺=295.

**Step 2:** The operation method was the same as that in the synthesis example of compound vii, using N-allyl-3-amino-8-bromoimidazo[1,2-a]pyridine-2-carboxamide (160 mg, 0.54 mmol) and (2-fluoro-6-methoxyphenyl)boronic acid (184 mg, 1.08 mmol) as starting materials to obtain the target product (16 mg, yield 8.7%) as a yellow solid.

¹H NMR (400MHz, CD₃OD) δ 8.07 (d, J = 6.8 Hz, 1 H), 7.48 - 7.41 (m, 1 H), 7.04 (d, J = 6.8 Hz, 1 H), 6.98 - 6.91 (m, 2 H), 6.85 (t, J = 8.6 Hz, 1 H), 5.89 (dt, J = 5.4, 11.0 Hz, 1 H), 5.21 (d, J = 17.1 Hz, 1 H), 5.10 (d, J = 10.3 Hz, 1 H), 3.94 (d, J = 4.9 Hz, 2 H), 3.74 (s, 3 H). LCMS: m/z [M+H]⁺= 341.

### Example 31

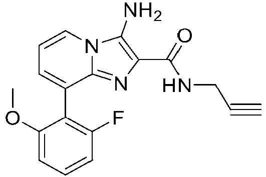

### N-Propargyl-3-amino-8-(2-fluoro-6-methoxyphenyl)imidazo[1,2-a]pyridine-2-carboxamide

### Step 1: N-propargyl-3-amino-8-bromoimidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (300 mg, 1.17 mmol) and prop-2-yn-1-amine (134 mg, 2.34 mmol) as starting materials to obtain the target product (129 mg, 38%) as a yellow solid. LCMS: m/z [M+H]⁺= 293.

**Step 2:** The operation method was the same as that in the synthesis example of compound vii, using N-propargyl-3-amino-8-bromoimidazo[1,2-a]pyridine-2-carboxamide (129 mg, 0.44 mmol) and (2-fluoro-6-methoxyphenyl)boronic acid (184 mg, 1.08 mmol) as starting materials to obtain the target product (36 mg, yield 24%).

¹H NMR (400 MHz, CD₃OD) δ 8.07 (d, J = 6.8 Hz, 1 H), 7.45 (q, J = 7.8 Hz, 1 H), 7.04 (d, J = 6.8 Hz, 1 H), 6.98 - 6.90 (m, 2 H), 6.86 (t, J = 8.6 Hz, 1 H), 4.10 (s, 2 H), 3.75 (s, 3 H), 2.57 (br. s, 1 H). LCMS: m/z [M+H]⁺= 339.

### Example 32

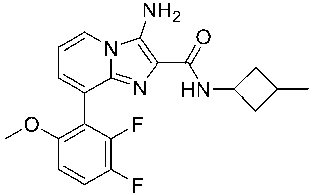

### 3-Amino-8-(2,3-difluoro-6-methoxyphenyl)-N-(3-methylcyclobutyl)imidazo[1,2-a]pyridine-2-carboxamide

The operation method was the same as that in the synthesis example of compound vii, using 3-amino-8-bromo-N-(3-methylcyclobutyl)imidazo[1,2-a]pyridine-2-carboxamide (100 mg, 0.30 mmol) and (2,3-difluoro-6-methoxyphenyl)boronic acid (85 mg, 0.45 mmol) as starting materials to obtain the target product (16 mg, 14%) as a yellow solid.
¹H NMR (400 MHz, DMSO-*d*₆) 8.18 (d, J=6.85 Hz, 1 H) 7.68 (d, J=8.80 Hz, 1 H) 7.49 (d, J=9.78 Hz, 1 H) 6.87 - 7.03 (m, 1 H) 6.20 (s, 2 H) 4.12 -4.30 (m, 1 H) 3.69 (s, 3 H) 2.27 (d, J=6.36 Hz, 2 H) 1.73 (d, J=9.29 Hz, 3 H) 1.01 (d, J=6.36 Hz, 3 H). LC-MS: m/z [M+1]⁺=387

### Example 33

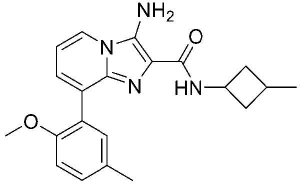

### 3-Amino-8-(2-methoxy-5-methylphenyl)-N-(3-methylcyclobutyl)imidazo[1,2-a]pyridine-2-carboxamide

### Step 1: 3-amino-8-bromo-N-(3-methylcyclobutyl)imidazo[1,2-a]pyridine-2-carboxamide

The operation method was the same as that in the synthesis of intermediate vi (route 1, step 2), using 3-amino-8-bromoimidazo[1,2-a]pyridine-2-carboxylic acid (400 mg, 1.56 mmol) and 3-methylcyclobutan-1-amine (200 mg, 2.36 mmol) as starting materials to obtain the target product (400 mg, crude) as a yellow-green solid.

Step 2: The operation method was the same as that in the synthesis example of compound vii, using 3-amino-8-bromo-N-(3-methylcyclobutyl)imidazo[1,2-a]pyridine-2-carboxamide (100 mg, 0.30 mmol) and (2-methoxy-5-methylphenyl)boronic acid (75 mg, 0.45 mmol) as starting materials to obtain the target product (11 mg, 10%) as a yellow solid.
¹H NMR (400 MHz, DMSO-*d*₆) 8.09 (d, J=6.85 Hz, 1 H) 7.47 - 7.61 (m, 1 H) 7.23 (br. s., 3 H) 7.03 (d, J=7.83 Hz, 1 H) 6.93 (br. s., 2 H) 6.85 (s, 2H) 6.13 (s, 2 H) 4.16 - 4.30 (m, 1 H) 3.67 (s, 4 H) 2.29 (s, 5 H) 1.62 - 2.01 (m, 3 H) 1.01 (d, J=6.36 Hz, 3 H).LC-MS: m/z [M+1]⁺= 365

### Example 34

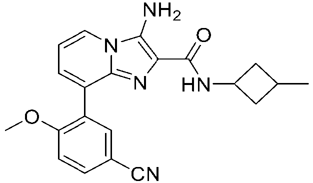

### 3-Amino-8-(5-cyano-2-methoxyphenyl)-N-(3-methylcyclobutyl)imidazo[1,2-a]pyridine-2-carboxamide

The operation method was the same as that in the synthesis example of compound vii, using 3-amino-8-bromo-N-(3-methylcyclobutyl)imidazo[1,2-a]pyridine-2-carboxamide (100 mg, 0.30 mmol) and (5-cyano-2-methoxyphenyl)boronic acid (80 mg, 0.45 mmol) as starting materials to obtain the target product (28 mg, 25%) as a yellow solid.
¹H NMR (400 MHz, DMSO-*d*₆) 8.15 (d, J=6.85 Hz, 2 H) 7.85 - 7.93 (m, 3 H) 7.70 (d, J=8.31 Hz, 1 H) 7.32 (d, J=9.29 Hz, 2 H) 7.04 (d, J=6.85 Hz, 2H)6.89 (t, J=6.85 Hz, 2 H) 6.18 (s, 2 H) 4.12 - 4.29 (m, 1 H) 3.81 (s, 3 H) 2.15 - 2.33 (m, 2 H) 1.66 (d, J=10.27 Hz, 3 H) 1.00 (d, J=6.36 Hz, 2 H).LC-MS: m/z [M+1]⁺= 376

### Example 35 (region B)

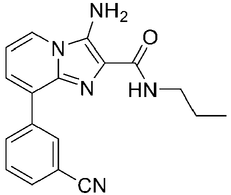

### 3-Amino-8-(3-cyanophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide(100 mg, 0.34 mmol) and 3-cyanophenylboronic acid (50 mg, 0.34 mmol) as starting materials to obtain the target product (105 mg, 97%) as a yellow solid.
¹H NMR (400 MHz, CHLOROFORM) 8.40 (s, 1 H) 8.21 (d, J=7.83 Hz, 1 H) 7.79 (d, J=6.85 Hz, 1 H) 7.72 (d, J=7.83 Hz, 1 H) 7.58 - 7.65 (m, 1 H) 7.25 (d, J=7.34 Hz, 1 H) 6.93 (t, J=7.09 Hz, 1 H) 4.99 (br. s., 2 H) 3.39 - 3.48 (m, 2 H) 1.64 - 1.72 (m, 2 H) 1.00 (t, J=7.34 Hz, 3 H).LC-MS: m/z [M+1]⁺= 320

### Example 36

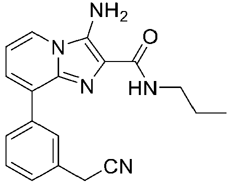

### 3-amino-8-(3-(cyanomethyl)phenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (50 mg, 0.17 mmol) and (3-(cyanomethyl)phenyl)boronic acid (41 mg, 0.26 mmol) as starting materials to obtain the target product (30 mg, 53%) as a yellow solid.
¹H NMR (400 MHz, DMSO-*d*₆) 8.40 (s, 1 H) 8.16 (d, J=6.85 Hz, 1 H) 8.04 (d, J=7.83 Hz, 1 H) 7.70 (s, 1 H) 7.52 (t, J=7.83 Hz, 1 H) 7.39 (d, J=6.85Hz, 2 H) 6.96 (t, J=6.85 Hz, 1 H) 6.17 (s, 2 H) 4.16 (s, 2 H) 3.17 - 3.28 (m, 2 H) 1.48 - 1.58 (m, 2 H) 0.88 (t, J=7.34 Hz, 3 H). LC-MS: m/z [M+1]⁺= 334

### Example 37

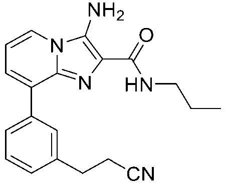

### 3-Amino-8-(3-(2-cyanoethyl)phenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide(200 mg, 0.67 mmol) and 3-(3-(4,4,5,5-tetramethyl-1,3,2-dioxaborolan-2-yl)phenyl)propanenitrile (181 mg, 0.68 mmol) as starting materials to obtain the target product (4 mg, 3%) as a yellow solid.
¹H NMR (400 MHz, DMSO-*d₆*)8.10 - 8.16 (m, 1 H) 8.01 - 8.07 (m, 1 H) 7.90 - 7.97 (m, 1 H) 7.75 - 7.83 (m, 1 H) 7.42 - 7.49 (m, 1 H) 7.25 - 7.37(m, 2 H) 6.90 - 6.98 (m, 1 H) 6.06 - 6.21 (m, 2 H) 3.16 - 3.25 (m, 2 H) 2.94 - 3.03 (m, 2 H) 2.83 - 2.91 (m, 2 H) 1.46 - 1.57 (m, 2 H) 0.78 - 0.92 (m, 3 H).LC-MS: m/z [M+1]⁺= 348

### Example 38

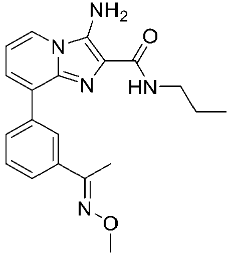

### (E)-3-Amino-8-(3-(1-(methoxyimino)ethyl)phenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (3-acetylphenyl)boronic acid (84 mg, 0.51 mmol) as starting materials to obtain 8-(3-acetylphenyl)-3-amino-N-propylimidazo[1,2-a]pyridine-2-carboxamide (100 mg, 88%). To methanol (2 mL) were sequentially added the intermediate, methoxyamine hydrochloride (50 mg, 0.6 mmol), and an aqueous sodium hydroxide solution (2N, 0.5 mL). The mixture was stirred at room temperature for 2 hours and added with water (20 mL), and extracted with dichloromethane (30 mL × 3). The organic phases were combined, dried over anhydrous sodium sulfate, concentrated, and purified by preparative TLC (petroleum ether:ethyl acetate = 1:1) to obtain the target product (79 mg, 73%) as a white solid.
¹H NMR (400 MHz, DMSO-*d*₆) 8.40 (s, 1 H) 8.16 (d, J=6.85 Hz, 1 H) 8.04 (d, J=7.83 Hz, 1 H) 7.70 (s, 1 H) 7.52 (t, J=7.83 Hz, 1 H) 7.39 (d, J=6.85Hz, 2 H) 6.96 (t, J=6.85 Hz, 1 H) 6.17 (s, 2 H) 4.16 (s, 2 H) 3.17 - 3.28 (m, 2 H) 1.48 - 1.58 (m, 2 H) 0.88 (t, J=7.34 Hz, 3 H). LC-MS: m/z [M+1]⁺= 366

### Example 39

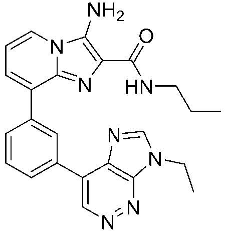

### 3-Amino-8-(3-(7-ethyl-7H-imidazo[4,5-c]pyridazin-4-yl)phenyl)-N-propylimidazo[1,2-a]pyridine-2-carboxamide

### Step 1: 4-(3-chlorophenyl)-7-ethyl-7H-imidazo[4,5-c]pyridazine

4-Chloro-7-ethyl-7-imidazopyridazine (100 mg, 0.55 mmol), 3-chlorophenylboronic acid (94 mg, 0.6 mmol), cesium carbonate (536 g, 1.65 mmol), and [1,1'-bis(di-tert-butylphosphino)ferrocene]dichloropalladium (CAS No.: 95408-45-0, 35 mg, 0.055 mmol) were sequentially added to a mixed solution of dioxane and water (5 mL/0.5 mL). The mixture was stirred at 90 °C under nitrogen atmosphere for 1 hour, and purified by column chromatography (petroleum ether:ethyl acetate = 2:1) to obtain the title product (120 mg, brown solid) in a yield of 85%. LC-MS: m/z [M+H]⁺ =259.

### Step 2: 7-Ethyl-4-(3-(4,4,5,5-tetramethyl-1,3,2-dioxaborolan-2-yl)phenyl)-7H-imidazo[4,5-c]pyridazine

Under nitrogen atmosphere, 4-(3-chlorophenyl)-7-ethyl-7H-imidazo[4,5-c]pyridazine (120 mg, 0.47 mmol), bis(pinacolato)diboron(143 mg, 0.56 mmol), potassium acetate (137 mg, 1.4 mmol), X-Phos (41 mg, 0.2 mmol), and Pd₂(dba)₃ (21 mg, 0.05 mmol) were sequentially added to dioxane (5 mL). The mixture was stirred at 100°C for 16 hours. The title product (120 mg, brown solid) was obtained in a yield of 72% by column chromatography (petroleum ether:ethyl acetate = 1:1). LC-MS: m/z [M+H]⁺ =351.

**Step 3:** The operation method was the same as that in the synthesis example of compound vii, using 7-ethyl-4-(3-(4,4,5,5-tetramethyl-1,3,2-dioxaborolan-2-yl)phenyl)-7H-imidazo[4,5-c]pyridazine (63 mg, 0.18 mmol) and 3-amino-8-bromo-N-propylimidazo[1,2-a]pyridine-2-carboxamide (50 mg, 0.17 mmol) as starting materials to obtain the title product as a yellow solid (30 mg, yield 40%).
¹HNMR(400 MHz, DMSO-d6) 9.59 (s, 1H), 9.05 (s, 1H), 8.87 (s, 1H), 8.40 (d, J = 7.9 Hz, 1H), 8.33 (d, J = 7.9 Hz, 1H), 8.20 (d, J = 6.8 Hz, 1H), 7.79 - 7.67 (m, 2H), 7.45 (d, J = 6.8 Hz, 1H), 7.00 (t, J = 6.9 Hz, 1H), 6.18 (s, 2H), 4.53 (q, J = 7.3 Hz, 2H), 3.25 (dd, J = 13.8, 6.5 Hz, 2H), 1.57 (t, J = 7.3 Hz, 3H), 1.51 (dd, J = 14.5, 7.3 Hz, 2H), 0.85 (dd, J = 9.5, 5.3 Hz, 3H). LC-MS: m/z [M+H]⁺ = 441

### Example 40

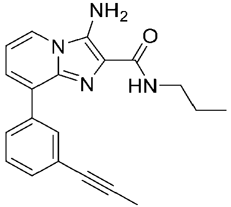

### 3-Amino-8-(3-(prop-1-yn-1-yl)phenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (3-(prop-1-yn-1-yl)phenyl)boronic acid (109 mg, 0.68 mmol) as starting materials to obtain the target product (75 mg, 67%) as a yellow solid.
¹HNMR (400MHz, DMSO-*d*₆) 8.15-8.11 (m, 2 H), 7.94 (s, 1 H), 7.72 (br. s., 1 H), 7.49 - 7.38 (m, 2 H), 7.30 (d, J = 6.8 Hz, 1 H), 6.92 (t, J = 6.8 Hz, 1 H), 6.15 (s, 2 H), 3.22 (d, J = 6.4 Hz, 2 H), 2.05 (s, 3 H), 1.57 - 1.46 (m, 2 H), 0.86 (t, J = 7.3 Hz, 3 H). LC-MS: m/z [M+H]⁺ =333

### Example 41

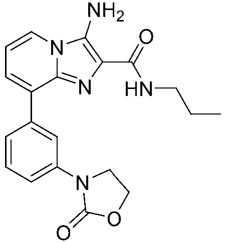

### 3-Amino-8-(3-(2-oxooxazolidin-3-yl)phenyl)-N-propylimidazo[1,2-a]pyridine-2-carboxamide

### Step 1: 3-(3-bromophenyl)oxazolidin-2-one

1- Bromo-3-iodobenzene (500 mg, 1.77 mmol), oxazolidin-2-one (427 mg, 5.32 mmol), cyclohexanediamine (16 mg, 0.14 mmol), and potassium carbonate (734 mg, 5.32 mmol) were sequentially added to dioxane (10 mL). The mixture was stirred at 110°C for 5 hours. The reaction mixture was poured into water (50 mL), extracted with ethyl acetate (50 mL × 3), concentrated, and purified by column chromatography to obtain the target product (320 mg, 68%). LC-MS: m/z [M+H]⁺ =242

### Step 2: 3-(3-(4,4,5,5-tetramethyl-1,3,2-dioxaborolan-2-yl)phenyl)oxazolidin-2-one

3-(3-Bromophenyl)oxazolidin-2-one (200 mg, 0.83 mmol), bis(pinacolato)diboron (420 mg, 1.65 mmol), potassium acetate (243 mg, 2.48 mmol), and [1,1'-bis(di-tert-butylphosphino)ferrocene]dichloropalladium (30 mg, 0.04 mmol) were sequentially added to dioxane (5 ml). Under argon atmosphere, the mixture was stirred at 100°C for 2 hours. It was concentrated and purified by column chromatography to obtain a pale yellow powder (80 mg, 33.5%). LC-MS: m/z [M+1] =290

**Step 3:** The operation method was the same as that in the synthesis example of compound vii, using 3-(3-(4,4,5,5-tetramethyl-1,3,2-dioxaborolan-2-yl)phenyl)oxazolidin-2-one (80 mg, 0.27 mmol) and 3-amino-8-bromo-N-propylimidazo[1,2-a]pyridine-2-carboxamide (82 mg, 0.27 mmol) as starting materials to obtain the target product as a yellow powder (20 mg, 19.5%).
¹H NMR (400 MHz, DMSO-*d*₆) 8.58 (s, 1H), 8.20 (d, J = 6.9 Hz, 1H), 7.87 - 7.76 (m, 2H), 7.63 (dd, J = 8.1, 1.7 Hz, 1H), 7.52 (t, J = 8.0 Hz, 1H), 7.43 (d, J = 6.9 Hz, 1H), 7.00 (s, 1H), 4.47 (dd, J = 8.9, 7.0 Hz, 2H), 4.24 - 4.10 (m, 2H), 3.25 (dd, J = 13.7, 6.5 Hz, 2H), 1.55 (dd ,J = 14.4, 7.3 Hz, 2H), 0.89 (t, J = 7.4 Hz, 3H). LC-MS: m/z [M+1] = 380

### Example 42

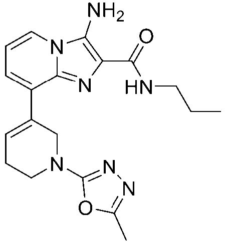

### 3-Amino-8-(1-(5-methyl-1,3,4-oxadiazol-2-yl)-1,2,5,6-tetrahydropyridin-3-yl)-N-propylimidazo[1,2-a]pyridine-2-carboxamide

### Step 1: tert-butyl 5-(3-amino-2-(propylcarbamoyl)imidazo[1,2-a]pyridin-8-yl)-3,6-dihydropyridine-1(2H)-carboxylate

The operation method was the same as that in the synthesis example of compound vii, using tert-butyl 5-(4,4,5,5-tetramethyl-1,3,2-dioxaborolan-2-yl)-3,6-dihydropyridine-1(2H)-carboxylate (300 mg, 0.97 mmol) and 3-amino-8-bromo-N-propylimidazo[1,2-a]pyridine-2-carboxamide (288 mg, 0.97 mmol) as starting materials to obtain the target product (200 mg, 52%). LC-MS: m/z [M+H]⁺ =400

### Step 2: 3-amino-N-propyl-8-(1,2,5,6-tetrahydropyridin-3-yl)imidazo[1,2-a]pyridine-2-carboxamide

At 25°C, 5-(3-amino-2-(propylcarbamoyl)imidazo[1,2-a]pyridin-8-yl)-3,6-dihydropyridine-1(2H)-carboxylic acid tert-butyl ester (200 mg, 0.5 mmol) was dissolved in dichloromethane (3 mL), then trifluoroacetic acid (1 mL) was added dropwise. The mixture was stirred for 16 hours. It was concentrated to obtain the target product as a brown oil (120 mg, crude). LC-MS: m/z [M+H]⁺ =300

**Step 3:** 3-amino-N-propyl-8-(1,2,5,6-tetrahydropyridin-3-yl)imidazo[1,2-a]pyridine-2-carboxamide (50 mg, 0.16 mmol), 2-bromo-5-methyl-1,3,4-oxadiazole (40 mg, 0.25 mmol), and triethylamine (50 mg, 0.48 mmol) were sequentially added to anhydrous ethanol (1.5 ml). The mixture was reacted at 130°C under microwave irradiation for 2 hours. The mixture was purified by column chromatography to obtain the title compound (25 mg, 41%).
¹H NMR (400 MHz, DMSO-*d*₆) 8.11 (d, J= 6.9 Hz, 1H), 7.90 *(t, J=* 5.8 Hz, 1H), 7.27 (s, 1H), 7.09 (d, *J= 7.0* Hz, 1H), 6.89 (t, *J=* 7.0 Hz, 1H), 5.56 (s, 2H), 4.49 (d, *J=* 1.7 Hz, 2H), 3.63 (t, *J* = 5.8 Hz, 2H), 3.24 (dd, *J=* 13.9, 6.5 Hz, 2H), 2.49 - 2.45 (m, 2H), 2.35 (s, 3H), 1.54 (dt, *J* = 14.5, 7.3 Hz, 2H), 0.89 (t, *J* = 7.4 Hz, 3H). LC-MS: m/z [M+H]⁺ =382

### Example 43

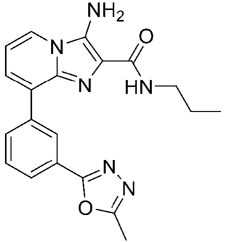

### 3-Amino-8-(3-(5-methyl-1,3,4-oxadiazol-2-yl)phenyl)-N-propylimidazo[1,2-a]pyridine-2-carboxamide

### Step 1: methyl 3-(3-amino-2-(propylcarbamoyl)imidazo[1,2-a]pyridin-8-yl)benzoate

The operation method was the same as that in the synthesis example of compound vii, using (3-(methoxycarbonyl)phenyl)boronic acid (100 mg, 0.56 mmol) and 3-amino-8-bromo-N-propylimidazo[1,2-a]pyridine-2-carboxamide (165 mg, 0.56 mmol) as starting materials to obtain a pale green solid (120 mg, 61%). LC-MS: m/z [M+H]⁺ =353

### Step 2: 3-(3-amino-2-(propylcarbamoyl)imidazo[1,2-a]pyridin-8-yl)benzoic acid

Methyl 3-(3-amino-2-(propylcarbamoyl)imidazo[1,2-a]pyridin-8-yl)benzoate (120 mg, 0.34 mmol) was dispersed in tetrahydrofuran/water (2 mL/2 mL). Lithium hydroxide monohydrate (54 mg, 1.36 mmol) was added, and the mixture was heated to 45°C and stirred for 16 hours, yielding an off-white solid (100 mg, 87%). LC-MS: m/z [M+H]⁺ = 339

### Step 3: 8-(3-(2-Acetylhydrazine-1-carbonyl)phenyl)-3-amino-N-propylimidazo[1,2-a]pyridine-2-carboxamide

3-(3-Amino-2-(propylcarbamoyl)imidazo[1,2-a]pyridin-8-yl)benzoic acid (80 mg, 0.23 mmol), HATU (131 mg, 0.345 mmol), acetohydrazide (25.5 mg, 0.345 mmol), and triethylamine (100 mg, 1 mmol) were sequentially added to N,N-dimethylformamide (5 mL). The mixture was stirred at room temperature for 2 hours. Water (20 mL) was added, and the mixture was extracted with ethyl acetate (20 mL × 3). The organic phases were combined, dried over anhydrous sodium sulfate, concentrated, and purified by column chromatography to obtain the title product (50 mg, 55%). LC-MS: m/z [M+H]⁺ = 395

**Step 4:** At 25°C, 8-(3-(2-acetylhydrazine-1-carbonyl)phenyl)-3-amino-N-propylimidazo[1,2-a]pyridine-2-carboxamide (20 mg, 0.05 mmol), p-toluenesulfonyl chloride (30 mg, 0.15 mmol), and triethylamine (15 mg, 0.15 mmol) were sequentially added to dichloromethane (3 mL). The mixture was stirred at 25°C for 16 hours. The mixture was concentrated and then purified by column chromatography to obtain the target compound (3 mg, 16%).
¹H NMR (400 MHz, DMSO-*d*₆) 8.70 (s, 1H), 8.41 (d, *J=* 8.0 Hz, 1H), 8.20 (d, *J= 6.9* Hz, 1H), 8.03 (d, *J* = 7.7 Hz, 1H), 7.77 - 7.61 (m, 2H), 7.44 (d, *J* = 6.9 Hz, 1H), 6.98 (t, *J* = 6.9 Hz, 1H), 6.19 (s, 2H), 3.24 (dd, *J* = 13.4, 6.8 Hz, 2H), 2.60 (s, 3H), 1.54 (dd, J= 14.4, 7.2 Hz, 2H), 0.89 (t, *J=* 7.4 Hz, 3H). LC-MS: m/z [M+H]⁺ = 377

### Example 44

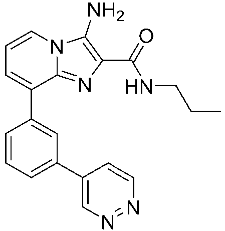

### 3-Amino-N-propyl-8-(3-pyridazin-4-yl)phenyl)imidazo[1,2-a]pyridine-2-carboxamide

### Step 1: 4-(3-chlorophenyl)pyridazine

3-Chlorophenylboronic acid (200 mg, 1.3 mmol), 4-bromopyridazine (226 mg, 1.4 mmol), cesium carbonate (1.26 g, 3.9 mmol), and [1,1'-bis(di-tert-butylphosphino)ferrocene]dichloropalladium (84 mg, 0.13 mmol) were sequentially added to a mixed solution of dioxane and water (5 mL/0.5 mL). The mixture was stirred at 90°C for 1 hour under nitrogen atmosphere. The mixture was concentrated and purified by column chromatography (petroleum ether:ethyl acetate = 2:1) to obtain the title product (120 mg, pale yellow solid) in a yield of 48%. LC-MS: m/z [M+H]⁺ =191

### Step 2: 4-(3-(4,4,5,5-Tetramethyl-1,3,2-dioxaborolan-2-yl)phenyl)pyridazine

4-(3-Chlorophenyl)pyridazine (120 mg, 0.63 mmol), bis(pinacolato)diboron (193 mg, 0.76 mmol), potassium acetate (185 mg, 1.9 mmol), X-Phos (56 mg, 0.13 mmol), and Pd₂(dba)₃ (28 mg, 0.03 mmol) were sequentially added to dioxane (5 mL). The mixture was stirred at 100°C for 16 hours under nitrogen atmosphere. It was concentrated and purified by column chromatography to obtain the title product (50 mg, yellow solid) in a yield of 28%. LC-MS: m/z [M+H]⁺ =283

**Step 3:** The operation method was the same as that in the synthesis example of compound vii, using 4-(3-(4,4,5,5-tetramethyl-1,3,2-dioxaborolan-2-yl)phenyl)pyridazine (50 mg, 0.177 mmol) and 3-amino-8-bromo-N-propylimidazo[1,2-a]pyridine-2-carboxamide (52 mg, 0.19 mmol) as starting materials to obtain the title product (10 mg, yellow solid) in a yield of 15%.
¹HNMR(400 MHz, DMSO-*d*₆) 9.75 (d, J = 1.2 Hz, 1H), 9.30 (d, J = 4.6 Hz, 1H), 8.53 (s, 1H), 8.38 (d, J = 7.9 Hz, 1H), 8.19 (d, J = 6.8 Hz, 1H), 8.12 (dd, J = 5.4, 2.5 Hz, 1H), 7.96 (d, J = 7.9 Hz, 1H), 7.78 (t, J = 6.0 Hz, 1H), 7.70 (t, J = 7.8 Hz, 1H), 7.50 (d, J = 6.6 Hz, 1H), 6.98 (t, J = 6.9 Hz, 1H), 6.18 (s, 2H), 3.24 (dd, J = 13.7, 6.6 Hz, 2H), 1.61 - 1.46 (m, 2H), 0.88 (t, J = 7.4 Hz, 3H). LC-MS: m/z [M+H]⁺= 373

### Example 45

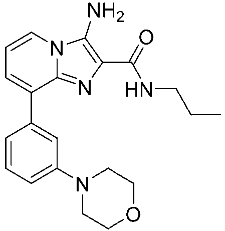

### 3-Amino-8-(3-morpholinophenyl)-N-propylimidazo[1,2-a]pyridine-2-carboxamide

The operation method was the same as that in the synthesis example of compound vii, using (3-morpholinophenyl)boronic acid (50 mg, 0.22 mmol) and 3-amino-8-bromo-N-propylimidazo[1,2-a]pyridine-2-carboxamide (65 mg, 0.22 mmol) as starting materials to obtain the title compound (35 mg, 42%).
¹H NMR (400 MHz, DMSO-*d*₆) 8.12 (d, J= 6.9 Hz, 1H), 7.67 (s, 1H), 7.62(t, J= 6.1 Hz, 1H), 7.50 (d, *J= 7.7* Hz, 1H), 7.34 (t, *J* = 8.0 Hz, 1H), 7.29 (d, *J* = 6.9 Hz, 1H), 7.02 (dd, *J* = 8.3, 2.4 Hz, 1H), 6.92 (t, *J* = 6.9 Hz, 1H), 6.13 (s, 2H), 3.82 - 3.71 (m, 4H), 3.24 (dd, *J* = 13.7, 6.7 Hz, 2H), 3.21 - 3.14 (m,4H), 1.52 (dd, *J=* 14.4, 7.2 Hz, 2H), 0.88 (t, *J* = 7.4 Hz, 3H). LC-MS: m/z [M+H]⁺ = 380

### Example 46

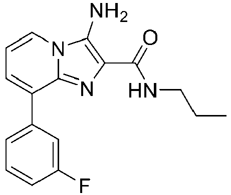

### 3-Amino-8-(3-fluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (3-fluorophenyl)boronic acid (71 mg, 0.51 mmol) to obtain the target product (35 mg, 33%) as a yellow solid.
¹H NMR (400 MHz, DMSO-*d*₆) 8.17 (d, J=6.85 Hz, 1 H) 7.95 - 8.09 (m, 2 H) 7.84 (br. s., 1 H) 7.46 - 7.60 (m, 1 H) 7.40 (d, J=6.85 Hz, 1 H) 7.25 (t, J=7.95 Hz, 1 H) 6.95 (t, J=6.72 Hz, 1 H) 6.19 (br. s., 2 H) 3.24 (d, J=6.36 Hz, 2 H) 1.42 - 1.61 (m, 2 H) 0.87 (t, J=7.34 Hz, 3 H).LC-MS: m/z [M+1]⁺= 313

### Example 47

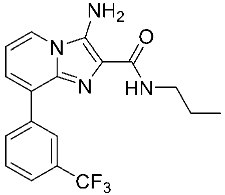

### 3-Amino-N-propyl-8-(3-(trifluoromethyl)phenyl)imidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (2-methoxyphenyl)boronic acid (62 mg, 0.41 mmol) as starting materials to obtain the target product (76 mg, 87%) as a yellow solid.
¹H NMR (400 MHz, DMSO-*d*₆) 8.52 (d, J=7.34 Hz, 1 H) 8.40 (s, 1 H) 8.20 (d, J=6.85 Hz, 1 H) 7.63 - 7.85 (m, 3 H) 7.45 (d, J=6.85 Hz, 1 H) 6.97 (t, J=6.72 Hz, 1 H) 6.21 (s, 2 H) 3.24 (q, J=6.68 Hz, 2 H) 1.44 - 1.61 (m, 2 H) 0.88 (t, J=7.34 Hz, 3 H). LC-MS: m/z [M+1]⁺= 363

### Example 48

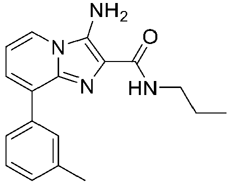

### 3-Amino-N-propyl-8-(3-methylphenyl)imidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (3-methylphenyl)boronic acid (55 mg, 0.41 mmol) as starting materials to obtain the target product (53 mg, 64%) as a yellow solid.
¹H NMR (400 MHz, DMSO-*d*₆) 8.13 (d, J=6.60 Hz, 1 H) 7.92 (d, J=7.34 Hz, 1 H) 7.85 (br. s., 1 H) 7.75 (br. s., 1 H) 7.36 (t, J=7.46 Hz, 1 H) 7.24(dd, J=17.97, 6.97 Hz, 2 H) 6.92 (t, J=6.85 Hz, 1 H) 6.16 (br. s., 2 H) 3.23 (q, J=6.44 Hz, 2 H) 2.39 (s, 3 H) 1.45 - 1.57 (m, 2 H) 0.86 (t, J=7.09 Hz, 3 H).LC-MS: m/z [M+1]⁺= 309

### Example 49

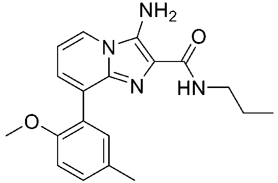

### 3-Amino-8-(2-methoxy-5-methylphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (2-methoxy-5-methylphenyl)boronic acid (45 mg, 0.27 mmol) as starting materials to obtain the target product (55 mg, 60%) as a yellow solid.
¹H NMR (400 MHz, DMSO-*d*₆) 8.09 (d, J=6.85 Hz, 1 H) 7.57 (br. s., 1 H) 7.15 - 7.29 (m, 2 H) 7.02 (d, J=8.31 Hz, 1 H) 6.95 (d, J=6.36 Hz, 1 H) 6.87(d, J=6.85 Hz, 1 H) 6.11 (s, 2 H) 3.67 (s, 3 H) 3.17 (d, J=6.85 Hz, 2 H) 2.28 (s, 3 H) 1.47 (d, J=6.85 Hz, 2 H) 0.82 (t, J=7.34 Hz, 3 H).LC-MS: m/z [M+1]⁺= 339

### Example 50

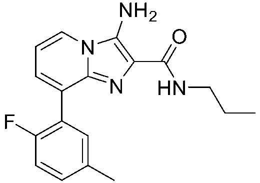

### 3-Amino-8-(2-fluoro-5-methylphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and 2-fluoro-5-methylphenylboronic acid (52 mg, 0.34 mmol) as starting materials to obtain the target product (76 mg, 68%) as a yellow solid.
¹H NMR (400 MHz, DMSO-*d*₆) 8.17 (d, J=6.85 Hz, 1 H) 7.62 (br. s., 1 H) 7.50 (d, J=5.87 Hz, 1 H) 7.15 - 7.31 (m, 2 H) 7.07 (d, J=6.85 Hz, 1 H) 6.91(t, J=6.60 Hz, 1 H) 6.18 (br. s., 2 H) 3.13 - 3.25 (m, 2 H) 2.34 (s, 3 H) 1.42 - 1.54 (m, 2 H) 0.83 (t, J=7.34 Hz, 3 H).LC-MS: m/z [M+1]⁺= 327

### Example 51

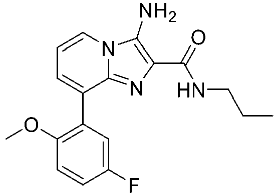

### 3-Amino-8-(3-fluoro-5-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (90 mg, 0.30 mmol) and (3-fluoro-6-methoxyphenyl)boronic acid (76 mg, 0.45 mmol) as starting materials to obtain the target product (25 mg, 24%) as a yellow solid.
¹H NMR (400 MHz, DMSO-*d*₆) 8.12 (d, J=6.85 Hz, 1 H) 7.63 (br. s., 1 H) 7.37 (d, J=6.36 Hz, 1 H) 7.22 (d, J=7.83 Hz, 1 H) 7.16 (d, J=4.40 Hz, 1 H) 7.06 (d, J=6.85 Hz, 1 H) 6.88 (t, J=6.85 Hz, 1 H) 6.14 (br. s., 2 H) 3.71 (s, 3 H) 3.18 (d, J=6.85 Hz, 2 H) 1.40 - 1.54 (m, 2 H) 0.83 (t, J=7.34 Hz, 3 H).LC-MS: m/z [M+1]⁺= 343

### Example 52

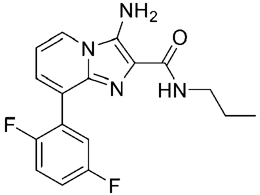

### 3-Amino-8-(2,5-difluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2,5-difluorophenyl)boronic acid (54 mg, 0.34 mmol) to obtain the target product (51 mg, 45%) as a yellow solid.
¹H NMR (400 MHz, DMSO-*d*₆) 8.20 (d, J=6.85 Hz, 1 H) 7.69 (br. s., 2 H) 7.28 - 7.46 (m, 2 H) 7.18 (d, J=6.36 Hz, 1 H) 6.94 (t, J=6.85 Hz, 1 H) 6.20(s, 2 H) 3.19 (q, J=6.52 Hz, 2 H) 1.44 - 1.55 (m, 2 H) 0.84 (t, J=7.34 Hz, 3 H).LC-MS: m/z [M+1]⁺= 331

### Example 53

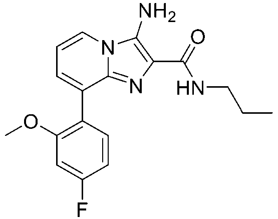

### 3-Amino-8-(4-fluoro-2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (4-fluoro-2-methoxyphenyl)boronic acid (87 mg, 0.51 mmol) as starting materials to obtain the target product (65 mg, 56%) as a yellow solid.
¹H NMR (400 MHz, DMSO-*d*₆) 8.11 (d, J=6.85 Hz, 1 H) 7.59 (t, J=6.11 Hz, 1 H) 7.52 (t, J=7.83 Hz, 1 H) 6.96 - 7.09 (m, 2 H) 6.80 - 6.90 (m, 2 H)6.12 (s, 2 H) 3.74 (s, 3 H) 3.17 (q, J=6.85 Hz, 2 H) 1.41 - 1.54 (m, 2 H) 0.82 (t, J=7.34 Hz, 3 H).LC-MS: m/z [M+1]⁺= 343

### Example 54

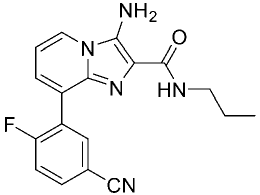

### 3-Amino-8-(5-cyano-2-fluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (60 mg, 0.20 mmol) and (5-cyano-2-fluorophenyl)boronic acid (49 mg, 0.30 mmol) as starting materials to obtain the target product (46 mg, 67%) as a yellow solid.
¹H NMR (400 MHz, DMSO-*d₆*)8.16 - 8.31 (m, 2 H) 8.01 (dd, J=5.38, 3.42 Hz, 1 H) 7.71 (s, 1 H) 7.60 (t, J=9.29 Hz, 1 H) 7.19 (d, J=6.85 Hz, 1 H)6.95 (t, J=6.85 Hz, 1 H) 6.22 (s, 2 H) 3.09 - 3.23 (m, 2 H) 1.42 - 1.55 (m, 2 H) 0.83 (t, J=7.34 Hz, 3 H). LC-MS: m/z [M+1]⁺= 338

### Example 55

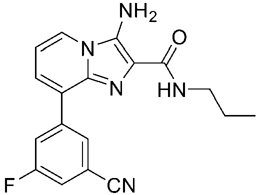

### 3-Amino-8-(3-cyano-5-fluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (3-cyano-5-fluorophenyl)boronic acid (67 mg, 0.41 mmol) to obtain the target product (86 mg, 95%) as a yellow solid.
¹H NMR (400 MHz, DMSO-*d*₆) 8.49 - 8.59 (m, 1 H) 8.44 (s, 1 H) 8.18 - 8.28 (m, 1 H) 7.81 - 7.98 (m, 2 H) 7.49 - 7.59 (m, 1 H) 6.93 - 7.03 (m, 1 H) 6.22 (s, 2 H) 3.19 - 3.29 (m, 2 H) 1.42 - 1.60 (m, 2 H) 0.79 - 0.93 (m, 3 H).LC-MS: m/z [M+1]⁺= 338

### Example 56

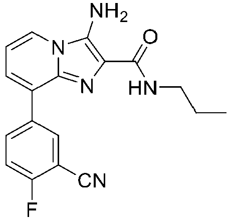

### 3-Amino-8-(3-cyano-4-fluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (3-cyano-4-fluorophenyl)boronic acid (112 mg, 0.68 mmol) to obtain the target product (88 mg, 77%) as a yellow solid.
¹H NMR (400 MHz, DMSO-*d*₆) 8.66 (br. s., 2 H) 8.09 - 8.28 (m, 1 H) 7.82 - 7.96 (m, 1 H) 7.55 - 7.73 (m, 1 H) 7.33 - 7.52 (m, 1 H) 6.80 - 7.04 (m, 1H) 6.20 (br. s., 2 H) 3.23 (d, J=6.36 Hz, 2 H) 1.53 (d, J=6.85 Hz, 2 H) 0.87 (t, J=7.09 Hz, 3 H). LC-MS: m/z [M+1]⁺= 338

### Example 57

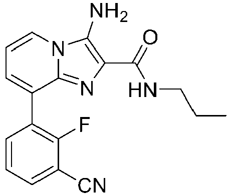

### 3-Amino-8-(3-cyano-2-fluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and 2-(5,5-dimethyl-1,3,2-dioxaborinan-2-yl)-3-methoxybenzonitrile (181 mg, 0.68 mmol) as starting materials to obtain the target product (28 mg, 24%) as a yellow solid.
¹H NMR (400 MHz, DMSO-*d*₆) 8.24 (d, J=6.85 Hz, 1 H) 8.13 - 8.20 (m, 1 H) 7.96 - 8.06 (m, 1 H) 7.68 - 7.77 (m, 1 H) 7.54 (s, 1 H) 7.21 (s, 1 H) 6.96 (s, 1 H) 6.23 (s, 2 H) 3.18 (d, J=7.34 Hz, 2 H) 1.49 (d, J=7.34 Hz, 2 H) 0.83 (t, J=7.34 Hz, 3 H). LC-MS: m/z [M+1]⁺= 338

### Example 58

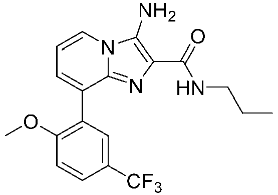

### 3-Amino-8-(2-methoxy-5-(trifluoromethyl)phenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2-methoxy-5-(trifluoromethyl)phenyl)boronic acid (110 mg, 0.51 mmol) as starting materials to obtain the target product (94 mg, 70%) as a yellow solid.
¹H NMR (400 MHz, DMSO-*d*₆) 8.15 (d, J=6.85 Hz, 1 H) 7.71 - 7.83 (m, 2 H) 7.52 (t, J=6.11 Hz, 1 H) 7.34 (d, J=8.80 Hz, 1 H) 7.07 (d, J=6.85 Hz, 1H) 6.89 (t, J=6.85 Hz, 1 H) 6.14 (s, 2 H) 3.80 (s, 3 H) 3.17 (q, J=6.85 Hz, 2 H) 1.40 - 1.53 (m, 2 H) 0.83 (t, J=7.58 Hz, 3 H).LC-MS: m/z [M+1]⁺= 393

### Example 59

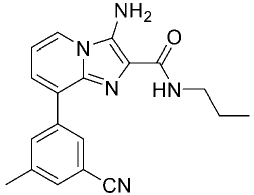

### 3-Amino-8-(3-cyano-5-methylphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (3-cyano-5-methylphenyl)boronic acid (111 mg, 0.68 mmol) to obtain the target product (50 mg, 44%) as a yellow solid.
¹H NMR (400 MHz, DMSO-*d*₆) 8.34 (s, 1 H) 8.27 (s, 1 H) 8.18 (s, 1 H) 7.77 - 7.86 (m, 1 H) 7.70 (s, 1 H) 7.41 (d, J=6.85 Hz, 1 H) 6.95 (s, 1 H) 6.20(s, 2 H) 3.24 (d, J=6.85 Hz, 2 H) 2.46 (s, 3 H) 1.53 (d, J=7.34 Hz, 2 H) 0.88 (t, J=7.34 Hz, 3 H). LC-MS: m/z [M+1]⁺=334

### Example 60

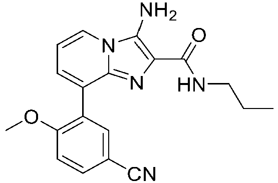

### 3-Amino-8-(5-cyano-2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (90 mg, 0.30 mmol) and (5-cyano-2-methoxyphenyl)boronic acid (80 mg, 0.45 mmol) as starting materials to obtain the target product (19 mg, 18%) as a yellow solid.
¹H NMR (400 MHz, DMSO-*d*₆) 8.15 (d, J=6.85 Hz, 1 H) 7.84 - 7.94 (m, 2 H) 7.66 (s, 1 H) 7.33 (d, J=9.29 Hz, 1 H) 7.05 (d, J=6.85 Hz, 1 H) 6.89 (t, J=6.85 Hz, 1 H) 6.16 (s, 2 H) 3.81 (s, 3 H) 3.16 (d, J=6.85 Hz, 2 H) 1.41 - 1.51 (m, 2 H) 0.82 (t, J=7.34 Hz, 3 H).LC-MS: m/z [M+1]⁺=350

### Example 61

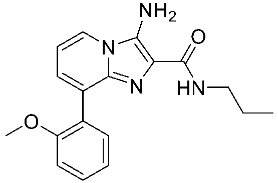

### 3-Amino-8-(2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2-methoxyphenyl)boronic acid (77 mg, 0.51 mmol) as starting materials to obtain the target product (49 mg, 44%) as a yellow solid.
¹H NMR (400 MHz, DMSO-*d*₆) 8.11 (d, J=6.85 Hz, 1 H) 7.56 (br. s., 1 H) 7.47 (d, J=6.85 Hz, 1 H) 7.38 (t, J=7.70 Hz, 1 H) 7.13 (d, J=8.56 Hz, 1 H)6.95 - 7.06 (m, 2 H) 6.87 (t, J=6.85 Hz, 1 H) 6.12 (br. s., 2 H) 3.72 (s, 3 H) 3.10 - 3.22 (m, 2 H) 1.37 - 1.54 (m, 2 H) 0.82 (t, J=7.21 Hz, 3 H).LC-MS: m/z [M+1]⁺=325

### Example 62

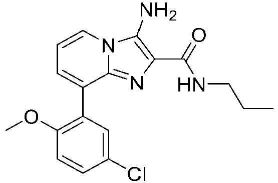

### 3-Amino-8-(5-chloro-2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (5-chloro-2-methoxyphenyl)boronic acid (95 mg, 0.51 mmol) as starting materials to obtain the target product (60 mg, 49%) as a yellow solid.
¹H NMR (400 MHz, DMSO-*d*₆) 8.13 (d, J=6.85 Hz, 1 H) 7.61 (s, 1 H) 7.38 - 7.51 (m, 2 H) 7.17 (d, J=8.80 Hz, 1 H) 7.02 (d, J=6.36 Hz, 1 H) 6.88 (t, J=6.85 Hz, 1 H) 6.15 (s, 2 H) 3.72 (s, 3 H) 3.17 (d, J=6.85 Hz, 2 H) 1.40 - 1.55 (m, 2 H) 0.83 (t, J=7.34 Hz, 3 H).LC-MS: m/z [M+1]⁺= 359

### Example 63

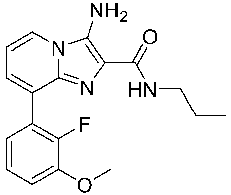

### 3-Amino-8-(2-fluoro-3-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2-fluoro-3-methoxyphenyl)boronic acid (58 mg, 0.34 mmol) as starting materials to obtain the target product (92 mg, 79%) as a yellow solid.
¹H NMR (400 MHz, DMSO-*d*₆) 8.18 (d, J=6.85 Hz, 1 H) 7.62 (t, J=5.87 Hz, 1 H) 7.22 (d, J=3.42 Hz, 3 H) 7.07 (d, J=6.85 Hz, 1 H) 6.92 (t, J=6.85Hz, 1 H) 6.18 (s, 2 H) 3.88 (s, 3 H) 3.18 (q, J=6.85 Hz, 2 H) 1.42 - 1.54 (m, 2 H) 0.83 (t, J=7.34 Hz, 3 H). LC-MS: m/z [M+1]⁺= 343

### Example 64

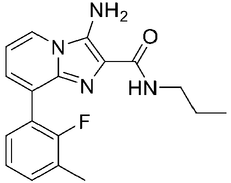

### 3-Amino-8-(2-fluoro-3-methylphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (2-fluoro-3-methylphenyl)boronic acid (62 mg, 0.41 mmol) as starting materials to obtain the target product (39 mg, 44%) as a yellow solid.
¹H NMR (400 MHz, DMSO-*d*₆) 8.17 (d, J=6.85 Hz, 1 H) 7.64 (t, J=5.87 Hz, 1 H) 7.52 (t, J=6.85 Hz, 1 H) 7.33 (t, J=7.34 Hz, 1 H) 7.14 - 7.24 (m, 1H) 7.08 (d, J=6.85 Hz, 1 H) 6.91 (t, J=6.85 Hz, 1 H) 6.18 (s, 2 H) 3.12 - 3.23 (m, 2 H) 2.30 (s, 3 H) 1.53-1.46 (m, 2 H) 0.83 (t, J=7.34 Hz, 3 H).LC-MS: m/z [M+1]⁺= 327

### Example 65

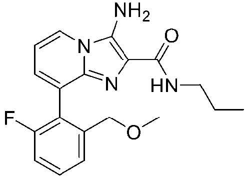

### 3-Amino-8-(2-fluoro-6-(methoxymethyl)phenyl)-N-propylimidazo[1,2-a]pyridine-2-carboxamide

### Step 1: 2-bromo-1-fluoro-3-(methoxymethyl)benzene

2-Bromo-1-(bromomethyl)-3-fluorobenzene (2 g, 7.46 mmol) and sodium methoxide (800 mg, 14.9 mmol) were dissolved in methanol (10 mL), and the mixture was stirred at room temperature for 2 hours. 20 mL of water was added, and the resulting mixture was extracted with dichloromethane (30 mL × 2). The organic phase was dried and concentrated to obtain 1.5 g of a white solid.

### Step 2: 2-(2-fluoro-6-(methoxymethyl)phenyl)-4,4,5,5-tetramethyl-1,3,2-dioxaborolane

2-Bromo-1-fluoro-3-(methoxymethyl)benzene (220 mg, 1 mmol), bis(pinacolato)diboron (500 mg, 2 mmol), potassium acetate (300 mg, 3 mmol), and [1,1'-bis(diphenylphosphino)ferrocene]dichloropalladium(II) dichloromethane complex (CAS No.: 95464-05-4, 86 mg, 0.1 mmol) were dissolved in 5 mL of dioxane, heated to 100 °C, and stirred for 16 hours. Water (20 mL) was added, and the mixture was extracted with dichloromethane (30 mL × 2). The organic phase was dried and concentrated to obtain the target product (181 mg, crude), which was used directly in the next step.

**Step 3:** The operation method was the same as that in the synthesis example of compound vii, using 3-amino-8-bromo-N-propylimidazo[1,2-a]pyridine-2-carboxamide (100 mg, 0.34 mmol) and 2-(2-fluoro-6-(methoxymethyl)phenyl)-4,4,5,5-tetramethyl-1,3,2-dioxaborolane (181 mg, 0.68 mmol) as starting materials to obtain the target product (12 mg, 10%) as a yellow solid.
¹H NMR (400 MHz, DMSO-*d*₆) 8.21 (d, J=6.85 Hz, 1 H) 7.61 (t, J=5.62 Hz, 1 H) 7.43 - 7.53 (m, 1 H) 7.37 (d, J=7.83 Hz, 1 H) 7.24 (t, J=8.80 Hz, 1 H) 6.89 - 7.03 (m, 2 H) 6.20 (s, 2 H) 4.10 - 4.33 (m, 2 H) 3.09 - 3.19 (m, 5 H) 1.38 - 1.52 (m, 2 H) 0.81 (t, J=7.09 Hz, 3 H). LC-MS: m/z [M+1]⁺= 357

### Example 66

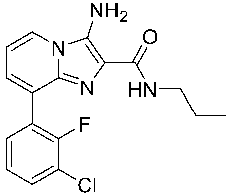

### 3-Amino-8-(3-chloro-2-fluorophenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (3-chloro-2-fluorophenyl)boronic acid (71 mg, 0.41 mmol) to obtain the target product (35 mg, 38%) as a yellow solid.
¹H NMR (400 MHz, DMSO-*d*₆) 8.21 (dd, J=6.85, 0.98 Hz, 1 H) 7.60 - 7.75 (m, 3 H) 7.34 (s, 1 H) 7.15 (d, J=6.85 Hz, 1 H) 6.90 - 6.97 (m, 1 H) 6.20(s, 2 H) 3.18 (d, J=7.34 Hz, 2 H) 1.48 (d, J=7.34 Hz, 2 H) 0.83 (t, J=7.58 Hz, 3 H).LC-MS: m/z [M+1]⁺= 347

### Example 67

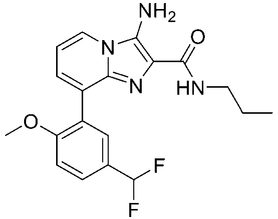

### 3-Amino-8-(5-(difluoromethyl)-2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (5-(difluoromethyl)-2-methoxyphenyl)boronic acid (101 mg, 0.51 mmol) as starting materials to obtain the target product (54 mg, 55%) as a yellow solid.
¹H NMR (400 MHz, DMSO-*d*₆) 8.14 (d, J=7.09 Hz, 1 H) 7.58 - 7.69 (m, 2 H) 7.55 (t, J=6.11 Hz, 1 H) 7.27 (d, J=8.56 Hz, 1 H) 6.85 - 7.17 (m, 3 H)6.14 (s, 2 H) 3.77 (s, 3 H) 3.10 - 3.22 (m, 2 H) 1.42 - 1.53 (m, 2 H) 0.83 (t, J=7.46 Hz, 3 H).LC-MS: m/z [M+1]⁺= 375

### Example 68

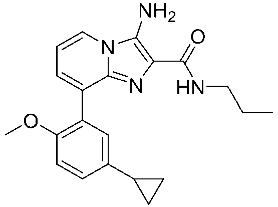

### 3-Amino-8-(5-cyclopropyl-2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (500 mg, 1.68 mmol) and (5-chloro-2-methoxyphenyl)boronic acid (470 mg, 2.52 mmol) as starting materials to obtain 3-amino-8-(5-chloro-2-methoxypheny1)-N-propylimidazo[1,2-a]pyridine-2-carboxamide (450 mg, 75%). Subsequently, the product (100 mg, 0.28 mmol) and cyclopropylboronic acid (36 mg, 0.42 mmol) were used as starting materials to obtain the target product (50 mg, 49%) as a yellow solid by referring to the synthesis method of compound vii.
¹H NMR (400 MHz, DMSO-*d*₆) 8.09 (d, J=6.85 Hz, 1 H) 7.54 (br. s., 1 H) 6.92 - 7.19 (m, 4 H) 6.86 (t, J=6.60 Hz, 1 H) 6.11 (br. s., 2 H) 3.67 (s, 3 H)3.17 (d, J=6.36 Hz, 2 H) 1.90 (br. s., 1 H) 1.37 - 1.55 (m, 2 H) 0.77 - 0.96 (m, 5 H) 0.62 (d, J=3.42 Hz, 2 H).LC-MS: m/z [M+1]⁺= 365

### Example 69

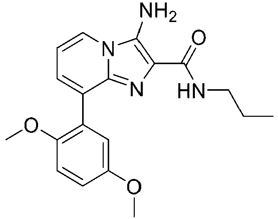

### 3-Amino-8-(2,5-dimethoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and 2,5-dimethoxyphenylboronic acid (62 mg, 0.34 mmol) as starting materials to obtain the target product (71 mg, 59%) as a yellow solid.
¹H NMR (400 MHz, CHLOROFORM) 7.76 (br. s., 1 H) 7.27 (br. s., 87 H) 6.92 - 7.04 (m, 2 H) 6.87 (br. s., 1 H) 5.02 (br. s., 1 H) 3.71 - 3.89 (m, 6H) 3.40 (d, J=6.85 Hz, 2 H) 1.56 - 1.73 (m, 2 H) 0.98 (t, J=7.34 Hz, 3 H).LC-MS: m/z [M+1]⁺= 355

### Example 70

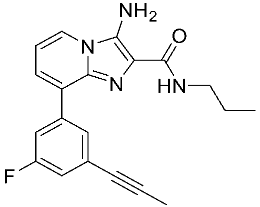

### 3-Amino-8-(3-fluoro-5-(prop-1-yn-1-yl)phenyl)-N-propylimidazo[1,2-a]pyridine-2-carboxamide

### Step 1: 1-bromo-3-fluoro-5-(prop-1-yn-1-yl)benzene

Bromo-3-fluoro-5-iodobenzene (0.5 g, 1.66 mmol), 1-(trimethylsilyl)-1-propyne (0.19 g, 1.66 mmol), copper(I) iodide (0.095 g, 0.50 mmol), and tetrakis(triphenylphosphine)palladium (0.15 g, 0.13 mmol) were dissolved in toluene (10 mL) and degassed. Triethylamine (0.55 g, 5.48 mmol) and tetrabutylammonium fluoride (0.43 g, 1.66 mmol) were then added to the reaction mixture, which was stirred at 100°C for 16 hours. The mixture was concentrated, then added with 60 mL of water, extracted with ethyl acetate (60 ml × 2), concentrated, and purified by column chromatography (petroleum ether) to obtain 300 mg of the product (85%, yellow liquid).

### Step 2: 2-(3-fluoro-5-(prop-1-yn-1-yl)phenyl)-4,4,5,5-tetramethyl-1,3,2-dioxaborolane

Bromo-3-fluoro-5-(prop-1-yn-1-yl)benzene (0.2 g, 0.94 mmol), bis(pinacolato)diboron (CAS No.: 73183-34-3, 0.36 g, 1.41 mmol), [1,1'-bis(diphenylphosphino)ferrocene]palladium(II) dichloride dichloromethane complex (0.077 g, 0.094 mmol), and potassium acetate (0.18 g, 1.88 mmol) were sequentially added to 1,4-dioxane (6 mL). The atmosphere was replaced with nitrogen, and the mixture was reacted under microwave irradiation at 100°C for 1 hour. The mixture was concentrated, added with 40 mL of water, extracted with ethyl acetate (40 mL × 2), and concentrated to obtain 150 mg of the product (89%, yellow liquid).

**Step 3:** The operation method was the same as that in the synthesis example of compound vii, using 2-(3-fluoro-5-(prop-1-yn-1-yl)phenyl)-4,4,5,5-tetramethyl-1,3,2-dioxaborolane (0.11 g, 0.42 mmol) and 3-amino-8-bromo-N-propylimidazo[1,2-a]pyridine-2-carboxamide (0.12 g, 0.42 mmol) as starting materials to obtain the product (40 mg, 27%) as a yellow solid.
¹HNMR (400MHz, DMSO-*d*₆) 8.17 (d, J = 5.9 Hz, 1 H), 8.09 (d, J = 9.8 Hz, 1 H), 7.83 ( s, 1 H), 7.77 (s, 1 H), 7.40 (d, J = 6.4 Hz, 1 H), 7.27 (d, J = 8.8 Hz, 1 H), 6.92 (s, 1 H), 6.18 (s, 2 H), 3.25-3.21 ( m, 2 H), 2.07 (s, 3 H), 1.52 (d, J = 6.8 Hz, 2 H), 0.92 - 0.81 (m, 3 H).LC-MS: m/z [M+1]⁺= 351

### Example 71

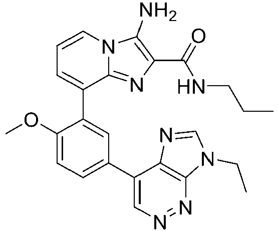

### 3-Amino-8-(5-(7-ethyl-7H-imidazo[4,5-c]pyridazin-4-yl)-2-methoxyphenyl)-N-propylimidazo[1,2-a]pyridine-2-carboxamide

### Step 1: 4-(3-Chloro-4-methoxyphenyl)-7-ethyl-7H-imidazo[4,5-c]pyridazine

The operation was the same as that in Example 39 (step 1), using 4-chloro-7-ethyl-7H-imidazo[4,5-c]pyridazine (546 mg, 3.0 mmol) and (3-chloro-4-methoxyphenyl)boronic acid (500 mg, 2.7 mmol) as a starting material to obtain a yellow oil (300 mg, 35%). LC-MS: m/z [M+H]⁺ = 289

### Step 2: 7-ethyl-4-(4-methoxy-3-(4,4,5,5-tetramethyl-1,3,2-dioxaborolan-2-yl)phenyl)-7H-imidazo[4,5-c]pyridazine

The operation was the same as that in Example 39 (step 2), using 4-(3-chloro-4-methoxyphenyl)-7-ethyl-7H-imidazo[4,5-c]pyridazine (136 mg, 0.47 mmol) as a starting material to obtain a gray solid (90 mg, 50%). LC-MS: m/z [M+H]⁺ =381

**Step 3:** The operation method was the same as that in the synthesis example of compound vii, using 7-ethyl-4-(4-methoxy-3-(4,4,5,5-tetramethyl-1,3,2-dioxaborolan-2-yl)phenyl)-7H-imidazo[4,5-c]pyridazine (100 mg, 0.26 mmol) and 3-amino-8-bromo-N-propylimidazo[1,2-a]pyridine-2-carboxamide (77 mg, 0.26 mmol) as starting materials to obtain the target product (9 mg, 7%).
¹H NMR (400 MHz, DMSO-*d*₆) 9.51 (s, 1H), 8.79 (s, 1H), 8.61 (d, *J=* 2.1 Hz, 1H), 8.49 (dd, *J* = 8.8, 2.2 Hz, 1H), 8.17 (d, *J* = 6.9 Hz, 1H), 7.59 (t, *J= 6.2* Hz, 1H), 7.39 (d, *J* = 8.9 Hz, 1H), 7.11 (d, *J* = 6.7 Hz, 1H), 6.93 (t, *J=* 6.9 Hz, 1H), 6.15 (s, 2H), 4.50 (q, *J* = 7.3 Hz, 2H), 3.84 (s, 3H), 3.15 (dd, *J* = 13.6, 6.6 Hz, 2H), 1.55 (t, *J* = 7.2 Hz, 3H), 1.44 (dd, *J* = 14.6, 7.2 Hz, 2H), 0.78 (t, *J=* 7.4 Hz, 3H). LC-MS: m/z [M+H]⁺ = 471

### Example 72

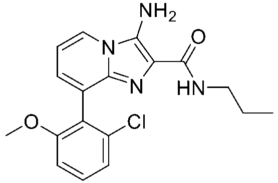

### 3-Amino-8-(2-chloro-6-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (2-chloro-6-methoxyphenyl)boronic acid (71 mg, 0.49 mmol) as starting materials to obtain the target product (33 mg, 34%) as a yellow solid.
¹H NMR (400 MHz, DMSO-*d*₆) 8.14 (d, J=5.87 Hz, 1 H) 7.59 (t, J=5.87 Hz, 1 H) 7.32 - 7.47 (m, 1 H) 7.13 (dd, J=16.63, 8.31 Hz, 2 H) 6.78 - 6.96(m, 2 H) 6.15 (s, 2 H) 3.66 (s, 3 H) 3.09 - 3.20 (m, 2 H) 1.38 - 1.52 (m, 2 H) 0.81 (t, J=7.34 Hz, 3 H).LC-MS: m/z [M+1]⁺=359

### Example 73

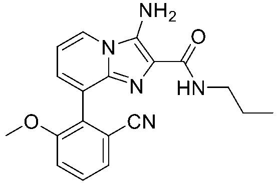

### 3-Amino-8-(2-cyano-6-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and 2-(5,5-dimethyl-1,3,2-dioxaborinan-2-yl)-3-methoxybenzonitrile (181 mg, 0.68 mmol) as starting materials to obtain the target product (28 mg, 24%) as a yellow solid.
¹H NMR (400 MHz, DMSO-*d₆*)8.15 - 8.24 (m, 1 H) 7.47-7.66 (m, 4 H) 6.90-7.06 (m, 2 H) 6.19 (s, 2 H) 3.73 (s, 3 H) 3.16-3.12 (m, 2 H) 1.46 (d, J=7.34 Hz, 2 H) 0.81 (t, J=7.58 Hz, 3 H). LC-MS: m/z [M+1]⁺= 350

### Example 74

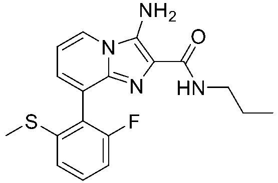

### 3-Amino-8-(2-fluoro-6-(methylthio)phenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and 6-fluoro-2-(methylsulfonyl)phenylboronic acid (50 mg, 0.27 mmol) as starting materials to obtain the target product (35 mg, 38%) as a yellow solid.
¹H NMR (400 MHz, DMSO-*d*₆) 8.20 (d, J=6.36 Hz, 1 H) 7.59 (br. s., 1 H) 7.41 - 7.51 (m, 1 H) 7.03 - 7.26 (m, 1 H) 6.84 - 7.01 (m, 1 H) 6.19 (br. s., 2H) 3.15 (d, J=6.85 Hz, 1 H) 2.36 (s, 3 H) 1.40 - 1.55 (m, 2 H) 0.81 (t, J=7.09 Hz, 3 H). LC-MS: m/z [M+1]⁺= 359

### Example 75

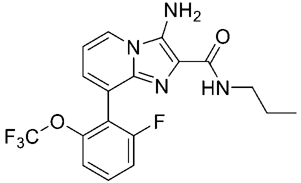

### 3-Amino-8-(2-fluoro-6-(trifluoromethoxy)phenyl)-N-propylimidazo[1,2-a]pyridine-2-carboxamide

### Step 1: (2-fluoro-6-(trifluoromethoxy)phenyl)boronic acid

1- Fluoro-3-(trifluoromethoxy)benzene (500 mg, 2.78 mmol) was dissolved in tetrahydrofuran (12 mL). Under argon atmosphere, the mixture was cooled to -78°C, and a solution of lithium diisopropylamide in tetrahydrofuran (2 M, 2.2 mL) was added dropwise. After stirring for 5 minutes, a solution of trimethyl borate (578 mg, 5.56 mmol) in tetrahydrofuran was added dropwise. The mixture was stirred for an additional 10 minutes. To the reaction mixture was added 1 M aqueous HCl solution to adjust the pH to 6, followed by extraction with diethyl ether. The organic phases were combined, dried over anhydrous sodium sulfate, filtered, and concentrated to a small volume of liquid. The residue was triturated with n-heptane, and filtered under reduced pressure to obtain a white solid (200 mg, 32%).

**Step 2:** The operation method was the same as that in the synthesis example of compound vii, using 3-amino-8-bromo-N-propylimidazo[1,2-a]pyridine-2-carboxamide (100 mg, 0.34 mmol) and (2-fluoro-6-(trifluoromethoxy)phenyl)boronic acid (99 mg, 0.44 mmol) as starting materials to obtain the target product as a pale yellow solid (32 mg, 24%).
¹HNMR (400MHz, DMSO-*d*₆) 8.36 - 8.24 (m, 1 H), 7.69 - 7.60 (m, 1 H), 7.58 - 7.50 (m, 1 H), 7.47 - 7.34 (m, 2 H), 7.09 - 7.01 (m, 1 H), 6.98 - 6.89 (m, 1 H), 6.33 - 6.14 (m, 2 H), 3.20 - 3.11 (m, 2 H), 1.50 - 1.42 (m, 2 H), 0.83-0.79 (m, 3 H). LC-MS: m/z [M+1]⁺= 397

### Example 76

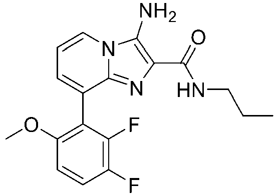

### 3-Amino-8-(2,3-difluoro-6-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (90 mg, 0.30 mmol) and (2,3-difluoro-6-methoxyphenyl)boronic acid (85 mg, 0.45 mmol) as starting materials to obtain the target product (9 mg, 8%) as a yellow solid.
¹H NMR (400 MHz, DMSO-*d*₆) 8.19 (d, J=6.36 Hz, 1 H) 7.64 (br. s., 1 H) 7.48 (d, J=9.29 Hz, 1 H) 6.87 - 7.06 (m, 3 H) 6.18 (br. s., 2 H) 3.69 (s, 3H) 3.15 (br. s., 2 H) 1.38 - 1.56 (m, 2 H) 0.81 (t, J=7.09 Hz, 3 H).LC-MS: m/z [M+1]⁺= 361

### Example 77

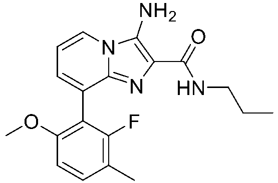

### 3-Amino-8-(2-fluoro-6-methoxy-3-methylphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (90 mg, 0.30 mmol) and (2-fluoro-6-methoxy-3-methylphenyl)boronic acid (83 mg, 0.45 mmol) as starting materials to obtain the target product (36 mg, 34%) as a yellow solid.
¹H NMR (400 MHz, DMSO-*d*₆) 8.02 - 8.22 (m, 1 H) 7.61 (s, 1 H) 7.28 (s, 1 H) 6.77 - 6.94 (m, 3 H) 6.15 (s, 2 H) 3.65 (s, 3 H) 3.16 (dd, J=9.05, 4.65Hz, 2 H) 2.20 (s, 3 H) 1.46 (d, J=7.34 Hz, 2 H) 0.81 (t, J=7.58 Hz, 3 H).LC-MS: m/z [M+1]⁺= 357

### Example 78

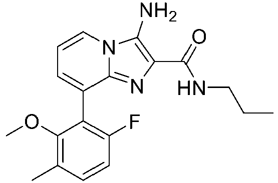

### 3-Amino-8-(6-fluoro-2-methoxy-3-methylphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (6-fluoro-2-methoxy-3-methylphenyl)boronic acid (94 mg, 0.51 mmol) as starting materials to obtain the target product (90 mg, 74%) as a yellow solid.
¹H NMR (400 MHz, DMSO-*d*₆) 8.18 (d, J=6.85 Hz, 1 H) 7.62 (s, 1 H) 7.30 (s, 1 H) 6.95 - 7.03 (m, 2 H) 6.86 - 6.94 (m, 1 H) 6.18 (s, 2 H) 3.41 (s, 3H) 3.14 (d, J=7.34 Hz, 2 H) 2.25 (s, 3 H) 1.39 - 1.55 (m, 2 H) 0.80 (t, J=7.34 Hz, 3 H).LC-MS: m/z [M+1]⁺= 357

### Example 79

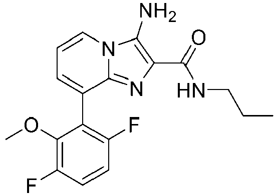

### 3-Amino-8-(3,6-difluoro-2-methoxyphenyl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (3,6-difluoro-2-methoxyphenyl)boronic acid (96 mg, 0.51 mmol) as starting materials to obtain the target product (55 mg, 45%) as a yellow solid.
¹H NMR (400 MHz, DMSO-*d*₆) 8.21 (d, J=5.87 Hz, 1 H) 7.70 (t, J=6.11 Hz, 1 H) 7.42 (ddd, J=11.25, 9.29, 5.38 Hz, 1 H) 7.11 (td, J=8.80, 3.91 Hz, 1H) 7.03 (d, J=5.87 Hz, 1 H) 6.88 - 6.95 (m, 1 H) 6.20 (s, 2 H) 3.74 (d, J=1.47 Hz, 3 H) 3.08 - 3.21 (m, 2 H) 1.41 - 1.53 (m, 2 H) 0.81 (t, J=7.34 Hz, 3 H).LC-MS: m/z [M+1]⁺= 361

### Example 80

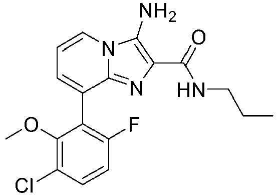

### 3-Amino-8-(2-fluoro-6-methoxyphenyl-5-chloro)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2-fluoro-6-methoxyphenyl-5-chloro)boronic acid (187 mg, 0.68 mmol) as starting materials to obtain the target product (20 mg, 16%) as a yellow solid.
¹H NMR (400 MHz, DMSO-*d₆*)8.22 (d, J=6.85 Hz, 1 H) 7.69 (s, 1 H) 7.63 (dd, J=8.80, 5.87 Hz, 1 H) 7.20 (t, J=8.80 Hz, 1 H) 7.06 (d, J=6.36 Hz, 1H) 6.89 - 6.97 (m, 1 H) 6.21 (s, 2 H) 3.58 (s, 3 H) 3.15 (d, J=7.34 Hz, 2 H) 1.41 - 1.51 (m, 2 H) 0.81 (t, J=7.58 Hz, 3 H). LC-MS: m/z [M+1]⁺= 377

### Example 81

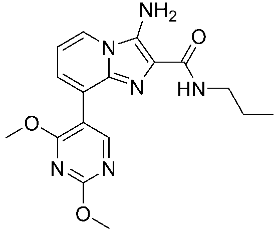

### 3-Amino-8-(2,4-dimethoxypyrimidin-5-yl)-N-propylimidazo[1,2-a]pyridine-2-carboxamide

The operation method was the same as that in the synthesis example of compound vii, using 3-amino-8-bromo-N-propylimidazo[ 1,2-a]pyridine-2-carboxamide (120 mg, 0.40 mmol) and 2,4-dimethoxypyrimidine-5-boronic acid (74 mg, 0.40 mmol) as starting materials to obtain the target product (108 mg, 76%) as a yellow solid.
¹H NMR (400 MHz, DMSO-*d*₆) 8.80 (s, 1 H) 8.13 (d, J=6.85 Hz, 1 H) 7.77 (s, 1 H) 7.22 (d, J=6.85 Hz, 1 H) 6.90 (t, J=7.09 Hz, 1 H) 6.15 (s, 2 H)3.97 (s, 3 H) 3.91 (s, 3 H) 3.12 - 3.22 (m, 2 H) 1.43 - 1.56 (m, 2 H) 0.84 (t, J=7.34 Hz, 3 H).LC-MS: m/z [M+1]⁺= 357

### Example 82

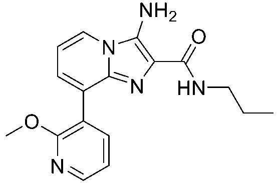

### 3-Amino-8-(2-methoxypyridin-3-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2-methoxypyridin-3-yl)boronic acid (52 mg, 0.34 mmol) as starting materials to obtain the target product (45 mg, 41%) as a yellow solid.
¹H NMR (400 MHz, DMSO-*d*₆) 8.22 (dd, J=4.89, 1.47 Hz, 1 H) 8.14 (d, J=6.36 Hz, 1 H) 8.05 (dd, J=7.34, 1.96 Hz, 1 H) 7.65 (s, 1 H) 7.07 - 7.20 (m,2 H) 6.90 (t, J=6.85 Hz, 1 H) 6.15 (s, 2 H) 3.83 (s, 3 H) 3.12 - 3.23 (m, 2 H) 1.41 - 1.54 (m, 2 H) 0.83 (t, J=7.34 Hz, 3 H).LC-MS: m/z [M+1]⁺=326

### Example 83

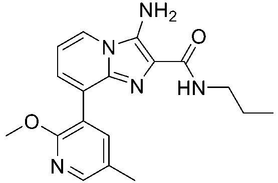

### 3-Amino-8-(2-methoxy-5-methylpyridin-3-yl)-N-propylimidazo[1,2-a]pyridine-2-carboxamide

### Step 1: 2-methoxy-5-methyl-3-(4,4,5,5-tetramethyl-1,3,2-dioxaborolan-2-yl)pyridine

3-Bromo-2-methoxy-5-methylpyridine (300 mg, 1.5 mmol), bis(pinacolato)diboron (455 mg, 1.8 mmol), potassium acetate (661 mg, 4.5 mmol), and [1,1'-bis(diphenylphosphino)ferrocene]dichloropalladium(II) (55 mg, 0.075 mmol) were sequentially added to N,N-dimethylformamide (5 mL). The mixture was stirred at 80°C for 16 hours under nitrogen atmosphere. It was purified by column chromatography (petroleum ether: ethyl acetate = 2:1) to obtain the title product as a white liquid (234 mg, yield 62.5%). LC-MS: m/z [M+H]⁺ =250.

**Step 2:** The operation method was the same as that in the synthesis example of compound vii, using 2-methoxy-5-methyl-3-(4,4,5,5-tetramethyl-1,3,2-dioxaborolan-2-yl)pyridine (50 mg, 0.2 mmol) and 3-amino-8-bromo-N-propylimidazo[1,2-a]pyridine-2-carboxamide (59 mg, 0.2 mmol) as starting materials to obtain the title product as a yellow solid (18 mg, yield 27%).

¹HNMR(400 MHz, DMSO-*d*₆) 8.18-8.12 (m, 1H), 8.04 (s, 1H), 7.82 (d, J = 2.2 Hz, 1H), 7.65-7.59 (m, 1H), 7.15-7.10 (m, 1H), 6.93-6.88 (m, 1H), 6.14 (s, 2H), 3.79 (s, 3H), 3.18 (dd, J = 13.6, 6.8 Hz, 2H), 2.29 (s, 3H), 1.49 (dd, J *=* 14.5, 7.3 Hz, 2H), 0.84 (t, J = 7.4 Hz, 3H) LC-MS: m/z [M+H]⁺ = 340.

### Example 84

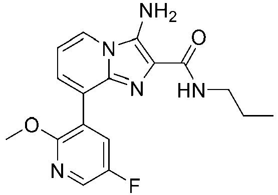

### 3-Amino-8-(5-fluoro-2-methoxypyridin-3-yl)-N-propylimidazo[1,2-a]pyridine-2-carboxamide

### Step 1: 5-fluoro-2-methoxy-3-(4,4,5,5-tetramethyl-1,3,2-dioxaborolan-2-yl)pyridine

The operation was the same as that in Example 83 (step 1), using 3-bromo-5-fluoro-2-methoxypyridine (2.0 g, 9.8 mmol) as a starting material to obtain a gray solid (2.0 g, 70%). LC-MS: m/z [M+H]⁺ =254

**Step 2:** The operation method was the same as that in the synthesis example of compound vii, using 5-fluoro-2-methoxy-3-(4,4,5,5-tetramethyl-1,3,2-dioxaborolan-2-yl)pyridine (64.8 mg, 0.26 mmol) and 3-amino-8-bromo-N-propylimidazo[1,2-a]pyridine-2-carboxamide (50 mg, 0.17 mmol) as starting materials to obtain the title compound (29 mg, 50%).
¹H NMR (400 MHz, DMSO) 8.22 (d, *J* = 3.0 Hz, 1H), 8.19 - 8.10 (m, 2H), 7.71 (t, *J* = 6.2 Hz, 1H), 7.27 (d, *J* = 6.3 Hz, 1H), 6.92 (t, *J* = 6.9 Hz, 1H), 6.17 (s, 2H), 3.84 (d, *J* = 7.8 Hz, 3H), 3.19 (dd, *J =* 14.0, 6.5 Hz, 2H), 1.49 (dd, *J* = 14.5, 7.3 Hz, 2H), 0.84 (t, *J =* 7.4 Hz, 3H). LC-MS: m/z [M+H]⁺ = 344

### Example 85

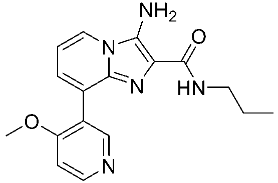

### 3-Amino-8-(4-methoxypyridin-3-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (4-methoxypyridin-3-yl)boronic acid (41 mg, 0.27 mmol) as starting materials to obtain the target product (39 mg, 44%) as a yellow solid.
¹H NMR (400 MHz, DMSO-*d*₆) 8.43 - 8.64 (m, 2 H) 8.15 (d, J=6.85 Hz, 1 H) 7.63 (br. s., 1 H) 7.17 - 7.28 (m, 1 H) 7.10 (d, J=6.36 Hz, 1 H) 6.85 -6.93 (m, 1 H) 6.15 (s, 2 H) 3.82 (s, 3 H) 3.17 (d, J=7.34 Hz, 2 H) 1.42 - 1.53 (m, 2 H) 0.82 (t, J=7.58 Hz, 3 H).LC-MS: m/z[M+1]⁺= 326

### Example 86

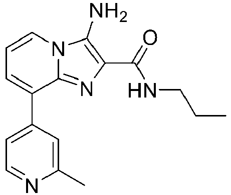

### 3-Amino-8-(2-methylpyridin-4-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and (2-methylpyridin-4-yl)boronic acid (55 mg, 0.41 mmol) as starting materials to obtain the target product (77 mg, 92%) as a yellow solid.
¹H NMR (400 MHz, DMSO-*d*₆) 8.53 (d, J=5.38 Hz, 1 H) 8.22 (d, J=6.85 Hz, 1 H) 7.97 - 8.12 (m, 2 H) 7.89 (t, J=5.87 Hz, 1 H) 7.52 (d, J=6.85 Hz, 1H) 6.97 (t, J=6.85 Hz, 1 H) 6.21 (s, 2 H) 3.20 - 3.28 (m, 2 H) 2.54 - 2.62 (m, 3 H) 1.47 - 1.62 (m, 2 H) 0.88 (t, J=7.34 Hz, 3 H).LC-MS: m/z [M+1]⁺= 310

### Example 87

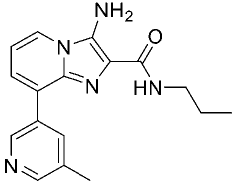

### 3-Amino-8-(5-methylpyridin-3-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (90 mg, 0.30 mmol) and (5-methylpyridin-3-yl)boronic acid (62 mg, 0.45 mmol) as starting materials to obtain the target product (68 mg, 72%) as a yellow solid.
¹H NMR (400 MHz, DMSO-*d*₆) 9.14 (br. s., 1 H) 8.46 (br. s., 1 H) 8.30 (br. s., 1 H) 8.18 (d, J=6.85 Hz, 1 H) 7.82 (t, J=5.62 Hz, 1 H) 7.39 (d, J=6.85Hz, 1 H) 6.95 (t, J=6.85 Hz, 1 H) 6.19 (s, 2 H) 3.23 (q, J=6.36 Hz, 2 H) 2.40 (s, 3 H) 1.42 - 1.63 (m, 2 H) 0.87 (t, J=7.34 Hz, 3 H).LC-MS: m/z [M+1]⁺= 310

### Example 88

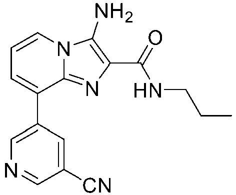

### 3-Amino-8-(5-cyanopyridin-3-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (90 mg, 0.30 mmol) and 5-cyano-3-pyridinylboronic acid (44 mg, 0.30 mmol) as starting materials to obtain the target product (66 mg, 62%) as a yellow solid.
¹H NMR (400 MHz, DMSO-*d*₆) 9.60 - 9.78 (m, 1 H) 9.05 (d, J=8.31 Hz, 2 H) 8.18 - 8.32 (m, 1 H) 7.90 - 8.07 (m, 1 H) 7.46 - 7.67 (m, 1 H) 6.91 -7.08 (m, 1 H) 6.24 (br. s., 2 H) 3.24 (d, J=5.38 Hz, 2 H) 1.44 - 1.61 (m, 2 H) 0.75 - 0.97 (m, 3 H).LC-MS: m/z [M+1]⁺= 321

### Example 89

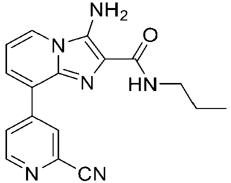

### 3-Amino-8-(2-cyanopyridin-4-yl)-N-propylimidazo[1,2-a]pyridine-2-carboxamide

The operation method was the same as that in the synthesis example of compound vii, using 4-(4,4,5,5-tetramethyl-1,3,2-dioxaborolan-2-yl)pyridinecarbonitrile (55 mg, 0.24 mmol) and 3-amino-8-bromo-N-propylimidazo[1,2-a]pyridine-2-carboxamide (71 mg, 0.24 mmol) as starting materials to obtain the title product as a yellow solid (4 mg, yield 5%).

¹HNMR (400 MHz, DMSO) 8.84 (d, J = 5.6 Hz, 3H), 8.31 (d, J = 6.9 Hz, 1H), 8.05 (t, J = 6.0 Hz, 1H), 7.78 (d, J = 7.0 Hz, 1H), 7.03 (t, J = 7.0 Hz, 1H), 6.26 (s, 2H), 3.26 (dd, J = 13.9, 6.6 Hz, 2H), 1.56 (dd, J = 14.5, 7.3 Hz, 2H), 0.90 (t, J = 7.4 Hz, 3H). LC-MS: m/z [M+H]⁺ = 321.

### Example 90

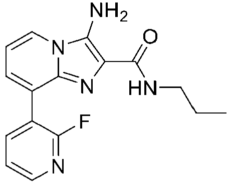

### 3-Amino-8-(2-fluoropyridin-3-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (100 mg, 0.34 mmol) and (2-methylpyridin-3-yl)boronic acid (96 mg, 0.68 mmol) as starting materials to obtain the target product (27 mg, 26%) as a white solid.

¹H NMR (400 MHz, DMSO-*d₆*)8.45 (br. s., 1 H) 8.16 - 8.36 (m, 1 H) 7.71 (br. s., 1 H) 7.50 (br. s., 1 H) 7.25 (d, J=5.38 Hz, 1 H) 6.96 (d, J=6.85 Hz,2 H) 6.20 (br. s., 1 H) 3.19 (d, J=6.85 Hz, 2 H) 1.49 (d, J=6.36 Hz, 2 H) 0.84 (br. s., 3 H).LC-MS: m/z [M+1]⁺= 314

### Example 91 (fused ring in region B)

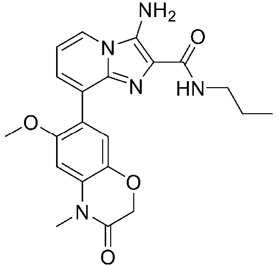

### 3-Amino-8-(6-methoxy-4-methyl-3-oxo-3,4-dihydro-2H-benzo[b][1,4]oxazin-7-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (80 mg, 0.27 mmol) and 6-methoxy-4-methyl-7-(tetramethyl-1,3,2-dioxaborolan-2-yl)-3,4-dihydro-2H-1,4-benzoxazin-3-one (86 mg, 0.27 mmol) as starting materials to obtain the target product (58 mg, 53%) as a yellow solid.
¹H NMR (400 MHz, DMSO-*d₆*) 8.06 - 8.12 (m, 1 H) 7.52 - 7.60 (m, 1 H) 7.15 (s, 1 H) 7.00 (s, 1 H) 6.92 (s, 1 H) 6.86 (s, 1 H) 6.11 (s, 2H) 4.63 (s, 2H) 3.76 (s, 3 H) 3.37 (br. s., 3 H) 3.18 (d, J=7.34 Hz, 2 H) 1.48 (d, J=6.85 Hz, 2 H) 0.83 (t, J=7.34 Hz, 3 H).LC-MS: m/z [M+1]⁺= 410

### Example 92

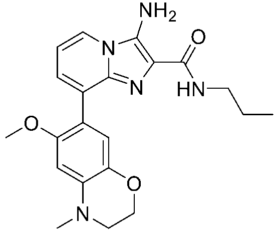

### 3-Amino-8-(6-methoxy-4-methyl-3,4-dihydro-2H-benzo[b][1,4]oxazin-7-yl)-N-propylimidazo[1,2-a]pyridine-2-carboxamide

### Step 1: 6-methoxy-4-methyl-7-(tetramethyl-1,3,2-dioxaborolan-2-yl)-3,4-dihydro-2H-1,4-benzoxazine

6-Methoxy-4-methyl-7-(tetramethyl-1,3,2-dioxaborolan-2-yl)-3,4-dihydro-2H-1,4-benzoxazin-3-one (600 mg, 1.88 mmol) was dissolved in 10 mL of 2M borane-tetrahydrofuran solution. The reaction mixture was sealed in a tube, heated to 80°C, and stirred for 16 hours. After cooling, the reaction mixture was concentrated, quenched by adding methanol, concentrated again, and purified by preparative TLC (petroleum ether:ethyl acetate = 10:1) to obtain the target product (310 mg, 52%).

**Step 2:** The operation method was the same as that in the synthesis example of compound vii, using 3-amino-8-bromo-N-propylimidazo[1,2-a]pyridine-2-carboxamide (300 mg, 1 mmol) and 6-methoxy-4-methyl-7-(tetramethyl-1,3,2-dioxaborolan-2-yl)-3,4-dihydro-2H-1,4-benzoxazine (310 mg, 1 mmol) as starting materials to obtain the target product (25 mg, 7%) as a yellow solid.
¹H NMR (400 MHz, DMSO-*d₆*) 7.94 - 8.07 (m, 1 H) 7.42 - 7.61 (m, 1 H) 6.91 - 6.99 (m, 1 H) 6.78 - 6.88 (m, 2 H) 6.46 (s, 1 H) 6.07 (s, 1 H) 4.19 (br.s., 2 H) 3.67 (s, 3 H) 3.27 (br. s., 2 H) 3.15 - 3.22 (m, 2 H) 2.92 (s, 3 H) 1.40 - 1.55 (m, 2 H) 0.84 (t, J=7.34 Hz, 3 H).LC-MS: m/z [M+1]⁺= 396

### Example 93

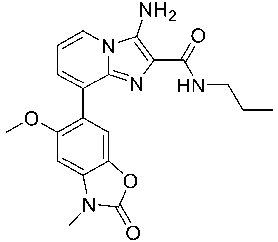

### 3-Amino-8-(5-methoxy-3-methyl-2-oxo-2,3-dihydrobenzo[d]oxazol-6-yl)-N-propylimidazo[1,2-a]pyridine-2-carboxamide

### Step 1: 5-methoxybenzo[d]azole-2(3H)-one

2-Amino-4-methoxyphenol (5.01 g, 35.93 mmol) was dissolved in anhydrous dichloromethane (100 mL), followed by the portion-wise addition of CDI (6.41 g, 39.53 mmol). The mixture was stirred at room temperature for 16 hours. After quenching with water (100 mL), the layers were separated, and the organic layer was collected. The aqueous phase was further extracted with dichloromethane (100 mL × 3) and dried in a rotary evaporator to obtain the crude product, which was purified by reverse-phase column chromatography (0.5% NH₃-H₂O to acetonitrile). The collected product was extracted with ethyl acetate (200 mL × 3), washed with saturated brine (100 mL), and dried over anhydrous sodium sulfate. After rotary evaporation, the resulting product was triturated with petroleum ether to obtain the title compound (3.41 g, white solid) in a yield of 57.5%.

### Step 2: 6-bromo-5-methoxybenzo[d]zol-2(3H)-one

To a solution of 5-methoxybenzo[*d*]oxazol-2(3*H*)-one (2.01 g, 12.12 mmol) in glacial acetic acid (20.0 mL) was added hydrobromic acid (33% in acetic acid) (7.43 g, 30.30 mmol), and the mixture was stirred at room temperature for 30 minutes. The reaction mixture was then cooled to 10°C, and hydrogen peroxide (30% concentration) (1.79 g, 15.76 mmol) was added dropwise slowly, ensuring the temperature did not exceed 10°C. The mixture was subsequently warmed to room temperature and reacted for 2 hours. The reaction mixture was poured into 100 mL of water, stirred for 30 minutes, and filtered. The filter cake was washed twice with water, collected, and dried under vacuum to obtain the title product (1.80 g, white solid) in a yield of 60.8%.

### Step 3: 6-bromo-5-methoxy-3-methylbenzo[d]zol-2(3H)-one

6-Bromo-5-methoxybenzo[*d*]oxazol-2(3*H*)-one (1.01 g, 4.10 mmol) was dissolved in a solution of DMSO (20.0 mL). At room temperature, iodomethane (7.66 mL, 123.01 mmol) and potassium carbonate (1.13 g, 8.20 mmol) were added, and the mixture was reacted at room temperature for 16 hours. After quenching with water (100 mL), the mixture was extracted with ethyl acetate (50 mL × 3), washed with saturated brine (50 mL), and dried in a rotary evaporator to obtain the crude product, which was purified by reverse-phase column chromatography (0.5% TFA to acetonitrile) to obtain the title product (510.1 mg, pink solid) in a yield of 48.2%.

### Step 4: 5-methoxy-3-methyl-6-(tetramethyl-1,3,2-dioxaborolan-2-yl)-2,3-dihydro-1,3-benzoxazol-2-one

6-Bromo-5-methoxy-3-methylbenzo[*d*]zol-2(3*H*)-one (110 mg, 0.43 mmol), bis(pinacolato)diboron (220 mg, 0.86 mmol), potassium acetate (130 mg, 1.29 mmol), triethylamine (130 mg, 1.29 mmol), 2-dicyclohexylphosphino-2',4',6'-triisopropylbiphenyl (CAS No.: 564483-18-7, 20 mg, 0.04 mmol), and bis(dibenzylideneacetone)dipalladium (CAS No.: 51364-51-3, 39 mg, 0.04 mmol) were dissolved in 5 mL of dioxane. The mixture was heated to 100°C and stirred for 16 hours. The organic phase was purified by preparative TLC (petroleum ether:ethyl acetate = 10:1) to obtain the target product (90 mg, white solid) in a yield of 77%.

**Step 5:** The operation method was the same as that in the synthesis example of compound vii, using 3-amino-8-bromo-N-propylimidazo[1,2-a]pyridine-2-carboxamide (80 mg, 0.27 mmol) and 5-methoxy-3-methyl-6-(tetramethyl-1,3,2-dioxaborolan-2-yl)-2,3-dihydro-1,3-benzoxazol-2-one (82 mg, 0.27 mmol) as starting materials to obtain the target product (18 mg, 17%) as a yellow solid.

¹H NMR (400 MHz, DMSO-*d₆*) 8.07 - 8.14 (m, 1 H) 7.57 - 7.63 (m, 1 H) 7.45 - 7.51 (m, 1 H) 7.16 - 7.21 (m, 1 H) 7.00 - 7.05 (m, 1 H) 6.85 - 6.91(m, 1 H) 6.08 - 6.15 (m, 2 H) 3.77 (s, 3 H) 3.41 (s, 3 H) 3.14 - 3.21 (m, 2 H) 1.43 - 1.51 (m, 2 H) 0.82 (s, 2 H).LC-MS: m/z[M+1]⁺=396.

### Example 94

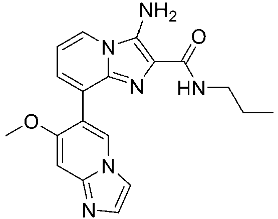

### 3'-Amino-7-methoxy-N-propyl-[6,8'-diimidazo[1,2-a]pyridine]-2'-carboxamide

### Step 1: 5-bromo-4-methoxy-2-aminopyridine

4-Methoxypyridin-2-amine (3 g, 24.2 mmol) was added to 30 mL of DMF, followed by portionwise addition of NBS (5.2 g, 29 mmol). The mixture was stirred at room temperature for 3 hours. The reaction mixture was added to 200 mL of water, and a red solid precipitated. The solid was filtered and dried to obtain 3 g of the title product in a yield of 71%. LC-MS: m/z [M+H]⁺ =203.

### Step 2: 6-bromo-7-methoxyimidazo[1,2-a]pyridine

5-Bromo-4-methoxy-2-aminopyridine (1 g, 4.95 mmol), chloroacetaldehyde (465 mg, 5.9 mmol), and sodium carbonate (1.24 g, 11.7 mmol) were added to a mixture of 16 mL of ethanol and 4 mL of water. The mixture was stirred at 80°C for 16 hours. The reaction mixture was added to 20 mL of water, extracted with ethyl acetate (30 mL × 3), concentrated, and purified by column chromatography (petroleum ether:ethyl acetate = 3:1) to obtain the title product (395 mg, brown solid) in a yield of 38%. LC-MS: m/z [M+H]⁺ =227.

### Step 3: 7-methoxy-6-(4,4,5,5-tetramethyl-1,3,2-dioxaborolan-2-yl)imidazo[1,2-a]pyridine

Under nitrogen atmosphere, 6-bromo-7-methoxyimidazo[1,2-a]pyridine (300 mg, 1.3 mmol), bis(pinacolato)diboron (363 mg, 1.43 mmol), potassium acetate (386 mg, 3.9 mmol), and [1,1'-bis(diphenylphosphino)ferrocene]dichloropalladium(II) dichloromethane complex (95 mg, 0.1 mmol) were sequentially added to 1,4-dioxane (5 mL). The mixture was stirred at 130°C under microwave irradiation for 2 hours. Water (20 mL) was added. The mixture was extracted with ethyl acetate (30 mL × 3) and concentrated to obtain the title compound (260 mg, crude). LC-MS: m/z [M+H]⁺ =275.

**Step 4:** The operation method was the same as that in the synthesis example of compound vii, using 7-methoxy-6-(4,4,5,5-tetramethyl-1,3,2-dioxaborolan-2-yl)imidazo[1,2-a]pyridine (260 mg crude, 0.95 mmol) and 3-amino-8-bromo-N-propylimidazo[1,2-a]pyridine-2-carboxamide (100 mg, 0.34 mmol) as starting materials to obtain the title product (4 mg, yellow solid) in a yield of 3%.

¹H NMR (400 MHz, DMSO) δ 8.72 (s, 1H), 8.16 (d, *J* = 6.4 Hz, 1H), 7.80 (s, 1H), 7.75 (t, *J =* 5.8 Hz, 1H), 7.45 (s, 1H), 7.14 (d, *J =* 6.6 Hz, 1H), 7.08 (s, 1H), 6.91 (t, *J =* 6.8 Hz, 1H), 6.15 (s, 2H), 3.78 (s, 3H), 3.16 (dd, *J =* 13.2, 6.4 Hz, 2H), 1.47 (dd, *J =* 14.4, 7.2 Hz, 2H), 0.82 (t, *J =* 7.3 Hz, 3H). LC-MS: m/z [M+H]⁺ =365.

### Example 95

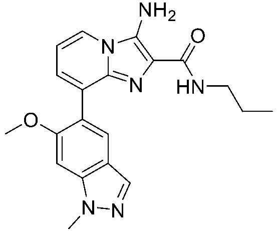

### 3-Amino-8-(6-methoxy-1-methyl-1H-indazol-5-yl)-N-propylimidazo[1,2-a]pyridine-2-carboxamide

### Step 1: 5-bromo-6-methoxy-1-methyl-1H-indazole (and 5-bromo-6-methoxy-2-methyl-2H-indazole)

5-Bromo-6-methoxy-1H-indazole (350 mg, 1.54 mmol) was dispersed in DMF (10 mL), followed by addition of sodium hydride (74.3 mg, 1.54 mmol). The mixture was stirred for 0.5 h, and then methyl iodide (440 mg, 3.09 mmol) was added. The mixture was stirred at room temperature for 5 hours. It was filtered, concentrated, and purified by column chromatography to obtain the target products, 5-bromo-6-methoxy-1-methyl-1H-indazole (95 mg) and 5-bromo-6-methoxy-2-methyl-2H-indazole (110 mg), with a total yield of 55%. LC-MS: m/z [M+H]⁺ =241

### Step 2: 6-methoxy-1-methyl-5-(4,4,5,5-tetramethyl-1,3,2-dioxaborolan-2-yl)-1H-indazole

The experimental operation was the same as that in Example 94 (step 3), using 5-bromo-6-methoxy-1-methyl-1H-indazole (50 mg, 0.21 mmol) as a starting material to obtain a white powder (35 mg, 58%). LC-MS: m/z [M+1] =289

**Step 3:** The operation method was the same as that in the synthesis example of compound vii, using 6-methoxy-1-methyl-5-(4,4,5,5-tetramethyl-1,3,2-dioxaborolan-2-yl)-1H-indazole (35 mg, 0.12 mmol) and 3-amino-8-bromo-N-propylimidazo[1,2-a]pyridine-2-carboxamide (36 mg, 0.12 mmol) as starting materials to obtain the target product (20 mg, 44%).
¹H NMR (400 MHz, DMSO-*d6*) 8.11 (d, J = 6.8 Hz, 1H), 7.96 (s, 1H), 7.74 (s, 1H), 7.55 (s, 1H), 7.24 (s, 1H), 6.99 (d, J = 6.7 Hz, 1H), 6.88 (s, 1H), 6.11 (s, 2H), 4.05 (s, 3H), 3.80 (s, 3H), 3.21 - 3.09 (m, 2H), 1.45 (d, J = 7.1 Hz, 2H), 0.81 (t, J = 7.4 Hz, 3H). LC-MS: m/z [M+1] =379

### Example 96

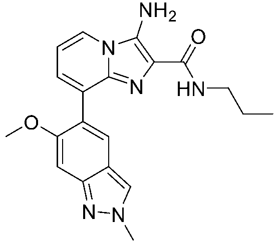

### 3-Amino-8-(6-methoxy-2-methyl-2H-indazol-5-yl)-N-propylimidazo[1,2-a]pyridine-2-carboxamide

### Step 1: 6-methoxy-2-methyl-5-(4,4,5,5-tetramethyl-1,3,2-dioxaborolan-2-yl)-2H-indazole

The experimental operation was the same as that in Example 94 (step 3), using 5-bromo-6-methoxy-2-methyl-2H-indazole (100 mg, 0.42 mmol) as a starting material to obtain a pale white powder (60 mg, 49.6%). LC-MS: m/z [M+1]⁺ =289

**Step 2:** The operation method was the same as that in the synthesis example of compound vii, using 6-methoxy-2-methyl-5-(4,4,5,5-tetramethyl-1,3,2-dioxaborolan-2-yl)-2H-indazole (50 mg, 0.17 mmol) and 3-amino-8-bromo-N-propylimidazo[1,2-a]pyridine-2-carboxamide (51 mg, 0.17 mmol) as starting materials to obtain the target compound (20 mg, 31%).
¹H NMR (400 MHz, DMSO-*d₆*) 8.25 (s, 1H), 8.10 (d, J = 6.1 Hz, 1H), 7.71 (s, 1H), 7.53 (t, J = 6.2 Hz, 1H), 7.04 (s, 1H), 6.98 (d ,J = 6.7 Hz, 1H), 6.87 (t, J = 6.9 Hz, 1H), 6.10 (s, 2H), 4.12 (s, 3H), 3.73 (d, J = 7.9 Hz, 3H), 3.14 (d, J = 19.4, 9.7 Hz, 2H), 1.55 - 1.38 (m, 2H), 0.89 - 0.71 (m, 3H). LC-MS: m/z [M+1]⁺ = 379

### Example 97

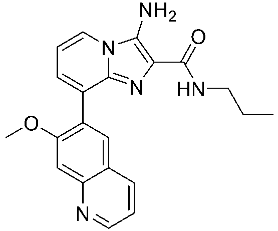

### 3-Amino-8-(7-methoxyquinolin-6-yl)-N-propylimidazo[1,2-a]pyridine-2-carboxamide

### Step 1: 7-methoxy-6-(4,4,5,5-tetramethyl-1,3,2-dioxaborolan-2-yl)quinoline

The experimental operation was the same as that in Example 94 (step 3), using 6-bromo-7-methoxyquinoline (200 mg, 0.84 mmol) as a starting material to obtain the title compound as a yellow solid (220 mg, 92%). LC-MS: m/z [M+H]⁺=286

**Step 2:** The operation method was the same as that in the synthesis example of compound vii, using 7-methoxy-6-(4,4,5,5-tetramethyl-1,3,2-dioxaborolan-2-yl)quinoline (200 mg, 0.7 mmol) and 3-amino-8-bromo-N-propylimidazo[1,2-a]pyridine-2-carboxamide (208 mg, 0.7 mmol) as starting materials to obtain the title compound as a yellow solid (100 mg, 35%).
¹H NMR (400 MHz, CHLOROFORM-d) 0.93 (t, J=7.34 Hz, 3 H) 1.58 (d, J=7.34 Hz, 2 H) 3.34 (d, J=6.85 Hz, 2 H) 3.97 (s, 3 H) 4.97 - 5.10 (m, 2 H)6.88 - 6.98 (m, 1 H) 7.36 - 7.49 (m, 2 H) 7.76 - 7.88 (m, 2 H) 8.15 (br. s., 1 H) 8.25 - 8.36 (m, 1 H) 8.83 - 8.93 (m, 1 H). LC-MS: m/z [M+H]⁺= 376

### Example 98

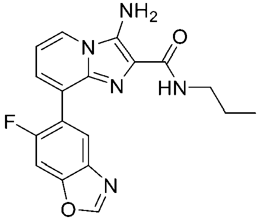

### 3-Amino-8-(6-fluorobenzo[d]azol-5-yl)-N-propylimidazo[1,2-a]pyridine-2-carboxamide

### Step 1: 2-amino-4-bromo-5-fluorophenol

Tin(II) chloride (3612 mg, 19.05 mmol) was added to a solution of 4-bromo-5-fluoro-2-nitrophenol (900 mg, 3.81 mmol) in ethanol (25 mL), and the mixture was heated to reflux and stirred for 2 hours. The reaction mixture was concentrated, and the residue was added to an aqueous sodium bicarbonate solution. The pH of the solution was adjusted to 8. The mixture was extracted with ethyl acetate (100 mL × 3). The organic phases were combined, dried over anhydrous sodium sulfate, filtered, concentrated, and purified by column chromatography to obtain the target product as a pale yellow solid (500 mg, 64%). LCMS: m/z [M+H]⁺= 206

### Step 2: 5-Bromo-6-fluorobenzo[d]oxazole

2-Amino-4-bromo-5-fluorophenol (300 mg, 1.46 mmol) and p-toluenesulfonic acid (251 mg, 1.46 mmol) were dissolved in trimethyl orthoformate (2 mL). The mixture was stirred at 90°C for 2 hours. The reaction mixture was concentrated and purified by column chromatography (petroleum ether:ethyl acetate = 30:1 to 10:1) to obtain the target product as a pale yellow solid (300 mg, 95%). LCMS: m/z [M+H]⁺= 216

### Step 3: 6-fluoro-5-(4,4,5,5-tetramethyl-1,3,2-diboran-2-yl)benzo[d]oxazole

5-Bromo-6-fluorobenzo[d]oxazole (60 mg, 0.28 mmol), bis(pinacolato)diboron (424 mg, 1.67 mmol), potassium acetate (273 mg, 2.78 mmol), and [1,1'-bis(diphenylphosphino)ferrocene]dichloropalladium(II) complex with dichloromethane (114 mg, 0.14 mmol) were sequentially added to 1,4-dioxane (12 mL). The mixture was stirred at 100°C for 16 hours under argon atmosphere. The reaction mixture was directly used in the next step.

**Step 4:** To the reaction mixture from the previous step were added 3-amino-8-bromo-N-propylimidazo[1,2-a]pyridine-2-carboxamide (100 mg, 0.34 mmol), cesium carbonate (371 mg, 1.14 mmol), [1,1'-bis(diphenylphosphino)ferrocene]dichloropalladium(II) dichloromethane complex (31 mg, 0.038 mmol), and water (2.5 mL). The mixture was stirred at 100°C for 5 hours. The reaction mixture was filtered through diatomite, and the resulting filtrate was concentrated, and purified by column chromatography to obtain the target product (38 mg, yield of 38% over two steps).
¹H NMR (400MHz, CHLOROFORM-d) 8.15 -8.02 (m, 3 H), 7.45 (d, J = 8.8 Hz, 1 H), 7.30 (br.s., 1 H), 6.97 (br. s., 1 H), 3.36-3.32 (m, 2 H), 1.68 - 1.50 (m, 2 H), 0.94 (t, J = 7.3 Hz, 3 H). LCMS: m/z [M+H]⁺= 354

### Example 99

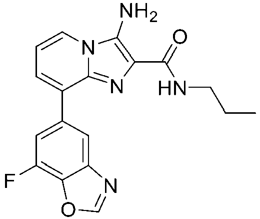

### 3-Amino-8-(7-fluorobenzo[d]azol-5-yl)-N-propylimidazo[1,2-a]pyridine-2-carboxamide

### Step 1: 2-amino-4-bromo-6-fluorophenol

Tin(II) chloride (8.03 g, 42.35 mmol) was added to a solution of 4-bromo-2-fluoro-6-nitrophenol (2 g, 8.47 mmol) in ethanol (60 mL). The reaction mixture was heated to reflux and stirred for 2 hours. The reaction mixture was then concentrated and added to an aqueous sodium bicarbonate solution. The pH of the solution was adjusted to 8. The mixture was extracted with ethyl acetate (50 mL × 3), and the organic phases were combined, dried over anhydrous sodium sulfate, and filtered. The filtrate was concentrated and purified by column chromatography to obtain a light yellow solid (800 mg, 46%). LCMS: m/z [M+H]⁺= 206

### Step 2: 5-Bromo-7-fluorobenzo[d]oxazole

2-Amino-4-bromo-6-fluorophenol (300 mg, 1.46 mmol) and p-toluenesulfonic acid (251 mg, 1.46 mmol) were dissolved in trimethyl orthoformate (6 mL). The mixture was stirred at 120°C for 3 hours. The reaction solution was concentrated, and an aqueous sodium bicarbonate solution was added to adjust the pH to 8. The mixture was extracted with ethyl acetate (50 mL × 3), and the organic phases were combined, dried over anhydrous sodium sulfate, filtered, concentrated, and purified by column chromatography to obtain the target product as a pale yellow solid (60 mg, 19%). LCMS: m/z [M+H]⁺= 216

### Step 3: 7-fluoro-5-(4,4,5,5-tetramethyl-1,3,2-dioxaborolan-2-yl)benzo[d]oxazole

5-Bromo-7-fluorobenzo[d]zole (60 mg, 0.28 mmol), bis(pinacolato)diboron (85 mg, 0.34 mmol), potassium acetate (55 mg, 0.56 mmol), and [1,1'-bis(diphenylphosphino)ferrocene]dichloropalladium(II) dichloromethane complex (23 mg, 0.028 mmol) were sequentially added to 1,4-dioxane (4 mL). The mixture was stirred at 100°C for 16 hours under argon atmosphere. The reaction mixture was directly used in the next step.

**Step 4:** To the reaction mixture from the previous step were added 3-amino-8-bromo-N-propylimidazo[1,2-a]pyridine-2-carboxamide (62 mg, 0.21 mmol), cesium carbonate (186 mg, 0.57 mmol), [1,1'-bis(diphenylphosphino)ferrocene]dichloropalladium(II) dichloromethane complex (16 mg, 0.019 mmol), and water (0.5mL). The mixture was stirred at 100°C for 5 hours. The reaction mixture was filtered, concentrated, and purified by column chromatography to obtain a pale yellow solid (12 mg, yield of 16% over two steps).
¹H NMR (400MHz, CHLOROFORM-d) 8.29(s, 1H), 8.19(s, 1H), 7.90(d, J = 10.8 Hz, 1H), 7.78(d, J = 6.4 Hz, 1H), 7.32-7.25 (m, 2H), 6.98-6.87(m, 1 H), 5.22-4.81(m, 1H), 3.55-3.31(m, 2H), 1.70-1.63(m, 2H), 0.99(t, J = 7.3 Hz, 3H). LCMS: m/z [M+H]⁺= 354

### Example 100

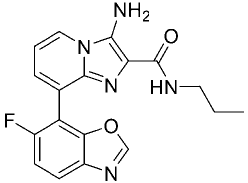

### 3-Amino-8-(6-fluorobenzoxazol-7-yl)-N-propylimidazopyridine-2-carboxamide

### Step 1: 6-amino-2-bromo-3-fluorophenol

2-Bromo-3-fluoro-6-nitrophenol (1 g, 4.25 mmol) and raney nickel (0.5 g) were added to 10 mL of methanol, and the mixture was stirred under a hydrogen atmosphere at room temperature for 16 hours. The mixture was filtered, and the filtrate was concentrated, purified by column chromatography (petroleum ether:ethyl acetate = 2:1) to obtain the title product (330 mg, yield 34%). LC-MS: m/z [M+H]⁺ =206.

### Step 2: 7-bromo-6-fluorobenzoxazole

Under nitrogen atmosphere, 6-amino-2-bromo-3-fluorophenol (300 mg, 1.46 mmol) and p-toluenesulfonic acid (25 mg, 0.146 mmol) were added to trimethyl orthoformate (5 ml). The mixture was stirred at 80°C for 3 hours. It was concentrated and purified by column chromatography (petroleum ether: ethyl acetate = 3:1) to obtain the title product as a pale yellow solid (270 g, yield 86%). LC-MS: m/z [M+H]⁺ =216.

### Step 3: 3-amino-N-propyl-8-(trimethylstannyl)imidazo[1,2-a]pyridine-2-carboxamide

3-Amino-N-propyl-8-bromoimidazo[1,2-a]pyridine-2-carboxamide (100 mg, 0.34 mmol), hexamethylditin (163 mg, 0.5 mmol), and tetrakis(triphenylphosphine)palladium (20 mg) were sequentially added to dioxane (3 mL). The mixture was stirred at 80°C for 16 hours. It was purified by column chromatography to obtain the title compound as a cyan solid (68 mg, 52%). LCMS: m/z [M+H]⁺ = 383

**Step 4:** 7-Bromo-6-fluorobenzoxazole (40 mg, 0.19 mmol), 3-amino-N-propyl-8-(trimethylstannyl)imidazo[1,2-a]pyridine-2-carboxamide (68 mg, 0.18 mmol), and bis(triphenylphosphine)palladium(II) dichloride (CAS No.: 13965-03-2, 20 mg) were sequentially added to dioxane (5 ml). The mixture was stirred at 100°C for 16 hours. The mixture was filtered to obtain the target compound as a yellow solid (5 mg, yield 7%).
¹HNMR (400 MHz, CDCl₃) δ 8.07 (s, 1H), 7.83 (dd, J = 8.7, 4.4 Hz, 2H), 7.35 - 7.28 (m, 2H), 7.01 (d, J = 37.2 Hz, 2H), 4.97 (s, 2H), 3.34 (dd, J = 13.8, 6.7 Hz, 2H), 1.26 (s, 2H), 0.93 (t, J = 7.4 Hz, 3H). LC-MS: m/z [M+H]⁺= 354

### Example 101

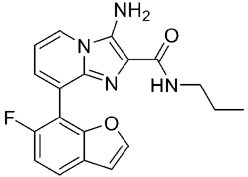

### 3-Amino-8-(6-fluorobenzofuran-7-yl)-N-propylimidazo[1,2-a]pyridine-2-carboxamide

### Step 1: 2-bromo-1-(2,2-dimethoxyethoxy)-3-fluorobenzene

2-Bromo-3-fluorophenol (3 g, 15.7 mmol), 2-bromo-1,1-dimethoxyethane (2.66 g, 15.7 mmol), and potassium carbonate (4.34 g, 31.4 mmol) were sequentially added to N,N-dimethylformamide (30 mL). The mixture was stirred at 135°C for 1 hour. To a mixture was added 150 ml of water. The mixture was extracted with ethyl acetate (150 ml × 2) and concentrated to obtain 2 g of the product (45%, yellow solid).

### Step 2: 7-bromo-6-fluorobenzofuran

2-Bromo-1-(2,2-dimethoxyethoxy)-3-fluorobenzene (1 g, 3.58 mmol) and polyphosphoric acid (1.22 g, 3.58 mmol) were dissolved in toluene (20 mL). The mixture was stirred at 130°C for 16 hours. The mixture was cooled to room temperature, added with 100 ml of water, extracted with ethyl acetate (100 ml × 2), concentrated, and purified by column chromatography (petroleum ether) to obtain 0.35 g of the product (45%, yellow oil).

### Step 3: (6-fluorobenzofuran-7-yl)boronic acid

Under nitrogen atmosphere and at -70°C, n-butyllithium (0.61 g, 9.6 mmol) was added dropwise to a solution of 7-bromo-6-fluoro-1-benzofuran (0.215 g, 1.0 mmol) in tetrahydrofuran (10 mL). After completion of the addition, the mixture was stirred at this temperature for 1 hour. Trimethyl borate (0.16 g, 1.5 mmol) was added to the mixture, and it was stirred for 30 minutes. To the reaction mixture was added an aqueous solution of sodium hydroxide (2 N, 2 mL), and the pH was adjusted to 2 with a hydrochloric acid solution (1 M). The mixture was extracted with ethyl acetate (50 mL × 2) and concentrated to obtain the 110 mg of the product (61%, yellow solid).

**Step 4:** The operation method was the same as that in the synthesis example of compound vii, using (6-fluorobenzofuran-7-yl)boronic acid (30 mg, 0.17 mmol) and 3-amino-8-bromo-N-propylimidazo[1,2-a]pyridine-2-carboxamide (49 mg, 0.17 mmol) as starting materials to obtain the title product (30 mg, 46%) as a pale yellow solid.
¹H NMR (400 MHz, DMSO-*d6*) 8.26 (dd, J = 7.0, 0.9 Hz, 1H), 7.96 (d, J = 2.2 Hz, 1H), 7.74 (dd, J = 8.6, 5.2 Hz, 1H), 7.54 (t, J = 6.2 Hz, 1H), 7.29 (dd, J = 10.1, 8.6 Hz, 1H), 7.18 (d, J = 6.0 Hz, 1H), 7.04 (d, J = 2.2 Hz, 1H), 6.96 (d, J = 6.9 Hz, 1H), 6.21 (s, 2H), 3.15 - 3.03 (m, 2H), 1.53 - 1.36 (m, 2H), 0.80 (t, J = 7.4 Hz, 3H). LCMS MS m/z (ESI): 353 [M+1]⁺

### Example 102

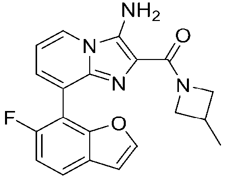

### (3-Amino-8-(6-fluorobenzofuran-7-yl)imidazo[1,2-a]pyridin-2-yl)(3-methylazetidin-1-yl)methanone

### Step 1: (3-amino-8-bromoimidazo[1,2-a]pyridin-2-yl)(3-methylazetidin-1-yl)methanone

The operation method was the same as that in the synthesis of intermediate vi (route 1, step 2), using 3-amino-8-bromoimidazo[1,2-a]pyridine-2-carboxylic acid (100 mg, 0.39 mmol) and 3-methylazetidine (41 mg, 0.59 mmol) as starting materials to obtain the target product (105 mg, crude) as a yellow-green solid

Step 2: The operation method was the same as that in the synthesis example of compound vii, using (3-amino-8-bromoimidazo[1,2-a]pyridin-2-yl)(3-methylazetidin-1-yl)methanone (0.15 g, 0.49 mmol) and (6-fluorobenzofuran-7-yl)boronic acid (0.088 g, 0.49 mmol) as starting materials to obtain the product (20 mg, 11%) as a yellow solid.
¹HNMR (400MHz, DMSO-d6) 8.25 (d, J = 6.4 Hz, 1 H), 7.98 (s, 1 H), 7.73 (s, 1 H), 7.33 - 7.20 (m, 2 H), 7.03 (s, 1 H), 6.98 (d, J = 5.9 Hz, 1 H), 6.26 (s, 2 H), 4.37-4.33 (m, 1 H), 4.08-4.04 (m, 1 H), 3.79-3.75 (m, 1 H), 3.52-3.48 (m, 1 H), 2.61-3.57 (m, 1 H), 1.11 (d, J = 5.9 Hz, 3 H). LC-MS: m/z [M+1]⁺= 365

### Example 103

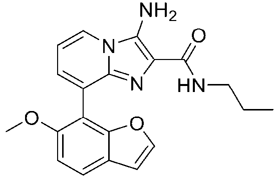

### 3-Amino-8-(6-methoxybenzofuran-7-yl)-N-propylimidazo[1,2-a]pyridine-2-carboxamide

### Step 1: 2-bromo-1-(2,2-dimethoxyethoxy)-3-methoxybenzene

2-Bromo-3-methoxyphenol (2 g, 10.47 mmol), 2-bromo-1,1-dimethoxyethane (1.95 g, 11.52 mmol), and cesium carbonate (6.82 g, 20.94 mmol) were dissolved in DMF (20 mL). The mixture was stirred at 100°C for 15 hours. The reaction mixture was diluted with ethyl acetate (50 mL), washed with saturated aqueous sodium chloride solution (20 mL × 3), and the organic phase was dried and concentrated to obtain the title product (2 g, 68%) as a yellow oil.

### Step 2: 7-bromo-6-methoxybenzofuran

2-Bromo-1-(2,2-dimethoxyethoxy)-3-methoxybenzene (500 mg, 1.72 mmol) and polyphosphoric acid (5 g) were added to toluene (100 mL). The mixture was stirred at 120°C for 15 hours. The mixture was concentrated, diluted with ethyl acetate (50 mL), and washed with saturated aqueous sodium chloride solution (30 mL × 3). The organic phase was dried, concentrated, and purified by column chromatography to obtain the title product (400 mg, crude) as a yellow oil.

### Step 3: (6-methoxybenzofuran-7-yl)boronic acid

7-Bromo-6-methoxybenzofuran (100 mg, 0.44 mmol) was dissolved in anhydrous tetrahydrofuran (4 mL) and freezed to -78°C, and n-butyllithium (0.33 mL, 0.53 mmol) was added dropwise slowly. The mixture was stirred at -78°C for 30 minutes. A solution of triisopropyl borate (124 mg, 0.66 mmol) in tetrahydrofuran (3 mL) was added dropwise slowly and the mixture was stirred at -78°C for 1 hour. The reaction mixture was quenched with a saturated aqueous ammonium chloride solution (20 mL) and extracted with ethyl acetate (30 mL × 3). The organic phase was dried, concentrated, and purified by column chromatography to obtain the title product (45 mg, 53%) as a yellow oil.

**Step 4:** The operation method was the same as that in the synthesis example of compound vii, using (6-methoxybenzofuran-7-yl)boronic acid (30 mg, 0.17 mmol) and 3-amino-8-bromo-N-propylimidazo[1,2-a]pyridine-2-carboxamide (49 mg, 0.17 mmol) as starting materials to obtain the title product (30 mg, 46%).
¹H NMR (400 MHz, DMSO-*d₆*) 8.44 (s, 1H), 7.84 (s, 1H), 7.75-7.71 (m, 2H), 7.22 (d, J = 8.6 Hz, 2H), 6.97 (s, 1H), 3.79 (s, 3H), 3.20-3.16 (m, 2H), 1.46 (dd, J = 14.4, 7.2 Hz, 2H), 0.83 (t, J = 7.3 Hz, 3H). LCMS MS m/z (ESI): 365 [M+1]

### Example 104

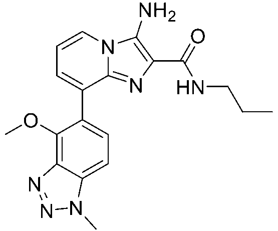

### 3-Amino-8-(4-methoxy-1-methyl-1H-benzo[d][1,2,3]triazol-5-yl)-N-propylimidazo[1,2-a]pyridine-2-carboxamide

### Step 1: 4-bromo-3-methoxy-N-methyl-2-nitroaniline

3-Methoxy-N-methyl-2-nitroaniline (700 mg, 3.8 mmol) was dissolved in acetonitrile (40 mL), followed by the addition of NBS (720 mg, 4.0 mmol). The mixture was stirred at room temperature for 15 minutes. The reaction mixture was directly concentrated and purified by column chromatography to obtain the title compound (914 mg, yield 91%) as a yellow oily liquid. LC-MS: m/z [M+H]⁺ =261

### Step 2: 4-bromo-3-methoxy-N¹-methylbenzene-1,2-diamine

4-Bromo-3-methoxy-N-methyl-2-nitroaniline (914 mg, 3.5 mmol) was dissolved in methanol (30 mL), followed by the addition of Raney nickel (250 mg). The atmosphere in the system was replaced three times using a hydrogen balloon, and the mixture was stirred at room temperature for 2 hours under hydrogen atmosphere. The mixture was filtered, and the filtrate was concentrated to obtain the title compound (696 mg, yield 86%) as a yellow solid. LC-MS: m/z [M+H]⁺ =231

### Step 3: 5-bromo-4-methoxy-1-methyl-1H-benzo[d][1,2,3]triazole

At 0°C, a solution of sodium nitrite in water (1.2 M, 5 mL) was added dropwise to a solution of 4-bromo-3-methoxy-N¹-methylbenzene-1,2-diamine in acetic acid (0.5 M, 6 mL). The mixture was maintained at 0°C and stirred for 30 minutes. The reaction mixture was concentrated and purified by column chromatography to obtain the title compound (365 mg, yield 50%) as a yellow solid. LC-MS: m/z [M+H]⁺= 242

### Step 4: 4-methoxy-1-methyl-5-(4,4,5,5-tetramethyl-1,3,2-dioxaborolan-2-yl)-1H-benzo[d][1,2,3]triazole

The experimental operation was the same as that in Example 94 (Step 3), using 5-bromo-4-methoxy-1-methyl-1H-benzo[d][1,2,3]triazole (100 mg, 0.41 mmol) as a starting material to obtain the title compound (50 mg, 42%). LC-MS: m/z [M+H]⁺ =290

**Step 5:** The operation method was the same as that in the synthesis example of compound vii, using 4-methoxy-1-methyl-5-(4,4,5,5-tetramethyl-1,3,2-dioxaborolan-2-yl)-1H-benzo[d][1,2,3]triazole (50.0 mg, 0.17 mmol) and 3-amino-8-bromo-N-propylimidazo[1,2-a]pyridine-2-carboxamide (50 mg, 0.17 mmol) as starting materials to obtain the title product (30 mg, 46%).
¹H NMR (400 MHz, DMSO) 8.14 (d, J = 6.8 Hz, 1H), 7.66 - 7.45 (m, 3H), 7.06 (d, J = 6.8 Hz, 1H), 6.91 (t, J = 6.9 Hz, 1H), 6.13 (s, 2H), 4.37 (s, 3H), 4.33 (s, 3H), 3.15 (dd, J = 13.9, 6.9 Hz, 2H), 1.45 (dd, J = 14.3, 7.0 Hz, 2H), 0.80 (t, J = 7.4 Hz, 3H). LCMS MS m/z (ESI): 380 [M+1]

### Example 105

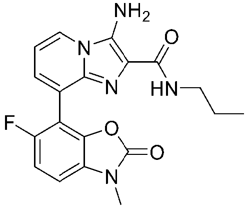

### 3-Amino-8-(6-fluoro-3-methyl-2-oxo-2,3-dihydrobenzo[d]oxazol-7-yl)-N-propylimidazo [1,2-a] pyridine-2-carboxamide

### Step 1: 7-bromo-6-fluorobenzo[d]oxazol-2(3H)-one

6-Amino-2-bromo-3-fluorophenol (700 mg, 3.41 mmol) and CDI (719 mg, 4.44 mmol) were added to tetrahydrofuran (25 mL) and the mixture was stirred at room temperature for 3 hours. The reaction mixture was concentrated and purified by column chromatography (petroleum ether/ethyl acetate = 3/1) to obtain the title compound (600 mg, 75%).

### Step 2: 7-bromo-6-fluoro-3-methylbenzo[d]oxazol-2(3H)-one

7-Bromo-6-fluorobenzo[d]azol-2(3H)-one (600 mg, 2.60 mmol), cesium carbonate (2.54 g, 7.79 mmol), and iodomethane (443 mg, 3.12 mmol) were sequentially added to DMF (10 mL) and the mixture was stirred at room temperature for 18 hours. The reaction mixture was diluted with water (50 mL) and extracted with ethyl acetate (50 mL × 2). The organic phases were combind, dried over anhydrous sodium sulfate, concentrated, and purified by column chromatography to obtain the title compound (350 mg, 55%). LC-MS: m/z [M+H]⁺ =246

### Step 3: 6-fluoro-3-methyl-7-(4,4,5,5-tetramethyl-1,3,2-dioxaborolan-2-yl)benzo[d]oxazol-2(3H)-one

The experimental operation was the same as that in Example 94 (step 3), using 7-bromo-6-fluoro-3-methylbenzo[d]oxazol-2(3H)-one (50 mg, 0.2 mmol) as a starting material to obtain the title compound (18 mg, 30%) as a yellow solid.

**Step 4:** The operation method was the same as that in the synthesis example of compound vii, using 6-fluoro-3-methyl-7-(4,4,5,5-tetramethyl-1,3,2-dioxaborolan-2-yl)benzo[d]oxazol-2(3H)-one (18 mg, 0.06 mmol) and 3-amino-8-bromo-N-propylimidazo[1,2-a]pyridine-2-carboxamide (18 mg, 0.06 mmol) as starting materials to obtain the title product (5 mg, 22%).
¹H NMR (400 MHz, DMSO) 7.82 (s, 1H), 7.23 -7.15 (m, 1H), 7.14 - 7.06 (m,2H), 7.01 - 6.95 (m, 1H), 6.91 (s, 1H), 4.92 (s, 2H), 3.45 (s, 3H), 3.35 (dd, J = 13.9, 6.7 Hz, 2H), 1.70 - 1.59 (m, 2H), 0.95 (t, J = 7.4 Hz, 3H). LCMS MS m/z (ESI): 384 [M+1]

### Example 106

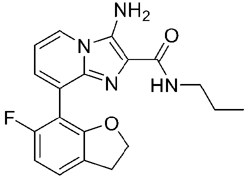

### 3-Amino-8-(6-fluoro-2,3-dihydrobenzofuran-7-yl)-N-propylimidazo[1,2-a]pyridine-2-carboxamide

### Step 1: 1-bromo-2-(2-bromoethoxy)-4-fluorobenzene

Under an argon atmosphere, 2-bromo-5-fluorophenol (10 g, 53.4 mmol), 1,2-dibromoethane (49 g, 262 mmol), and potassium carbonate (8 g, 57.6 mmol) were sequentially added to N,N-dimethylformamide (150 mL). The mixture was stirred at 60°C for 2 hours. The mixture was filtered. The stock solution was diluted with water (250 ml), and extracted with ethyl acetate (250 ml × 3). The organic phases were combined, dried over anhydrous sodium sulfate, concentrated, and purified by column chromatography to afford the product (8 g, 51%) as a colorless liquid.

¹H NMR (400 MHz, CDCl₃) δ 7.53 - 7.44 (m, 1H), 6.67 - 6.58 (m, 2H), 4.30 (t, J = 6.4 Hz, 2H), 3.69 (t, J = 6.4 Hz, 2H).

### Step 2: 6-fluoro-2,3-dihydro-1-benzofuran

Under an argon atmosphere, 1-bromo-2-(2-bromoethoxy)-4-fluorobenzene (1 g, 3.36 mmol) was dissolved in anhydrous tetrahydrofuran (30 mL). The mixture was cooled to - 78°C, and a solution of n-butyllithium in tetrahydrofuran (1 M, 3.7 mL) was added dropwise slowly. The reaction mixture was maintained at -78°C and stirred for 3 hours. The mixture was then warmed to 0°C and stirred for 1 hour. A portion of the reaction mixture was taken, concentrated, and analyzed by NMR. The remaining reaction mixture was directly used in the next step.

¹H NMR (400 MHz, CDCl₃) δ 7.13 - 7.08 (m, 1H), 6.61 - 6.50 (m, 2H), 4.62 (t, J = 8.7 Hz, 2H), 3.17 (t, J = 8.7 Hz, 2H).

### Step 3: (6-fluoro-2,3-dihydrobenzofuran-7-yl)boronic acid

Under argon atmosphere, a solution of 6-fluoro-2,3-dihydro-1-benzofuran (0.4 g, 2.9 mmol) in anhydrous tetrahydrofuran (30 mL) was cooled to -78°C. A solution of lithium diisopropylamide in tetrahydrofuran (2 M, 2.2 mL) was added dropwise. After the mixture was stirred for 10 minutes, a solution of trimethyl borate (0.33 g, 3.19 mmol) in tetrahydrofuran (10 mL) was added dropwise to the reaction mixture. The temperature was maintained at - 78°C, and the mixture was stirred for an additional 10 minutes. The reaction mixture was quenched with 1 M hydrochloric acid aqueous solution and extracted with ethyl acetate (20 ml × 3). The organic phase was concentrated and washed with diethyl ether to obtain a white solid (0.49 g, 93%).

¹HNMR (400 MHz, CDCl₃) δ 7.24 (t, J = 7.0 Hz, 1H), 6.60 (dd, J = 10.4, 8.4 Hz, 1H), 5.98-5.94 (m, 2H), 4.74 (t, J = 8.7 Hz, 2H), 3.21 (t, J = 8.7 Hz, 2H).

**Step 4:** The operation method was the same as that in the synthesis example of compound vii, using 3-amino-8-bromo-N-propylimidazo[1,2-a]pyridine-2-carboxamide (100 mg, 0.34 mmol) and (6-fluoro-2,3-dihydro-1-benzofuran-7-yl)boronic acid (124 mg, 0.68 mmol) to obtain the target product (25 mg, 20%) as a yellow solid.
¹H NMR (400 MHz, DMSO-*d₆*) δ 8.16 (d, J = 6.4 Hz, 1H), 7.58 (d, J = 5.5 Hz, 1H), 7.29 - 7.21 (m, 1H), 6.99 (d, J = 6.2 Hz, 1H), 6.88 (t, J = 6.7 Hz, 1H), 6.74 (t, J *=* 9.0 Hz, 1H), 6.15 (s, 2H), 4.53 (s, 2H), 3.19 (dd, J = 19.1, 8.0 Hz, 4H), 1.47 (dd, J = 14.2, 7.1 Hz, 2H), 0.82 (t, J = 7.1 Hz, 3H). LCMS: m/z [M+H]⁺= 355

### Example 107

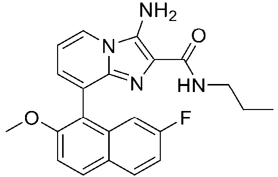

### 3-Amino-8-(7-fluoro-2-methoxynaphthalen-1-yl)-N-propylimidazo[1,2-a]pyridine-2-carboxamide

### Step 1: 8-bromo-7-methoxy-3,4-dihydronaphthalen-1(2H)-one

7-Methoxy-3,4-dihydronaphthalen-1(2H)-one (10.0 g, 56.82 mmol) was added to acetonitrile (150 mL), and NBS (10.1 g, 57.38 mmol) was subsequently added. The mixture was stirred at room temperature for 5 hours. The reaction mixture was quenched by adding sodium sulfite solution. The reaction mixture was concentrated and purified by column chromatography (petroleum ether/ethyl acetate = 10/1) to obtain the title compound (7.4 g, 51%). ¹H NMR (400 MHz, CDCl₃) δ 7.20 - 7.14 (m, 1H), 7.01 (dd, *J =* 8.4, 1.8 Hz, 1H), 3.90 (d, *J* = 2.7 Hz, 3H), 2.93 - 2.85 (m, 2H), 2.76 - 2.61 (m, 2H), 2.17 - 1.95 (m, 2H).

### Step 2: 8-bromo-2-fluoro-7-methoxy-3,4-dihydronaphthalen-1(2H)-one

8-Bromo-7-methoxy-3,4-dihydronaphthalen-1(2H)-one (1.0 g, 3.95 mmol) was added to methanol (15 mL), and 1-chloromethyl-4-fluoro-1,4-diazabicyclo[2.2.2]octane bis(tetrafluoroborate) (1.7 g, 4.74 mmol) was subsequently added. The mixture was stirred at room temperature overnight. The reaction mixture was concentrated and purified by column chromatography (petroleum ether/ethyl acetate = 10/1) to obtain the title compound (850 mg, 79%).

¹H NMR (400 MHz, DMSO-*d₆*) 7.37-7.33 (m, 2H), 5.68 - 5.04 (m, 1H), 3.87 (s, 3H), 3.19 - 2.99 (m, 2H), 2.49 - 2.11 (m, 2H).

### Step 3: 8-bromo-2-fluoro-7-methoxy-1,2,3,4-tetrahydronaphthalen-1-ol

8-Bromo-2-fluoro-7-methoxy-3,4-dihydronaphthalen-1(2H)-one (750 mg, 2.75 mmol) was added to methanol (10 mL). The mixture was cooled to 0°C, and sodium borohydride (125 mg, 3.29 mmol) was added. The mixture was stirred at room temperature for 2 hours. The reaction mixture was added with water and filtered. The filtrate was concentrated and purified by column chromatography (petroleum ether/ethyl acetate = 5/1) to obtain the title compound (600 mg, 79%).

¹H NMR (400 MHz, DMSO-*d₆*) 7.12 (d, *J =* 8.5 Hz, 1H), 7.03 (d, *J =* 8.5 Hz, 1H), 5.41 (d, *J =* 5.9 Hz, 1H), 5.02 - 4.86 (m, 1H), 4.73 (ddt, *J =* 47.4, 12.4, 3.7 Hz, 1H), 3.81 (s, 3H), 2.83 (tdd, *J* = 17.2, 14.6, 5.3 Hz, 2H), 2.29 - 2.10 (m, 1H), 1.86 (d, *J* = 3.9 Hz, 1H).

### Step 4: 5-bromo-3-fluoro-6-methoxy-1,2-dihydronaphthalene

8-Bromo-2-fluoro-7-methoxy-1,2,3,4-tetrahydronaphthalen-1-ol (600 mg, 2.18 mmol) was added to toluene (10 mL), and p-toluenesulfonic acid (80 mg, 0.44 mmol) was subsequently added. The mixture was stirred at 50°C for 3 hours. The reaction mixture was concentrated and purified by column chromatography (petroleum ether/ethyl acetate = 10/1) to obtain the title compound (520 mg, 92%).

¹H NMR (400 MHz, DMSO-*d₆*) 7.14 (t, *J* = 7.8 Hz, 1H), 6.89 - 6.78 (m, 1H), 6.42 (d, *J* = 14.3 Hz, 1H), 3.80 (s, 3H), 2.96 (dt, *J =* 8.4, 4.1 Hz, 2H), 2.55 (td, *J* = 8.3, 4.5 Hz, 2H).

### Step 5: 1-bromo-7-fluoro-2-methoxynaphthalene

5-Bromo-3-fluoro-6-methoxy-1,2-dihydronaphthalene (520 mg, 2.04 mmol) was added to dichloromethane, and 2,3-dichloro-5,6-dicyano-1,4-benzoquinone (DDQ, 551 mg, 2.42 mmol) was subsequently added. The reaction mixture was stirred at 40°C for 3 hours, then concentrated and purified by column chromatography (petroleum ether/ethyl acetate = 10/1) to obtain the title compound (470 mg, 91%).

¹H NMR (400 MHz, DMSO-*d₆*) δ 8.14 - 7.99 (m, 2H), 7.71 (dd, *J =* 11.7, 9.8 Hz, 1H), 7.54 (dd, *J =* 18.6, 9.0 Hz, 1H), 7.40 - 7.25 (m, 1H), 4.01 (s, 3H).

### Step 6: 2-(7-fluoro-2-methoxynaphthalen-1-yl)-4,4,5,5-tetramethyl-1,3,2-dioxaborolane

The experimental operation was the same as that in Example 94 (step 3), using 1-bromo-7-fluoro-2-methoxynaphthalene (470 mg, 1.84 mmol) as a starting material, followed by column chromatography (petroleum ether/ethyl acetate = 5/1) to obtain the title compound (350 mg, 62%).

¹H NMR (400 MHz, CD₃OD) 7.88 (t, *J* = 8.0 Hz, 1H), 7.80 - 7.68 (m, 1H), 7.54 - 7.39 (m, 1H), 7.25 (dd, *J =* 15.1, 6.0 Hz, 1H), 7.04 (td, *J =* 8.7, 2.5 Hz, 1H), 3.83 (d, *J* = 4.7 Hz, 3H), 1.37 (s, 12H).

**Step 7:** The operation method was the same as that in the synthesis example of compound vii, using 2-(7-fluoro-2-methoxynaphthalen-1-yl)-4,4,5,5-tetramethyl-1,3,2-dioxaborolane (80 mg, 0.26 mmol) and 3-amino-8-bromo-N-propylimidazo[1,2-a]pyridine-2-carboxamide (70 mg, 0.24 mmol) as starting materials to obtain the title compound as a solid (5 mg, 4%).
¹H NMR (400 MHz, CD₃OD) 8.17-8.16 (d, *J*=4.0 Hz, 1H), 8.09-8.07 (d, *J =* 8.0Hz, 1H), 7.97-7.94 (m, 1H), 7.54-7.52 (d, *J =* 8.0Hz, 1H), 7.18-7.14 (t, 1H), 7.09-7.02 (m, 2 H), 6.89-6.86 (d, *J =* 12.0 Hz, 1H), 3.86 (s, 3 H), 3.25-3.22 (t, 2H), 1.56-1.47 (m, 2 H), 0.92-0.88 (t, 3H). LCMS: m/z [M+H]⁺=393

### Example 108

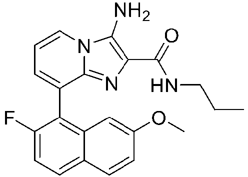

### 3-Amino-8-(2-fluoro-7-methoxynaphthalen-1-yl)-N-propylimidazo[1,2-a]pyridine-2-carboxamide

### Step 1: 2-fluoro-7-methoxy-3,4-dihydronaphthalen-1-yl trifluoromethanesulfonate

Under nitrogen atmosphere, 2-fluoro-7-methoxy-3,4-dihydronaphthalen-1(2H)-one (200 mg, 1.03 mmol) was dissolved in 10 ml of anhydrous tetrahydrofuran. The solution was cooled to -78°C using a dry ice-acetone bath. At this temperature, LiHMDS (1 M, 2.1 ml) was slowly added. The reaction was maintained at this temperature for 1 hour. Then, PhNTf₂ (736 mg, 2.06 mmol) was dissolved in anhydrous tetrahydrofuran (2 ml) and the resulting solution was slowly added dropwise. The mixture was allowed to warm to room temperature and stirred for 16 hours. After the reaction was completed, the mixture was poured into 100 ml of water and extracted with ethyl acetate (100 ml × 3). It was concentrated and purified by column chromatography (petroleum ether/ethyl acetate = 95/5) to obtain the title product (150 mg, yellow oil) in a yield of 44.7%. LCMS MS m/z (ESI): 327 [M+1]

### Step 2: 2-fluoro-7-methoxynaphthalen-1-yl trifluoromethanesulfonate

Under nitrogen atmosphere, 2-fluoro-7-methoxy-3,4-dihydronaphthalen-1-yl trifluoromethanesulfonate (2000 mg, 6.13 mmol) and 2,3-dichloro-5,6-dicyano-1,4-benzoquinone (2785 mg, 12.27 mmol) were dissolved in toluene and reacted at 70°C for 16 hours. After the reaction was completed, the mixture was added into 100 mL of water and extracted with ethyl acetate (100 mL × 3). The organic phase was dried in a rotary evaporator, and the residue was purified by column chromatography (petroleum ether/ethyl acetate = 95/5) to obtain the title product (1500 mg, yellow oil) in a yield of 75.5%. LCMS MS m/z (ESI): 325 [M+1]

**Step 3:** 2-fluoro-7-methoxynaphthalen-1-yl trifluoromethanesulfonate (20 mg, 0.06 mmol), 3-amino-8-(trimethylstannyl)-N-propylimidazo[1,2-a]pyridine-2-carboxamide (23 mg, 0.06 mmol), and Pd(PPh₃)₄ (14 mg, 0.012 mmol) were sequentially added to DMF (1 mL), and the mixture was reacted under microwave irradiation at 120°C for 2 hours. The reaction mixture was diluted with ethyl acetate (20 mL) and washed with saturated aqueous sodium chloride solution (10 mL × 3). The organic phase was dried over anhydrous sodium sulfate, concentrated under reduced pressure, and purified by column chromatography to obtain the title product (2 mg, 8%) as a yellow solid.
¹H NMR (400 MHz, CDCl₃) 8.29 (s, 1H), 7.90 (s, 1H), 7.78 (d, J = 9.0 Hz, 1H), 7.69 - 7.43 (m, 2H), 7.24 (s, 2H), 7.10 (d, J = 7.9 Hz, 1H), 6.86 (s, 1H), 3.71 (d, J = 26.3 Hz, 3H), 3.22 (s, 2H), 1.57 - 1.51 (m, 2H), 0.87 (t, J = 7.1 Hz, 3H). LCMS MS m/z (ESI): 393 [M+1]

### Example 109

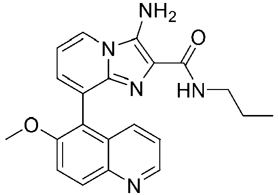

### 3-Amino-8-(6-methoxyquinolin-5-yl)-N-propylimidazo[1,2-a]pyridine-2-carboxamide

### Step 1: 5-bromo-6-methoxyquinoline

6-Methoxyquinoline (2.0 g, 12.6 mmol) was added to acetonitrile (20 mL), and NBS (2.24 g, 12.6 mmol) was subsequently added in batches. The mixture was stirred at room temperature for 16 hours. The mixture was poured into water (50 mL), and extracted with ethyl acetate (50 mL × 3). The mixture was purified by column chromatography to obtain a gray solid (1 g, 33%). LC-MS: m/z [M+H]⁺ =238

**Step 2:** 5-Bromo-6-methoxyquinoline (100 mg, 0.42 mmol) was dispersed in dioxane (2 mL), and 3-amino-N-propyl-8-(trimethylstannyl)imidazo[1,2-a]pyridine-2-carboxamide (100 mg, 0.26 mmol) and Pd(PPh₃)₂Cl₂ (15 mg, 0.021 mmol) were sequentially added. After nitrogen displacement, the tube was sealed, and the mixture was stirred at 100°C for 18 hours. The mixture was then purified by column chromatography to obtain the target product (6 mg, 6%).
¹H NMR (400 MHz, DMSO) 8.75 (d, *J* = 2.8 Hz, 1H), 8.28 - 8.18 (m, 1H), 8.14 (d, *J* = 9.4 Hz, 1H), 7.81 (d, *J =* 9.4 Hz, 1H), 7.63 (d, *J* = 8.4 Hz, 1H), 7.45 (d, *J* = 6.1 Hz, 1H), 7.34 (dd, *J =* 8.5, 4.1 Hz, 1H), 6.96 (d, *J =* 3.3 Hz, 2H), 6.19 (s, 2H), 3.82 (s, 3H), 3.08 (dd, *J* = 11.8, 5.9 Hz, 2H), 1.38 (dd, *J* = 14.6, 7.2 Hz, 2H), 0.75 (t, *J* = 7.4 Hz, 3H). LC-MS: m/z [M+H]⁺ =376

### Example 110

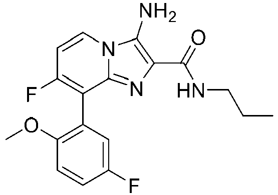

### 3-Amino-7-fluoro-8-(5-fluoro-2-methoxyphenyl)-N-propylimidazo[1,2-a]pyridine-2-carboxamide

3-Amino-7-fluoro-8-bromo-N-propylimidazo[1,2-a]pyridine-2-carboxamide (100 mg, 0.32 mmol), (5-fluoro-2-methoxyphenyl)boronic acid (115 mg, 0.68 mmol), cesium carbonate (221 mg, 0.68 mmol), and (2-dicyclohexylphosphino-2',6'-dimethoxybiphenyl)[2-(2'-amino-1,1'-biphenyl)]palladium(II) methanesulfonate (SPhos-Pd-G3, CAS No.: 1445085-82-4, 31 mg, 0.04 mmol) were sequentially added to a mixed solution of dioxane and water (2 mL/0.4 mL). The mixture was stirred at 100°C for 2 hours under argon atmosphere. 20 mL of water was added. The mixture was extracted with dichloromethane (20 mL × 3). The organic phase was concentrated and purified by column chromatography (petroleum ether:ethyl acetate = 1:1 to 1:5) to obtain the target product (74 mg, 65%) as a yellow solid.
¹H NMR (400MHz, DMSO-*d₆*) 8.23-8.19 (m, 1 H), 7.59 (br. s., 1 H), 7.29-7.25 (m, 2 H), 7.16 (br. s., 1 H), 7.01 (br. s., 1 H), 6.17 (s, 2 H), 3.71 (s, 3 H), 3.17-3.13 (m, 2 H), 1.48-1.44 (m, 2 H), 0.83-0.79 (m, 3 H). LC-MS: m/z [M+H]⁺ = 361

### Example 111

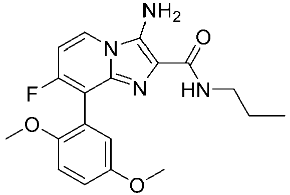

### 3-Amino-8-(2,5-dimethylphenyl)-7-fluoro-N-propylimidazo[1,2-a]pyridine-2-carboxamide[1,2-a]

The operation method was the same as the synthesis method of Example 110, using 3-amino-7-fluoro-8-bromo-N-propylimidazo[1,2-a]pyridine-2-carboxamide (100 mg, 0.32 mmol) and (2,5-dimethoxyphenyl)boronic acid (124 mg, 0.68 mmol) as starting materials to obtain the target product (67 mg, 57%) as a yellow solid.
¹H NMR (400MHz, DMSO-*d₆*) 8.20 (br. s., 1 H), 7.58 (br. s., 1 H), 7.07 (d, J = 8.3 Hz, 1 H), 7.03 - 6.92 (m, 3 H), 6.16 (br. s., 2 H), 3.72 (s, 3 H), 3.66 (s, 3 H), 3.20 - 3.11 (m, 2 H), 1.46 (d, J = 6.4 Hz, 2 H), 0.84 - 0.78 (m, 3 H). LC-MS: m/z [M+H]⁺ = 373

### Example 112

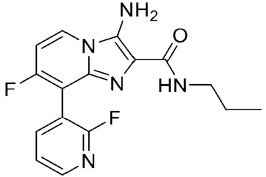

### 3-Amino-7-fluoro-8-(2-fluoropyridin-3-yl)-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (2-fluoropyridin-3-yl)boronic acid (96 mg, 0.68 mmol) as starting materials to obtain the target product (70 mg, 67%) as a yellow solid
¹H NMR (400MHz, DMSO-*d6*) 8.37 (t, J = 6.4 Hz, 1 H), 8.34 - 8.21 (m, 2 H), 7.65 (br. s., 1 H), 7.54 (br. s., 1 H), 7.10 (br. s., 1 H), 6.24 (s, 2 H), 3.16 (d, J = 5.9 Hz, 2 H), 1.52 - 1.39 (m, 2 H), 0.85 - 0.79 (m, 3 H). LC-MS: m/z [M+H]⁺ =332

### Example 113

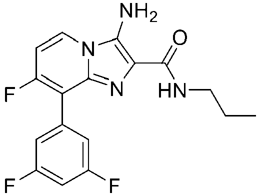

### 3-Amino-8-(3,5-difluorophenyl)-7-fluoro-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (3,5-difluorophenyl)boronic acid (107 mg, 0.68 mmol) as starting materials to obtain the target product (59 mg, 53%) as a yellow solid.
¹H NMR (400MHz, DMSO-*d₆*) 8.2(br. s., 1 H), 7.72 (br. s., 1 H), 7.52 (d, J = 6.4 Hz, 2 H), 7.32 (br. s., 1 H), 7.06 (br. s., 1 H), 6.23 (br. s., 2 H), 3.19 (d, J = 5.9 Hz, 2 H), 1.54 - 1.44 (m, 2 H), 0.87 - 0.81 (m, 3 H). LC-MS: m/z [M+H]⁺ = 349

### Example 114

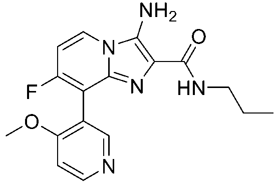

### 3-Amino-7-fluoro-8-(4-methoxypyridin-3-yl)-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (4-methoxypyridin-3-yl)boronic acid (104 mg, 0.68 mmol) as starting materials to obtain the target product (69 mg, 63%) as a yellow solid
¹H NMR (400MHz, DMSO-*d₆*) 8.53 (br. s., 1 H), 8.44 (br. s., 1 H), 8.24 (br. s., 1 H), 7.59 (br. s., 1 H), 7.22 (br. s., 1 H), 7.03 (br. s., 1 H), 6.18 (br. s., 2 H), 3.82 (br. s., 3 H), 3.14 (br. s., 2 H), 1.45 (br. s., 2 H), 0.81 (br. s., 3 H). LC-MS: m/z [M+H]⁺ = 344

### Example 115

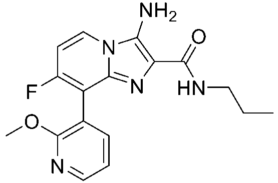

### 3-Amino-7-fluoro-8-(2-methoxypyridin-3-yl)-N-propylimidazo[1,2-A]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (2-methoxypyridin-3-yl)boronic acid (104 mg, 0.68 mmol) as starting materials to obtain the target product (27 mg, 25%) as a yellow solid
¹H NMR (400MHz, DMSO-*d₆*) 8.36 - 8.14 (m, 2 H), 7.84 (d, J = 7.3 Hz, 1 H), 7.58 (br. s., 1 H), 7.14 (t, J = 5.6 Hz, 1 H), 7.02 (t, J = 7.6 Hz, 1 H), 6.17 (br. s., 2 H), 3.82 (s, 3 H), 3.14 (d, J = 6.8 Hz, 2 H), 1.58 - 1.39 (m, 2 H), 0.84 - 0.77 (m, 3 H). LC-MS: m/z [M+H]⁺ = 344

### Example 116

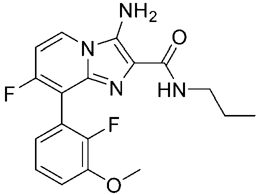

### 3-Amino-7-fluoro-8-(3-fluoro-2-methoxyphenyl)-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (3-fluoro-2-methoxyphenyl)boronic acid (115 mg, 0.68 mmol) as starting materials to obtain the target product (37 mg, 32%) as a yellow solid.
¹H NMR (400MHz, DMSO-*d₆*) 8.27 (t, J = 6.6 Hz, 1 H), 7.57 (t, J = 5.6 Hz, 1 H), 7.30 - 7.21 (m, 2 H), 7.16 - 7.09 (m, 1 H), 7.06 (t, J = 7.8 Hz, 1 H), 6.20 (s, 2 H), 3.89 (s, 3 H), 3.15 (q, J = 6.8 Hz, 2 H), 1.57 - 1.39 (m, 2 H), 0.81 (t, J = 7.3 Hz, 3 H). LC-MS: m/z [M+H]⁺ = 361

### Example 117

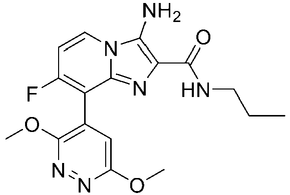

### 3-Amino-8-(3,6-dimethoxypyridazin-4-yl)-7-fluoro-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (3,6-dimethoxypyridazin-4-yl)boronic acid (125 mg, 0.68 mmol) as starting materials to obtain the target product (41 mg, 35%) as a yellow solid
¹HNMR (400MHz, DMSO-*d₆*) 8.31 (br. s., 1 H), 7.74 (br. s., 1 H), 7.44 (br. s., 1 H), 7.07 (br. s., 1 H), 6.23 (br. s., 2 H), 4.00 (s, 3 H), 3.92 (s, 3 H), 3.18 - 3.08 (m, 2 H), 1.44 (d, J = 6.8 Hz, 2 H), 0.83 - 0.77 (m, 3 H). LC-MS: m/z [M+H]⁺ = 375

### Example 118

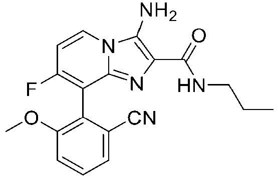

### 3-Amino-8-(2-cyano-6-methoxyphenyl)-7-fluoro-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and 2-(5,5-dimethyl-1,3,2-dioxaborinan-2-yl)-3-methoxybenzonitrile (167 mg, 0.68 mmol) as starting materials to obtain the target product (9 mg, 8%) as a yellow solid
¹H NMR (400MHz, DMSO-*d₆*) 8.34-8.30 (m, 1 H), 7.67 (br. s., 1 H), 7.57 (d, J = 7.3 Hz, 3 H), 7.12-7.08 (m, 1 H), 6.23 (s, 2 H), 3.77 (s, 3 H), 3.17-3.13 (m, 2 H), 1.52 - 1.41 (m, 2 H), 0.81 (br. s., 3 H). LC-MS: m/z [M+H]⁺ = 368

### Example 119

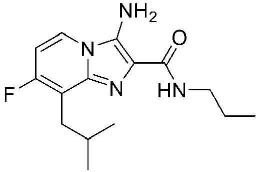

### 3-Amino-7-fluoro-8-isobutyl-N-propionylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (2-methylpropyl)boronic acid (173 mg, 1.7 mmol) as starting materials to obtain the target product (32 mg, 34%) as a yellow solid
¹H NMR (400MHz, DMSO-*d₆*) 8.05 (t, J = 6.6 Hz, 1 H), 7.71 (t, J = 5.9 Hz, 1 H), 6.89 (t, J = 7.8 Hz, 1 H), 6.09 (s, 2 H), 3.22 (q, J = 6.7 Hz, 2 H), 2.70 (d, J = 6.8 Hz, 2 H), 2.28 - 2.08 (m, 1 H), 1.60 - 1.44 (m, 2 H), 0.93 - 0.84 (m, 9 H). LC-MS: m/z [M+H]⁺ = 293

### Example 120

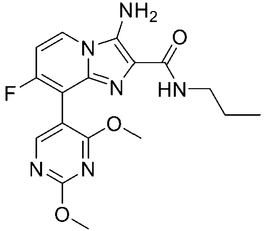

### 3-Amino-8-(2,4-dimethoxypyrimidin-5-yl)-7-fluoro-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (2,4-dimethoxypyrimidin-5-yl)boronic acid (125 mg, 0.68 mmol) as starting materials to obtain the target product (83 mg, 70%) as a yellow solid
¹H NMR (400MHz, DMSO-*d₆*) 8.48 (t, J = 6.6 Hz, 1 H), 8.26-8.22 (m, 1 H), 7.71-7.67 (m, 1 H), 7.04 (t, J = 7.6 Hz, 1 H), 6.19 (s, 2 H), 3.98 (s, 3 H), 3.90 (s, 3 H), 3.15 (d, J = 6.4 Hz, 2 H), 1.52 - 1.42 (m, 2 H), 0.85 - 0.79 (m, 3 H). LC-MS: m/z [M+H]⁺ = 375

### Example 121

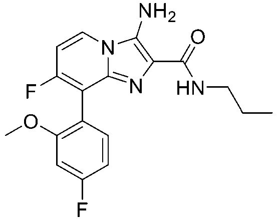

### 3-Amino-7-fluoro-8-(4-fluoro-2-methoxyphenyl)-N-propylimidazo[1,2-a]pyridine-2-carboxamide

The operation method was the same as the synthesis method of Example 110, using 3-amino-7-fluoro-8-bromo-N-propylimidazo[1,2-a]pyridine-2-carboxamide (100 mg, 0.32 mmol) and (4-fluoro-2-methoxyphenyl)boronic acid (115 mg, 0.68 mmol) as starting materials to obtain the target product (45 mg, 39%) as a yellow solid.
¹H NMR (400MHz, DMSO-*d₆*) 8.19 (br. s., 1 H), 7.54 (br. s., 1 H), 7.39 (br. s., 1 H), 7.05 (br. s., 1 H), 6.98 (br. s., 1 H), 6.87 (br. s., 1 H), 6.14 (br. s., 2 H), 3.73 (br. s., 3 H), 3.14 (br. s., 2 H), 1.45 (br. s., 2 H), 0.81 (br. s., 3 H). LC-MS: m/z [M+H]⁺ = 361

### Example 122

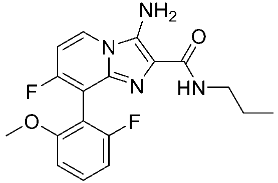

### 3-Amino-7-fluoro-8-(2-fluoro-6-methoxyphenyl)-N-propylimidazo[1,2-a]pyridine-2-carboxamide

The operation method was the same as that in the synthesis example of compound vii, using 3-amino-7-fluoro-8-bromo-N-propylimidazo[1,2-a]pyridine-2-carboxamide (100 mg, 0.32 mmol) and (2-fluoro-6-methoxyphenyl)boronic acid (115 mg, 0.68 mmol) as starting materials to obtain the target product (64 mg, 56%) as a yellow solid.
¹HNMR (400MHz, DMSO-*d₆*) 8.27 (br. s., 1 H), 7.61 (br. s., 1 H), 7.50 (br. s., 1 H), 7.04 (br. s., 2 H), 6.97 (br. s., 1 H), 6.20 (br. s., 2 H), 3.74 (br. s., 3 H), 3.15 (br. s., 2 H), 1.46 (br. s., 2 H), 0.82 (br. s., 3 H). LC-MS: m/z [M+H]⁺ = 361

### Example 123

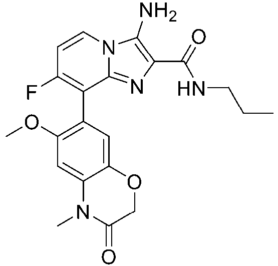

### 3-Amino-7-fluoro-8-(6-methoxy-4-methyl-3-oxo-3,4-dihydro-2H-benzo[b][1,4]oxazin-7-yl)-N-propylimidazo[1,2-a]pyridine-2-carboxamide

### Step 1: 6-methoxy-2H-benzo[b][1,4]oxazin-3(4H)-one

2-Amino-4-methoxyphenol (10.0 g, 71 mmol) was added to acetonitrile (500 ml), and chloroacetyl chloride (9.8 g, 86 mmol) and cesium carbonate (58.4 g, 179 mmol) were sequentially added. The mixture was stirred at 80°C for 16 hours, then filtered. The filtrate was dried in a rotary evaporator and purified by column chromatography (petroleum ether:ethyl acetate = 1:1) to obtain the title compound (9.4 g, 74%) as a red solid. LC-MS: m/z [M+H]⁺ = 180

### Step 2: 7-bromo-6-methoxy-2H-benzo[b][1,4]oxazin-3(4H)-one

6-Methoxy-2H-benzo[b][1,4]oxazin-3(4H)-one (10 g, 55.8 mmol) was dissolved in DMF (100 ml). NBS (10 g, 55.8 mmol) was added at 0°C. After completion of the addition, the mixture was stirred at room temperature for 2 hours. The reaction mixture was poured into ice-water (500 mL) and filtered. The resulting solid was washed with water (50 mL × 3) and dried under vacuum to obtain the title compound (9 g, 63%) as a red solid. LC-MS: m/z [M+H]⁺ =258

### Step 3: 7-bromo-6-methoxy-4-methyl-2H-benzo[b][1,4]oxazin-3(4H)-one

7-Bromo-6-methoxy-2H-benzo[b][1,4]oxazin-3(4H)-one (10 g, 38.7 mmol), cesium carbonate (37.2 g, 116 mmol), and methyl iodide (4.17 g, 29.4 mmol) were added to acetonitrile (100 mL) respectively. The mixture was stirred at 35°C for 16 hours. The reaction mixture was poured into ice water (200 mL), extracted with ethyl acetate (300 mL × 3), concentrated, and purified by column chromatography (petroleum ether:ethyl acetate = 2:1) to obtain the title compound (6 g, 57%) as an off-white solid. LC-MS: m/z [M+H]⁺ =272

### Step 4: 6-methoxy-4-methyl-7-(tetramethyl-1,3,2-dioxaborolan-2-yl)-3,4-dihydro-2H-1,4-benzoxazin-3-one

7-Bromo-6-methoxy-4-methyl-2H-benzo[b][1,4]oxazin-3(4H)-one (1.04 g, 3.85 mmol), bis(pinacolato)diboron (1.47 g, 5.78 mmol), potassium acetate (754 mg, 7.7 mmol), and [1,1'-bis(diphenylphosphino)ferrocene]palladium(II) dichloride dichloromethane complex (314 mg, 0.39 mmol) were sequentially added to dioxane (30 mL). The mixture was stirred at 100°C for 16 hours under argon atmosphere. The mixture was concentrated and purified by column chromatography (petroleum ether:ethyl acetate=1:1) to obtain the target product (500 mg, 41%) as a white solid. LC-MS: m/z [M+H]⁺ =320

**Step 5:** The operation method was the same as that in the synthesis example of compound vii, using 3-amino-7-fluoro-8-bromo-N-propylimidazo[1,2-a]pyridine-2-carboxamide (100 mg, 0.32 mmol) and 6-methoxy-4-methyl-7-(tetramethyl-1,3,2-dioxaborolan-2-yl)-3,4-dihydro-2H-1,4-benzoxazin-3-one (217 mg, 0.68 mmol) as starting materials to obtain the target product (12 mg, 9%) as a yellow solid.
¹HNMR (400MHz, DMSO-*d₆*) 8.19 (t, J = 6.4 Hz, 1 H), 7.57 (t, J = 4.9 Hz, 1 H), 7.05 - 6.96 (m, 2 H), 6.92 (s, 1 H), 6.15 (br. s., 2 H), 4.64 (s, 2 H), 3.76 (s, 3 H), 3.38 (s, 3 H),3.22 - 3.10 (m, 2 H), 1.50 - 1.43 (m, 2 H), 0.84 - 0.79 (m, 3 H). LC-MS: m/z [M+H]⁺ = 428

### Example 124

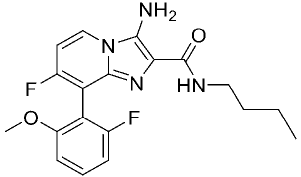

### 3-Amino-N-butyl-7-fluoro-8-(2-fluoro-6-methoxyphenyl)imidazo[1,2-a]pyridine-2-carboxamide

The operation method was the same as the synthesis method of Example 110, using 3-amino-7-fluoro-8-bromo-N-butylimidazo[1,2-a]pyridine-2-carboxamide (100 mg, 0.32 mmol) and (2-fluoro-6-methoxyphenyl)boronic acid (115 mg, 0.68 mmol) as starting materials to obtain the target product (26 mg, 23%) as a yellow solid.
¹H NMR (400MHz , DMSO-*d₆*) 8.27 (t, J = 6.6 Hz, 1 H), 7.58 (t, J = 5.6 Hz, 1 H), 7.49 (q, J = 7.8 Hz, 1 H), 7.08 - 7.00 (m, 2 H), 6.96 (t, J = 8.8 Hz, 1 H), 6.19 (s, 2 H), 3.74 (s, 3 H), 3.19 (q, J = 6.4 Hz, 2 H), 1.43 (td, J = 7.0, 14.4 Hz, 2 H), 1.23 (t, J = 7.1 Hz, 2 H), 0.85 (t, J = 7.1 Hz, 3 H). LC-MS: m/z [M+H]⁺ = 375

### Example 125

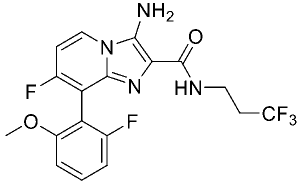

### 3-Amino-7-fluoro-8-(2-fluoro-6-methoxyphenyl)-N-(3,3,3-trifluoropropyl)propylimidazo[1,2-a]pyridine-2-carboxamide

The operation method was the same as the synthesis method of Example 110, using 3-amino-7-fluoro-8-bromo-N-(3,3,3-trifluoropropyl)imidazo[1,2-a]pyridine-2-carboxamide (100 mg, 0.32 mmol) and (2-fluoro-6-methoxyphenyl)boronic acid (115 mg, 0.68 mmol) as starting materials to obtain the target product (69 mg, 61%) as a yellow solid.
¹H NMR (400MHz, DMSO-*d₆*) 8.27 (t, J = 6.6 Hz, 1 H), 7.79 (t, J = 5.6 Hz, 1 H), 7.56 - 7.43 (m, 1 H), 7.07 - 6.99 (m, 2 H), 6.95 (t, J = 8.6 Hz, 1 H), 6.23 (s, 2 H), 3.73 (s, 3 H), 3.44 (d, J = 6.8 Hz, 2 H), 2.56 - 2.48 (m, 2 H). LC-MS: m/z [M+H]⁺ = 415

### Example 126

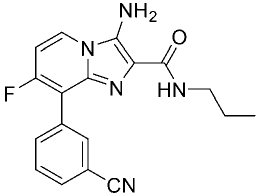

### 3-Amino-8-(3-cyanophenyl)-7-fluoro-N-propylimidazo[1,2-a]pyridine-2-carboxamide

The operation method was the same as that in the synthesis example of compound vii, using 3-amino-7-fluoro-8-bromo-N-propylimidazo[1,2-a]pyridine-2-carboxamide (100 mg, 0.32 mmol) and (3-cyanophenyl)boronic acid (100 mg, 0.68 mmol) as starting materials to obtain the target product (58 mg, 54%) as a yellow solid.
¹H NMR (400MHz, DMSO-*d₆*) 8.27 (t, J = 6.1 Hz, 1 H), 8.20 (br. s., 1 H), 8.12 (d, J = 7.3 Hz, 1 H), 7.92 (d, J = 7.8 Hz, 1 H), 7.76 - 7.67 (m, 2 H), 7.09 (t, J = 8.3 Hz, 1 H), 6.23 (br. s., 2 H), 3.23 - 3.14 (m, 2 H), 1.55 - 1.43 (m, 2 H), 0.87 - 0.82 (m, 3 H). LC-MS: m/z [M+H]⁺ = 338

### Example 127

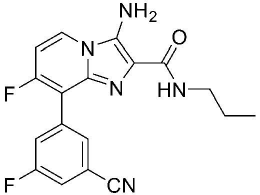

### 3-Amino-8-(3-methoxy-5-fluorophenyl)-7-fluoro-N-propylimidazo[1,2-a]pyridine-2-carboxamide

The operation method was the same as that in the synthesis example of compound vii, using 3-amino-7-fluoro-8-bromo-N-propylimidazo[1,2-a]pyridine-2-carboxamide (100 mg, 0.32 mmol) and (3-cyano-5-fluorophenyl)boronic acid (112 mg, 0.68 mmol) to obtain the target product (52 mg, 46%) as a yellow solid.
¹H NMR (400MHz, DMSO-*d₆*) 8.29 (t, J = 5.9 Hz, 1 H), 8.14 - 8.03 (m, 2 H), 7.96 (d, J = 8.3 Hz, 1 H), 7.76 (br. s., 1 H), 7.11 (t, J = 8.1 Hz, 1 H), 6.24 (br. s., 2 H), 3.20 (d, J = 6.4 Hz, 2 H), 1.55 - 1.44 (m, 2 H), 0.85 (t, J = 6.8 Hz, 3 H). LC-MS: m/z [M+H]⁺ = 356

### Example 128

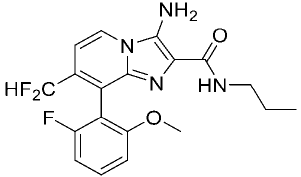

### 3-Amino-7-(difluoromethyl)-8-(2-fluoro-6-methoxyphenyl)-N-propylimidazo[1,2-a]pyridine-2-carboxamide

### Step 1: 3-bromo-2-chloro-4-(difluoromethyl)pyridine

3-Bromo-2-chloroisonicotinaldehyde (5 g, 22.8 mmol) was dissolved in dichloromethane (50 mL). Diethylaminosulfur trifluoride (DAST, CAS No.: 38078-09-0, 3.67 g, 22.8 mmol) was added dropwise under an ice bath. After addition, the reaction mixture was stirred at room temperature for 16 hours. The reaction mixture was quenched by adding saturated ammonium chloride (20 mL), and the layers were separated. The aqueous phase was extracted with dichloromethane (50 mL × 2). The organic phases were combined, dried over anhydrous sodium sulfate, concentrated, and purified by column chromatography (petroleum ether : ethyl acetate = 2 : 1) to obtain the target compound (4.3 g, 78.2%). LC-MS: m/z [M+H]⁺ = 242

### Step 2: 3-bromo-4-(difluoromethyl)-N-(4-methoxybenzyl)pyridin-2-amine

3-Bromo-2-chloro-4-(difluoromethyl)pyridine (4.1 g, 16.9 mmol) was dissolved in DMF (40 mL). p-Methoxybenzylamine (3.02 g, 22.0 mmol) and potassium carbonate (7.01 g, 50.7 mmol) were added sequentially. The mixture was stirred at 120°C for 16 hours. The reaction mixture was quenched by adding water (50 mL), and the resulting layers were separated. The aqueous phase was extracted with ethyl acetate (50 mL × 3). The organic phases were combined, dried over anhydrous sodium sulfate, filtered, concentrated, and purified by column chromatography (petroleum ether : ethyl acetate = 5 : 1) to obtain the target compound (3.2 g, 55%). LC-MS: m/z [M+H]⁺=343

### Step 3: 3-bromo-4-(difluoromethyl)pyridin-2-amine

3-Bromo-4-(difluoromethyl)-N-(4-methoxybenzyl)pyridin-2-amine (3.1 g, 9.03 mmol) was dissolved in trifluoroacetic acid (30 mL). The mixture was stirred at 80°C for 2 hours. The reaction mixture was concentrated, followed by the addition of water (20 mL) and saturated sodium bicarbonate solution (10 mL) to adjust the pH to 8. The aqueous phase was extracted with ethyl acetate (50 mL × 3). The organic phases were combined, dried over anhydrous sodium sulfate, filtered, concentrated, and purified by column chromatography (petroleum ether : ethyl acetate = 5 : 1) to obtain the target compound (1.1 g, 54.6%). LC-MS: m/z [M+H]⁺ =223

### Step 4: 3-amino-7-(difluoromethyl)-8-bromo-N-propylimidazo[1,2-a]pyridine-2-carboxamide

The operation method was the same as that for the synthesis of Intermediate iv (route 1) and intermediate vi (route 1), using 3-bromo-4-(difluoromethyl)pyridin-2-amine as a starting material to obtain the target product as a gray solid.

**Step 5:** The operation method was the same as the synthesis method of Example 110, using 3-amino-7-(difluoromethyl)-8-bromo-N-propylimidazo[1,2-a]pyridine-2-carboxamide (100 mg, 0.32 mmol) and (2-fluoro-6-methoxyphenyl)boronic acid (115 mg, 0.68 mmol) as starting materials to obtain the target product (31 mg, 27%) as a yellow solid.

¹H NMR (400MHz, DMSO-*d₆*) 8.35 (d, J = 2.0 Hz, 1 H), 7.63 (br.s., 1 H), 7.53 (q, J = 7.8 Hz, 1 H), 7.10 - 6.95 (m, 3 H), 6.60-6.28 (m, 3 H), 3.72 (s, 3 H), 3.18-3.14 (m, 2 H), 1.49-1.43 (m, 2 H), 0.85-0.77 (m, 3 H). LC-MS: m/z [M+H]⁺ = 393

### Example 129

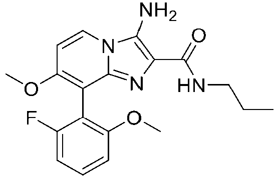

### 3-Amino-8-(2-fluoro-6-methoxyphenyl)-7-methoxy-N-propylimidazo[1,2-a]pyridine-2-carboxamide

### Step 1: 3-amino-7-methoxy-8-bromo-N-propylimidazo[1,2-a]pyridine-2-carboxylic acid

Ethyl 3-amino-7-fluoro-8-bromoimidazo[1,2-a]pyridine-2-carboxylate (1.3 g, 4.59 mmol) and aqueous sodium hydroxide solution (4 N, 5 mL) were sequentially added to MeOH (30 mL). The mixture was stirred at 60°C for 16 hours. The mixture was neutralized to pH 6 with 1 M dilute hydrochloric acid, then methanol was evaporated. The mixture was filtered, and the resulting filter cake was dried. The filter cake was then dissolved in N,N-dimethylformamide (10 mL), and HATU (CAS No.: 148893-10-1, 1.5 g, 3.96 mmol), n-propylamine (383 mg, 6.6 mmol), and triethylamine (660 mg, 6.6 mmol) were sequentially added. The mixture was stirred for 16 hours. The reaction mixture was poured into water, extracted with dichloromethane (50 mL × 3), dried over anhydrous sodium sulfate, concentrated, and purified by column chromatography to obtain the title compound (200 mg, 13%) as a gray solid. LC-MS: m/z [M+H]⁺ = 327

**Step 2:** The operation method was the same as the synthesis method of Example 110, using 3-amino-7-methoxy-8-bromo-N-propylimidazo[1,2-a]pyridine-2-carboxamide (105 mg, 0.32 mmol) and (2-fluoro-6-methoxyphenyl)boronic acid (115 mg, 0.68 mmol) as starting materials to obtain the target product (24 mg, 21%) as a yellow solid.
¹H NMR (400MHz, DMSO-*d₆*) 8.19 (d, J = 7.8 Hz, 1 H), 7.45 - 7.36 (m, 2 H), 7.06 (d, J = 7.3 Hz, 1 H), 6.94 (d, J = 8.3 Hz, 1 H), 6.87 (t, J = 8.3 Hz, 1 H), 6.02 (br. s., 2 H), 3.76 (s, 3 H), 3.69 (s, 3 H), 3.14 (d, J = 6.4 Hz, 2 H), 1.49 - 1.40 (m, 2 H), 0.81 (t, J = 7.1 Hz, 3 H). LC-MS: m/z [M+H]⁺ = 373

### Example 130

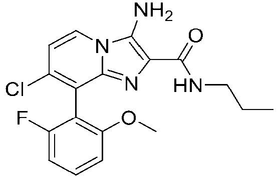

### 3-Amino-7-chloro-8-(2-fluoro-6-methoxyphenyl)-N-propylimidazo[1,2-a]pyridine-2-carboxamide

### Step 1: ethyl 8-bromo-7-chloro-3-nitroimidazo[1,2-a]pyridine-2-carboxylate

The operation method was the same as that in the synthesis of intermediate iv (route 1, step 1 and step 2), using 4-chloro-3-bromopyridin-2-amine (1 g, 4.85 mmol) as a starting material to obtain the title product (842 mg, 50%). LCMS: m/z [M+H]⁺= 348

### Step 2: 8-bromo-7-chloro-3-nitro-N-propylimidazo[1,2-a]pyridine-2-carboxamide

The operation method was the same as that in the synthesis of intermediate vi (route 1), using ethyl 8-bromo-7-chloro-3-nitroimidazo[1,2-a]pyridine-2-carboxylate (842 mg, 2.43 mmol) as a starting material to obtain the title compound (350 mg, 0.97 mmol). LCMS: m/z [M+H]⁺=361

### Step 3: 7-chloro-8-(2-fluoro-6-methoxyphenyl)-3-nitro-N-propylimidazo [1,2-a]pyridine-2-carboxamide

The operation method was the same as the synthesis method of Example 110, using 8-bromo-7-chloro-3-nitro-N-propylimidazo[1,2-a]pyridine-2-carboxamide (350 mg, 0.97 mmol) and (2-fluoro-6-methoxyphenyl)boronic acid (200 mg, 1.18 mmol) as starting materials to obtain the target product (70 mg, 18%). LCMS: m/z [M+H]⁺=407

**Step 4:** 7-Chloro-8-(2-fluoro-6-methoxyphenyl)-3-nitro-N-propylimidazo[1,2-a]pyridine-2-carboxamide (70 mg, 0.17 mmol) was added to a solution of methanol (5 mL), and iron powder (47.8 mg, 0.85 mmol) and ammonium chloride (91 mg, 1.70 mmol) were sebsequently added. The mixture was stirred at 80°C for 2 hours. It was filtered and concentrated. The residue was purified by thin-layer chromatography (petroleum ether:ethyl acetate = 2:1) to obtain the target product (10 mg, yield 15%) as a yellow solid.

¹H NMR (400 MHz, CD₃OD) 8.10 (d, J = 7.4 Hz, 1H), 7.52 (d, J = 7.4 Hz, 1H), 7.01 (d, J = 7.7 Hz, 2H), 6.92 - 6.85 (m, 1H), 3.78 (s, 3H), 3.27 (d, J = 6.9 Hz, 2H), 1.58 (dd, J = 14.5, 7.1 Hz, 2H), 0.95 (t, J = 7.2 Hz, 3H). LCMS: m/z [M+H]⁺= 377.

### Example 131

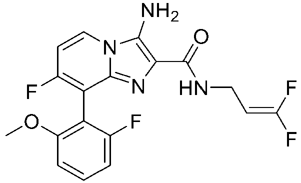

### 3-Amino-N-(3,3-difluoroallyl)-7-fluoro-8-(2-fluoro-6-methoxyphenyl)imidazo[1,2-a]pyridine-2-carboxamide

### Step 1: 3-amino-7-fluoro-8-bromo-N-(3,3-difluoroallyl)imidazo[1,2-a]pyridine-2-carboxamide

The operation method was the same as that in the synthesis of intermediate vi (route 1, step 2), using 3-amino-7-fluoro-8-bromoimidazo[1,2-a]pyridine-2-carboxylic acid and 3,3-difluoroprop-2-en-1-amine as starting materials to obtain the target product.

**Step 2:** The operation method was the same as the synthesis method of Example 110, using 3-amino-7-fluoro-8-bromo-N-(3,3-difluoroallyl)imidazo[1,2-a]pyridine-2-carboxamide (100 mg, 0.29 mmol) and (2-fluoro-6-methoxyphenyl)boronic acid (115 mg, 0.68 mmol) as starting materials to obtain the target product (58 mg, 51%) as a yellow solid.
¹HNMR (400MHz, DMSO-*d₆*) 8.28 (t, J = 6.6 Hz, 1 H), 7.85 (t, J = 5.4 Hz, 1 H), 7.56 - 7.43 (m, 1 H), 7.10 - 6.88 (m, 3 H), 6.25 (br. s., 2 H), 4.73 - 4.47 (m, 1 H), 3.81 (br. s., 2 H), 3.74 (s, 3 H). LC-MS: m/z [M+H]⁺ = 395

### Example 132

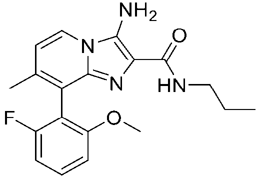

### 3-Amino-8-(2-fluoro-6-methoxyphenyl)-7-methyl-N-propylimidazo[1,2-a]pyridine-2-carboxamide

### Step 1: 3-amino-8-bromo-7-methyl-N-propylimidazo[1,2-a]pyridine-2-carboxamide

The operation method was the same as those described for the synthesis of compound iv (route 2) and compound vi (route 2), using 3-bromo-4-methylpyridin-2-amine (1.0 g, 5.35 mmol) solution as a starting material to obtain the target product (86 mg, yield 5.2%). LCMS: m/z [M+H]⁺=311.

**Step 2:** 3-Amino-8-bromo-7-methyl-N-propylimidazo[1,2-a]pyridine-2-carboxamide (53 mg, 0.17 mmol) was dissolved in a mixed solvent of dioxane and water (4 mL/0.5 mL). Cesium carbonate (166 mg, 0.51 mmol), (2-fluoro-6-methoxyphenyl)boronic acid (87 mg, 0.51 mmol), tris(dibenzylideneacetone)dipalladium (Pd₂(dba)₃, CAS No.: 52409-22-0, 16 mg, 0.02 mmol), and 2-dicyclohexylphosphino-2',4',6'-triisopropylbiphenyl (X-Phos, CAS No.: 564483-18-7, 16 mg, 0.03 mmol) were sequentially added. The reaction mixture was stirred at 100°C for 2 hours under argon atmosphere. The mixture was added with water (100 mL) and extracted with ethyl acetate (100 mL × 2). The organic phases were combined, dried over anhydrous sodium sulfate, concentrated, and purified by column chromatography (petroleum ether:ethyl acetate = 1:0 to 0:1) to obtain the target product (12 mg, yield 19%) as a yellow solid.

¹H NMR (400 MHz, CDCl₃) δ 7.68 (d, J = 6.8 Hz, 1H), 7.39 (d, J = 6.7 Hz, 1H), 7.15 (s, 1H), 6.85 (t, J = 8.7 Hz, 2H), 6.73 (d, J = 6.8 Hz, 1H), 4.86 (s, 2H), 3.76 (s, 3H), 3.33 (d, J = 6.4 Hz, 2H), 2.13 (s, 3H), 1.62 - 1.54 (m, 2H), 0.94 (t, J = 7.3 Hz, 3H). LCMS: m/z [M+H]⁺= 357.

### Example 133

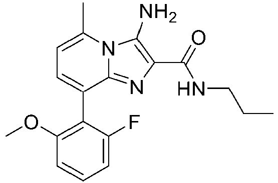

### 3-Amino-8-(2-fluoro-6-methoxyphenyl)-5-methyl-N-propylimidazo[1,2-a]pyridine-2-carboxamide

### Step 1: ethyl 3-amino-8-bromo-5-methylimidazo[1,2-a]pyridine-2-carboxylate

The operation was the same as that for intermediate iv (route 2), using 3-bromo-6-methylpyridin-2-amine (1.5 g, 8 mmol) as a starting material to obtain the target product (700 mg, 30%). LC-MS: m/z [M+H]⁺ =298

### Step 2: 33-amino-8-bromo-5-methyl-N-propylimidazo[1,2-a]pyridine-2-carboxamide

The operation was the same as that for intermediate vi (route 1), using ethyl 3-amino-8-bromo-5-methylimidazo[1,2-a]pyridine-2-carboxylate (300 mg, 1.0 mmol) to obtain the compound (120 mg, 39%). LC-MS: m/z [M+H]⁺ =311

**Step 3:** The operation method was the same as that in the synthesis example of compound vii, using 3-amino-8-bromo-5-methyl-N-propylimidazo[1,2-a]pyridine-2-carboxamide (30 mg, 0.1 mmol) and (2-fluoro-6-methoxyphenyl)boronic acid (25 mg, 0.15 mmol) as starting materials to obtain the title compound (10 mg, 28%).
¹H NMR (400 MHz, DMSO-d6) 7.54 (s, 1H), 7.41 (d, *J =* 7.1 Hz, 1H), 6.97 (d, *J=* 8.4 Hz, 1H), 6.89 (t, *J =* 8.6 Hz, 1H), 6.78 (d, *J =* 6.8 Hz, 1H), 6.48 (d, *J =* 6.8 Hz, 1H), 5.87 (s, 2H), 3.69 (s, 3H), 3.21 - 3.08 (m, 2H), 2.94 (s, 3H), 1.46 (dd, *J =* 14.6, 7.3 Hz, 2H), 0.81 (t, *J* = 7.4 Hz, 3H). LC-MS: m/z [M+H]⁺ =357

### Example 134

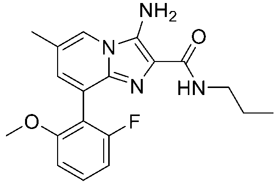

### 3-amino-8-(2-fluoro-6-methoxyphenyl)-6-methyl-N-propylimidazo[1,2-a]pyridine-2-carboxamide

### Step 1: ethyl 3-amino-8-bromo-6-methylimidazo[1,2-a]pyridine-2-carboxylate

The operation was the same as that for intermediate iv (route 2), using 3-bromo-5-methylpyridin-2-amine (500 mg, 2.7 mmol) as a starting material to obtain the target product (170 mg, 31%). LC-MS: m/z [M+H]⁺ =298

### Step 2: 3-amino-8-bromo-6-methyl-N-propylimidazo[1,2-a]pyridine-2-carboxamide

The operation was the same as that for intermediate vi (route 1), using ethyl 3-amino-8-bromo-6-methylimidazo[1,2-a]pyridine-2-carboxylate (150 mg, 0.5 mmol) to obtain the compound (55 mg, 35%). LC-MS: m/z [M+H]⁺ =311

**Step 3:** The operation method was the same as that in the synthesis example of compound vii, using 3-amino-8-bromo-6-methyl-N-propylimidazo[1,2-a]pyridine-2-carboxamide (55 mg, 0.17 mmol) and 2-fluoro-6-methoxyphenylboronic acid (33 mg, 0.19 mmol) to obtain the title product (17 mg, cyan solid) in a yield of 28%.

¹HNMR(400 MHz, DMSO-*d₆*) 7.96 (s, 1H), 7.54 (t, J *=* 6.1 Hz, 1H), 7.43 (dd, J *=* 15.3, 8.3 Hz, 1H), 6.98 (d, J = 8.4 Hz, 1H), 6.91 (t, J = 8.6 Hz, 1H), 6.81 (d, J = 1.2 Hz, 1H), 6.03 (d, J = 9.6 Hz, 2H), 3.70 (s, 3H), 3.14 (dd, J = 14.1, 6.5 Hz, 2H), 2.28 (s, 3H), 1.46 (dd, J = 14.5, 7.3 Hz, 2H), 0.81 (t, J = 7.4 Hz, 3H) LC-MS: m/z [M+H]⁺ = 357.

### Example 135

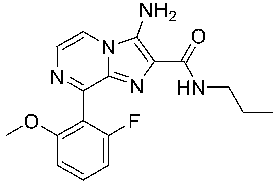

### Step 1: ethyl 3-amino-8-chloroimidazo[1,2-a]pyrazine-2-carboxylate

3-Chloropyrazin-2-amine (1 g, 7.7 mmol), ethyl glyoxylate (1.2 g, 11.6 mmol), and DBU (1.3 g, 11.6 mmol) were added to 8 mL of tetrahydrofuran. The mixture was cooled to 0°C, and trimethylsilyl cyanide (1.15 g, 11.6 mmol) was subsequently added. The mixture was reacted under microwave irradiation at 120°C for 2 hours. The mixture was concentrated and purified by column chromatography (petroleum ether:ethyl acetate = 4:1) to obtain the title product (170 mg, green solid) in a yield of 9.2%. LC-MS: m/z [M+H]⁺ =241.

### Step 2: ethyl 3-amino-8-(2-fluoro-6-methoxyphenyl)imidazo[1,2-a]pyrazine-2-carboxylate

The operation method was the same as that in the synthesis example of compound vii, using ethyl 3-amino-8-chloroimidazo[1,2-a]pyrazine-2-carboxylate (200 mg, 0.83 mmol) and 2-fluoro-6-methoxyphenylboronic acid (156 mg, 0.92 mmol) as starting materials to obtain the title product (113 mg, cyan solid) in a yield of 41%. LC-MS: m/z [M+H]⁺ =331

### Step 3: 3-amino-8-(2-fluoro-6-methoxyphenyl)imidazo[1,2-a]pyrazine-2-carboxylic acid

Ethyl 3-amino-8-(2-fluoro-6-methoxyphenyl)imidazo[1,2-a]pyrazine-2-carboxylate (60 mg, 0.18 mmol) and lithium hydroxide monohydrate (62 mg, 1.5 mmol) were sequentially added to a mixed solution of 4 mL tetrahydrofuran and 1 mL water. The mixture was kept at room temperature for 2 hours. The mixture was added with trifluoroacetic acid to adjust the pH to 6, thus yielding the title product (50 mg, cyan solid). LC-MS: m/z [M+H]⁺ =303

### Step 4: 3-amino-8-(2-fluoro-6-methoxyphenyl)-N-propylimidazo[1,2-a]pyrazine-2-carboxamide

3-Amino-8-(2-fluoro-6-methoxyphenyl)imidazo[1,2-a]pyrazine-2-carboxylic acid (50 mg, 0.17 mmol) and HATU (97 mg, 0.25 mmol) were added to DMF (5 mL). The mixture was stirred at room temperature for 30 minutes, and triethylamine (50 mg, 0.5 mmol) and n-propylamine (12 mg, 0.20 mmol) were then added. The mixture was stirred at room temperature for another 2 hours to obtain the target product (18 mg, yellow solid) in a yield of 33%.
1HNMR(400 MHz, DMSO-*d₆*) 8.22 (d, J = 4.8 Hz, 1H), 7.90 (t, J = 6.0 Hz, 1H), 7.80 (d, J = 4.8 Hz, 1H), 7.50 (dd, J = 15.4, 8.3 Hz, 1H), 7.02 (d, J = 8.5 Hz, 1H), 6.94 (t, J = 8.7 Hz, 1H), 6.49 (s, 2H), 3.70 (s, 3H), 3.16 (dd, J = 13.8, 6.6 Hz, 2H), 1.47 (dd, J = 14.5, 7.3 Hz, 2H), 0.82 (t, J = 7.4 Hz, 3H) . LC-MS: m/z [M+H]⁺ = 344

### Example 136

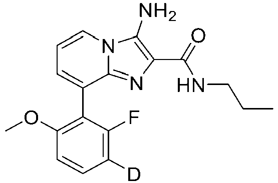

### 3-Amino-8-(2-fluoro-6-methoxyphenyl-3-d)-N-propylimidazo[1,2-a]pyridine-2-carboxamide

### Step 1: (2-fluoro-6-methoxyphenyl-3-d)boronic acid

(2-Fluoro-6-methoxyphenyl-3-bromo)boronic acid (200 mg, 0.8 mmol) was dissolved in deuterated methanol (CD₃OD, 2 mL). Palladium on carbon (30 mg, pre-stirred in deuterated methanol for 24 hours) was added. The mixture was stirred at room temperature under deuterium atmosphere for 2 hours. The mixture was filtered through diatomite. The organic phase was concentrated, and the resulting product was directly used in the next step.

**Step 2:** The operation method was the same as that in the synthesis example of compound vii, using 3-amino-8-bromo-N-propylimidazo[1,2-a]pyridine-2-carboxamide (100 mg, 0.34 mmol) and (2-fluoro-6-methoxyphenyl-3-d)boronic acid (115 mg, 0.68 mmol) as starting materials to obtain the target product (30 mg, 25%) as a yellow solid.
¹H NMR (400 MHz, DMSO-*d₆*) 8.09 - 8.24 (m, 1 H) 7.52 - 7.68 (m, 1 H) 7.39 - 7.51 (m, 1 H) 6.97 - 7.03 (m, 1 H) 6.82 - 6.95 (m, 2 H) 6.14 (s, 2 H)3.70 (s, 3 H) 3.12 - 3.19 (m, 2 H) 1.41 - 1.52 (m, 2 H) 0.82 (t, J=7.34 Hz, 3 H). LC-MS: m/z [M+1]⁺= 344

### Example 137

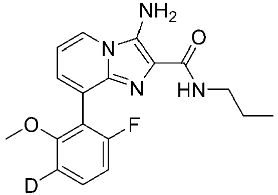

### 3-Amino-8-(2-fluoro-6-methoxyphenyl-5-d)-N-propylimidazo[1,2-a]pyridine-2-carboxamide

### Step 1: (2-fluoro-6-methoxy-5-deutero)boronic acid

(2-Fluoro-6-methoxyphenyl-5-bromo)boronic acid (200 mg, 0.8 mmol) was dissolved in deuterated methanol (CD₃OD, 2 mL). Palladium on carbon (30 mg, pre-stirred in deuterated methanol for 24 hours) was added. The mixture was stirred at room temperature under deuterium atmosphere for 2 hours. The mixture was filtered through diatomite. The organic phase was concentrated, and the resulting product was directly used in the next step.

**Step 2:** The operation method was the same as that in the synthesis example of compound vii, using 3-amino-8-bromo-N-propylimidazo[1,2-a]pyridine-2-carboxamide (100 mg, 0.34 mmol) and (2-fluoro-6-methoxy-5-deutero)boronic acid (120 mg, 0.68 mmol) as starting materials to obtain the target product (26 mg, 23%) as a white solid.
¹H NMR (400 MHz, DMSO-*d₆*) 8.15 (d, J=6.85 Hz, 1 H) 7.58 (br. s., 1 H) 7.43 (s, 1 H) 6.84 - 6.96 (m, 3 H) 6.14 (s, 2 H) 3.70 (s, 3 H) 3.15 (d, J=6.85 Hz, 2 H) 1.46 (d, J=7.34 Hz, 2 H) 0.81 (t, J=7.34 Hz, 3 H).LC-MS: m/z [M+1]⁺= 344

### Example 138

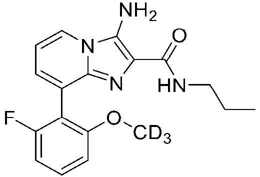

### 3-Amino-8-(2-fluoro-6-(methoxy-d3)phenyl)-N-propylimidazo[1,2-a]pyridine-2-carboxamide

### Step 1: 2-bromo-1-fluoro-3-(methoxy-d3)benzene

2-Bromo-3-fluorophenol (1 g, 5.26 mmol) was dissolved in acetonitrile (30 mL), and potassium carbonate (1.45 g, 10.5 mmol) and deuterated iodomethane (CAS No.: 865-50-9, 2.28 g, 15.75 mmol) were added. The mixture was stirred at 50°C for 5 hours, filtered, and concentrated. Water (50 mL) was added, and the mixture was extracted with dichloromethane (50 mL × 3). The organic phases were combined, dried over anhydrous sodium sulfate, and concentrated to obtain 0.93 g of crude product.

### Step 2: 2-(2-fluoro-6-(methoxy-d3)phenyl)-4,4,5,5-tetramethyl-1,3,2-dioxaborolane

2-Bromo-1-fluoro-3-(methoxy-d3)benzene (800 mg, 3.85 mmol), bis(pinacolato)diboron (1.47 g, 5.78 mmol), potassium acetate (754 mg, 7.7 mmol), and [1,1'-bis(diphenylphosphino)ferrocene]palladium(II) dichloride dichloromethane complex (314 mg, 0.39 mmol) were sequentially added to dioxane. The mixture was stirred at 100°C for 16 hours under argon atmosphere. The mixture was concentrated and purified by column chromatography (petroleum ether:ethyl acetate = 10:1) to obtain the target product (610 mg, 62%) as a white solid.

**Step 3:** The operation method was the same as that in the synthesis example of compound vii, using 3-amino-8-bromo-N-propylimidazo[1,2-a]pyridine-2-carboxamide (100 mg, 0.34 mmol) and 2-(2-fluoro-6-(methoxy-d3)phenyl)-4,4,5,5-tetramethyl-1,3,2-dioxaborolane (173 mg, 0.68 mmol) as starting materials to obtain the target product (70 mg, 60%) as a yellow solid.
¹H NMR (400MHz, DMSO-*d₆*) 8.16 (d, J = 6.4 Hz, 1 H), 7.57 (br. s., 1 H), 7.49 - 7.38 (m, 1 H), 7.03 - 6.85 (m, 4 H), 6.15 (br. s., 2 H), 3.16 (d, J = 6.8 Hz, 2 H), 1.52 - 1.43 (m, 2 H), 0.82 (t, J = 7.1 Hz, 3 H). LC-MS: m/z [M+H]⁺ = 346

### Example 139

### 3-Amino-8-(1,3-dimethyl-1H-pyrazol-4-yl)-N-propylimidazo[1,2-a]pyridine-2-carboxamide

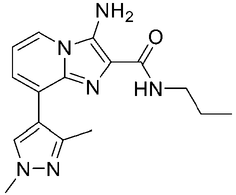

The operation method was the same as that in the synthesis example of compound vii, using 3-amino-8-bromo-N-propylimidazo[1,2-a]pyridine-2-carboxamide (0.1 g, 0.34 mmol) and 1,3-dimethyl-4-(tetramethyl-1,3,2-dioxaborolan-2-yl)-1H-pyrazole (0.11 g, 0.51 mmol) as starting materials to obtain the target product (25 mg, 24%) as a yellow solid.
¹HNMR (400MHz, DMSO-*d₆*) 8.86 (s, 1 H), 8.01 (d, *J* = 5.4 Hz, 2 H), 7.19 (d, *J* = 4.9 Hz, 1 H), 6.92 - 6.76 (m, 1 H), 6.10 (br. s., 2 H), 3.87 (s, 3 H), 3.27 (d, *J =* 5.4 Hz, 2 H), 2.41 (s, 3 H), 1.57 (d, *J =* 6.4 Hz, 2 H), 0.97 - 0.84 (m, 3 H).LC-MS: m/z [M+1]⁺= 313

### Example 140

### 3-Amino-8-(1-methyl-1H-imidazol-5-yl)-N-propylimidazo[1,2-a]pyridine-2-carboxamide

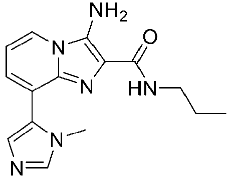

The operation method was the same as that in the synthesis example of compound vii, using 3-amino-8-bromo-N-propylimidazo[1,2-a]pyridine-2-carboxamide (0.1 g, 0.64 mmol) and 1-methyl-5-(tetramethyl-1,3,2-dioxaborolan-2-yl)-1H-imidazole (0.11 g, 0.51 mmol) as starting materials to obtain the target product (20 mg, 19%) as a yellow solid.
¹HNMR (400MHz, DMSO-*d₆*) 8.17 (d, *J =* 6.8 Hz, 1 H), 7.89 (s, 1 H), 7.84 (s, 1 H), 7.14 (d, *J =* 6.8 Hz, 1 H), 6.91 (t, *J =* 8 Hz, 1 H), 6.19 (s, 2 H), 3.74 (s, 3H), 3.23 - 3.11 (m, 2 H), 1.56 - 1.43 (m, 2 H), 0.86 (t, *J* = 7.3 Hz, 3 H). LC-MS: m/z [M+1]⁺= 299

### Example 141

### 3-Amino-8-(5-methyl-1,2-oxazol-4-yl)-N-propylimidazo[1,2-a]pyridine-2-carboxamide

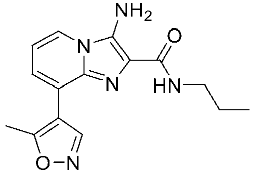

The operation method was the same as that in the synthesis example of compound vii, using 3-amino-8-bromo-N-propylimidazo[1,2-a]pyridine-2-carboxamide (0.1 g, 0.34 mmol) and 5-methyl-4-(tetramethyl-1,3,2-dioxaborolan-2-yl)-1,2-oxazole (0.11 g, 0.51 mmol) to obtain the target product (35 mg, 34%) as a yellow solid.
¹H NMR (400MHz, DMSO-*d₆*) 9.49 (s, 1 H), 8.15 (d, *J* = 6.8 Hz, 1 H), 8.07 (t, *J* = 5.6 Hz, 1 H), 7.23 (d, *J =* 6.8 Hz, 1 H), 6.92 (t, *J =* 6.8 Hz, 1 H), 6.18 (s, 2 H), 3.24 (q, *J=* 6.7 Hz, 2 H), 2.67 (s, 3 H), 1.61 - 1.47 (m, 2 H), 0.89 (t, *J* = 7.3 Hz, 3H). LC-MS: m/z [M+1]⁺ = 300

### Example 142

### 3-Amino-8-(2-methylpyridin-3-yl)-N-propylimidazo[1,2-a]pyridine-2-carboxamide

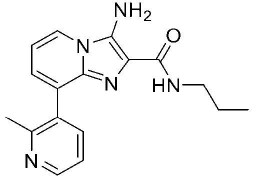

The operation method was the same as that in the synthesis example of compound vii, using 3-amino-8-bromo-N-propylimidazo[1,2-a]pyridine-2-carboxamide (0.1 g, 0.34 mmol) and (2-methylpyridin-3-yl)boronic acid (0.07 g, 0.51 mmol) as starting materials to obtain the target product (40 mg, 38%) as a yellow solid
¹H NMR (400MHz, DMSO-*d₆*) 8.52 (d, *J* = 4.9 Hz, 1 H), 8.21 (d, *J* = 6.8 Hz, 1 H), 7.72 (d, *J =* 7.3 Hz, 1 H), 7.68 - 7.61 (m, 1 H), 7.33 (dd, *J =* 4.9, 7.3 Hz, 1 H), 7.00 (d, *J =* 6.8 Hz, 1 H), 6.95 - 6.88 (m, 1 H), 6.21 (s, 2 H), 3.21 - 3.09 (m, 2 H), 2.35 (s, 3 H), 1.51 - 1.40 (m, 2 H), 0.85 - 0.75 (m, 3H). LC-MS: m/z [M+1]⁺= 310

### Example 143

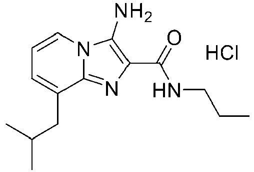

### 3-Amino-8-isobutyl-N-propylimidazo[1,2-a]pyridine-2-carboxamide hydrochloride

3-Amino-8-bromo-N-propylimidazo[1,2-a]pyridine-2-carboxamide (100 mg, 0.34 mmol), (2-methylpropyl)boronic acid (173 mg, 1.7 mmol), cesium carbonate (221 mg, 0.68 mmol), and [1,1'-bis(diphenylphosphino)ferrocene]dichloropalladium(II) dichloromethane complex (CAS No.: 95464-05-4, 25 mg, 0.03 mmol) were sequentially added to a mixed solution of toluene and water (2 mL/0.2 mL). The reaction mixture was stirred at 100°C for 2 hours under argon atmosphere. 20 mL of water was added. The mixture was extracted with dichloromethane (20 mL × 3). The organic phase was concentrated and purified by column chromatography (petroleum ether:ethyl acetate = 1:1 to 1:5) to obtain the target product. The product was dissolved in dichloromethane (10 mL), followed by the addition of hydrochloric acid (4M in methanol, 2 mL). The mixture was stirred for 5 minutes and then concentrated to obtain the target product (70 mg, 66%) as an off-white solid.
¹H NMR (400MHz, DMSO-*d₆*) 8.49 (br. s., 1 H), 7.55 (br. s., 1 H), 7.31 (br. s., 1 H), 3.29 (br. s., 2 H), 2.91-2.87 (m, 2 H), 2.09-2.05 (m, 1 H), 1.58 (d, J = 6.4 Hz, 2 H), 0.95-0.91 (m, 9 H). LC-MS: m/z [M+H]⁺ = 275

### Example 144

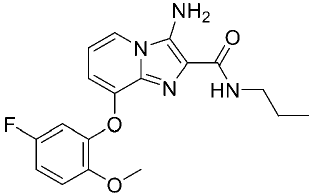

### 3-Amino-8-(5-fluoro-2-methoxyphenoxy)-N-propylimidazo[1,2-a]pyridine-2-carboxamide

### Step 1: 3-(5-fluoro-2-methoxyphenoxy)-2-nitropyridine

3-Fluoro-2-nitropyridine (1 g, 7.04 mmol), 5-fluoro-2-methoxyphenol (800 mg, 7.04 mmol), and cesium carbonate (4.6 g, 14.08 mmol) were dissolved in 20 mL of acetonitrile. The mixture was stirred at room temperature for 2 hours. 20mL of water was added, and the resulting mixture was extracted with dichloromethane (30 mL × 2). The organic phase was dried, concentrated, and purified by column chromatography (dichloromethane) to obtain 1.6 g of a pale yellow solid. LC-MS: m/z [M+1]⁺=265

### Step 2: 3-(5-fluoro-2-methoxyphenoxy)pyridin-2-amine

Under hydrogen atmosphere, 3-(5-fluoro-2-methoxyphenoxy)-2-nitropyridine (1.6 g, 6.06 mmol) and Raney nickel (100 mg) were dissolved in 20 mL of methanol and the mixture was stirred at room temperature for 2 hours. The solids were filtered off, and the organic phase was concentrated to obtain 1.2 g of crude product, which was directly used in the next step. LC-MS: m/z [M+1]⁺=235

### Step 3: Ethyl 8-(5-fluoro-2-methoxyphenoxy)imidazo[1,2-a]pyridine-2-carboxylate

3-(5-Fluoro-2-methoxyphenoxy)pyridin-2-amine (1.2 g, 5.12 mmol) and ethyl bromopyruvate (1.2 g, 6.14 mmol) were dissolved in ethanol (20 mL). The mixture was heated to 90°C and stirred for 16 hours. The organic phase was concentrated and purified by column chromatography (petroleum ether: ethyl acetate = 10:1) to obtain 2 g of a yellow oil. LC-MS: m/z [M+1]⁺=331

### Step 4: Ethyl 8-(5-fluoro-2-methoxyphenoxy)-3-nitroimidazo[1,2-a]pyridine-2-carboxylate

Ethyl 8-(5-fluoro-2-methoxyphenoxy)imidazo[1,2-a]pyridine-2-carboxylate (2 g, 6.05 mmol), trifluoroacetic acid (0.8 g, 7.26 mmol), and trifluoroacetic anhydride (1.5 g, 7.26 mmol) were dissolved in 1,2-dichloroethane (20 mL). The mixture was cooled to 0°C, and tetrabutylammonium nitrate (CAS No.: 1941-27-1, 1.8 g, 6.05 mmol) was added in batches. The reaction mixture was stirred at 0°C for 1 hour, then added with water (20 mL), and neutralized to pH 7 with an aqueous sodium bicarbonate solution. The mixture was extracted with dichloromethane (50 mL × 2). The organic phase was dried over anhydrous sodium sulfate, concentrated, and purified by column chromatography (petroleum ether: ethyl acetate = 1:1) to obtain 300 mg of a yellow solid. LC-MS: m/z [M+1]⁺=376

### Step 5: Ethyl 3-amino-8-(5-fluoro-2-methoxyphenoxy)imidazo[1,2-a]pyridine-2-carboxylate

Ethyl 8-(5-fluoro-2-methoxyphenoxy)-3-nitroimidazo[1,2-a]pyridine-2-carboxylate (300 mg, 0.8 mmol) and zinc powder (260 mg, 4 mmol) were dissolved in 10 mL of methanol. Then, 2 mL of a saturated aqueous ammonium chloride solution was added, and the mixture was stirred at room temperature for 2 hours. The solid was filtered off, and 30 mL of water was added. The mixture was extracted with dichloromethane (50 mL × 2). The organic phase was dried over anhydrous sodium sulfate and concentrated to obtain 200 mg of crude product, which was directly used in the next step. LC-MS: m/z [M+1]⁺=346

**Step 6:** Ethyl 3-amino-8-(5-fluoro-2-methoxyphenoxy)imidazo[1,2-a]pyridine-2-carboxylate (200 mg, 0.58 mmol) was dissolved in 5 mL of n-propylamine. The mixture was heated to 120°C in a sealed tube and stirred overnight. The reaction mixture was concentrated and purified by preparative thin-layer chromatography (petroleum ether:ethyl acetate = 1:1) to obtain the target product (32 mg, 15%) as a gray solid.
¹H NMR (400 MHz, DMSO-*d₆*) δ 7.97 (t, J = 6.0 Hz, 1H), 7.88 (d, J = 6.6 Hz, 1H), 7.22 (dd, J = 9.7, 5.3 Hz, 1H), 7.14 - 7.07 (m, 2H), 6.67 (t, J = 7.2 Hz, 1H), 6.18 (s, 2H), 6.12 (d, J = 7.3 Hz, 1H), 3.74 (s, 3H), 3.22 (dd, J = 13.7, 6.5 Hz, 2H), 1.59 - 1.47 (m, 2H), 0.87 (t, J = 7.4 Hz, 3H). LC-MS: m/z [M+1]⁺=359

### Example 145

### 3-Amino-8-(6-fluoropyridin-2-yl)-N-propylimidazo[1,2-a]pyridine-2-carboxamide

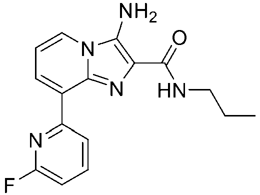

The operation method was the same as that in the synthesis example of compound vii, using 3-amino-8-bromo-N-propylimidazo[1,2-a]pyridine-2-carboxamide (0.1 g, 0.34 mmol) and (6-fluoropyridin-2-yl)boronic acid (0.72 g, 0.51 mmol) as starting materials to obtain the target product (67 mg, 63%) as a yellow solid
¹H NMR (400MHz, DMSO-*d₆*) 9.40 (d, *J* = 7.3 Hz, 1 H), 8.29 (d, *J* = 6.8 Hz, 1 H), 8.12 (td, *J =* 8.1*,* 16.5 Hz, 3 H), 7.24 - 7.16 (m, 1 H), 7.08 - 6.97 (m, 1 H), 6.23 (s, 2 H), 3.31 - 3.19 (m, 2 H), 1.65 - 1.49 (m, 2 H), 0.98 - 0.85 (m, 3 H). LC-MS: m/z [M+1]⁺ = 314

### Example 146

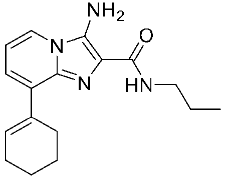

### 3-Amino-8-(cyclohex-1-en-1-yl)-N-propylimidazo[1,2-a]pyridine-2-carboxamide

The operation method was the same as that in the synthesis example of compound vii, using 3-amino-8-bromo-N-propylimidazo[1,2-a]pyridine-2-carboxamide (0.1 g, 0.34 mmol) and 2-(cyclohex-1-en-1-yl)-4,4,5,5-tetramethyl-1,3,2-dioxaborolane (218 mg, 1.05 mmol) as starting materials to obtain the target product (50 mg, 51%) as a yellow solid.
1H NMR (400MHz ,DMSO-d₆) 7.99 (d, *J* = 6.8 Hz, 1 H), 7.80 (t, *J* = 6.1 Hz, 1 H), 7.30 (d, *J =* 5.9 Hz, 1 H), 6.99 (d, *J =* 6.8 Hz, 1 H), 6.79 (t, *J =* 6.8 Hz,1 H), 6.06 (s, 2 H), 3.28 - 3.19 (m, 2 H), 2.56 - 2.51 (m, 2 H), 2.28 (br. s., 2 H), 1.79 - 1.71 (m, 2 H), 1.65 (d, *J* = 5.4 Hz, 2 H), 1.59 - 1.48 (m, 2 H), 0.93 - 0.84 (m, 3 H) LC-MS: m/z [M+1]⁺ = 285

### Example 147

### 3-Amino-8-cyano-N-propylimidazo[1,2-a]pyridine-2-carboxamide

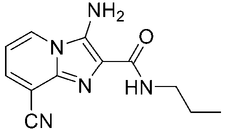

3-Amino-8-bromo-N-propylimidazo[1,2-a]pyridine-2-carboxamide (0.1 g, 0.34 mmol), zinc cyanide (0.020 g, 0.17 mmol), zinc powder (0.0022 g, 0.034 mmol), tris(dibenzylideneacetone)dipalladium(0) (0.016 g, 0.017 mmol), and X-Phos (CAS No.: 564483-18-7, 0.016 g, 0.034 mmol) were sequentially added to N,N-dimethylacetamide (4 mL). The mixture was stirred at 140°C for 3 hours under argon atmosphere. 40 ml of water was added. The mixture was extracted with ethyl acetate (40 ml × 2), concentrated, and purified by column chromatography to obtain the target product (61 mg, 52%) as a yellow solid.
¹HNMR (400MHz, DMSO-*d₆*) 8.48-8.46 (m, 1 H), 8.02-7.97 (m, 1 H), 7.83-7.77 (m, 1 H), 7.02-6.95 (m, 1 H), 6.38 ( s, 2 H), 3.28 - 3.20 (m, 2 H), 1.58-1.50 (m, 2 H), 0.92-0.84 (m, 3H). LC-MS: m/z [M+1]⁺ = 244

### Example 148

### 3-Amino-7-cyano-8-(2-fluoro-6-methoxyphenyl)-N-propylimidazo[1,2-a]pyridine-2-carboxamide

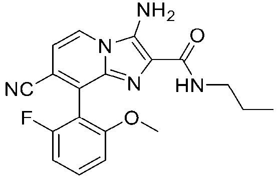

### Step 1: Ethyl 8-chloro-7-iodo-3-nitroimidazo[1,2-a]pyridine-2-carboxylate

The operation method was the same as that in the synthesis of intermediate iv (route 1, step 1 and step 2), using 3-chloro-4-iodopyridin-2-amine (10 g, 39.3 mmol) as a starting material to obtain the title compound (7.75 g, yield 50%) as a yellow solid. LCMS: m/z [M+H]⁺=396.

### Step 2: 8-chloro-7-iodo-3-nitroimidazo[1,2-a]pyridine-2-carboxylic acid

Ethyl 8-chloro-7-iodo-3-nitroimidazo[1,2-a]pyridine-2-carboxylate (1 g, 2.5 mmol) was added to a mixed solution of tetrahydrofuran (10 mL) and water (10 mL), and lithium hydroxide (181 mg, 7.6 mmol) was subsequently added. The mixture was stirred at 25°C for 2 hours. The mixture was added to water (100 ml) and extracted with ethyl acetate (100 ml). The aqueous phase was adjusted to pH <7 with 1M hydrochloric acid. The mixture was extracted with ethyl acetate (100 mL×2). The organic phases were combined and concentrated to obtain the title compound (804 mg, yield 86.5%) as a yellow solid. LCMS: m/z [M+H]⁺=368.

### Step 3: 8-chloro-7-iodo-3-nitro-N-propylimidazo[1,2-a]pyridine-2-carboxamide

8-Chloro-7-iodo-3-nitroimidazo[1,2-a]pyridine-2-carboxylic acid (800 mg, 2.2 mmol) was added to dichloromethane (20 mL), and triethylamine (661 mg, 6.5 mmol) and propylphosphonic anhydride (2.7 g, 4.4 mmol) were sequentially added. The mixture was stirred at 25°C for 0.5 hours. Then, n-propylamine (193 mg, 3.3 mmol) was added to the above mixture, and the resulting mixture was stirred at 25°C for 2 hours. The mixture was added with a saturated aqueous solution of ammonium chloride (100 mL) and extracted with dichloromethane (100 mL × 2). The organic layer was washed with brine and dried over anhydrous sodium sulfate. It was then purified by column chromatography (petroleum ether:ethyl acetate = 10:1 to 2:1) to obtain the title compound (580 mg, yield 65.2%) as a yellow solid. LCMS: m/z [M+H]⁺=409.

### Step 4: 8-chloro-7-cyano-3-nitro-N-propylimidazo[1,2-a]pyridine-2-carboxamide

8-Chloro-7-iodo-3-nitro-N-propylimidazo[1,2-a]pyridine-2-carboxamide (560 mg, 1.4 mmol) was added to N,N-dimethylformamide (5 mL), and copper(I) cyanide (368 mg, 4.1 mmol) was subsequently added. The mixturer was stirred under microwave irradiation at 120°C for 1 hour. The mixture was added dropwise into 20 mL of water and filtered. The filter cake was concentrated to obtain the title compound (445 mg, crude) as a yellow solid. LCMS: m/z [M+H]⁺=308.

### Step 5: 3-amino-8-chloro-7-cyano-N-propylimidazo[1,2-a]pyridine-2-carboxamide

8-Chloro-7-cyano-3-nitro-N-propylimidazo[1,2-a]pyridine-2-carboxamide (200 mg, 0.65 mmol) was added to ethanol (3 mL), and zinc powder (0.43 g, 6.5 mmol) and ammonium chloride (347 mg, 6.5 mmol) were subsequently added. The resulting mixture was stirred at 25°C for 2 hours. The mixture was filtered. The filtrate was concentrated to obtain the title compound (120 mg, crude) as a yellow solid. LCMS: m/z [M+H]⁺=278.

**Step 6:** 3-Amino-8-chloro-7-cyano-N-propylimidazo[1,2-a]pyridine-2-carboxamide (60 mg, 0.22 mmol) was added to a mixed solvent of 1,4-dioxane (1.5 mL) and water (0.5 mL), and cesium carbonate (215 mg, 0.66 mmol), (2-fluoro-6-methoxyphenyl)boronic acid (112 mg, 0.66 mmol), tris(dibenzylideneacetone)dipalladium (20 mg, 0.022 mmol), and X-Phos (11 mg, 0.022 mmol) were sequentially added. The reaction mixture was stirred at 100°C for 2 hours under argon atmosphere. The mixture was added with water (100 mL) and extracted with ethyl acetate (100 mL). The organic layer was rinsed with brine and dried over anhydrous sodium sulfate. It was purified by column chromatography (petroleum ether:ethyl acetate = 10:1 to 2:1) to obtain the target product (24 mg, yield 30%) as a yellow solid.

¹H NMR (400 MHz, DMSO-*d₆*) δ 8.33 (d, *J* = 5.7 Hz, 1H), 7.85-7.81 (m, 1H), 7.59 - 7.50 (m, 1H), 7.20 (d, *J* = 5.9Hz, 1H), 7.09 (d, *J* = 6.9 Hz, 1H), 7.01 (t, *J* = 8.5 Hz, 1H), 6.59 (s, 2H), 3.76 (s, 3H), 3.18-3.13 (m, 2H), 1.47 (dd, *J* = 14.0, 7.0 Hz, 2H), 0.81 (t, *J* = 6.6 Hz, 3H). LCMS: m/z [M+H]⁺= 368.

### Example 149

### 3-Amino-8-(3-methylmorpholino)-N-propylimidazo[1,2-a]pyridine-2-carboxamide

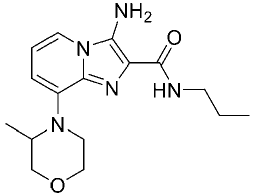

### Step 1: Ethyl 8-fluoro-3-nitroimidazo[1,2-a]pyridine-2-carboxylate

The operation method was the same as that in the synthesis of intermediate iv (route 1, step 1 and step 2), using 3-fluoropyridin-2-amine (1.5 g, 13.4 mmol) and ethyl bromopyruvate (3.9 g, 20 mmol) as starting materials to obtain the title product as a yellow solid (500 mg, yield 15%). LC-MS: m/z [M+1]⁺ =254

### Step 2: Ethyl 8-(3-methylmorpholino)-3-nitroimidazo[1,2-a]pyridine-2-carboxylate

Ethyl 8-fluoro-3-nitroimidazo[1,2-a]pyridine-2-carboxylate (200 mg, 0.79 mmol) and 3-methylmorpholine (160 mg, 1.58 mmol) were dissolved in dimethyl sulfoxide (5 mL). The mixture was heated to 100°C and stirred for 12 hours. The reaction mixture was cooled, diluted with 50 mL of water, and extracted with ethyl acetate (20 mL × 3). The organic phase was dried over anhydrous sodium sulfate, concentrated, and purified by column chromatography (petroleum ether : ethyl acetate = 5 : 1) to obtain the target product as a white solid (200 mg, yield 76%).

### Step 3: 8-(3-methylmorpholino)-3-nitro-N-propylimidazo[1,2-a]pyridine-2-carboxamide

Ethyl 8-(3-methylmorpholino)-3-nitroimidazo[1,2-a]pyridine-2-carboxylate (200 mg, 0.60 mmol) was dissolved in n-propylamine (5 mL). The mixture was stirred at 100°C in a sealed tube for 12 hours. The reaction mixture was concentrated to obtain the target product as a white solid (200 mg, crude).

**Step 4:** 8-(3-methylmorpholino)-3-nitro-N-propylimidazo[1,2-a]pyridine-2-carboxamide (150 mg, 0.58 mmol), zinc powder (379 mg, 5.8 mmol), and saturated aqueous ammonium chloride solution (1 mL) were dissolved in methanol (5 mL). The mixture was stirred at room temperature for 1 hour. Water (20 mL) was added, and the mixture was extracted with dichloromethane (20 mL × 3). The organic phase was dried over anhydrous sodium sulfate, concentrated, and purified by preparative thin-layer chromatography (ethyl acetate) to obtain the target product as a white solid (20 mg, yield 11%).
¹H NMR (400 MHz, DMSO-*d₆*) 7.72 - 7.80 (m, 1 H) 7.64 - 7.70 (m, 1 H) 6.66 - 6.72 (m, 1 H) 6.21 - 6.26 (m, 1 H) 5.99 (s, 2 H) 5.10 - 5.19 (m, 1 H) 3.83 - 3.96 (m, 2 H) 3.59 - 3.69 (m, 2 H) 3.15 - 3.26 (m, 4 H) 1.48 - 1.58 (m, 2 H) 0.94 (d, J=6.85 Hz, 3 H) 0.85 - 0.90 (m, 3 H). LC-MS: m/z [M+1]⁺ = 318

### Example 150

### 3-Amino-8-(2-fluoro-6-methoxyphenyl)-N-propylimidazo[1,2-a]pyridine-7-deuterio-2-carboxamide

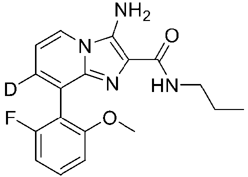

### Step 1: 3-chloropyridine-4-deuterium-2-amine

3-Chloro-4-iodopyridin-2-amine (2 g, 8 mmol) was dissolved in deuterated methanol (10 mL). Raney nickel (200 mg, pre-stirred in deuterated methanol for 10 minutes and filtered, which was repeated three times) was added. The mixture was stirred under deuterium atmosphere at 50°C for 72 hours. After filtration, the filtrate was concentrated and purified by column chromatography to obtain an off-white solid (500 mg, yield 48%). LC-MS: m/z [M+1]⁺ =130.

### Step 2: 3-amino-8-chloro-N-propylimidazo[1,2-a]pyridine-7-deuterium-2-carboxamide

The operation was the same as that for intermediate iv (route 1) and intermediate vi (route 2), using 3-chloropyridine-4-deuterium-2-amine (500 mg, 3.85 mmol) as a starting material to obtain the target product as a yellow-green solid (200 mg, 20%). LC-MS: m/z [M+1]⁺ =254.

**Step 3:** The operation method was the same as the synthesis method of Example 110, using 3-amino-8-chloro-N-propylimidazo[1,2-a]pyridine-7-deuterium-2-carboxamide (100 mg, 0.32 mmol) and (2-fluoro-6-methoxyphenyl)boronic acid (115 mg, 0.68 mmol) as starting materials to obtain the target product (70 mg, 64%) as a yellow solid.

¹H NMR (400MHz, DMSO-*d₆*) 8.15 (d, *J* = 6.8 Hz, 1 H), 7.57 (br. s., 1 H), 7.45-7.40 (m, 1 H), 6.98 (d, *J* = 8.3 Hz, 1 H), 6.94 - 6.85 (m, 2 H), 6.13(br. s., 2 H), 3.70 (s, 3 H), 3.17-3.13 (m, 2 H), 1.55 - 1.37 (m, 2 H), 0.81 (t, *J =* 7.3 Hz, 3 H). LC-MS: m/z [M+1]⁺ = 344.

### Example 151

### 3-Amino-8-((2-fluoro-6-methoxybenzyl)oxy)-N-propylimidazo[1,2-a]pyridine-2-carboxamide

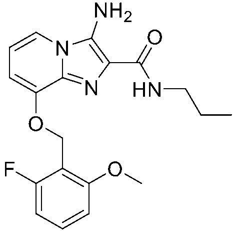

### Step 1: 8-((2-fluoro-6-methoxybenzyl)oxy)-3-nitroimidazo[1,2-a]pyridine-2-carboxylic acid

Ethyl 8-fluoro-3-nitroimidazo[1,2-a]pyridine-2-carboxylate (200 mg, 0.79 mmol), (2-fluoro-6-methoxyphenyl)methanol (247 mg, 1.58 mmol), and cesium carbonate (772 mg, 2.37 mmol) were dissolved in N,N-dimethylformamide (5 mL). The mixture was heated to 100°C and stirred for 2 hours. The reaction mixture was cooled, diluted with 50 mL of water, and neutralized with dilute hydrochloric acid. The mixture was extracted with dichloromethane (20 mL × 3). The organic phase was dried over anhydrous sodium sulfate and concentrated to obtain the target product as a white solid (250 mg, crude).

### Step 2: 8-((2-fluoro-6-methoxybenzyl)oxy)-3-nitro-N-propylimidazo[1,2-a]pyridine-2-carboxamide

8-((2-Fluoro-6-methoxybenzyl)oxy)-3-nitroimidazo[1,2-a]pyridine-2-carboxylic acid (250 mg, 0.69 mmol) was dissolved in dichloromethane (10 mL), and oxalyl chloride (1 mL) and one drop of N,N-dimethylformamide were subsequently added. The mixture was stirred at room temperature for 1 hour. The reaction mixture was concentrated, dissolved in dichloromethane (10 mL), and then added with n-propylamine (0.5 mL) and triethylamine (0.5 mL). The mixture was stirred at room temperature for 2 hours. The reaction mixture was washed with a saturated aqueous ammonium chloride solution, and the organic phase was dried, concentrated, and purified by column chromatography (petroleum ether:ethyl acetate = 1:1) to obtain the target product as a yellow solid (200 mg, yield 72%).

**Step 3:** 8-((2-Fluoro-6-methoxybenzyl)oxy)-3-nitro-N-propylimidazo[1,2-a]pyridine-2-carboxamide (200 mg, 0.5 mmol), zinc powder (327 mg, 5 mmol), and a saturated aqueous ammonium chloride solution (1 mL) were dissolved in methanol (5 mL). The mixture was stirred at room temperature for 1 hour. Water (30 mL) was added, and the mixture was extracted with dichloromethane (30 mL × 3). The organic phase was dried over anhydrous sodium sulfate, concentrated, and purified by preparative thin-layer chromatography (petroleum ether:ethyl acetate = 1:1) to obtain the target product as a white solid (54 mg, yield 29%).

¹H NMR (400 MHz, DMSO-*d₆*)7.91 (br. s., 1 H) 7.78 (d, J=6.85 Hz, 1 H) 7.41 - 7.52 (m, 1 H) 6.97 (d, J=8.31 Hz, 1 H) 6.91 (s, 1 H) 6.65 - 6.78 (m, 2 H) 6.09 (s, 2 H) 5.14 (s, 2 H) 3.84 (s, 3 H) 3.11 - 3.19 (m, 2 H) 1.47 (d, J=7.34 Hz, 2 H) 0.81 (t, J=7.34 Hz, 3 H). LC-MS: m/z [M+1]⁺ = 373.

### Example 154

### 3-Amino-8-(2-fluoro-6-methoxyphenyl)-N-propylimidazo[1,2-a]pyridine-6-deuterio-2-carboxamide

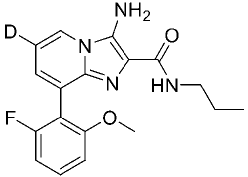

### Step 1: 3-chloropyridine-5-deuterium-2-amine

3-Chloro-5-iodopyridin-2-amine (2 g, 8 mmol) was dissolved in deuterated methanol (10 mL). Raney nickel (200 mg, pre-stirred in deuterated methanol for 10 minutes and filtered, which was repeated three times) was added. The mixture was stirred under deuterium atmosphere at 50°C for 72 hours. After filtration, the filtrate was concentrated and purified by column chromatography to obtain an off-white solid (400 mg, yield 39%). LC-MS: m/z [M+1]⁺ =130.

### Step 2: 3-amino-8-chloro-N-propylimidazo[1,2-a]pyridine-6-deuterium-2-carboxamide

The operation was the same as that for intermediate iv (route 1) and intermediate vi (route 2), using 3-chloropyridine-5-deuterium-2-amine (400 mg, 3.1 mmol) as a starting material to obtain the target product as a yellow-green solid (150 mg, 19%). LC-MS: m/z [M+1]⁺ = 254.

**Step 3:** The operation method was the same as the synthesis method of Example 110, using 3-amino-8-chloro-N-propylimidazo[1,2-a]pyridine-6-deuterium-2-carboxamide (100 mg, 0.32 mmol) and (2-fluoro-6-methoxyphenyl)boronic acid (115 mg, 0.68 mmol) as starting materials to obtain the target product (68 mg, 64%) as a yellow solid.

¹H NMR (400MHz, DMSO-*d₆*) 8.17 (br. s., 1 H), 7.60 (br. s., 1 H), 7.46-7.41 (m, 1 H), 7.00 (d, *J* = 8.3 Hz, 1 H), 6.97 - 6.88 (m, 2 H), 6.15 (br. s., 2H), 3.71 (s, 3 H), 3.24 - 3.11 (m, 2 H), 1.50-1.46 (m, 2 H), 0.88 - 0.77 (m, 3 H).LC-MS: m/z [M+1]⁺ = 344.

### Example 155

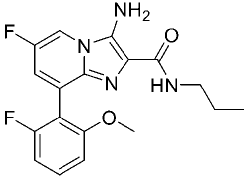

### 3-Amino-6-fluoro-8-(2-fluoro-6-methoxyphenyl)-N-propylimidazo[1,2-a]pyridine-2-carboxamide

The operation was the same as that for intermediate iv (route 1), intermediate vi (route 2), and compound vii, using 3-bromo-5-fluoropyridin-2-amine (1 g, 5.26 mmol) as a starting material to obtain the title product as a yellow solid (100 mg, yield 5%).

¹H NMR (400MHz, DMSO-*d₆*) = 8.38 (br. s., 1 H), 7.65 (br. s., 1 H), 7.48 (d, *J* = 7.8 Hz, 1 H), 7.12 (d, *J =* 8.3 Hz, 1 H), 7.03 (d, *J* = 8.3 Hz, 1 H), 6.95 (t, *J=* 8.3 Hz, 1 H), 6.20 (br. s., 1 H), 3.74 (s, 3 H), 3.23 - 3.11 (m, 2 H), 1.49-1.45 (m, 2 H), 0.83 (br. s., 3 H). LC-MS: m/z [M+1]⁺ = 361.

### Example 156

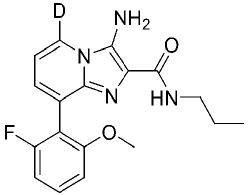

### Step 1: 3-chloropyridine-6-deuterium-2-amine

3-Chloro-6-bromopyridin-2-amine (1 g, 4.85 mmol) was dissolved in deuterated methanol (10 mL). Raney nickel (200 mg, pre-stirred in deuterated methanol for 10 minutes and filtered, which was repeated three times) was added. The mixture was stirred under deuterium atmosphere at 50°C for 72 hours. After filtration, the filtrate was concentrated and purified by column chromatography to obtain an off-white solid (200 mg, yield 32%). LC-MS: m/z [M+1]⁺=130.

### Step 2: 3-amino-8-chloro-N-propylimidazo[1,2-a]pyridine-5-deuterium-2-carboxamide

The operation was the same as that for intermediate iv (route 1) and intermediate vi (route 2), using 3-chloropyridine-5-deuterium-2-amine (200 mg, 1.55 mmol) as a starting material to obtain the target product as a yellow-green solid (100 mg, 25%). LC-MS: m/z [M+1]⁺ =254.

### Step 3: 3-amino-8-(2-fluoro-6-methoxyphenyl)-N-propylimidazo[1,2-a]pyridine-5-deuterium-2-carboxamide

The operation method was the same as the synthesis method of Example 110, using 3-amino-8-chloro-N-propylimidazo[1,2-a]pyridine-5-deuterium-2-carboxamide (100 mg, 0.32 mmol) and (2-fluoro-6-methoxyphenyl)boronic acid (115 mg, 0.68 mmol) as starting materials to obtain the target product (60 mg, 54%) as a yellow solid.

¹H NMR (400MHz, DMSO-*d₆*) 7.58 (br. s., 1 H), 7.46-7.40 (m, 1 H), 6.98 (d, *J* = 8.3 Hz, 1 H), 6.95 - 6.84 (m, 3 H), 6.14(br. s., 2 H), 3.70 (s, 3 H), 3.18-3.12 (m, 2 H), 1.53 - 1.39 (m, 2 H), 0.81 (t, *J* = 7.3 Hz, 3 H). LC-MS: m/z [M+1]⁺ = 344.

### Bioactivity Data

### Cell line establishment and subculture

The α subunit, β subunit, and γ subunit are indispensable for forming a fully functional GABA_{A} receptor. In this example, HEK293 cells stably expressing α2-GABA_{A} receptors (α2-GABAAR) were constructed and screened respectively via liposome transfection (Felgner, P.L., et al. Proceedings of the National Academy of Sciences, 1987, 84: 7413-7417). The α2-GABA_{A}R-HEK293 cell model co-expresses the α2 subunit (protein sequence referenced under GenBank accession number: NM_000807.4), β3 subunit (protein sequence referenced under GenBank accession number: NM_000814.5), and γ2 subunit (protein sequence referenced under GenBank accession number: NM_000816.3).

The above cell lines were subcultured. α2-GABA_{A}R-HEK293 cells were amplified for testing the affinity of compounds to the benzodiazepine (BZD) site of GABA_{A} receptors. Some of the α2-GABA_{A}R-HEK293 cells in suspension during subculturing were plated on glass slides pre-treated with Poly-D-Lysine for electrophysiological testing (method reference: Patent CN 107344936 A, paragraph 0586).

### Affinity activity of the compound of the present disclosure for the α2-GABA_{A} receptor

The affinity of the compound for the α2-GABA_{A} receptor was determined by competing with ³H-flunitrazepam (isotope ³H-labeled flunitrazepam) for binding to the BZD site of membrane proteins in HEK293 cells stably expressing human α2-GABA_{A}R.

Membrane preparation: Cells were suspended in 50 mM Tris-HCl buffer (pH=7.4), homogenized on ice using a homogenizer for 20 seconds with 10 repetitions, and centrifuged at 4°C, 1000g for 10 minutes. The supernatant was collected, and the above steps were repeated. The supernatant was centrifuged at 4°C (33,800 × g; Thermo, rotor: A27-8x50) for 60 minutes, and the pellet was resuspended in Tris buffer (50 mM Tris-HCl, 10 mM MgCl₂, 0.5 mM EDTA, 10% glycerol). The protein concentration was determined using the bicinchoninic acid (BCA) protein assay based on the copper ion reduction method (BCA kit purchased from Pierce (Annoron)). Aliquots of 1 mL were prepared and stored at -80°C.

Radioactive ligand competitive binding assay: This assay was conducted in a 200 µL system (96-well plate), containing 100 µL of cell membrane. The concentration of ³H-flunitrazepam was 1 nM, and the concentration of the test compound ranged from 1 × 10⁻⁵ to 10⁻⁶ M. Flumazenil was used as a control. The low signal control well (Low Control, LC) was added with 1 µL of 2 mM flumazenil (final concentration 10 µM), while the high signal control well (High Control, HC) was added with 1 µL of dimethyl sulfoxide. The final concentration of the target membrane protein was 5 µg/well. All test compound stock solutions were prepared as 10 mM solutions in dimethyl sulfoxide. The working concentration of the samples was prepared by diluting all samples to 0.2 mM with dimethyl sulfoxide, followed by a 4-fold serial dilution to obtain a total of 8 concentration gradients. The 96-well plate was sealed with sealing film and then incubated on a shaker at room temperature for 1 hour. The GF/C filter plate was also soaked with a soaking buffer (0.3% PEI (polyethyleneimine, purchased from: Sigma-Aldrich, model: P314), stored at 4°C) for at least 0.5 hours. After the incubation was completed, the mixture was collected onto a GF/C filter plate using a cell harvester and washed four times with wash buffer (50 mM Tris-HCl, pH 7.4, stored at 4°C). After drying in a 50°C oven for 1 hour, the dried GF/C filter plate was sealed at the bottom with a film. The residual radioactivity on the filter membrane was detected using liquid scintillation counting. Each well was filled with 50 µL of scintillation fluid and sealed. Readings were taken using a Microbeta² (microplate counter, purchased from PerkinElmer, model: CNLL0153). The inhibitory activity of the test sample on the binding of ³H-flunitrazepam to GABA_{A} receptor membrane protein was calculated. Specifically, the IC₅₀ of each test sample was determined by dose-response curve fitting (GraphPad Prism 5 software), and the Kᵢ of the sample was calculated based on the IC₅₀ value, thereby evaluating the binding ability of the compound to BZD site of the α2-GABA_{A} receptor.

Representative test results obtained through the above approach for measuring the binding affinity of the compound to the BZD site of the human α2-GABA_{A}R-HEK293 cell membrane protein are shown in Table 1.

### Functional activity of the compound of the present disclosure on different subtypes of GABA_{A} receptors

The positive modulatory activity of the test drug on the α2-GABA_{A} receptor was assessed using electrophysiological methods. The specific approach is as follows:

With regards to the concentration of the compound, the final concentration of all compounds used in the screening was 100 nM. For cell lines expressing α2-GABA_{A} receptors, the GABA concentration was 0.10 to 0.11 µM (approximately EC₇₋₈). Electrophysiological experiments were conducted using the whole-cell patch-clamp technique, which can be referenced to the method reported in the literature (Nickolls, S. A., et al. *British Journal of Pharmacology*, 2018, 175: 708-725). The composition of the extracellular solution (ECS) for electrophysiology was as follows: 150 mM NaCl, 5 mM KCl, 2.5 mM CaCl₂, 1 mM MgCl₂, 10 mM HEPES, and 10 mM glucose (pH 7.4). The intracellular solution (ICS) for electrophysiology was formulated as follows: 140 mM CsCl, 11 mM EGTA, 10 mM HEPES, 2 mM CaCl₂, 1 mM MgCl₂, 4 mM MgATP, and 2 mM TEA (pH 7.3). GABA (γ-aminobutyric acid) powder was dissolved in pure water to prepare a stock solution, which was subsequently diluted in ECS. The compound was initially dissolved in dimethyl sulfoxide to prepare a 4 mM stock solution, followed by stepwise dilution into GABA-ECS at corresponding concentrations. All solutions were freshly prepared prior to electrophysiological testing.

Electrophysiological signals were acquired using an EPC 10 amplifier and PatchMaster software (HEKA) or an Axon 700B amplifier and Clampex software (AXON). The recording electrode was pulled from borosilicate glass, with an electrode resistance of 4 to 6 MΩ. Extracellular administration was performed using the ALA-VC-8PG^{™} system. A single independently growing cell was selected. After forming a tight seal between the glass electrode and the cell membrane, the membrane was ruptured to establish the whole-cell configuration. The cell membrane potential was clamped at -60 mV and recorded in Gap-free mode. During the test, extracellular fluid was first applied externally for approximately 20 seconds. After the baseline (I_{prebaseline}) was stabilized, the extracellular solution was switched to GABA-ECS. At this point, the current induced by GABA (I_{gaba}) can be detected. After approximately 10 to 20 seconds, once the current had stabilized, the extracellular solution was switched to a mixture of the compound and GABA-ECS until the current induced by both the compound and GABA (Iₜᵣₑₐₜₘₑₙₜ) could be detected. Finally, the solution was switched to extracellular fluid, and the recording was terminated after 20 to 40 seconds. Only cells with a stable baseline, an absolute control current magnitude (I_{gaba} - I_{prebaseline}) greater than 40 pA, and a relatively stable current within 10 to 20 seconds were used for compound testing.

The experimental results of the positive allosteric modulatory activity of α2-GABA_{A}R were analyzed using PatchMaster v2x90.1 or PatchMaster v2x90.3 software (EPC 10 amplifier) and Clampex 10.6 software (Axon 700B amplifier). The current value at each stage was calculated as the average of the current after stabilization under the corresponding conditions. The control current was defined as I_{gaba} - I_{prebaseline}, and the current after compound treatment was defined as Iₜᵣₑₐₜₘₑₙₜ - I_{prebaseline}. The allosteric modulatory activity of the compound is expressed as a percentage and calculated according to the following formula: Functional Activity = [(Iₜᵣₑₐₜₘₑₙt - I_{gaba}) / (I_{gaba} - I_{prebaseline})] × 100%. If the value is negative, it indicates that the test compound exerts an inverse allosteric modulation on the GABA receptor. If the value is positive, it indicates that the test compound exerts a positive allosteric modulation on the GABA receptor. For ease of comparison, the positive compound CVL-865 (i.e., Example 4 of WO2014091368A1) was used as a reference compound for normalization, such that the positive allosteric modulatory activity = [functional activity of the compound / functional activity of the reference compound] × 100%.

The results showing the affinity activity of the compounds of the present disclosure for the α2-GABA_{A} receptor and the positive allosteric modulatory activity for the α2-GABA_{A} receptor are shown in Table 1 below.

It should be noted that the absolute values of the functional activity of compounds may vary across different assay systems and are therefore not directly comparable. To facilitate comparison, the experiments in the present disclosure were conducted under the same assay system, with controls and relevant compounds tested simultaneously. The positive compound CVL-865 was employed as a reference for normalization, such that positive allosteric modulatory activity = [functional activity of the compound / functional activity of the reference compound] × 100%. Specific results are shown in Table 1 below.

**Table 1. Affinity activity of some compounds for the α2-GABA_{A} receptor and their positive allosteric modulatory activity for the α2-GABA_{A} receptor**

| No. | α2 Kᵢ | α2-GABA_{A}R |
|---|---|---|
| | (Unit: nM) | Positive allosteric modulatory activity |
| CVL-865 | - | 100% |
| Example 1 | - | - |
| Example 2 | - | 35% |
| Example 3 | 24 | 105% |
| Example 4 | - | - |
| Example 5 | 39 | 65% |
| Example 6 | 24 | 57% |
| Example 7 | 14.92 | 52% |
| Example 8 | 11.85 | 66% |
| Example 9 | 93 | 52% |
| Example 10 | - | - |
| Example 11 | - | 27% |
| Example 12 | 34.1 | 32% |
| Example 13 | 44 | 107% |
| Example 14 | - | 54% |
| Example 15 | - | 61% |
| Example 16 | 19.32 | 200% |
| Example 17 | - | - |
| Example 18 | - | - |
| Example 19 | 13.85 | 65% |
| Example 20 | - | 35% |
| Example 21 | - | 43% |
| Example 22 | - | 25% |
| Example 23 | - | 57% |
| Example 24 | - | 39% |
| Example 25 | 28.77 | 91% |
| Example 26 | 17.83 | 97% |
| Example 27 | 32 | 84% |
| Example 28 | - | - |
| Example 29 | - | - |
| Example 30 | - | 81% |
| Example 31 | - | 26% |
| Example 32 | 7.33 | 50% |
| Example 33 | 15.48 | 155% |
| Example 34 | 5.07 | 101% |
| Example 35 | 13.26 | 108% |
| Example 36 | 21.18 | 181% |
| Example 37 | 33.6 | 105% |
| Example 38 | - | 50% |
| Example 39 | - | - |
| Example 40 | - | 25% |
| Example 41 | - | 30% |
| Example 42 | - | - |
| Example 43 | - | - |
| Example 44 | - | 31% |
| Example 45 | - | - |
| Example 46 | 18.7 | 50% |
| Example 47 | 35.2 | - |
| Example 48 | 14.2 | 71% |
| Example 49 | 19.15 | 156% |
| Example 50 | 3.95 | 104% |
| Example 51 | 26 | 121% |
| Example 52 | 11.18 | 63% |
| Example 53 | 57 | 92% |
| Example 54 | - | - |
| Example 55 | 27.85 | 89% |
| Example 56 | 38.22 | 95% |
| Example 57 | 3.32 | 167% |
| Example 58 | 34.5 | 103% |
| Example 59 | - | - |
| Example 60 | 5.7 | 140% |
| Example 61 | 39.4 | 91% |
| Example 62 | 5.29 | 192% |
| Example 63 | 10.21 | 71% |
| Example 64 | 3.37 | 93% |
| Example 65 | - | 36% |
| Example 66 | 2.44 | - |
| Example 67 | 15 | 138% |
| Example 68 | 51 | 92% |
| Example 69 | 30.8 | 70% |
| Example 70 | - | - |
| Example 71 | 7.43 | 130% |
| Example 72 | 95 | 44% |
| Example 73 | - | 64% |
| Example 74 | - | 73% |
| Example 75 | - | 101% |
| Example 76 | 12.9 | 101% |
| Example 77 | 5.17 | 126% |
| Example 78 | 31.66 | 74% |
| Example 79 | 40.1 | 71% |
| Example 80 | - | 77% |
| Example 81 | - | 48% |
| Example 82 | 22.3 | 93% |
| Example 83 | 5.9 | 164% |
| Example 84 | 42 | 133% |
| Example 85 | 17.16 | 100% |
| Example 86 | - | 73% |
| Example 87 | - | 48% |
| Example 88 | - | 63% |
| Example 89 | - | 44% |
| Example 90 | - | 60% |
| Example 91 | 27 | 93% |
| Example 92 | - | 33% |
| Example 93 | - | 62% |
| Example 94 | - | 51% |
| Example 95 | - | 27% |
| Example 96 | - | 52% |
| Example 97 | - | 52% |
| Example 98 | - | 64% |
| Example 99 | - | 31% |
| Example 100 | - | 94% |
| Example 101 | - | 112% |
| Example 102 | - | 44% |
| Example 103 | - | 87% |
| Example 104 | - | - |
| Example 105 | 39.55 | 160% |
| Example 106 | - | 100% |
| Example 107 | - | 90% |
| Example 108 | - | 50% |
| Example 109 | - | - |
| Example 110 | - | 141% |
| Example 111 | - | 77% |
| Example 112 | - | 53% |
| Example 113 | - | 49% |
| Example 114 | - | 124% |
| Example 115 | - | 123% |
| Example 116 | - | 92% |
| Example 117 | - | 88% |
| Example 118 | - | 51% |
| Example 119 | - | 33% |
| Example 120 | - | 59% |
| Example 121 | - | 88% |
| Example 122 | 11.4 | 105% |
| Example 123 | 4.97 | 71% |
| Example 124 | - | 90% |
| Example 125 | - | 70% |
| Example 126 | - | 99% |
| Example 127 | - | 77% |
| Example 128 | - | 49% |
| Example 129 | - | - |
| Example 130 | - | 53% |
| Example 131 | - | 82% |
| Example 132 | - | 48% |
| Example 133 | - | - |
| Example 134 | - | - |
| Example 135 | 81.1 | 60% |
| Example 136 | 17.6 | 91% |
| Example 137 | 13.45 | 97% |
| Example 138 | 11.13 | 77% |
| Example 139 | - | - |
| Example 140 | - | - |
| Example 141 | - | 44% |
| Example 142 | - | 45% |
| Example 143 | - | 48% |
| Example 144 | 4.97 | 95% |
| Example 145 | - | - |
| Example 146 | 30.5 | 55% |
| Example 147 | - | - |
| Example 148 | 4.46 | 107% |
| Example 149 | 90 | 44% |
| Example 150 | - | 85% |
| Example 151 | 87 | 35% |
| Example 154 | - | 84% |
| Example 155 | - | 56% |
| Example 156 | - | 100% |

| | | |
|---|---|---|
| "-" indicates not detected. | | |

The structures of the comparative compounds are shown below:

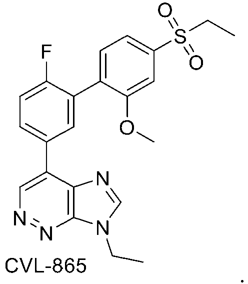

## Claims

1. A compound of formula (I), an isotopic derivative thereof, a nitrogen oxide thereof, a pharmaceutically acceptable salt thereof, a solvate thereof, or a solvate of the pharmaceutically acceptable salt thereof: 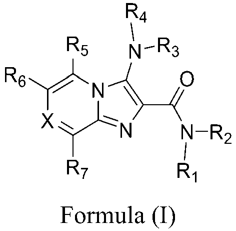 wherein
R₁ and R₂ are independently hydrogen, C₁₋₆ alkyl, C₃₋₆ cycloalkyl, C₂₋₆ alkenyl, C₂₋₆ alkynyl, 4- to 12-membered heterocycloalkyl, C₆₋₁₀ aryl, or 5- to 12-membered heteroaryl, wherein the C₁₋₆ alkyl, C₃₋₆ cycloalkyl, C₂₋₆ alkenyl, C₂₋₆ alkynyl, 4- to 12-membered heterocycloalkyl, C₆₋₁₀ aryl, and 5- to 12-membered heteroaryl are each substituted by 0, 1, 2, 3, 4 or 5 R₉;
alternatively, R₁ and R₂ together with the N atom to which they are attached form a 4- to 12-membered heterocycloalkyl or a 4- to 12-membered heterocycloalkyl substituted by 1, 2, or 3 R₁₋₁; R₁₋₁ is independently deuterium, -CN, -OH, halogen, C₁₋₆ alkyl, or -O-C₁₋₆ alkyl;
R₃ and R₄ are H, C₁₋₆ alkyl, C₃₋₆ cycloalkyl, or -O-C₁₋₆ alkyl, wherein the C₁₋₆ alkyl, C₃₋₆ cycloalkyl, and -O-C₁₋₆ alkyl are each substituted by 0, 1, 2, or 3 R₃₋₁; R₃₋₁ is independently deuterium, -CN, -OH, halogen, C₁₋₆ alkyl, or -O-C₁₋₆ alkyl;
R₅ and R₆ are independently hydrogen, deuterium, halogen, or C₁₋₆ alkyl;
X is N or CR₈;
R₈ is hydrogen, deuterium, halogen, -CN, C₁₋₆ alkyl, C₃₋₆ cycloalkyl, or -O-C₁₋₆ alkyl, wherein the C₁₋₆ alkyl, C₃₋₆ cycloalkyl, and -O-C₁₋₆ alkyl are each substituted by 0, 1, 2, or 3 R₈₋₁; R₈₋₁ is independently deuterium, -CN, -OH, halogen, C₁₋₆ alkyl, or -O-C₁₋₆ alkyl;
R₇ is -CN, C₁₋₆ alkyl, C₃₋₆ cycloalkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, -Z-(CRₐR_{b})ₘ-C₆₋₁₀ aryl, or -Z-(CRₐR_{b})ₘ-5- to 12-membered heteroaryl, wherein the C₁₋₆ alkyl, C₃₋₆ cycloalkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, -Z-(CRₐR_{b})ₘ-C₆₋₁₀ aryl, and -Z-(CRₐR_{b})ₘ-5- to 12-membered heteroaryl are each substituted by 0, 1, 2, 3, 4, or 5 R₁₀;
Z is independently -O- or -NR_{c}; Rₐ and R_{b} are independently H or C₁₋₆ alkyl; R_{c} is H or C₁₋₆ alkyl;
m is independently 0, 1, or 2;
R₉ is halogen, -OH, -CN, C₁₋₆ alkyl, -O-C₁₋₆ alkyl, C₃₋₆ cycloalkyl, 5- to 12-membered heterocycloalkyl, C₆₋₁₀ aryl, or 5- to 12-membered heteroaryl, wherein the C₁₋₆ alkyl, -O-C₁₋₆ alkyl, C₃₋₆ cycloalkyl, 5- to 12-membered heterocycloalkyl, C₆₋₁₀ aryl, and 5- to 12-membered heteroaryl are each substituted by 0, 1, 2, or 3 R₉₋₁; R₉₋₁ is independently deuterium, -CN, -OH, halogen, C₁₋₆ alkyl, or -O-C₁₋₆ alkyl;
R₁₀ is oxo (-C=O), deuterium, halogen, -OH, -CN, 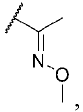 C₁₋₆ alkyl, C₂₋₆ alkenyl, C₂₋₆ alkynyl, -O-C₁₋₆ alkyl, -S-C₁₋₆ alkyl, C₆₋₁₀ aryl, 5- to 12-membered heteroaryl, C₃₋₆ cycloalkyl, or 5- to 12-membered heterocycloalkyl, wherein the C₁₋₆ alkyl, C₂₋₆ alkenyl, C₂₋₆ alkynyl, -O-C₁₋₆ alkyl, -S-C₁₋₆ alkyl, C₆₋₁₀ aryl, 5- to 12-membered heteroaryl, C₃₋₆ cycloalkyl, and 5- to 12-membered heterocycloalkyl are each substituted by 0, 1, 2, 3, 4, or 5 R₁₁;
R₁₁ is deuterium, halogen, -CN, -OH, C₁₋₆ alkyl, -O-C₁₋₆ alkyl, or oxo (-C=O);
each of said 5- to 12-membered heteroaryl is independently 5- to 12-membered heteroaryl containing 1, 2, 3, or 4 heteroatoms independently selected from the group consisting of N, O, and S; each of said 4- to 12-membered heterocycloalkyl is independently 4- to 12-membered heterocycloalkyl containing 1, 2, 3, or 4 heteroatoms independently selected from the group consisting of N, O, and S; each of said 5- to 12-membered heterocycloalkyl is independently 5- to 12-membered heterocycloalkyl containing 1, 2, 3, or 4 heteroatoms independently selected from the group consisting of N, O, and S; each of said 5- to 12-membered heterocycloalkenyl is independently 5- to 12-membered heterocycloalkenyl containing 1, 2, 3, or 4 heteroatoms independently selected from the group consisting of N, O, and S.

2. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof: 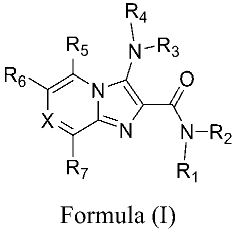 wherein
R₁ and R₂ are independently hydrogen, C₁₋₆ alkyl, C₃₋₆ cycloalkyl, C₂₋₆ alkenyl, C₂₋₆ alkynyl, 5- to 12-membered heterocycloalkyl, C₆₋₁₀ aryl, or 5- to 12-membered heteroaryl, wherein the C₁₋₆ alkyl, C₃₋₆ cycloalkyl, C₂₋₆ alkenyl, C₂₋₆ alkynyl, 5- to 12-membered heterocycloalkyl, C₆₋₁₀ aryl, and 5- to 12-membered heteroaryl are each substituted by 0, 1, 2, 3, 4 or 5 R₉;
alternatively, R₁ and R₂ together with the N atom to which they are attached form a 5- to 12-membered heterocycloalkyl;
R₃ and R₄ are H;
R₅ and R₆ are independently hydrogen or methyl;
X is N or CR₈;
R₈ is hydrogen, deuterium, halogen, -CN, methoxy, methyl, or -CHF₂;
R₇ is -CN, C₁₋₆ alkyl, C₃₋₆ cycloalkyl, C₃₋₆ cycloalkenyl, C₆₋₁₀ aryl, 5- to 12-membered heteroaryl, 5- to 12-membered heterocycloalkyl, 5- to 12-membered heterocycloalkenyl, or - Z-(CRₐR_{b})ₘ₋C₆₋₁₀ aryl, wherein the C₁₋₆ alkyl, C₃₋₆ cycloalkyl, C₃₋₆ cycloalkenyl, C₆₋₁₀ aryl, 5-to 12-membered heteroaryl, 5- to 12-membered heterocycloalkyl, 5- to 12-membered heterocycloalkenyl, and -Z-(CRₐR_{b})ₘ-C₆₋₁₀ aryl are each substituted by 0, 1, 2, 3, 4, or 5 R₁₀;
Z is -O- or -NR_{c}; Rₐ and R_{b} are independently H or C₁₋₆ alkyl; R_{c} is H or C₁₋₆ alkyl;
m is 0, 1, or 2;
R₉ is halogen, -OH, -CN, C₁₋₆ alkyl, -O-C₁₋₆ alkyl, C₃₋₆ cycloalkyl, 5- to 12-membered heterocycloalkyl, C₆₋₁₀ aryl, or 5- to 12-membered heteroaryl;
R₁₀ is oxo (-C=O), deuterium, halogen, hydroxyl, -CN, 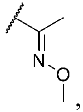 C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, or 5- to 12-membered heterocycloalkyl, wherein the C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, and 5- to 12-membered heterocycloalkyl are each substituted by 0, 1, 2, 3, 4, or 5 R₁₁;
R₁₁ is halogen, -CN, -OH, C₁₋₆ alkyl, or oxo (-C=O);
each of said 5- to 12-membered heteroaryl is independently 5- to 12-membered heteroaryl containing 1, 2, or 3 heteroatoms independently selected from the group consisting of N, O, and S; each of said 5- to 12-membered heterocycloalkyl is independently 5- to 12-membered heterocycloalkyl containing 1, 2, or 3 heteroatoms independently selected from the group consisting of N, O, and S; each of said 5- to 12-membered heterocycloalkenyl is independently 5- to 12-membered heterocycloalkenyl containing 1, 2, or 3 heteroatoms independently selected from the group consisting of N, O, and S.

3. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof, wherein the compound of formula (I) satisfies one or more of the following conditions:
(1) R₁ is H;
(2) R₂ is C₁₋₆ alkyl, C₃₋₆ cycloalkyl, C₂₋₆ alkenyl, C₂₋₆ alkynyl, 4- to 12-membered heterocycloalkyl, C₆₋₁₀ aryl, or 5- to 12-membered heteroaryl, wherein the C₁₋₆ alkyl, C₃₋₆ cycloalkyl, C₂₋₆ alkenyl, 4- to 12-membered heterocycloalkyl, and 5- to 12-membered heteroaryl are each substituted by 0, 1, 2, 3, 4, or 5 R₉;
(3) R₁₋₁ is C₁₋₆ alkyl;
(4) R₃ and R₄ are H;
(5) R₈ is hydrogen, deuterium, halogen, -CN, C₁₋₆ alkyl, -O-C₁₋₆ alkyl, or C₁₋₆ alkyl substituted by 1, 2, or 3 R₈₋₁;
(6) R₇ is -CN, C₁₋₆ alkyl, C₃₋₆ cycloalkenyl, C₆₋₁₀ aryl, 5- to 12-membered heteroaryl, 5-to 12-membered heterocycloalkyl, 5- to 12-membered heterocycloalkenyl, or -Z-(CRₐR_{b})ₘ-C₆₋₁₀ aryl, wherein the C₆₋₁₀ aryl, 5- to 12-membered heteroaryl, 5- to 12-membered heterocycloalkyl, 5- to 12-membered heterocycloalkenyl, and -Z-(CRₐR_{b})ₘ-C₆₋₁₀ aryl are each substituted by 1, 2, 3, 4, or 5 R₁₀;
(7) Rₐ and R_{b} are H;
(8) Z is -O-;
(9) m is 0 or 1;
(10) R₉ is halogen, -OH, -CN, C₁₋₆ alkyl, -O-C₁₋₆ alkyl, C₃₋₆ cycloalkyl, 5- to 12-membered heterocycloalkyl, C₆₋₁₀ aryl, 5- to 12-membered heteroaryl, or 5- to 12-membered heteroaryl substituted by 1, 2, or 3 R₉₋₁;
(11) R₉₋₁ is independently C₁₋₆ alkyl;
(12) R₁₀ is oxo (-C=O), deuterium, halogen, -OH, -CN, 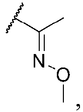 C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, or 5- to 12-membered heterocycloalkyl, wherein the C₁₋₆ alkyl, C₂₋₆ alkynyl, -O-C₁₋₆ alkyl, -S-C₁₋₆ alkyl, 5- to 12-membered heteroaryl, C₃₋₆ cycloalkyl, and 5- to 12-membered heterocycloalkyl are each substituted by 0, 1, 2, 3, 4, or 5 R₁₁; and
(13) R₁₁ is deuterium, halogen, -CN, -OH, C₁₋₆ alkyl, or oxo (-C=O).

4. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof, wherein the compound of formula (I) satisfies one or more of the following conditions:
(1) in R₁, R₂, R₃, R₄, R₅, R₆, R₇, R₈, R₉, R₁₀, R₁₁, R₁₋₁, R₃₋₁, R₈₋₁, R₉₋₁, Rₐ, and R_{b}, the C₁₋₆ alkyl in said C₁₋₆ alkyl and substituted C₁₋₆ alkyl is independently methyl, ethyl, n-propyl, isopropyl, n-butyl, isobutyl, or tert-butyl;
(2) in R₁, R₂, R₃, R₄, R₇, R₈, R₉, and R₁₀, the C₃₋₆ cycloalkyl in said C₃₋₆ cycloalkyl and substituted C₃₋₆ cycloalkyl is independently cyclopropyl, cyclobutyl, cyclopentyl, 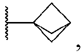 or cyclohexyl;
(3) in R₁, R₂, and R₁₀, the C₂₋₆ alkenyl in said C₂₋₆ alkenyl and substituted C₂₋₆ alkenyl is independently vinyl, n-propenyl, isopropenyl, n-butenyl, or isobutenyl;
(4) in R₁, R₂, and R₁₀, the C₂₋₆ alkynyl in said C₂₋₆ alkynyl and substituted C₂₋₆ alkynyl is independently ethynyl, n-propynyl, or n-butynyl;
(5) in R₁, R₂, R₇, R₈, R₉, R₁₀, and R₁₁, the C₆₋₁₀ aryl in said C₆₋₁₀ aryl and substituted C₆₋₁₀ aryl is independently phenyl or naphthyl;
(6) in R₃, R₄, R₈, R₉, R₁₀, R₁₁, R₁₋₁, R₃₋₁, R₈₋₁, and R₉₋₁, the C₁₋₆ alkyl in said -O-C₁₋₆ alkyl and substituted -O-C₁₋₆ alkyl is independently methyl, ethyl, n-propyl, isopropyl, n-butyl, isobutyl, or tert-butyl;
(7) in R₃, R₄, R₅, R₆, R₈, R₉, R₁₀, R₁₁, R₁₋₁, R₃₋₁, R₈₋₁, and R₉₋₁, the halogen is independently F, Cl, or Br;
(8) in R₁ and R₂, the 4- to 12-membered heterocycloalkyl in said 4- to 12-membered heterocycloalkyl and 4- to 12-membered heterocycloalkyl substituted by 1, 2, 3, 4, or 5 R₉ is independently 4- to 6-membered heterocycloalkyl;
(9) in R₁ and R₂, the 4- to 12-membered heterocycloalkyl in said 4- to 12-membered heterocycloalkyl and 4- to 12-membered heterocycloalkyl substituted by 1, 2, 3, 4, or 5 R₉ is independently 4- to 12-membered heterocycloalkyl containing 1, 2, or 3 heteroatoms, wherein the heteroatom is N.
(10) in R₁ and R₂, the 5- to 12-membered heteroaryl in said 5- to 12-membered heteroaryl and 5- to 12-membered heteroaryl substituted by 1, 2, 3, 4, or 5 R₉ is independently 5- to 6-membered heteroaryl;
(11) in R₁ and R₂, the 5- to 12-membered heteroaryl in said 5- to 12-membered heteroaryl and 5- to 12-membered heteroaryl substituted by 1, 2, 3, 4, or 5 R₉ is independently 5- to 12-membered heteroaryl containing 1, 2, or 3 heteroatoms selected from the group consisting of N and O;
(12) when R₁ and R₂ together with the N atom to which they are attached form a 4- to 12-membered heterocycloalkyl or a 4- to 12-membered heterocycloalkyl substituted by 1, 2, or 3 R₁₋₁, the 4- to 12-membered heterocycloalkyl in said 4- to 12-membered heterocycloalkyl and 4- to 12-membered heterocycloalkyl substituted by 1, 2, or 3 R₁₋₁ is independently 4- to 6-membered heterocycloalkyl;
(13) when R₁ and R₂ together with the N atom to which they are attached form a 4- to 12-membered heterocycloalkyl or a 4- to 12-membered heterocycloalkyl substituted by 1, 2, or 3 R₁₋₁, the 4- to 12-membered heterocycloalkyl in said 4- to 12-membered heterocycloalkyl and 4- to 12-membered heterocycloalkyl substituted by 1, 2, or 3 R₁₋₁ is independently 4- to 12-membered heterocycloalkyl containing 1, 2, or 3 heteroatoms, wherein the heteroatom is N;
(14) in R₇, the C₃₋₆ cycloalkenyl in said C₃₋₆ cycloalkenyl and C₃₋₆ cycloalkenyl substituted by 1, 2, 3, 4, or 5 R₁₀ is independently cyclopropenyl, cyclopentenyl, or cyclohexenyl;
(15) in R₇, the 5- to 12-membered heteroaryl in said 5- to 12-membered heteroaryl and 5-to 12-membered heteroaryl substituted by 1, 2, 3, 4, or 5 R₁₀ is independently 5- to 12-membered heteroaryl containing 1, 2, or 3 heteroatoms selected from the group consisting of N and O;
(16) in R₇, the 5- to 12-membered heterocycloalkyl in said 5- to 12-membered heterocycloalkyl and 5- to 12-membered heterocycloalkyl substituted by 1, 2, 3, 4, or 5 R₁₀ is independently 5- to 6-membered heterocycloalkyl;
(17) in R₇, the 5- to 12-membered heterocycloalkyl in said 5- to 12-membered heterocycloalkyl and 5- to 12-membered heterocycloalkyl substituted by 1, 2, 3, 4, or 5 R₁₀ is independently 5- to 12-membered heterocycloalkyl containing 1, 2, or 3 heteroatoms selected from the group consisting of N and O;
(18) in R₇, the 5- to 12-membered heterocycloalkenyl in said 5- to 12-membered heterocycloalkenyl and 5- to 12-membered heterocycloalkenyl substituted by 1, 2, 3, 4, or 5 R₁₀ is independently 5- to 6-membered heterocycloalkyl;
(19) in R₇, the 5- to 12-membered heterocycloalkenyl in said 5- to 12-membered heterocycloalkenyl and 5- to 12-membered heterocycloalkenyl substituted by 1, 2, 3, 4, or 5 R₁₀ is independently 5- to 12-membered heterocycloalkyl containing 1, 2, or 3 heteroatoms, wherein the heteroatom is N;
(20) in R₇, the -Z-(CRₐR_{b})ₘ-C₆₋₁₀ aryl in said -Z-(CRₐR_{b})ₘ-C₆₋₁₀ aryl and -Z-(CRₐR_{b})ₘ-C₆₋₁₀ aryl substituted by 1, 2, 3, 4, or 5 R₁₀ is independently 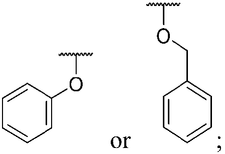
(21) in R₉, the 5- to 12-membered heteroaryl in said 5- to 12-membered heteroaryl and 5-to 12-membered heteroaryl substituted by 1, 2, or 3 R₉₋₁ is independently 5- to 6-membered heteroaryl;
(22) in R₉, the 5- to 12-membered heteroaryl in said 5- to 12-membered heteroaryl and 5-to 12-membered heteroaryl substituted by 1, 2, or 3 R₉₋₁ is independently 5- to 12-membered heteroaryl containing 1, 2, or 3 heteroatoms selected from the group consisting of N and O;
(23) in R₁₀, the 5- to 12-membered heteroaryl in said 5- to 12-membered heteroaryl and 5- to 12-membered heteroaryl substituted by 1, 2, or 3 R₉₋₁ is independently 5- to 12-membered heteroaryl containing 1, 2, 3, or 4 heteroatoms selected from the group consisting of N and O;
(24) in R₁₀, the 5- to 12-membered heterocycloalkyl in said 5- to 12-membered heterocycloalkyl and 5- to 12-membered heterocycloalkyl substituted by 1, 2, or 3 R₉₋₁ is independently 5- to 6-membered heterocycloalkyl;
(25) in R₁₀, the 5- to 12-membered heterocycloalkyl in said 5- to 12-membered heterocycloalkyl and 5- to 12-membered heterocycloalkyl substituted by 1, 2, or 3 R₉₋₁ is independently 5- to 12-membered heterocycloalkyl containing 1, 2, or 3 heteroatoms selected from the group consisting of N and O; and
(26) in R₁₀, the C₁₋₆ alkyl in said -S-C₁₋₆ alkyl and -S-C₁₋₆ alkyl substituted by 1, 2, 3, 4, or 5 R₁₁ is independently methyl, ethyl, n-propyl, isopropyl, n-butyl, isobutyl, or tert-butyl.

5. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof, wherein the compound of formula (I) satisfies one or more of the following conditions:
(1) in R₁, R₂, R₃, R₄, R₅, R₆, R₇, R₈, R₉, R₁₀, R₁₁, R₁₋₁, R₃₋₁, R₈₋₁, R₉₋₁, Rₐ, and R_{b}, the C₁₋₆ alkyl in said C₁₋₆ alkyl and substituted C₁₋₆ alkyl is independently methyl, ethyl, n-propyl, or isopropyl;
(2) in R₁, R₂, R₇, R₈, R₉, R₁₀, and R₁₁, the C₆₋₁₀ aryl in said C₆₋₁₀ aryl and substituted C₆₋₁₀ aryl is independently phenyl;
(3) in R₃, R₄, R₈, R₉, R₁₀, R₁₁, R₁₋₁, R₃₋₁, R₈₋₁, and R₉₋₁, the C₁₋₆ alkyl in said -O-C₁₋₆ alkyl and substituted -O-C₁₋₆ alkyl is independently methyl, ethyl, n-propyl, or isopropyl;
(4) in R₃, R₄, R₅, R₆, R₈, R₉, R₁₀, R₁₁, R₁₁, R₃₋₁, R₈₋₁, and R₉₋₁, the halogen is independently F or Cl;
(5) in R₁ and R₂, the 4- to 12-membered heterocycloalkyl in said 4- to 12-membered heterocycloalkyl and 4- to 12-membered heterocycloalkyl substituted by 1, 2, 3, 4, or 5 R₉ is independently 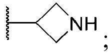
(6) in R₁ and R₂, the 5- to 12-membered heteroaryl in said 5- to 12-membered heteroaryl and 5- to 12-membered heteroaryl substituted by 1, 2, 3, 4, or 5 R₉ is independently 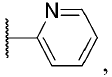 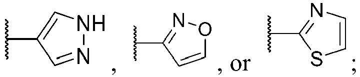
(7) when R₁ and R₂ together with the N atom to which they are attached form a 4- to 12-membered heterocycloalkyl or a 4- to 12-membered heterocycloalkyl substituted by 1, 2, or 3 R₁₋₁, the 4- to 12-membered heterocycloalkyl in said 4- to 12-membered heterocycloalkyl and 4- to 12-membered heterocycloalkyl substituted by 1, 2, or 3 R₁₋₁ is independently 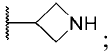
(8) in R₇, the C₃₋₆ cycloalkenyl in said C₃₋₆ cycloalkenyl and C₃₋₆ cycloalkenyl substituted by 1, 2, 3, 4, or 5 R₁₀ is independently 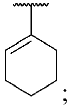
(9) in R₇, the 5- to 12-membered heteroaryl in said 5- to 12-membered heteroaryl and 5-to 12-membered heteroaryl substituted by 1, 2, 3, 4, or 5 R₁₀ is independently 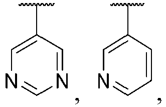 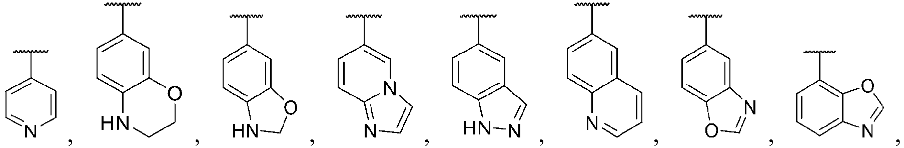 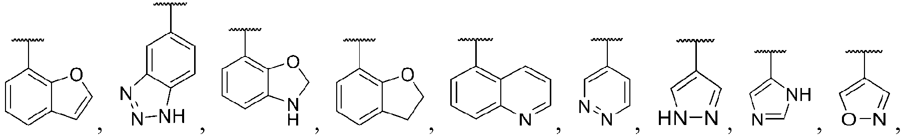 or 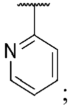
(10) in R₇, the 5- to 12-membered heterocycloalkyl in said 5- to 12-membered heterocycloalkyl and 5- to 12-membered heterocycloalkyl substituted by 1, 2, 3, 4, or 5 R₁₀ is independently 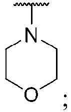
(11) in R₇, the 5- to 12-membered heterocycloalkenyl in said 5- to 12-membered heterocycloalkenyl and 5- to 12-membered heterocycloalkenyl substituted by 1, 2, 3, 4, or 5 R₁₀ is independently 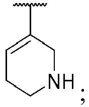
(12) in R₉, the 5- to 12-membered heteroaryl in said 5- to 12-membered heteroaryl and 5-to 12-membered heteroaryl substituted by 1, 2, or 3 R₉₋₁ is independently 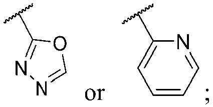
(13) in R₁₀, the 5- to 12-membered heteroaryl in said 5- to 12-membered heteroaryl and 5- to 12-membered heteroaryl substituted by 1, 2, or 3 R₉₋₁ is independently 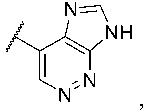 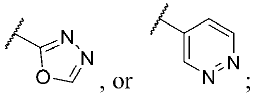
(14) in R₁₀, the 5- to 12-membered heterocycloalkyl in said 5- to 12-membered heterocycloalkyl and 5- to 12-membered heterocycloalkyl substituted by 1, 2, or 3 R₉₋₁ is independently 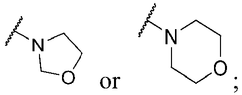 and
(15) in R₁₀, the C₁₋₆ alkyl in said -S-C₁₋₆ alkyl and -S-C₁₋₆ alkyl substituted by 1, 2, 3, 4, or 5 R₁₁ is independently methyl.

6. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof, wherein the compound of formula (I) satisfies one or more of the following conditions:
(1) R₂ is 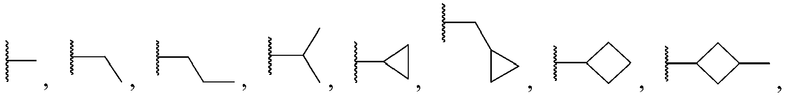 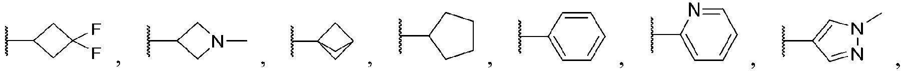 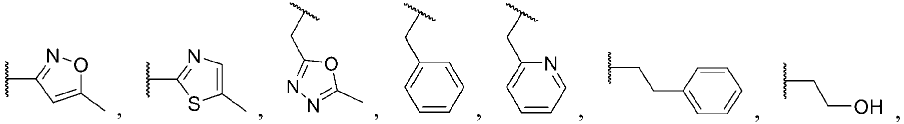 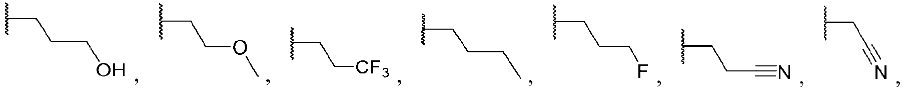 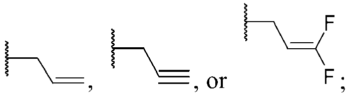 alternatively, R₁ and R₂ together with the nitrogen atom to which they are attached form 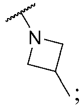
(2) R₅ is H, deuterium, or methyl;
(3) R₆ is H, deuterium, F, or methyl;
(4) R₇ is 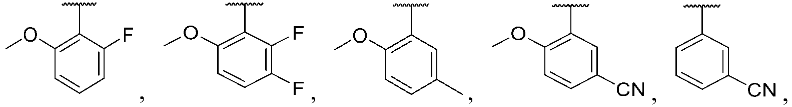 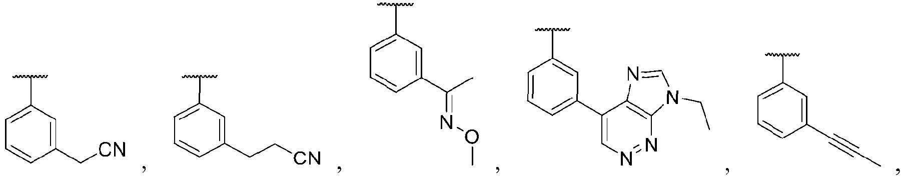 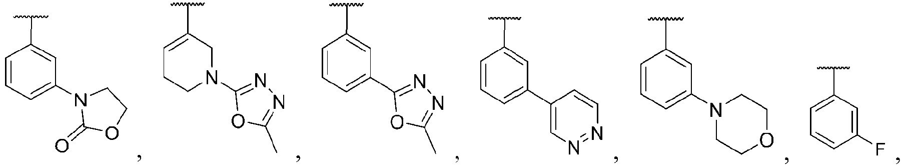 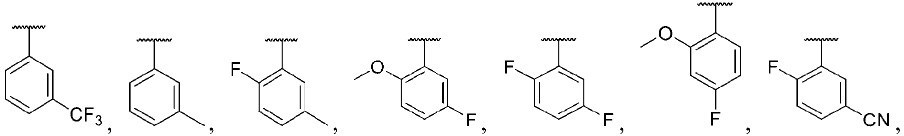 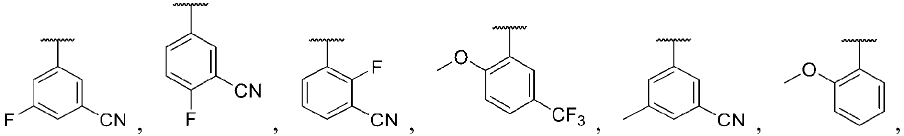 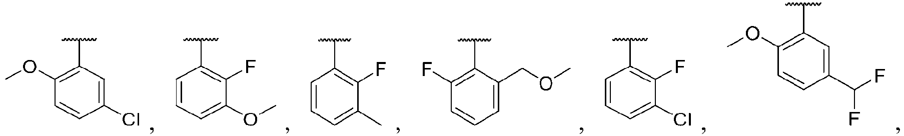 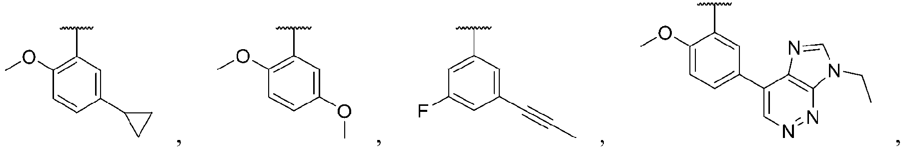 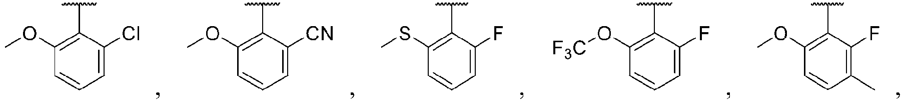 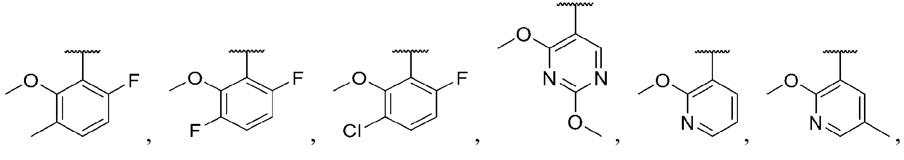 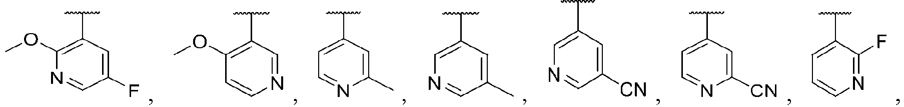 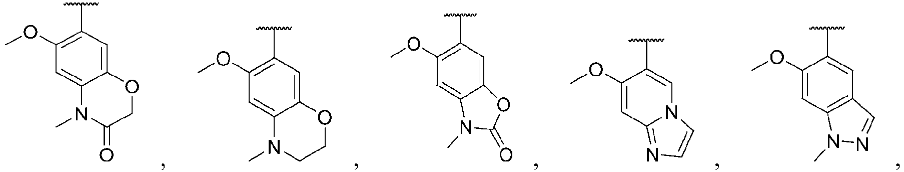 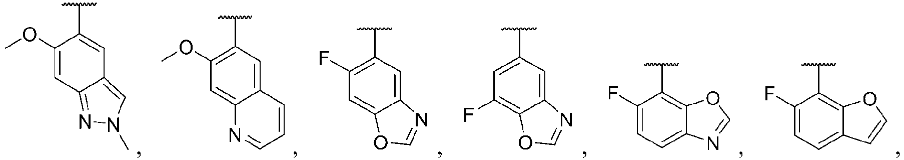 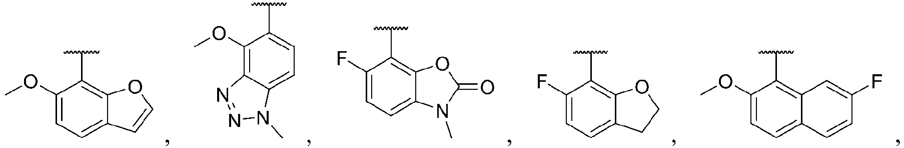 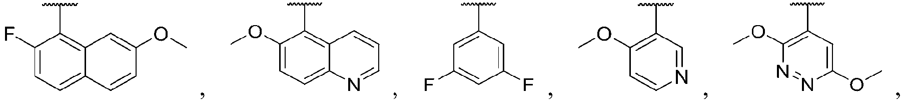 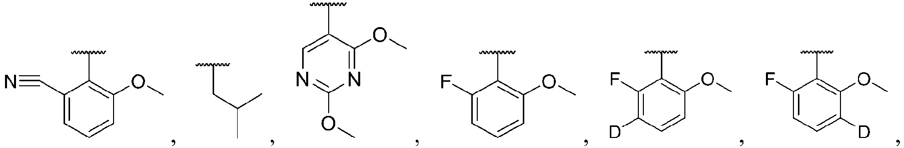 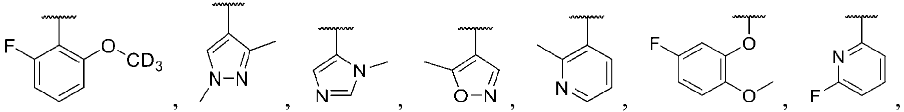 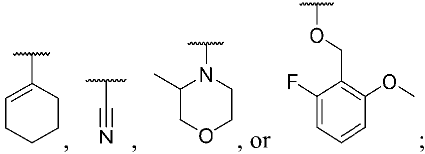 and
(5) R₈ is H, D, F, Cl, -CN, -CH₃, -OCH₃, or -CHF₂.

7. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof, wherein the compound of formula (I) satisfies one or more of the following conditions:
(1) 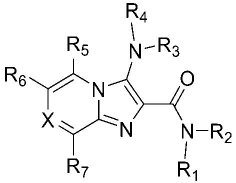 is 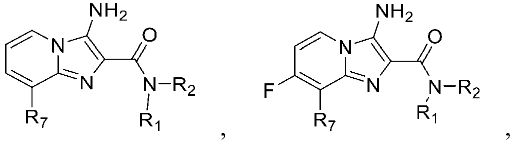 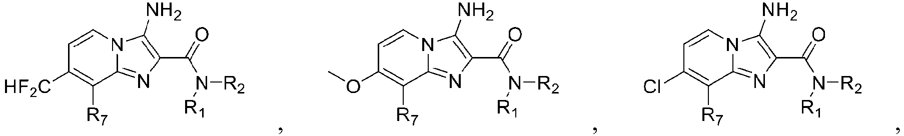 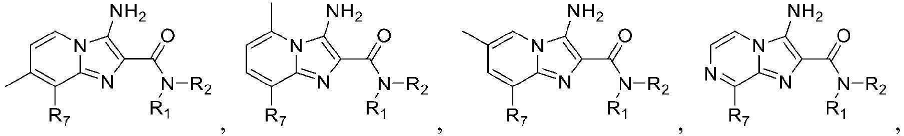 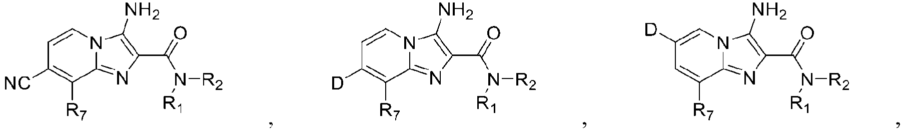 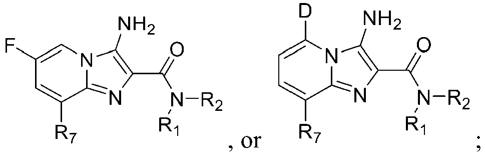
(2) R₉ is F, -OH, -CN, methyl, methoxy, cyclopropyl, phenyl, 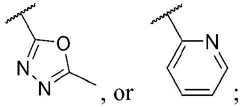
(3) R₁₀ is oxo (-C=O), deuterium, F, Cl, -OH, -CN, 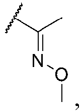 methyl, methoxy, -CF₃, 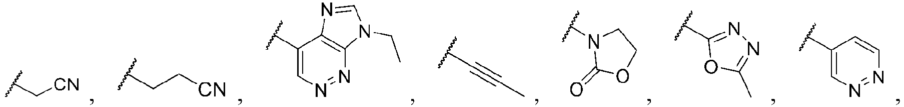 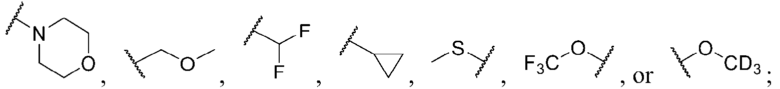 and
(4) R₁₁ is deuterium, F, -CN, methyl, ethyl, or oxo (-C=O); preferably, 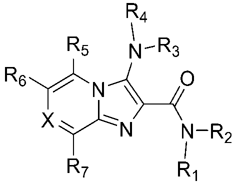 is 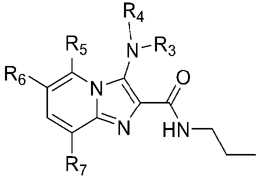 (e.g., 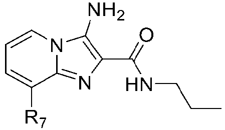 );
R₇ is 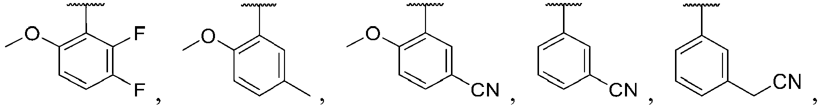 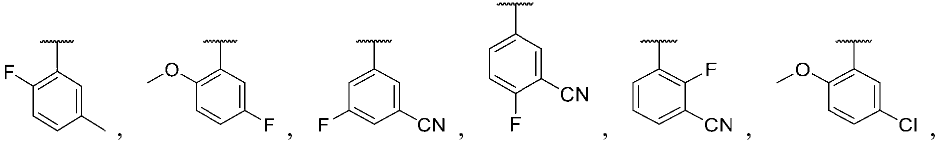 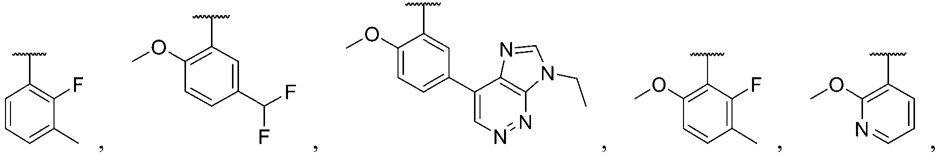 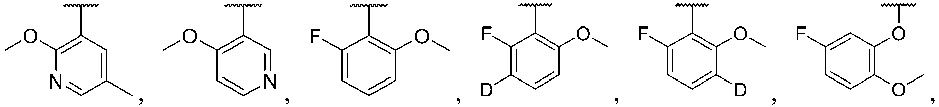 or

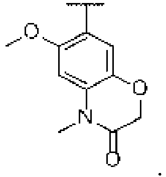

8. The compound of formula (I) according to claim 1, an isotopic derivative thereof, a nitrogen oxide thereof, a pharmaceutically acceptable salt thereof, a solvate thereof, or a solvate of the pharmaceutically acceptable salt thereof, wherein the compound of formula (I) is any one selected from the group consisting of the following compounds:
| No. | Structure | No. | Structure |
|---|---|---|---|
| 1 | 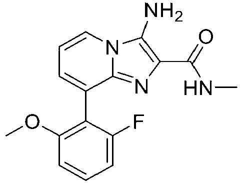 | 81 | 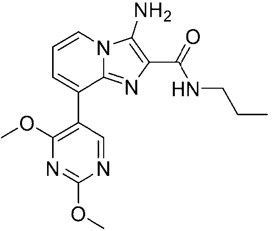 |
| 2 | 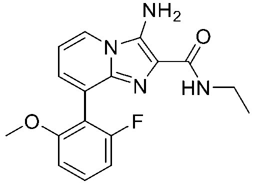 | 82 | 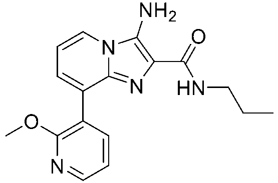 |
| 3 | 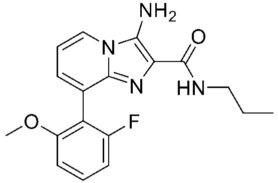 | 83 | 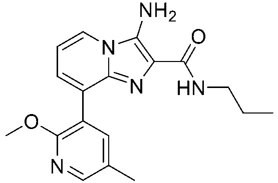 |
| 4 | 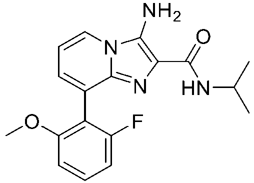 | 84 | 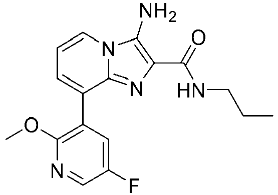 |
| 5 | 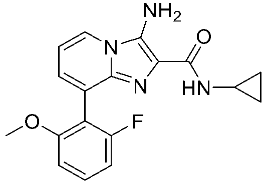 | 85 | 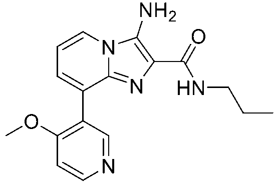 |
| 6 | 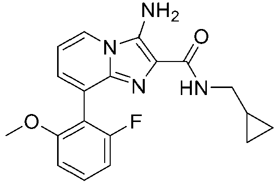 | 86 | 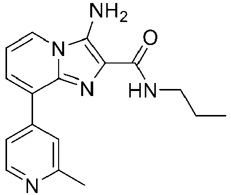 |
| 7 | 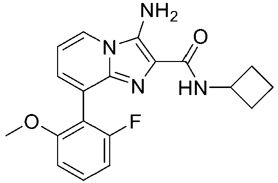 | 87 | 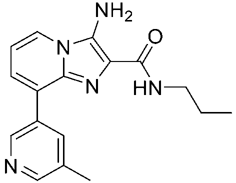 |
| 8 | 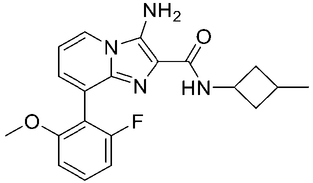 | 88 | 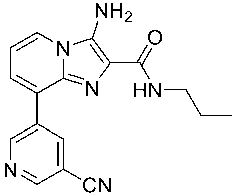 |
| 9 | 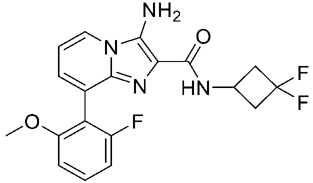 | 89 | 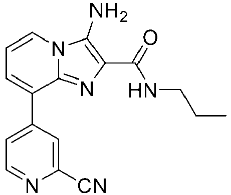 |
| 10 | 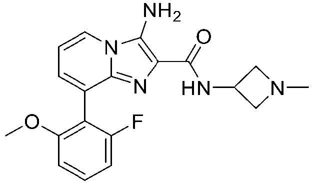 | 90 | 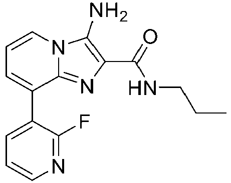 |
| 11 | 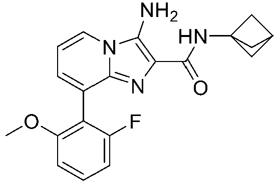 | 91 | 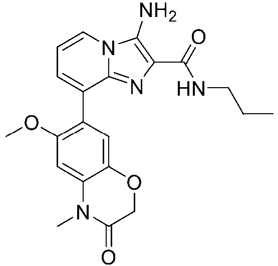 |
| 12 | 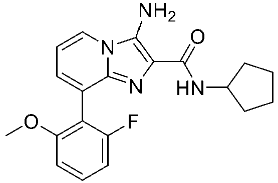 | 92 | 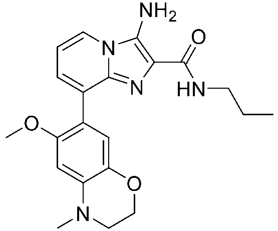 |
| 13 | 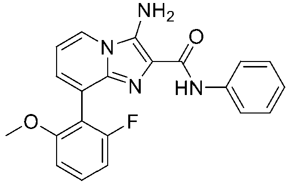 | 93 | 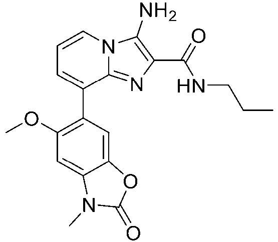 |
| 14 | 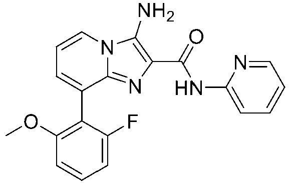 | 94 | 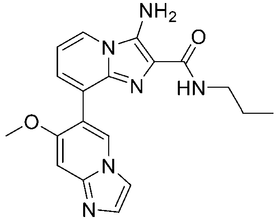 |
| 15 | 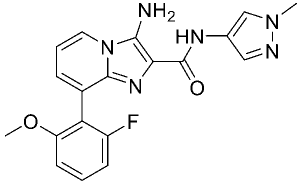 | 95 | 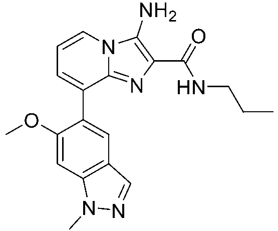 |
| 16 | 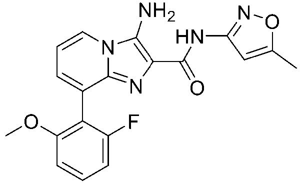 | 96 | 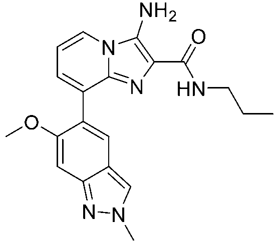 |
| 17 | 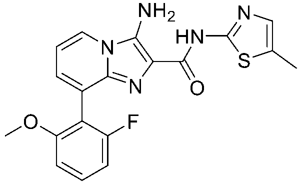 | 97 | 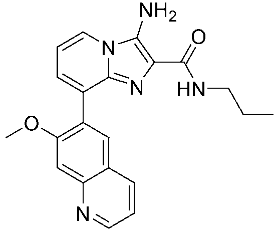 |
| 18 | 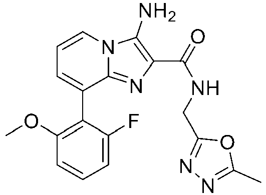 | 98 | 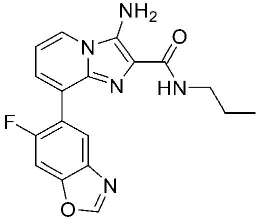 |
| 19 | 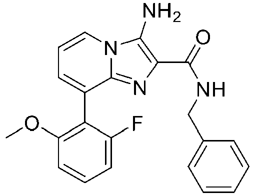 | 99 | 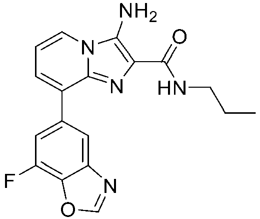 |
| 20 | 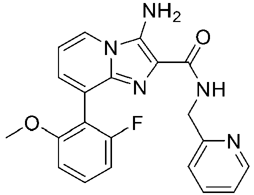 | 100 | 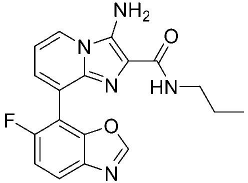 |
| 21 | 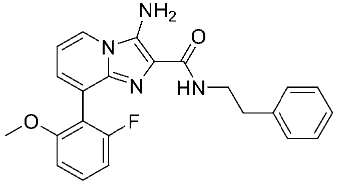 | 101 | 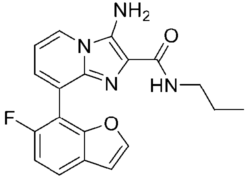 |
| 22 | 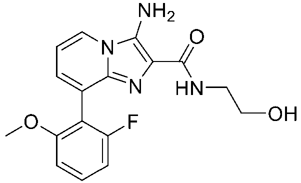 | 102 | 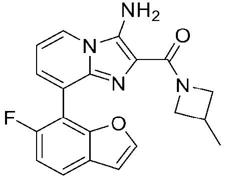 |
| 23 | 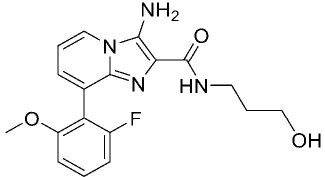 | 103 | 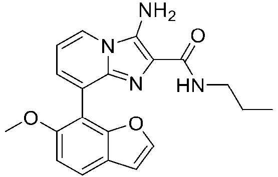 |
| 24 | 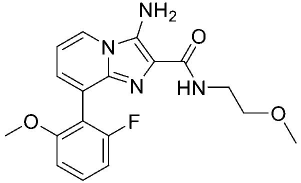 | 104 | 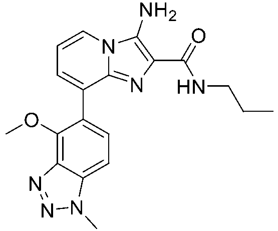 |
| 25 | 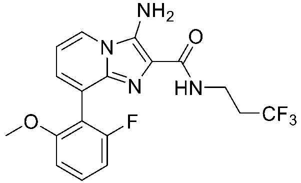 | 105 | 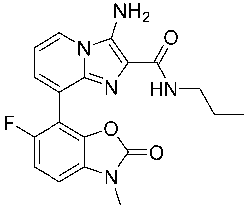 |
| 26 | 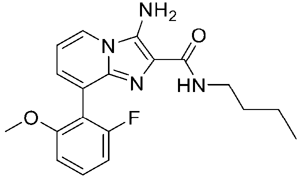 | 106 | 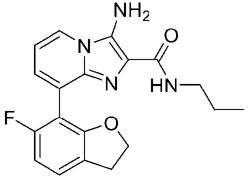 |
| 27 | 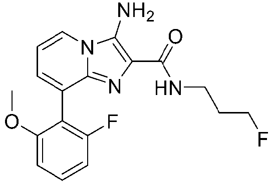 | 107 | 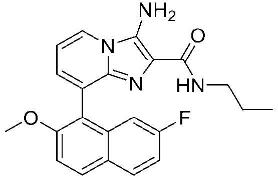 |
| 28 | 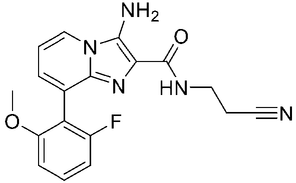 | 108 | 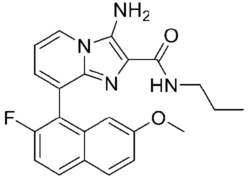 |
| 29 | 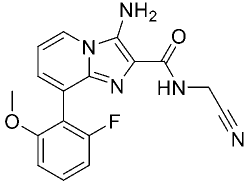 | 109 | 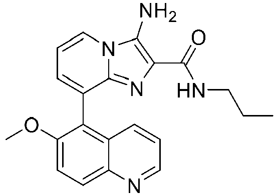 |
| 30 | 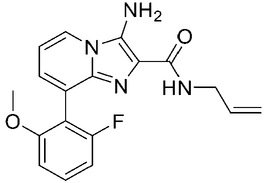 | 110 | 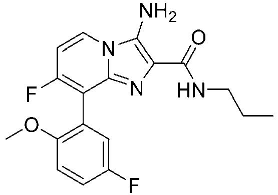 |
| 31 | 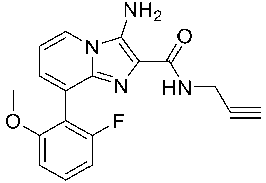 | 111 | 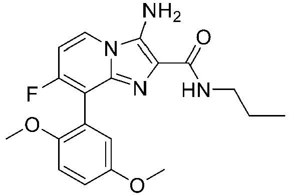 |
| 32 | 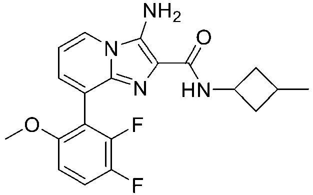 | 112 | 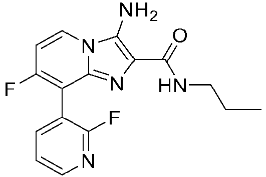 |
| 33 | 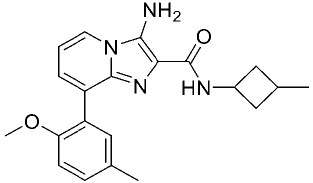 | 113 | 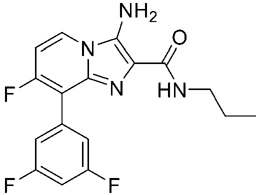 |
| 34 | 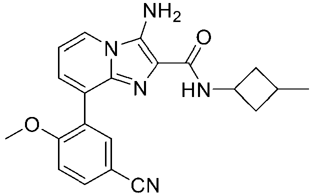 | 114 | 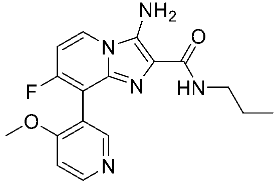 |
| 35 | 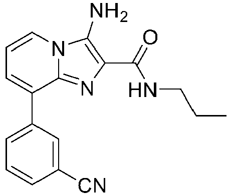 | 115 | 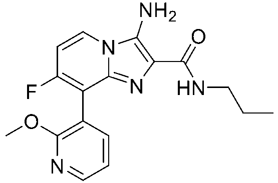 |
| 36 | 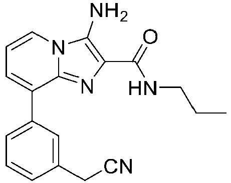 | 116 | 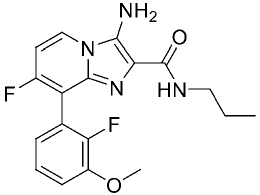 |
| 37 | 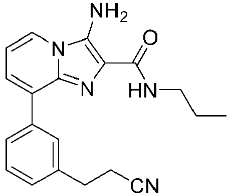 | 117 | 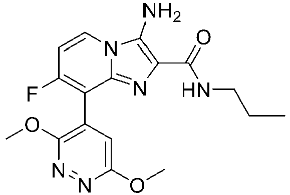 |
| 38 | 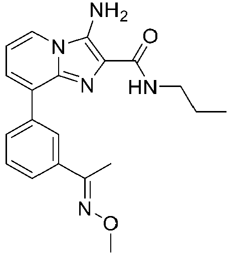 | 118 | 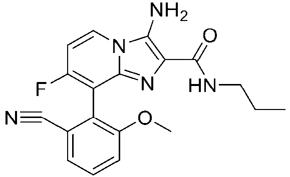 |
| 39 | 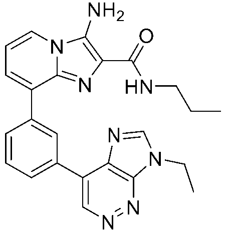 | 119 | 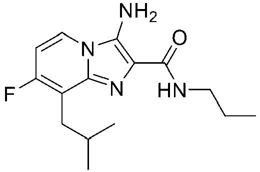 |
| 40 | 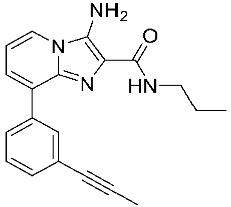 | 120 | 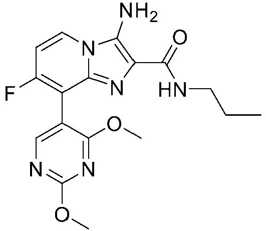 |
| 41 | 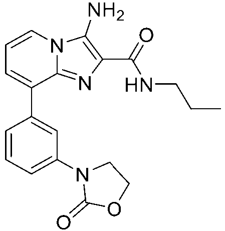 | 121 | 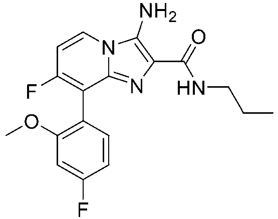 |
| 42 | 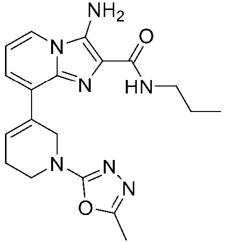 | 122 | 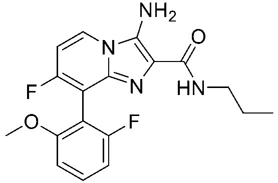 |
| 43 | 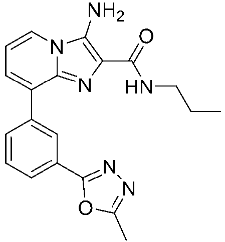 | 123 | 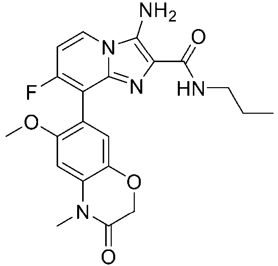 |
| 44 | 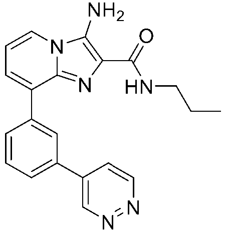 | 124 | 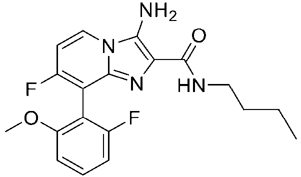 |
| 45 | 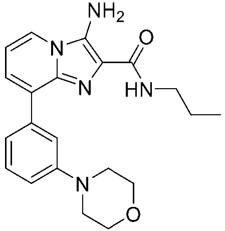 | 125 | 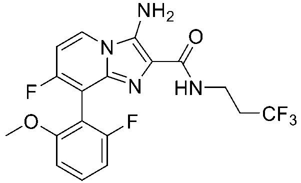 |
| 46 | 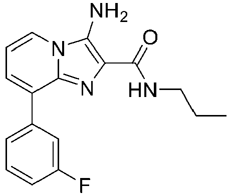 | 126 | 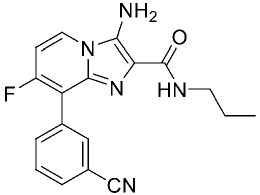 |
| 47 | 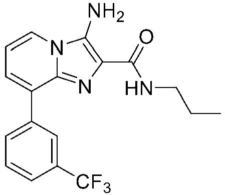 | 127 | 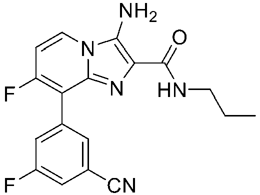 |
| 48 | 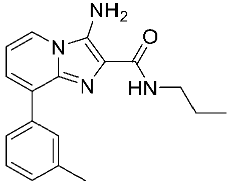 | 128 | 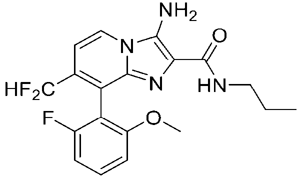 |
| 49 | 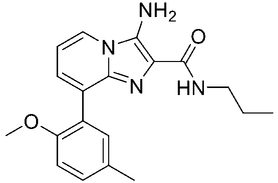 | 129 | 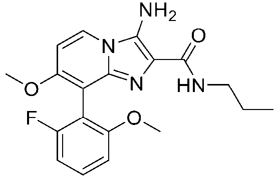 |
| 50 | 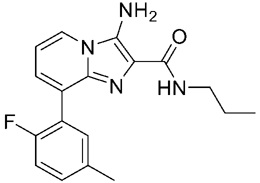 | 130 | 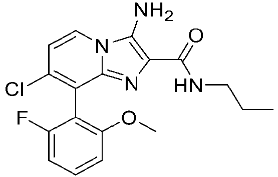 |
| 51 | 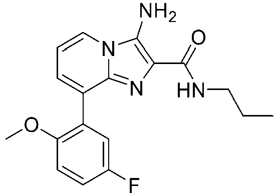 | 131 | 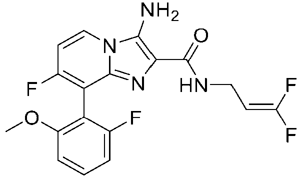 |
| 52 | 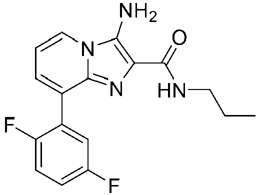 | 132 | 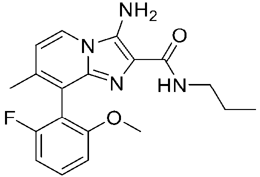 |
| 53 | 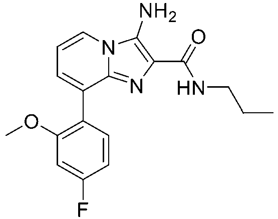 | 133 | 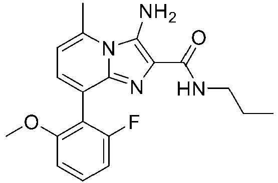 |
| 54 | 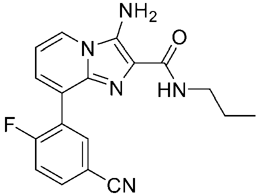 | 134 | 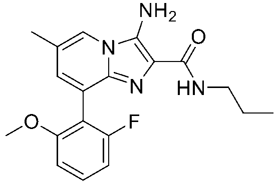 |
| 55 | 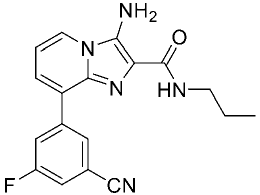 | 135 | 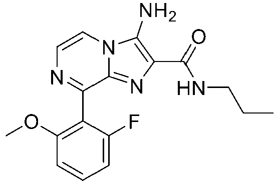 |
| 56 | 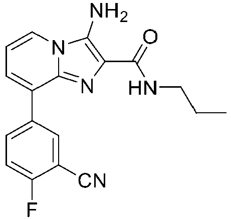 | 136 | 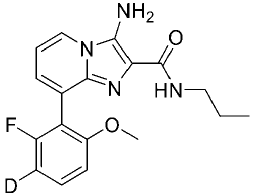 |
| 57 | 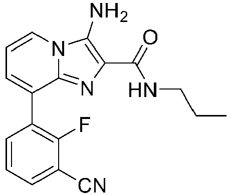 | 137 | 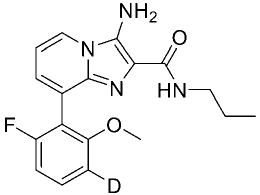 |
| 58 | 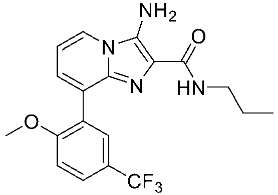 | 138 | 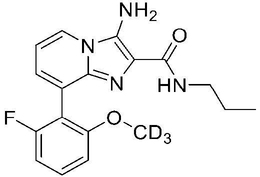 |
| 59 | 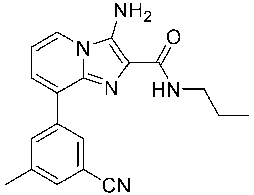 | 139 | 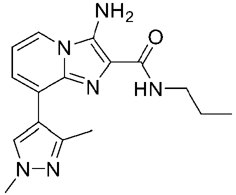 |
| 60 | 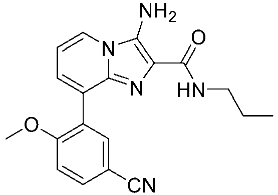 | 140 | 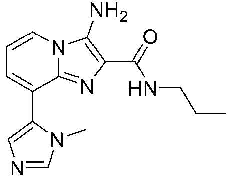 |
| 61 | 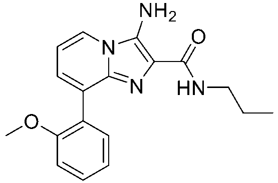 | 141 | 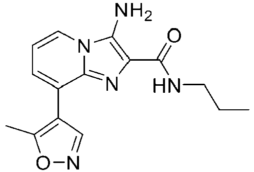 |
| 62 | 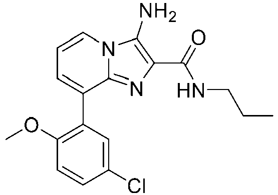 | 142 | 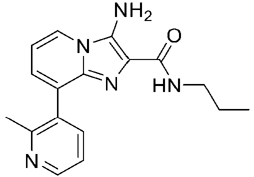 |
| 63 | 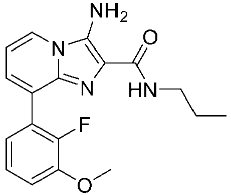 | 143 | 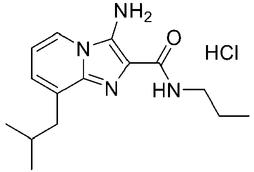 |
| 64 | 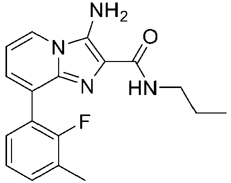 | 144 | 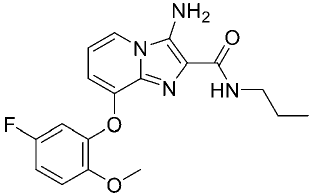 |
| 65 | 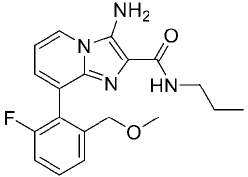 | 145 | 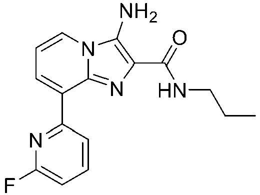 |
| 66 | 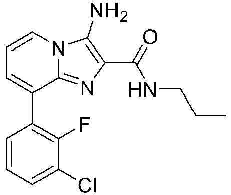 | 146 | 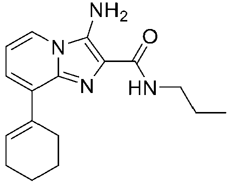 |
| 67 | 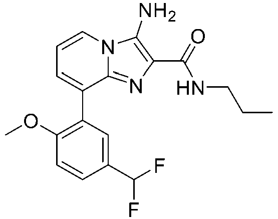 | 147 | 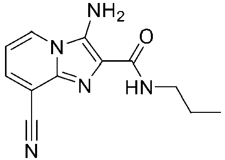 |
| 68 | 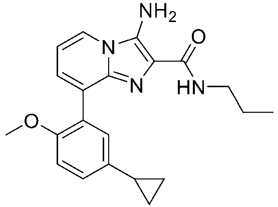 | 148 | 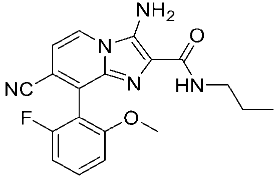 |
| 69 | 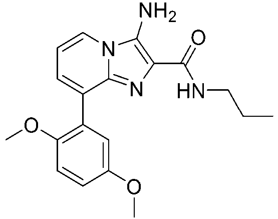 | 149 | 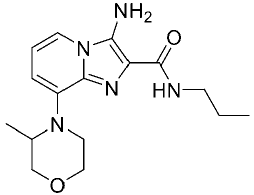 |
| 70 | 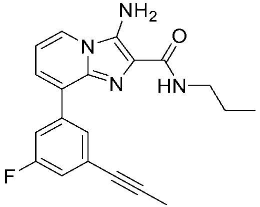 | 150 | 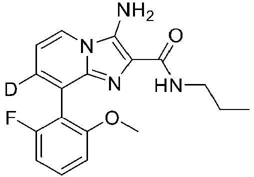 |
| 71 | 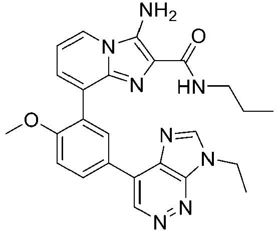 | 151 | 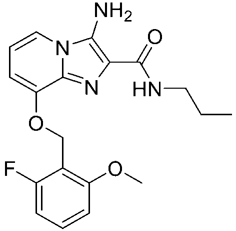 |
| 72 | 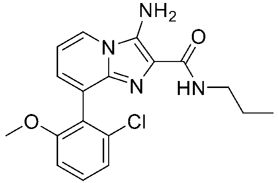 | 154 | 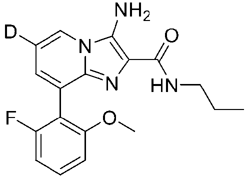 |
| 73 | 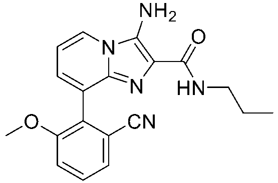 | 155 | 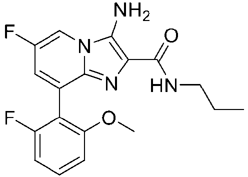 |
| 74 | 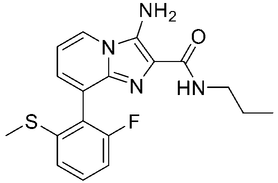 | 156 | 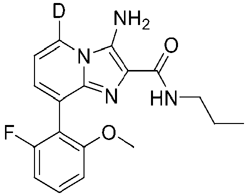 |
| 75 | 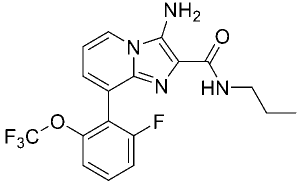 | | |
| 76 | 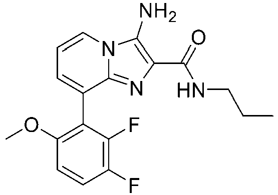 | | |
| 77 | 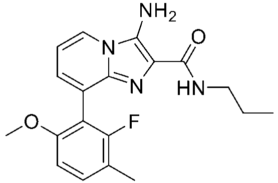 | | |
| 78 | 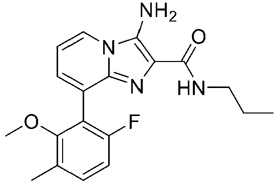 | | |
| 79 | 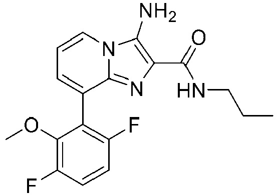 | | |
| 80 | 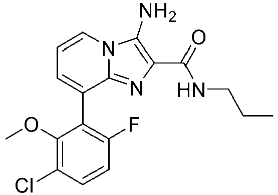 | | |

9. A pharmaceutical composition comprising a substance X and a pharmaceutically acceptable excipient, wherein the substance X is the compound as defined in any one of claims 1 to 8, an isotopic derivative thereof, a nitrogen oxide thereof, a pharmaceutically acceptable salt thereof, a solvate thereof, or a solvate of the pharmaceutically acceptable salt thereof.

10. A use of a substance X or the pharmaceutical composition as defined in claim 9 in the manufacture of a medicament for treating or preventing a GABA_{A} receptor-associated disease; wherein the substance X is the compound as defined in any one of claims 1 to 8, an isotopic derivative thereof, a nitrogen oxide thereof, a pharmaceutically acceptable salt thereof, a solvate thereof, or a solvate of the pharmaceutically acceptable salt thereof.

11. A use of the substance X as defined in claim 10 or the pharmaceutical composition as defined in claim 9 in the manufacture of a medicament for treating or preventing a GABA_{A} receptor-associated disease, wherein the GABA_{A} receptor-associated disease is selected from the group consisting of pain, Alzheimer's disease, multi-infarct dementia, stroke, pain, epilepsy, anxiety, pruritus, and depression.

12. A use of the substance X as defined in claim 10 or the pharmaceutical composition as defined in claim 9 in the manufacture of a medicament for treating or preventing a GABA_{A} receptor-associated disease, wherein the GABA_{A} receptor-associated disease is a disease associated with α2/3-GABA_{A} receptors, such as pain, epilepsy, anxiety, pruritus, and depression.

13. A use of the substance X as defined in claim 10 or the pharmaceutical composition as defined in claim 9 in the manufacture of a medicament for treating or preventing pain, Alzheimer's disease, multi-infarct dementia, stroke, epilepsy, anxiety, pruritus, or depression.
